# EUROPEAN PATENT APPLICATION

(11) **EP 4 567 118 A2**
(43) Date of publication of application: **11.06.2025**
(21) Application number: 25153185.1
(22) Date of filing: 30.06.2020
(51) Int. Cl.: C12N 15/85

(54) **TRANSGENIC MAMMALS AND METHODS OF USE THEREOF**

(30) Priority: 01.07.2019 US 201962869415 P
(62) Divisional of application: 20747552.6
(71) Applicant: Trianni, Inc., Wilmington, DE 19801 (US)
(72) Inventor: DUONG, Bao, Pacifica (US); MÜLLER, Werner, Köln (DE); BURROWS, Peter Daniel, Birmingham (US); ESPOSITO, Gloria, Vienna (AT); WABL, Matthias, San Francisco (US)
(74) Representative: Clark, Claudia

(57) **Abstract**

Transgenic mammals that express bovine-based immunoglobulins are described herein, including transgenic rodents that express bovine-based immunoglobulins for the development of bovine therapeutic antibodies.

## Description

### CROSS-REFERENCE TO RELATED APPLICATIONS

This application claims priority to U.S. Provisional Application No. 62/869,415, filed July 1, 2019, the disclosure of which is incorporated herein by reference.

### SEQUENCE LISTING

The instant application contains a Sequence Listing which has been submitted electronically in ASCII format and is hereby incorporated by reference in its entirety. Said ASCII copy, created on June 23, 2020, is named 0133-0007WO1_SL.txt and is 119,740 bytes in size.

### FIELD OF THE INVENTION

This invention relates to production of immunoglobulin molecules, including methods for generating transgenic mammals capable of producing bovine antigen-specific antibody-secreting cells for the generation of monoclonal antibodies.

### BACKGROUND

In the following discussion certain articles and methods are described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Antibodies have emerged as important biological pharmaceuticals because they (i) exhibit exquisite binding properties that can target antigens of diverse molecular forms, (ii) are physiological molecules with desirable pharmacokinetics that make them well tolerated in treated humans and animals, and (iii) are associated with powerful immunological properties that naturally ward off infectious agents. Furthermore, established technologies exist for the rapid isolation of antibodies from laboratory animals, which can readily mount a specific antibody response against virtually any foreign substance not present natively in the body.

In their most elemental form, antibodies are composed of two identical heavy (H) chains that are each paired with an identical light (L) chain. The N-termini of both H and L chains includes a variable domain (V_{H} and V_{L}, respectively) that together provide the paired H-L chains with a unique antigen-binding specificity.

The exons that encode the antibody V_{H} and V_{L} domains do not exist in the germline DNA. Instead, each V_{H} exon is generated by the recombination of randomly selected V_{H}, D, and J_{H} gene segments present in the immunoglobulin H chain locus (IGH); likewise, individual V_{L} exons are produced by the chromosomal rearrangements of randomly selected V_{L} and J_{L} gene segments in a light chain locus.

The bovine genome contains two alleles that can express the H chain (one allele from each parent), two alleles that can express the kappa (κ) L chain, and two alleles that can express the lambda (λ) L chain. There are multiple V_{H}, D, and J_{H} gene segments at the H chain locus as well as multiple V_{L} and J_{L} gene segments at both the immunoglobulin κ (IGK) and immunoglobulin λ (IGL) L chain loci (Collins and Watson (2018) Immunoglobulin Light Chain Gene Rearrangements, Receptor Editing and the Development of a Self-Tolerant Antibody Repertoire. Front. Immunol. 9:2249. (doi: 10.3389/fimmu.2018.02249)).

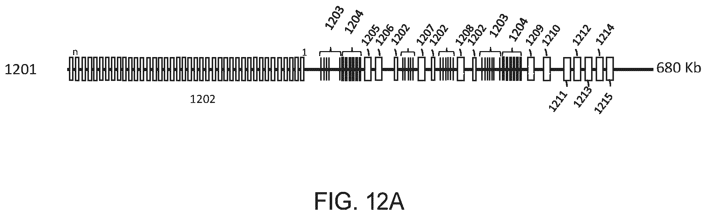

In a typical immunoglobulin heavy chain variable region locus, V_{H} gene segments lie upstream (5') of J_{H} gene segments, with D gene segments located between the V_{H} and J_{H} gene segments. Downstream (3') of the J_{H} gene segments of the IGH locus are clusters of exons that encode the constant region (C_{H}) of the antibody. Each cluster of C_{H} exons encodes a different antibody class (isotype). Eight classes of antibody exist in mouse: IgM, IgD, IgG3, IgG1, IgG2a (or IgG2c), IgG2b, IgE, and IgA (at the nucleic acid level, they are respectively referred to as: µ, δ, γ3, γ1, γ2a/c, γ2b, ε, and α). In bovine animals, the putative isotypes are IgM, IgD, IgG, IgE, and IgA (FIG. 12A). There are three sub-types of bovine IgG and two IgM. The functional consequence of having two IgM sub-types is unclear, although it appears that ultralong CDR H3 antibodies appear to exclusively use IgM2. (Stanfield et al. (2018) The unusual genetics and biochemistry of bovine immunoglobulins Adv. Immunol. 137:136-164 (doi: 10.1016/bs.ai.2017.12.004). The bovine IGH locus is small, ~680 Kb.

The question of whether there is bovine IgD has been controversial. Early studies found no evidence for a Cδ gene or IgD protein in cows (Butler, et al. (1996) The swine Ig heavy chain locus has a single JH and no identifiable IgD. Int. Immunol. 8:1897-1094 (DOI: 10.1093/intimm/8.12.1897); Naessens J (1997) Surface Ig on B lymphocytes from cattle and sheep. Int. Immunol. 9:349-354 (DOI:10.1093/intimm/9.3.349). Subsequently, evidence for the existence of bovine Cδ genes (Zhao, et al. (2002) J. Immunol. Artiodactyl IgD: The missing link. 160:4408-4416 (DOI:10.4049/jimmunol.169.8.4408) and expression of bovine IgD (Xu, et al. (2012) PLoS ONE Expressional Analysis of Immunoglobulin D in Cattle (Bos taurus), a Large Domesticated Ungulate. 7:e44719. (doi.org/10.1371/journal.pone.0044719) have been reported, although the frequency of IgD⁺ cells was much lower than in mice. The current annotation of the bovine IGH locus lists five Cδ genes, IGHDDIP, IGHDD2P, IGHDD39, and IGHD, none of which are functional. There are two IGHD alleles, one is a pseudogene due to a frameshift in M1 and the other is an ORF due to a non-canonical splice donor, NGC instead of NGT. Perhaps the low frequency of IgD⁺ cells observed by Xu et al. is due to occasional leaky splicing from the ORF allele.

At the IGK locus of most mammalian species, a cluster of V_{κ} gene segments are located upstream of a small number of J_{κ} gene segments, with the J_{κ} gene segment cluster located upstream of a single C_{κ} gene. This organization of the κ locus can be represented as (V_{κ})ₐ ... (J_{κ})_{b} ... C_{κ}, wherein a and b, independently, are an integer of 1 or more. The bovine IGK locus is small, ~400 Kb.

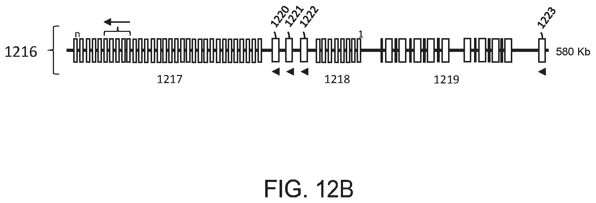

The IGL locus of most species includes a set of V_{λ}, gene segments that are located 5' to a variable number of J-C tandem cassettes, each made up of a J_{λ} gene segment and a C_{λ}, gene segment (see schematic of the bovine IGL locus in FIG. 12B). The organization of the λ locus can be represented as (V_{λ})ₐ... (J_{λ}-C_{λ})_{b}, wherein a and b are, independently, an integer of 1 or more. The mouse IGL locus is unusual in that it contains two units of (V_{λ})ₐ... (J_{λ}-C_{λ})_{b}. The bovine IGL locus is small, ~580 Kb.

During B cell development, gene rearrangements occur first on one of the two homologous chromosomes that contain the H chain variable gene segments. The resultant V_{H} exon is then spliced at the RNA level to the C_{µ} exons for IgM H chain expression. Subsequently, the V_{L}-J_{L} rearrangements occur on one L chain allele at a time until a functional L chain is produced, after which the L chain polypeptides can associate with the IgM H chain homodimers to form a fully functional B cell receptor (BCR) for antigen. In mouse and humans, as B cells continue to mature, IgD is co-expressed with IgM, with IgD being expressed at a level 10 times higher than IgM in the main B cell population.

It is widely accepted by experts in the field that in mouse and human, V_{L}-J_{L} rearrangements first occur at the IGK locus on both chromosomes before the IGL light chain locus on either chromosome becomes receptive for V_{L}-J_{L} recombination. This is supported by the observation that in mouse B cells that express κ light chains, the λ locus on both chromosomes is usually inactivated by non-productive rearrangements. This may explain the predominant κ L chain usage in mouse, which is >90% κ and <10% λ.

However, the ratio of κ to λ immunoglobulins varies between species. Many livestock and companion animals are highly λ dominant, with cattle producing approximately 91% λ chains. Arun et al. (1996) Immunohistochemical examination of light chain expression (lambda/kappa ratio) in canine, feline, equine, bovine and porcine plasma cells. Zentralblatt für Veterinärmedizin Reihe A. 43:573-576.

Upon encountering an antigen, the B cell then undergoes another round of DNA recombination at the IGH locus to remove the C_{µ} and C_{δ} exons, effectively switching the C_{H} region to one of the downstream isotypes (this process is called class switching).

The genes encoding various bovine (e.g., domestic cattle) and mouse immunoglobulins have been characterized, although the sequence and annotation of the bovine Ig loci in the genome databases is not yet complete. For example, Sinclair, et al., describe the bovine IgG repertoire as being dominated by a single diversified VH gene segment family in J. Immunol., 159(8):3883-89 (1997); Lopez, et al., describe a single VH family and long CDR3 as being the targets for hypermutation in bovine IgG heavy chains in Immunol. Rev. 162(1):55-66 (1998); Hosseini, et al., demonstrate that duplicated copies of the bovine JH locus contribute to the Ig repertoire in Int. Immunol. 16(6):355-63 (1998); Wang, et al., describe antigen-binding sites in certain bovine antibodies as ultralong CDR3 loops, each with a stalk with a projecting knob that can be further somatically diversified by changing the number of Cys residues, as well as the patterns and connectivities of the somatically generated disulfide bonds in Cell, 153(6):1379-1393 (2013). Blankenstein and Krawinkel describe the mouse variable heavy chain region in Eur. J. Immunol. 17:1351-1357 (1987).

The generation of transgenic animals-such as mice having varied immunoglobulin loci-has allowed the use of such transgenic animals in various research and development applications, e.g., in drug discovery and basic research into various biological systems. For example, the generation of transgenic mice bearing human immunoglobulin genes is described in International Application WO 90/10077 and WO 90/04036. WO 90/04036 describes a transgenic mouse with an integrated human immunoglobulin "mini" locus. WO 90/10077 describes a vector containing the immunoglobulin dominant control region for use in generating transgenic animals.

Numerous methods have been developed for modifying the mouse endogenous immunoglobulin variable region gene locus with, e.g., human immunoglobulin sequences to create partly or fully human antibodies for drug discovery purposes. Examples of such mice include those described in, e.g., U.S. Pat. Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669. However, many of the fully humanized immunoglobulin transgenic mice exhibit suboptimal antibody production because B cell development in these mice is severely hampered by inefficient V(D)J recombination, and by inability of the fully human antibodies/BCRs to function optimally with mouse signaling proteins. Other humanized immunoglobulin transgenic mice, in which the mouse coding sequences have been "swapped" with human sequences, are very time consuming and expensive to create due to the approach of replacing individual mouse exons with the syntenic human counterpart.

The use of antibodies that function as drugs is not necessarily limited to the prevention or therapy of human disease. The high population density of modern intensively managed livestock operations results in sharing of both commensal flora and pathogens, which results in rapid dissemination of infectious agents. As a result, livestock commonly require aggressive infection management strategies, which often include the use of antibiotics. The massive use of antibiotics favors the outgrowth of resistant microbes, endangering both farm animals and the humans who consume them. As such, there is increasing pressure to reduce or eliminate the use of antibiotics in domestic livestock, such as cattle.

Based on the foregoing, a clear need exists for efficient and cost-effective methods to produce bovine antibodies for the treatment of diseases in domestic cattle. More particularly, there is a need for small, rapidly breeding mammals capable of producing antigen-specific bovine immunoglobulins that can be used to generate hybridomas capable of large-scale production of bovine monoclonal antibodies.

### SUMMARY

This Summary is provided to introduce a selection of concepts in a simplified form that are further described below in the Detailed Description. This Summary is not intended to identify key or essential features of the claimed subject matter, nor is it intended to be used to limit the scope of the claimed subject matter. Other features, details, utilities, and advantages of the claimed subject matter will be apparent from the following written Detailed Description including those aspects illustrated in the accompanying drawings and defined in the appended claims.

Described herein is a non-bovine mammalian cell and a non-bovine mammal having a genome comprising an exogenously introduced, partly bovine immunoglobulin locus, where the introduced locus comprises coding sequences of the bovine immunoglobulin variable region gene segments and non-coding sequences based on the endogenous immunoglobulin variable region locus of the non-bovine mammalian host. Thus, the non-bovine mammalian cell or mammal is capable of expressing a chimeric B cell receptor (BCR) or antibody comprising H and L chain variable regions that are fully bovine in conjunction with the respective constant regions that are native to the non-bovine mammalian host cell or mammal. In one aspect, the transgenic cells and animals have genomes in which part or all of the endogenous immunoglobulin variable region gene locus is removed.

At a minimum, the production of chimeric bovine monoclonal antibodies in a non-bovine mammalian host requires the host to have at least one locus that expresses a chimeric bovine immunoglobulin H or L chain. In most aspects, there are one heavy chain locus and two light chain loci that, respectively, express chimeric bovine immunoglobulin H and L chains.

In some aspects, the partly bovine immunoglobulin locus comprises bovine V_{H} coding sequences and non-coding regulatory or scaffold sequences present in the endogenous V_{H} gene locus of the non-bovine mammalian host. In these aspects, the partly bovine immunoglobulin locus further comprises bovine D and J_{H} gene segment coding sequences in conjunction with the non-coding regulatory or scaffold sequences present in the vicinity of the endogenous D and J_{H} gene segments of the non-bovine mammalian host cell genome.

In one aspect, the partly bovine immunoglobulin locus comprises bovine V_{H}, D and J_{H} gene segment coding sequences embedded in non-coding regulatory or scaffold sequences present in an endogenous immunoglobulin heavy chain locus of the non-bovine mammalian host. In one aspect, the partly bovine immunoglobulin locus comprises bovine V_{H}, D and J_{H} gene segment coding sequences embedded in non-coding regulatory or scaffold sequences present in an endogenous immunoglobulin heavy chain locus of a rodent, such as a mouse. In other aspects, the partly bovine immunoglobulin locus comprises bovine V_{L} coding sequences and non-coding regulatory or scaffold sequences present in the endogenous V_{L} gene locus of the non-bovine mammalian host. In one aspect, the exogenously introduced, partly bovine immunoglobulin locus comprising bovine V_{L} coding sequences further comprises bovine L-chain J gene segment coding sequences and non-coding regulatory or scaffold sequences present in the vicinity of the endogenous L-chain J gene segments of the non-bovine mammalian host cell genome. In one aspect, the partly bovine immunoglobulin locus comprises bovine V_{λ} and J_{λ} gene segment coding sequences embedded in non-coding regulatory or scaffold sequences present in an immunoglobulin light chain locus of the non-bovine mammalian host cell. In one aspect, the partly bovine immunoglobulin locus comprises bovine V_{κ} and J_{κ} gene segment coding sequences embedded in non-coding regulatory or scaffold sequences present in an immunoglobulin locus of the non-bovine mammalian host. In one aspect, the endogenous κ locus of the non-bovine mammalian host is inactivated or replaced by sequences encoding bovine λ chain, to increase production of bovine λ immunoglobulin light chain over bovine κ chain. In one aspect, the endogenous κ locus of the non-bovine mammalian host is inactivated but not replaced by sequences encoding bovine λ chain.

In certain aspects, the non-bovine mammal is a rodent, for example, a mouse or rat.

In one aspect, the engineered immunoglobulin locus includes a partly bovine immunoglobulin light chain locus that includes one or more bovine λ variable region gene segment coding sequences. In one aspect, the engineered immunoglobulin locus is a partly bovine immunoglobulin light chain locus that includes one or more bovine κ variable region gene segment coding sequences.

In one aspect, a transgenic rodent or rodent cell is provided that has a genome comprising an engineered partly bovine immunoglobulin locus. In one aspect, a transgenic rodent or rodent cell is provided that has a genome comprising an engineered partly bovine immunoglobulin light chain locus. In one aspect, the partly bovine immunoglobulin light chain locus of the rodent or rodent cell includes one or more bovine immunoglobulin λ variable region gene segment coding sequences. In one aspect, the partly bovine immunoglobulin light chain locus of the rodent or rodent cell includes one or more bovine immunoglobulin κ variable region gene segment coding sequences. In one aspect, the engineered immunoglobulin locus is capable of expressing immunoglobulin comprising bovine variable domains.

In one aspect, a transgenic rodent that produces more immunoglobulin comprising λ light chain than immunoglobulin comprising κ light chain is provided. In one aspect, the transgenic rodent produces at least about 25%, 30%, 35%, 40%, 45%, 50%, 55%, 60%, 65%, 70%, 75%, 80%, 85%, 90% or 95% and up to about 100% λ light chain immunoglobulin. In one aspect, the transgenic rodent produces at least about 25%, 30%, 35%, 40%, 45%, 50%, 55%, 60%, 65%, 70%, 75%, 80%, 85%, 90% or 95% and up to about 100% λ light chain immunoglobulin comprising a bovine variable domain. In one aspect, more λ light chain-producing cells than κ light chain-producing cells are likely to be isolated from the transgenic rodent. In one aspect, more cells producing λ light chain with a bovine variable domain are likely to be isolated from the transgenic rodent than cells producing κ light chain with a bovine variable domain.

In one aspect, a transgenic rodent cell is provided that is more likely to produce immunoglobulin comprising λ light chain than immunoglobulin comprising κ light chain. In one aspect, the rodent cell is isolated from a transgenic rodent described herein. In one aspect, the rodent cell is recombinantly produced as described herein. In one aspect, the transgenic rodent cell or its progeny, has at least about a 25%, 30%, 35%, 40%, 45%, 50%, 55%, 60%, 65%, 70%, 75%, 80%, 85%, 90%, or 95% and up to about 100%, probability of producing λ light chain immunoglobulin. In one aspect, the transgenic rodent cell or its progeny, has at least about about a 25%, 30%, 35%, 40%, 45%, 50%, 55%, 60%, 65%, 70%, 75%, 80%, 85%, 90%, or 95%, and up to about 100%, probability of producing λ light chain immunoglobulin with a bovine variable domain.

In one aspect, the engineered partly bovine immunoglobulin locus comprises bovine V_{λ} gene segment coding sequences and J_{λ} gene segment coding sequences and non-coding sequences such as regulatory or scaffold sequences of a rodent immunoglobulin light chain variable region gene locus.

In one aspect, the engineered immunoglobulin locus comprises bovine V_{λ}, and J_{λ} gene segment coding sequences embedded in rodent non-coding regulatory or scaffold sequences of a rodent immunoglobulin λ light chain variable region gene locus. In one aspect, the engineered immunoglobulin locus comprises bovine V_{λ}, and J_{λ} gene segment coding sequences embedded in non-coding regulatory or scaffold sequences of the rodent immunoglobulin κ light chain variable region gene locus. In one aspect, the partly bovine immunoglobulin locus comprises one or more bovine V_{λ} gene segment coding sequences and J_{λ} gene segment coding sequences and one or more rodent immunoglobulin λ constant region coding sequences.

In one aspect, the engineered immunoglobulin variable region locus comprises one or more bovine V_{λ} gene segment coding sequences and one or more J-C units wherein each J-C unit comprises a bovine J_{λ} gene segment coding sequence and rodent region C_{λ}, coding sequence. In one aspect, the engineered immunoglobulin variable region locus comprises one or more bovine V_{λ} gene segment coding sequences and one or more J-C units wherein each J-C unit comprises a bovine J_{λ} gene segment coding sequence and rodent C_{λ}, region coding and non-coding sequences. In one aspect, the rodent C_{λ}, region coding sequence is selected from a rodent C_{λ1}, C_{λ2} or C_{λ3} coding sequence. In one aspect, one or more bovine V_{λ} gene segment coding sequences are located upstream of one or more J-C units, wherein each J-C unit comprises a bovine J_{λ} gene segment coding sequence and a rodent C_{λ} gene segment coding sequence. In one aspect, one or more bovine V_{λ} gene segment coding sequences are located upstream of one or more J-C units, wherein each J-C unit comprises a bovine J_{λ} gene segment coding sequence and a rodent C_{λ} gene segment coding sequence and rodent C_{λ}, non-coding sequences. In one aspect, the J-C units comprise bovine J_{λ} gene segment coding sequences and rodent C_{λ} region coding sequences embedded in non-coding regulatory or scaffold sequences of a rodent immunoglobulin κ light chain locus.

In one aspect, a transgenic rodent or rodent cell is provided with an engineered immunoglobulin locus that includes a rodent immunoglobulin κ locus in which one or more rodent V_{κ} gene segment coding sequences and one or more rodent J_{κ} gene segment coding sequences have been deleted and replaced with one or more bovine V_{λ} gene segment coding sequences and one or more J_{λ} gene segment coding sequences, respectively, and in which rodent C_{κ} coding sequence in the locus has been replaced by rodent C_{λ1}, C_{λ2}, or C_{λ3} coding sequence(s).

In one aspect, the engineered immunoglobulin locus includes one or more bovine V_{λ} gene segment coding sequences upstream and in the same transcriptional orientation as one or more bovine J_{λ} gene segment coding sequences which are upstream of one or more rodent C_{λ} coding sequences.

In one aspect, the engineered immunoglobulin locus includes one or more bovine V_{λ} gene segment coding sequences upstream and in the opposite transcriptional orientation as one or more bovine J_{λ} gene segment coding sequences which are upstream of one or more rodent C_{λ} coding sequences.

In one aspect, a transgenic rodent or rodent cell is provided in which an endogenous rodent immunoglobulin κ light chain locus is deleted, inactivated, or made nonfunctional by one or more of:
a. deleting or mutating all endogenous rodent V_{κ} gene segment coding sequences;
b. deleting or mutating all endogenous rodent J_{κ} gene segment coding sequences;
c. deleting or mutating an endogenous rodent C_{κ} coding sequence;
d. deleting or mutating a splice donor site, pyrimidine tract, or splice acceptor site within the intron between a J_{κ} gene segment and C_{κ} exon; and
e. deleting, mutating, or disrupting an endogenous intronic κ enhancer (iE_{κ}), a 3' enhancer sequence (3'E_{κ}), or a combination thereof.

In one aspect, a transgenic rodent or rodent cell is provided in which expression of an endogenous rodent immunoglobulin λ light chain variable domain is suppressed or inactivated by one or more of:
a. deleting or mutating all endogenous rodent V_{λ} gene segments;
b. deleting or mutating all endogenous rodent J_{λ} gene segments;
c. deleting or mutating all endogenous rodent C_{λ} coding sequences; and
d. deleting or mutating a splice donor site, pyrimidine tract, splice acceptor site within the intron between a J_{λ} gene segment and C_{λ}, exon or a combination thereof.

In one aspect, a transgenic rodent or rodent cell is provided in which the engineered immunoglobulin locus expresses immunoglobulin light chains comprising a bovine variable domain and a rodent constant domain. In one aspect, a transgenic rodent or rodent cell is provided in which the engineered immunoglobulin locus expresses immunoglobulin light chains comprising a bovine λ variable domain and rodent λ constant domain. In one aspect, a transgenic rodent or rodent cell is provided in which the engineered immunoglobulin locus expresses immunoglobulin light chains comprising a bovine κ variable domain and rodent κ constant domain.

In one aspect, a transgenic rodent or rodent cell is provided in which the genome of the transgenic rodent or rodent cell comprises an engineered immunoglobulin locus comprising bovine V_{κ} and J_{κ} gene segment coding sequences. In one aspect, the bovine V_{κ} and J_{κ} gene segment coding sequences are inserted into a rodent immunoglobulin κ light chain locus. In one aspect, the bovine V_{κ} and J_{κ} gene segment coding sequences are embedded in rodent non-coding regulatory or scaffold sequences of the rodent immunoglobulin κ light chain variable region gene locus. In one aspect, the bovine V_{κ} and J_{κ} coding sequences are inserted upstream of a rodent immunoglobulin κ light chain constant region coding sequence.

In one aspect, a transgenic rodent or rodent cell is provided in which the genome of the transgenic rodent or rodent cell comprises an engineered immunoglobulin locus comprising bovine V_{κ} and J_{κ} gene segment coding sequences inserted into a rodent immunoglobulin λ light chain locus. In one aspect, the bovine V_{κ} and J_{κ} gene segment coding sequences are embedded in rodent non-coding regulatory or scaffold sequences of the rodent immunoglobulin λ light chain variable region gene locus. In one aspect, the genome of the transgenic rodent or rodent cell includes a rodent immunoglobulin κ light chain constant region coding sequence inserted downstream of the bovine V_{κ} and J_{κ} gene segment coding sequences. In one aspect, the rodent immunoglobulin κ light chain constant region is inserted upstream of an endogenous rodent C_{λ} coding sequence. In one aspect, the rodent immunoglobulin κ light chain constant region is inserted upstream of an endogenous rodent C_{λ2} coding sequence. In one aspect, expression of an endogenous rodent immunoglobulin λ light chain variable domain is suppressed or inactivated by one or more of:
a. deleting or mutating all endogenous rodent V_{λ} gene segment coding sequences.
b. deleting or mutating all endogenous rodent J_{λ} gene segment coding sequences;
c. deleting or mutating all endogenous C_{λ} coding sequences; and
d. deleting or mutating a splice donor site, pyrimidine tract, or splice acceptor site within the intron between a J_{λ} gene segment and C_{λ} exon.

In one aspect, the engineered partly bovine immunoglobulin light chain locus comprises a rodent intronic κ enhancer (iE_{κ}) and 3' κ enhancer (3'E_{κ}) regulatory sequences.

In one aspect, the transgenic rodent or rodent cell further comprises an engineered partly bovine immunoglobulin heavy chain locus comprising bovine immunoglobulin heavy chain variable region gene segment coding sequences and non-coding regulatory and scaffold sequences of the rodent immunoglobulin heavy chain locus. In one aspect, the engineered bovine immunoglobulin heavy chain locus comprises bovine V_{H}, D and J_{H} gene segment coding sequences. In one aspect, each bovine/rodent chimeric V_{H}, D or J_{H} gene segment comprises V_{H}, D or J_{H} coding sequence embedded in non-coding regulatory and scaffold sequences of the rodent immunoglobulin heavy chain locus. In one aspect, the heavy chain scaffold sequences are interspersed by one or both functional ADAM6 genes.

In one aspect, the rodent regulatory and scaffold sequences comprise one or more enhancers, promoters, splice sites, introns, recombination signal sequences, or a combination thereof.

In one aspect, an endogenous rodent immunoglobulin locus of the transgenic rodent or rodent cell has been inactivated. In one aspect, an endogenous rodent immunoglobulin locus of the transgenic rodent or rodent cell has been deleted and replaced with the engineered partly bovine immunoglobulin locus.

In one aspect, the rodent is a mouse or a rat. In one aspect, the rodent cell is an embryonic stem (ES) cell or a cell of an early stage embryo. In one aspect, the rodent cell is a mouse or rat embryonic stem (ES) cell, or mouse or rat cell of an early stage embryo.

In one aspect, a cell of B lymphocyte lineage is provided that is obtained from a transgenic rodent described herein, wherein the B cell expresses or is capable of expressing a chimeric immunoglobulin heavy chain or light chain comprising a bovine variable region and a rodent immunoglobulin constant region. In one aspect, a hybridoma cell or immortalized cell line is provided that is derived from a cell of B lymphocyte lineage obtained from a transgenic rodent or rodent cell described herein.

In one aspect, antibodies or antigen binding portions thereof are provided that are produced by a cell from a transgenic rodent or rodent cell described herein.

In one aspect, a nucleic acid sequence of a V_{H}, D, or J_{H}, or a V_{L} or J_{L} gene segment coding sequence is provided that is derived from an immunoglobulin produced by a transgenic rodent or rodent cell described herein. In one aspect, a method for generating a non-bovine mammalian cell comprising a partly bovine immunoglobulin locus is provided, said method comprising: a) introducing two or more recombinase targeting sites into the genome of a non-bovine mammalian host cell and integrating at least one site upstream and at least one site downstream of a genomic region comprising endogenous immunoglobulin variable region genes wherein the endogenous immunoglobulin variable genes comprise V_{H}, D and J_{H} gene segments, or V_{κ} and J_{κ} gene segments, or V_{λ} and J_{λ} gene segments, or V_{λ}, J_{λ} and C_{λ} gene segments; and b) introducing into the non-bovine mammalian host cell via recombinase-mediated cassette exchange (RMCE) an engineered partly bovine immunoglobulin variable gene locus comprising bovine immunoglobulin variable region gene coding sequences and non-coding regulatory or scaffold sequences corresponding to the non-coding regulatory or scaffold sequences present in the endogenous immunoglobulin variable region gene locus of the non-bovine mammalian host.

In another aspect, the method further comprises deleting the genomic region flanked by the two exogenously introduced recombinase targeting sites prior to step b.

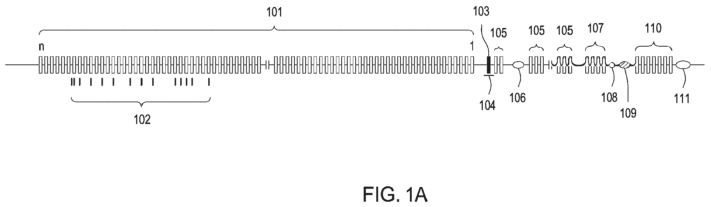

In a specific aspect, the exogenously introduced, engineered partly bovine immunoglobulin heavy chain locus is provided that comprises bovine V_{H} gene segment coding sequences, and further comprises i) bovine D and J_{H} gene segment coding sequences and ii) non-coding regulatory or scaffold sequences upstream of the bovine D gene segments (pre-D sequences, FIG. 1A) that correspond to the sequences present upstream of the endogenous D gene segments in the genome of the non-bovine mammalian host. In one aspect, these upstream scaffold sequences are interspersed by non-immunoglobulin genes, such as ADAM6A or ADAM6B (FIG. 1A) needed for male fertility (Nishimura et al. Developmental Biol. 233(1): 204-213 (2011)). The partly bovine immunoglobulin heavy chain locus is introduced into the host cell using recombinase targeting sites that have been previously introduced upstream of the endogenous immunoglobulin V_{H} gene locus and downstream of the endogenous J_{H} gene locus on the same chromosome.

In other aspects, the non-coding regulatory or scaffold sequences derive (at least partially) from other sources, e.g., they could be rationally designed artificial sequences or otherwise conserved sequences of unknown functions, sequences that are a combination of bovine and artificial or other designed sequences, or sequences from other species. As used herein, "artificial sequence" refers to a sequence of a nucleic acid not derived from a sequence naturally occurring at a genetic locus. In one aspect, the non-coding regulatory or scaffold sequences are derived from non-coding regulatory or scaffold sequences of a rodent immunoglobulin heavy chain variable region locus. In one aspect, the non-coding regulatory or scaffold sequences have at least about 75%, 80%, 85%, 90%, 95% or 100% sequence identity to non-coding regulatory or scaffold sequences of a rodent immunoglobulin heavy chain variable region locus. In another aspect, the non-coding regulatory or scaffold sequences are rodent immunoglobulin heavy chain variable region non-coding or scaffold sequences.

In yet another specific aspect of the method, the introduced engineered partly bovine immunoglobulin locus comprises bovine immunoglobulin V_{L} gene segment coding sequences, and further comprises i) bovine L-chain J gene segment coding sequences and ii) non-coding regulatory or scaffold sequences corresponding to the non-coding regulatory or scaffold sequences present in the endogenous L chain locus of the non-bovine mammalian host cell genome. In one aspect, the engineered partly bovine immunoglobulin locus is introduced into the host cell using recombinase targeting sites that have been previously introduced upstream of the endogenous immunoglobulin V_{L} gene locus and downstream of the endogenous J gene locus on the same chromosome.

In a more particular aspect, an exogenously introduced, engineered partly bovine immunoglobulin light chain locus is provided that comprises bovine V_{λ}, gene segment coding sequences and bovine J_{λ} gene segment coding sequences. In one aspect, the partly bovine immunoglobulin light chain locus is introduced into the host cell using recombinase targeting sites that have been previously introduced upstream of the endogenous immunoglobulin V_{λ} gene locus and downstream of the endogenous J_{λ} gene locus on the same chromosome.

In one aspect, the exogenously introduced, engineered partly bovine immunoglobulin light chain locus comprises bovine V_{κ} gene segment coding sequences and bovine J_{κ} gene segment coding sequences. In one aspect, the partly bovine immunoglobulin light chain locus is introduced into the host cell using recombinase targeting sites that have been previously introduced upstream of the endogenous immunoglobulin V_{κ} gene locus and downstream of the endogenous J_{κ} gene locus on the same chromosome.

In one aspect, the non-coding regulatory or scaffold sequences are derived from non-coding regulatory or scaffold sequences of a rodent λ immunoglobulin light chain variable region locus. In one aspect, the non-coding regulatory or scaffold sequences have at least about 75%, 80%, 85%, 90%, 95% or 100% sequence identity to non-coding regulatory or scaffold sequences of a rodent immunoglobulin λ light chain variable region locus. In another aspect, the non-coding regulatory or scaffold sequences are rodent immunoglobulin λ light chain variable region non-coding or scaffold sequences.

In one aspect, the non-coding regulatory or scaffold sequences are derived from non-coding regulatory or scaffold sequences of a rodent immunoglobulin κ light chain variable region locus. In one aspect, the non-coding regulatory or scaffold sequences have at least about 75%, 80%, 85%, 90%, 95% or 100% sequence identity to non-coding regulatory or scaffold sequences of a rodent immunoglobulin κ light chain variable region locus. In another aspect, the non-coding regulatory or scaffold sequences are rodent immunoglobulin κ light chain variable region non-coding or scaffold sequences.

In one aspect, the engineered partly bovine immunoglobulin locus is synthesized as a single nucleic acid, and introduced into the non-bovine mammalian host cell as a single nucleic acid region. In one aspect, the engineered partly bovine immunoglobulin locus is synthesized in two or more contiguous segments, and introduced to the mammalian host cell as discrete segments. In another aspect, the engineered partly bovine immunoglobulin locus is produced using recombinant methods and isolated prior to being introduced into the non-bovine mammalian host cell.

In another aspect, methods for generating a non-bovine mammalian cell comprising an engineered partly bovine immunoglobulin locus are provided, said method comprising: a) introducing into the genome of a non-bovine mammalian host cell two or more sequence-specific recombination sites that are not capable of recombining with one another, wherein at least one recombination site is introduced upstream of an endogenous immunoglobulin variable region gene locus while at least one recombination site is introduced downstream of the endogenous immunoglobulin variable region gene locus on the same chromosome; b) providing a vector comprising an engineered partly bovine immunoglobulin locus having i) bovine immunoglobulin variable region gene coding sequences and ii) non-coding regulatory or scaffold sequences based on an endogenous immunoglobulin variable region gene locus of the host cell genome, wherein the partly bovine immunoglobulin locus is flanked by the same two sequence-specific recombination sites that flank the endogenous immunoglobulin variable region gene locus of the host cell of a); c) introducing into the host cell the vector of step b) and a site specific recombinase capable of recognizing the two recombinase sites; d) allowing a recombination event to occur between the genome of the cell of a) and the engineered partly bovine immunoglobulin locus, resulting in a replacement of the endogenous immunoglobulin variable region gene locus with the engineered partly bovine immunoglobulin variable region gene locus.

In one aspect, the partly bovine immunoglobulin locus comprises V_{H} immunoglobulin gene segment coding sequences, and further comprises i) bovine D and J_{H} gene segment coding sequences, ii) non-coding regulatory or scaffold sequences surrounding the codons of individual V_{H}, D, and J_{H} gene segments present endogenously in the genome of the non-bovine mammalian host, and iii) pre-D sequences based on the endogenous genome of the non-bovine mammalian host cell. The recombinase targeting sites are introduced upstream of the endogenous immunoglobulin V_{H} gene locus and downstream of the endogenous D and J_{H} gene locus.

In one aspect, there is provided a transgenic rodent with a genome deleted of a rodent endogenous immunoglobulin variable gene locus and in which the deleted rodent endogenous immunoglobulin variable gene locus has been replaced with an engineered partly bovine immunoglobulin locus comprising bovine immunoglobulin variable gene coding sequences and non-coding regulatory or scaffold sequences based on the rodent endogenous immunoglobulin variable gene locus, wherein the engineered partly bovine immunoglobulin locus of the transgenic rodent is functional and expresses immunoglobulin chains with bovine variable domains and rodent constant domains. In some aspects, the engineered partly bovine immunoglobulin locus comprises bovine V_{H}, D, and J_{H} coding sequences, and in some aspects, the engineered partly bovine immunoglobulin locus comprises bovine V_{L} and J_{L} coding sequences. In one aspect, the partly bovine immunoglobulin locus comprises bovine V_{λ} and J_{λ} coding sequences. In another aspect, the partly bovine immunoglobulin locus comprises bovine V_{κ} and J_{κ} coding sequences.

Some aspects provide a cell of B lymphocyte lineage from the transgenic rodent, a part or whole immunoglobulin molecule comprising bovine variable domains and rodent constant domains obtained from the cell of B lymphocyte lineage, a hybridoma cell derived from the cell of B lymphocyte lineage, a part or whole immunoglobulin molecule comprising bovine variable domains and rodent constant domains obtained from the hybridoma cell, a part or whole immunoglobulin molecule comprising bovine variable domains derived from an immunoglobulin molecule obtained from the hybridoma cell, an immortalized cell derived from the cell of B lymphocyte lineage, a part or whole immunoglobulin molecule comprising bovine variable domains and rodent constant domains obtained from the immortalized cell, a part or whole immunoglobulin molecule comprising bovine variable domains derived from an immunoglobulin molecule obtained from the immortalized cell.

In one aspect, a transgenic rodent is provided, wherein the engineered partly bovine immunoglobulin locus comprises bovine V_{L} and J_{L} coding sequences, and a transgenic rodent, wherein the engineered partly bovine immunoglobulin loci comprise bovine V_{H}, D, and J_{H} or V_{L} and J_{L} coding sequences. In some aspects, the rodent is a mouse. In some aspects, the non-coding regulatory sequences comprise the following sequences of endogenous host origin: promoters preceding each V gene segment coding sequence, introns, splice sites, and recombination signal sequences for V(D)J recombination; in other aspects, the engineered partly bovine immunoglobulin locus further comprises one or more of the following sequences of endogenous host origin: ADAM6A or ADAM6B gene, a Pax-5-Activated Intergenic Repeat (PAIR) elements, or CTCF binding sites from a heavy chain intergenic control region 1.

In one aspect, the non-bovine mammalian cell for use in each of the above methods is a mammalian cell, for example, a mammalian embryonic stem (ES) cell. In one aspect, the mammalian cell is a cell of an early stage embryo. In one aspect, the non-bovine mammalian cell is a rodent cell. In one aspect, the non-bovine mammalian cell is a mouse cell.

Once the cells have been subjected to the replacement of the endogenous immunoglobulin variable region gene locus by the introduced partly bovine immunoglobulin variable region gene locus, the cells can be selected and isolated. In one aspect, the cells are non-bovine mammalian ES cells, for example, rodent ES cells, and at least one isolated ES cell clone is then utilized to create a transgenic non-bovine mammal expressing the engineered partly bovine immunoglobulin variable region gene locus.

In one aspect, a method for generating the transgenic rodent is provided, said method comprising: a) integrating at least one target site for a site-specific recombinase in a rodent cell's genome upstream of an endogenous immunoglobulin variable gene locus and at least one target site for a site-specific recombinase downstream of the endogenous immunoglobulin variable gene locus, wherein the endogenous immunoglobulin variable locus comprises V_{H}, D and J_{H} gene segments, or V_{κ} and J_{κ} gene segments, or V_{λ} and J_{λ} gene segments, or V_{λ}, J_{λ} and C_{λ} gene segments; b) providing a vector comprising an engineered partly bovine immunoglobulin locus, said engineered partly bovine immunoglobulin locus comprising chimeric bovine immunoglobulin gene segments, wherein each of the partly bovine immunoglobulin gene segment comprises bovine immunoglobulin variable gene coding sequences and rodent non-coding regulatory or scaffold sequences, with the partly bovine immunoglobulin variable gene locus being flanked by target sites for a site-specific recombinase wherein the target sites are capable of recombining with the target sites introduced into the rodent cell; c) introducing into the cell the vector and a site-specific recombinase capable of recognizing the target sites; d) allowing a recombination event to occur between the genome of the cell and the engineered partly bovine immunoglobulin locus resulting in a replacement of the endogenous immunoglobulin variable gene locus with the engineered partly bovine immunoglobulin locus; e) selecting a cell that comprises the engineered partly bovine immunoglobulin variable locus generated in step d); and utilizing the cell to create a transgenic rodent comprising partly bovine the engineered partly bovine immunoglobulin variable locus. In some aspects, the cell is a rodent embryonic stem (ES) cell, and in some aspects the cell is a mouse embryonic stem (ES) cell. Some aspects of this method further comprise after, after step a) and before step b), a step of deleting the endogenous immunoglobulin variable gene locus by introduction of a recombinase that recognizes a first set of target sites, wherein the deleting step leaves in place at least one set of target sites that are not capable of recombining with one another in the rodent cell's genome. In some aspects, the vector comprises bovine V_{H}, D, and J_{H}, coding sequences, and in some aspects the vector comprises bovine V_{L} and J_{L} coding sequences. In some aspects, the vector further comprises rodent promoters, introns, splice sites, and recombination signal sequences of variable region gene segments.

In another aspect, a method for generating a transgenic non-bovine mammal comprising an exogenously introduced, engineered, partly bovine immunoglobulin variable region gene locus is provided, said method comprising: a) introducing into the genome of a non-bovine mammalian host cell one or more sequence-specific recombination sites that flank an endogenous immunoglobulin variable region gene locus and are not capable of recombining with one another; b) providing a vector comprising a partly bovine immunoglobulin locus having i) bovine variable region gene coding sequences and ii) non-coding regulatory or scaffold sequences based on the endogenous host immunoglobulin variable region gene locus, wherein the coding and non-coding regulatory or scaffold sequences are flanked by the same sequence-specific recombination sites as those introduced to the genome of the host cell of a); c) introducing into the cell the vector of step b) and a site-specific recombinase capable of recognizing one set of recombinase sites; d) allowing a recombination event to occur between the genome of the cell of a) and the engineered partly bovine immunoglobulin variable region gene locus, resulting in a replacement of the endogenous immunoglobulin variable region gene locus with the partly bovine immunoglobulin locus; e) selecting a cell which comprises the partly bovine immunoglobulin locus; and f) utilizing the cell to create a transgenic animal comprising the partly bovine immunoglobulin locus.

In a specific aspect, the engineered partly bovine immunoglobulin locus comprises bovine V_{H}, D, and J_{H} gene segment coding sequences, and non-coding regulatory and scaffold pre-D sequences (including a fertility-enabling gene) present in the endogenous genome of the non-bovine mammalian host. In one aspect, the sequence-specific recombination sites are then introduced upstream of the endogenous immunoglobulin V_{H} gene segments and downstream of the endogenous J_{H} gene segments.

In one aspect, a method for generating a transgenic non-bovine animal comprising an engineered partly bovine immunoglobulin locus is provided, said method comprising: a) providing a non-bovine mammalian cell having a genome that comprises two sets of sequence-specific recombination sites that are not capable of recombining with one another, and which flank a portion of an endogenous immunoglobulin variable region gene locus of the host genome; b) deleting the portion of the endogenous immunoglobulin locus of the host genome by introduction of a recombinase that recognizes a first set of sequence-specific recombination sites, wherein such deletion in the genome retains a second set of sequence-specific recombination sites; c) providing a vector comprising an engineered partly bovine immunoglobulin variable region gene locus having bovine coding sequences and non-coding regulatory or scaffold sequences based on the endogenous immunoglobulin variable region gene locus, where the coding and non-coding regulatory or scaffold sequences are flanked by the second set of sequence-specific recombination sites; d) introducing the vector of step c) and a site-specific recombinase capable of recognizing the second set of sequence-specific recombination sites into the cell; e) allowing a recombination event to occur between the genome of the cell and the partly bovine immunoglobulin locus, resulting in a replacement of the endogenous immunoglobulin locus with the engineered partly bovine immunoglobulin variable locus; f) selecting a cell that comprises the partly bovine immunoglobulin variable region gene locus; and g) utilizing the cell to create a transgenic animal comprising the engineered partly bovine immunoglobulin variable region gene locus.

In one aspect, a method for generating a transgenic non-bovine mammal comprising an engineered partly bovine immunoglobulin locus is provided, said method comprising: a) providing a non-bovine mammalian embryonic stem ES cell having a genome that contains two sequence-specific recombination sites that are not capable of recombining with each other, and which flank the endogenous immunoglobulin variable region gene locus; b) providing a vector comprising an engineered partly bovine immunoglobulin locus comprising bovine immunoglobulin variable gene coding sequences and non-coding regulatory or scaffold sequences based on the endogenous immunoglobulin variable region gene locus, where the partly bovine immunoglobulin locus is flanked by the same two sequence-specific recombination sites that flank the endogenous immunoglobulin variable region gene locus in the ES cell; c) bringing the ES cell and the vector into contact with a site-specific recombinase capable of recognizing the two recombinase sites under appropriate conditions to promote a recombination event resulting in the replacement of the endogenous immunoglobulin variable region gene locus with the engineered partly bovine immunoglobulin variable region gene locus in the ES cell; d) selecting an ES cell that comprises the engineered partly bovine immunoglobulin locus; and e) utilizing the cell to create a transgenic animal comprising the engineered, partly bovine immunoglobulin locus.

In one aspect, the transgenic non-bovine mammal is a rodent, e.g., a mouse or a rat.

In one aspect, a non-bovine mammalian cell and a non-bovine transgenic mammal are provide that express an introduced immunoglobulin variable region gene locus having bovine variable region gene coding sequences and non-coding regulatory or scaffold sequences based on the endogenous non-bovine immunoglobulin locus of the host genome, where the non-bovine mammalian cell and transgenic animal express chimeric antibodies with fully bovine H or L chain variable domains in conjunction with their respective constant regions that are native to the non-bovine mammalian cell or animal.

Further, B cells from transgenic animals are provided that are capable of expressing partly bovine antibodies having fully bovine variable sequences, wherein such B cells are immortalized to provide a source of a monoclonal antibody specific for a particular antigen. In one aspect, a cell of B lymphocyte lineage from a transgenic animal is provided that is capable of expressing partly bovine heavy or light chain antibodies comprising a bovine variable region and a rodent constant region.

In one aspect, bovine immunoglobulin variable region gene sequences cloned from B cells are provided for use in the production or optimization of antibodies for diagnostic, preventative and therapeutic uses.

In one aspect, hybridoma cells are provided that are capable of producing partly bovine monoclonal antibodies having fully bovine immunoglobulin variable region sequences. In one aspect, a hybridoma or immortalized cell line of B lymphocyte lineage is provided.

In another aspect, antibodies or antigen binding portions thereof produced by a transgenic animal or cell described herein are provided. In another aspect, antibodies or antigen binding portions thereof comprising variable heavy chain or variable light chain sequences derived from antibodies produced by a transgenic animal or cell described herein are provided.

In one aspect, methods for determining the sequences of the H and L chain immunoglobulin variable domains from the monoclonal antibody-producing hybridomas or primary plasma cells or B cells and combining the V_{H} and V_{L} sequences with bovine constant regions are provided for creating a fully bovine antibody that is not immunogenic when injected into cattle.

These and other aspects, objects and features are described in more detail below.

### BRIEF DESCRIPTION OF THE FIGURES

FIG. 1A is a schematic diagram of the endogenous mouse IGH locus located at the telomeric end of chromosome 12.
FIG. 1B is a schematic diagram of the endogenous mouse IGL locus located on chromosome 16.
FIG. 1C is a schematic diagram of the endogenous mouse IGK locus located on chromosome 6.
FIG. 2 is a schematic diagram illustrating the strategy of targeting by homologous recombination to introduce a first set of sequence-specific recombination sites into a region upstream of the H chain variable region gene locus in the genome of a non-bovine mammalian host cell.
FIG. 3 is another schematic diagram illustrating the strategy of targeting by homologous recombination to introduce a first set of sequence-specific recombination sites into a region upstream of the H chain variable region gene locus in the genome of a non-bovine mammalian host cell.
FIG. 4 is a schematic diagram illustrating the introduction of a second set of sequence-specific recombination sites into a region downstream of the H chain variable region gene locus in the genome of a non-bovine mammalian cell via a homology targeting vector.
FIG. 5 is a schematic diagram illustrating deletion of the endogenous immunoglobulin H chain variable region gene locus from the genome of the non-bovine mammalian host cell.
FIG. 6 is a schematic diagram illustrating the RMCE strategy to introduce an engineered partly bovine immunoglobulin H chain locus into the non-bovine mammalian host cell genome that has been previously modified to delete the endogenous immunoglobulin H chain variable region gene locus.
FIG. 7 is a schematic diagram illustrating the RMCE strategy to introduce an engineered partly bovine immunoglobulin H chain locus comprising additional regulatory sequences into the non-bovine mammalian host cell genome that has been previously modified to delete the endogenous immunoglobulin H chain variable region genes.
FIG. 8 is a schematic diagram illustrating the introduction of an engineered partly bovine immunoglobulin H chain variable region gene locus into the endogenous immunoglobulin H chain locus of the mouse genome.
FIG. 9 is a schematic diagram illustrating the introduction of an engineered, partly bovine immunoglobulin κ L chain variable region gene locus into the endogenous immunoglobulin κ L chain locus of the mouse genome.
FIG. 10 is a schematic diagram illustrating the introduction of an engineered, partly bovine immunoglobulin λ L chain variable region gene locus into the endogenous immunoglobulin λ L chain locus of the mouse genome.
FIG. 11 is a schematic diagram illustrating the introduction of an engineered, partly bovine immunoglobulin locus comprising a bovine V_{H} minilocus via RMCE.
FIG. 12A is a schematic diagram of the endogenous bovine IGH locus (1201) located on chromosome 21. V_{H} (1202), D (1203) and J_{H} (1204) gene segments are indicated as are exons encoding the constant regions for M1 (1205), DDIP (1206), DD2P (1207), DD3P (1208), M2 (1209), D (1210), G3 (1211), G1 (1212), G2 (1213), E (1214), and A (1215).
FIG. 12B is a schematic diagram of the endogenous bovine IGL locus (1217) located on chromosome 17. V_{λ} (1218) and tandem clusters J_{λ}-C_{λ} (1219) gene segments-exons are indicated. Other non-Ig genes interspersed in the locus are Zinc Finger Protein 280B (1220), Zinc Finger Protein 280A (1221) and Preferentially Expressed Antigen in Melanoma (1222). VPREB (1223) encodes a V_{λ}-like component of the preB cell receptor. Genes 1220-1223 are in the opposite transcriptional orientation to the Ig genes.
FIG. 12C is a schematic diagram of the endogenous bovine IGK locus (1224) located on chromosome 11. V_{κ} (1225) and J_{κ} (1226) gene segments are indicated as is the exon encoding C_{κ} (1227). The gene encoding Ribose 5-Phosphate Isomerase A (1228) is present downstream of in the locus in opposite transcriptional orientation.
FIG. 13 is a schematic diagram illustrating an engineered partly bovine immunoglobulin light chain variable region locus in which one or more bovine V_{λ} gene segment coding sequences are inserted into a rodent immunoglobulin κ light chain locus upstream of one or more bovine J_{λ} gene segment coding sequences, which are upstream of one or more rodent C_{λ} region coding sequences.
FIG. 14 is a schematic diagram illustrating the introduction of an engineered partly bovine light chain variable region locus in which one or more bovine V_{λ} gene segment coding sequences are inserted into a rodent immunoglobulin κ light chain locus upstream of an array of J_{λ}-C_{λ} tandem cassettes in which the J_{λ} is of bovine origin and the C_{λ} is of mouse origin, C_{λ1}, C_{λ2} or C_{λ3}.
FIG. 15 shows the results of flow cytometry analysis of cells expressing bovine BLV1H12, the VH domain with an ultra-long HCDR3 with a mouse IgM backbone paired with bovine (b) V_{λ} (AF023843.1) or V_{κ} (BC122795) linked to the constant region of mouse (m) C_{κ}, C_{λ1}, C_{λ2}, or C_{λ3}.
FIG. 16 shows the results of flow cytometry analysis of cells expressing bovine IGHV B4, the VH domain with a normal-length HCDR3 with a mouse IgM backbone paired with bovine (b) V_{λ} (AF023843.1) or V_{κ} (BC122795) linked to the constant region of mouse (m) C_{κ}, C_{λ1}, C_{λ2}, or C_{λ3}.
FIG. 17A shows western blots of supernatants (1701) and FIG. 17B shows western blots of cell lysates (1702) of cells expressing bovine BLV1H12, the VH domain with an ultra-long HCDR3, with a mouse IgG2a HC backbone paired with bovine (b) V_{λ} (AF023843.1) or V_{κ} (BC122795) linked to the constant region of mouse (m) C_{κ}, C_{λ1}, C_{λ2}, or C_{λ3}.
FIG. 18A shows loading controls using Myc (1802) and FIG. 18B shows loading controls using GAPDH (1803) of the western blots shown in FIG. 17A and 17B.
FIG. 19A shows western blots of supernatants (1901) and FIG. 19B shows western blots of cell lysates (1902) of cells expressing bovine BLV1H12, the VH domain with an average length HCDR3, with a mouse IgG2a HC backbone paired with bovine (b) V_{λ}, (AF023843.1) or V_{κ} (BC122795) linked to the constant region of mouse (m) C_{κ}, C_{λ1}, C_{λ2}, or C_{λ3}.
FIG. 20A shows loading controls using Myc (2001) and FIG. 20B shows loading controls using GAPDH (2002) of the western blots shown in FIG. 19A and 19B.
FIG. 21 shows expression of intracellular bovine IGHV BLV1H12 (2101) and bovine IGHV BLV5B8 (2102) with a mouse IgD backbone and bovine V_{λ}-mouse C_{λ1} (2103), C_{λ2} (2104) or C_{λ3} (2105).
FIG. 22 shows expression of cell surface of the same bovine constructs as in FIG.21, stained with the same antibodies and with the data arranged the same as in FIG. 21.
FIG. 23 shows expression of intracellular bovine IGHV B4, which has an average length HCDR3, with a mouse IgD backbone and bovine V_{λ} attached to mouse C_{λ1} (2302), C_{λ2} (2303) or C_{λ3} (2304).
FIG. 24 shows expression bovine IGHV B4 with a mouse IgD backbone and bovine V_{λ} attached to mouse C_{λ1} (2402), C_{λ2} (2403) or C_{λ3} (2404) with cell surface staining, in which the cell surface staining data is arranged the same as in FIG. 23.

### DEFINITIONS

The terms used herein are intended to have the plain and ordinary meaning as understood by those of ordinary skill in the art. The following definitions are intended to aid the reader in understanding the present invention but are not intended to vary or otherwise limit the meaning of such terms unless specifically indicated.

The term "locus" as used herein refers to a chromosomal segment or nucleic acid sequence that, respectively, is present endogenously in the genome or is (or about to be) exogenously introduced into the genome. For example, an immunoglobulin locus may include part or all of the genes (i.e., V, D, J gene segments as well as constant region genes) and intervening sequences (i.e., introns, enhancers, etc.) supporting the expression of immunoglobulin H or L chain polypeptides. Thus, a locus (e.g., immunoglobulin heavy chain variable region gene locus) may refer to a specific portion of a larger locus (e.g., a portion of the immunoglobulin H chain locus that includes the V_{H}, D and J_{H} gene segments). Similarly, an immunoglobulin light chain variable region gene locus may refer to a specific portion of a larger locus (e.g., a portion of the immunoglobulin L chain locus that includes the V_{L} and J_{L} gene segments). The term "immunoglobulin variable region gene" as used herein refers to a V, D, or J gene segment that encodes a portion of an immunoglobulin H or L chain variable domain. The term "immunoglobulin variable region gene locus" as used herein refers to part of, or the entire, chromosomal segment or nucleic acid strand containing clusters of the V, D, or J gene segments and may include the non-coding regulatory or scaffold sequences.

The term "gene segment" as used herein, refers to a nucleic acid sequence that encodes a part of the heavy chain or light chain variable domain of an immunoglobulin molecule. A gene segment can include coding and non-coding sequences. The coding sequence of a gene segment is a nucleic acid sequence that can be translated into a polypeptide, such the leader peptide and the N-terminal portion of a heavy chain or light chain variable domain. The non-coding sequences of a gene segment are sequences flanking the coding sequence, which may include the promoter, 5' untranslated sequence, intron intervening the coding sequences of the leader peptide, recombination signal sequence(s) (RSS), and splice sites. The gene segments in the immunoglobulin heavy chain (IGH) locus comprise the V_{H}, D and J_{H} gene segments (also referred to as IGHV, IGHD and IGHJ, respectively). The light chain variable region gene segments in the immunoglobulin κ and λ light loci can be referred to as V_{L} and J_{L} gene segments. In the κ light chain, the V_{L} and J_{L} gene segments can be referred to as V_{κ} and J_{κ} gene segments or IGKV and IGKJ. Similarly, in the λ light chain, the V_{L} and J_{L} gene segments can be referred to as V_{λ} and J_{λ} gene segments or IGLV and IGLJ.

The heavy chain constant region can be referred to as C_{H} or IGHC. The C_{H} region exons that encode IgM, IgD, IgG1-4, IgE, or IgA can be referred to as, respectively, C_{µ,} C_{δ}, C_{γ1-4}, C_{ε} or C_{α}. Similarly, the immunoglobulin κ or λ constant region can be referred to as C_{κ} or C_{λ}, as well as IGKC or IGLC, respectively.

"Partly bovine" as used herein refers to a strand of nucleic acids, or their expressed protein and RNA products, comprising sequences corresponding to the sequences found in a given locus of both a bovine and a non-bovine mammalian host. "Partly bovine" as used herein also refers to an animal comprising nucleic acid sequences from both a bovine and a non-bovine mammal, for example, a rodent. In one aspect, the partly bovine nucleic acids have coding sequences of bovine immunoglobulin H or L chain variable region gene segments and sequences based on the non-coding regulatory or scaffold sequences of the endogenous immunoglobulin locus of the non-bovine mammal.

The term "based on" when used with reference to endogenous non-coding regulatory or scaffold sequences from a non-bovine mammalian host cell genome refers to the non-coding regulatory or scaffold sequences that are present in the corresponding endogenous locus of the mammalian host cell genome. In one aspect, the term "based on" means that the non-coding regulatory or scaffold sequences that are present in the partly bovine immunoglobulin locus share a relatively high degree of homology with the non-coding regulatory or scaffold sequences of the endogenous locus of the host mammal. In one aspect, the non-coding sequences in the partly bovine immunoglobulin locus share at least about 80%, 90%, 95%, 96%, 97%, 98%, 99% or 100% homology with the corresponding non-coding sequences found in the endogenous locus of the host mammal. In one aspect, the non-coding sequences in the partly bovine immunoglobulin locus are retained from an immunoglobulin locus of the host mammal. In one aspect, the bovine coding sequences are embedded in the non-regulatory or scaffold sequences of the immunoglobulin locus of the host mammal. In one aspect, the host mammal is a rodent, such as a rat or mouse.

"Non-coding regulatory sequences" refer to sequences that are known to be essential for (i) V(D)J recombination, (ii) isotype switching, (iii) proper expression of the full-length immunoglobulin H or L chains following V(D)J recombination, and (iv) alternate splicing to generate, e.g., membrane and secreted forms of the immunoglobulin H chain. "Non-coding regulatory sequences" may further include the following sequences of endogenous origin: enhancer and locus control elements such as the CTCF and PAIR sequences (Proudhon, et al., Adv. Immunol. 128:123-182 (2015)); promoters preceding each endogenous V gene segment; splice sites; introns; recombination signal sequences flanking each V, D, or J gene segment. In one aspect, the "non-coding regulatory sequences" of the partly bovine immunoglobulin locus share at least about 70%, 75%, 80%, 85%, 90%, 95%, 96%, 97%, 98%, 99% and up to 100% homology with the corresponding non-coding sequences found in the targeted endogenous immunoglobulin locus of the non-bovine mammalian host cell.

"Scaffold sequences" refer to sequences intervening the gene segments present in the endogenous immunoglobulin locus of the host cell genome. In certain aspects, the scaffold sequences are interspersed by sequences essential for the expression of a functional non-immunoglobulin gene, for example, ADAM6A or ADAM6B. In certain aspects, the scaffold sequences are derived (at least partially) from other sources-e.g., they could be rationally designed or artificial sequences, sequences present in the immunoglobulin locus of the bovine genome, sequences present in the immunoglobulin locus of another species, or combinations thereof. It is to be understood that the phrase "non-coding regulatory or scaffold sequence" is inclusive in meaning (i.e., referring to both the non-coding regulatory sequence and the scaffold sequence existing in a given locus).

The term "homology targeting vector" refers to a nucleic acid sequence used to modify the endogenous genome of a mammalian host cell by homologous recombination; such nucleic acid sequence may comprise (i) targeting sequences with significant homologies to the corresponding endogenous sequences flanking a locus to be modified that is present in the genome of the non-bovine mammalian host, (ii) at least one sequence-specific recombination site, (iii) non-coding regulatory or scaffold sequences, and (iv) optionally one or more selectable marker genes. As such, a homology targeting vector can be used to introduce a sequence-specific recombination site into particular region of a host cell genome.

"Site-specific recombination" or "sequence-specific recombination" refers to a process of DNA rearrangement between two compatible recombination sequences (also referred to as "sequence-specific recombination sites" or "site-specific recombination sequences") including any of the following three events: a) deletion of a preselected nucleic acid flanked by the recombination sites; b) inversion of the nucleotide sequence of a preselected nucleic acid flanked by the recombination sites, and c) reciprocal exchange of nucleic acid sequences proximate to recombination sites located on different nucleic acid strands. It is to be understood that this reciprocal exchange of nucleic acid segments can be exploited as a targeting strategy to introduce an exogenous nucleic acid sequence into the genome of a host cell.

The term "targeting sequence" refers to a sequence homologous to DNA sequences in the genome of a cell that flank or are adjacent to the region of an immunoglobulin locus to be modified. The flanking or adjacent sequence may be within the locus itself or upstream or downstream of coding sequences in the genome of the host cell. Targeting sequences are inserted into recombinant DNA vectors which are used to transfect, e.g., ES cells, such that sequences to be inserted into the host cell genome, such as the sequence of a recombination site, are flanked by the targeting sequences of the vector.

The term "site-specific targeting vector" as used herein refers to a vector comprising a nucleic acid encoding a sequence-specific recombination site, an engineered partly bovine locus, and optionally a selectable marker gene, which is used to modify an endogenous immunoglobulin locus in a host using recombinase-mediated site-specific recombination. The recombination site of the targeting vector is suitable for site-specific recombination with another corresponding recombination site that has been inserted into a genomic sequence of the host cell (e.g., via a homology targeting vector), adjacent to an immunoglobulin locus that is to be modified. Integration of an engineered partly bovine sequence into a recombination site in an immunoglobulin locus results in replacement of the endogenous locus by the exogenously introduced partly bovine region.

The term "transgene" is used herein to describe genetic material that has been or is about to be artificially inserted into the genome of a cell, and particularly a cell of a mammalian host animal. The term "transgene" as used herein refers to a partly bovine nucleic acid, e.g., a partly bovine nucleic acid in the form of an engineered expression construct or a targeting vector.

"Transgenic animal" refers to a non-bovine animal, usually a mammal, having an exogenous nucleic acid sequence present as an extrachromosomal element in a portion of its cells or stably integrated into its germ line DNA (i.e., in the genomic sequence of most or all of its cells). In one aspect, a partly bovine nucleic acid is introduced into the germ line of such transgenic animals by genetic manipulation of, for example, embryos or embryonic stem cells of the host animal according to methods well known in the art.

A "vector" includes plasmids and viruses and any DNA or RNA molecule, whether self-replicating or not, which can be used to transform or transfect a cell.

Note that as used herein and in the appended claims, the singular forms "a," "an," and "the" include plural referents unless the context clearly dictates otherwise. Thus, for example, reference to "a locus" refers to one or more loci, and reference to "the method" includes reference to equivalent steps and methods known to those skilled in the art, and so forth.

As used herein, the term "or" can mean "and/or", unless explicitly indicated to refer only to alternatives or the alternatives are mutually exclusive. The terms "including," "includes" and "included", are not limiting.

Unless defined otherwise, all technical and scientific terms used herein have the same meaning as commonly understood by one of ordinary skill in the art to which this invention belongs. All publications mentioned herein are incorporated by reference for the purpose of describing and disclosing devices, formulations and methodologies that may be used in connection with the presently described invention.

Where a range of values is provided, it is understood that each intervening value, between the upper and lower limit of that range and any other stated or intervening value in that stated range is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either both of those included limits are also included in the invention.

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Such conventional techniques include polymer array synthesis, hybridization and ligation of polynucleotides, polymerase chain reaction, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach and Veksler, Eds. (2007), PCR Primer: A Laboratory Manual; Bowtell and Sambrook (2003), DNA Microarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleotide Synthesis: A Practical Approach" 1984, IRL Press, London; Nelson and Cox (2000), Lehninger, Principles of Biochemistry 3.sup.rd Ed., W. H. Freeman Pub., New York, N.Y.; and Berg et al. (2002) Biochemistry, 5.sup.th Ed., W.H. Freeman Pub., New York, N.Y., all of which are herein incorporated in their entirety by reference for all purposes.

### DETAILED DESCRIPTION

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Described herein is a transgenic rodent or rodent cell having a genome comprising an engineered partly bovine immunoglobulin heavy chain or light chain locus. In one aspect, the partly bovine immunoglobulin heavy chain locus comprises one or more bovine immunoglobulin heavy chain variable region gene segments. In one aspect, the partly bovine immunoglobulin light chain locus comprises one or more bovine immunoglobulin λ light chain variable region gene segments. In one aspect, the partly bovine immunoglobulin light chain locus comprises one or more bovine immunoglobulin κ light chain variable region gene segments. In one aspect, the partly bovine immunoglobulin heavy chain or light chain locus comprise immunoglobulin region gene segments from *Bos Taurus.*

In one aspect, non-bovine mammalian cells are provided that comprise an exogenously introduced, engineered partly bovine nucleic acid sequence comprising coding sequences for bovine variable regions and non-coding regulatory or scaffold sequences present in the immunoglobulin locus of the mammalian host genome, e.g., mouse genomic non-coding sequences when the host mammal is a mouse. In one aspect, one or more coding sequences for bovine variable region gene segments are embedded in non-coding regulatory or scaffold sequences corresponding to those of an immunoglobulin locus in a mammalian host genome. In one aspect, the coding sequences for bovine variable region gene segments are embedded in non-coding regulatory or scaffold sequences of a rodent or mouse immunoglobulin locus.

In one aspect, the partly bovine immunoglobulin locus is synthetic and comprises bovine V_{H}, D, or J_{H} or V_{L} or J_{L} gene segment coding sequences that are under the control of regulatory elements of the endogenous host. In one aspect, the partly bovine immunoglobulin locus comprises bovine V_{H}, D, or J_{H} or V_{L} or J_{L} gene segment coding sequences embedded in non-coding regulatory or scaffold sequences corresponding to those of an immunoglobulin locus in a mammalian host genome.

Methods are also provided for generating a transgenic rodent or rodent ES cell comprising exogenously introduced, engineered partly bovine immunoglobulin loci, wherein the resultant transgenic rodent is capable of producing more immunoglobulin comprising λ light chain than immunoglobulin comprising κ light chain.

### Immunoglobulin Loci in mice and cattle

In the humoral immune system, a diverse antibody repertoire is produced by combinatorial and junctional diversity of IGH and IGL chain gene loci by a process termed V(D)J recombination. In the developing B cell, the first recombination event to occur is between one D and one J_{H} gene segment of the heavy chain locus, and the DNA between these two gene segments is deleted. This D-J_{H} recombination is followed by the joining of one V_{H} gene segment from a region upstream of the newly formed DJ_{H} complex, forming a rearranged V_{H}DJ_{H} exon. All other sequences between the recombined V_{H} and D gene segments of the newly generated V_{H}DJ_{H} exon are deleted from the genome of the individual B cell. This rearranged exon is ultimately expressed on the B cell surface as the variable region of the H-chain polypeptide, which is associated with an L-chain polypeptide to form the B cell receptor (BCR).

The light chain repertoire in the mouse is believed to be shaped by the order of gene rearrangements. The IGK light chain locus on both chromosomes is believed to undergo V_{κ}-J_{κ} rearrangements first before the IGL light chain locus on either chromosome becomes receptive for V_{λ}-J_{λ} recombination. If an initial κ rearrangement is unproductive, additional rounds of secondary rearrangement can proceed, in a process known as receptor editing (Collins and Watson. (2018) Immunoglobulin light chain gene rearrangements, receptor editing and the development of a self-tolerant antibody repertoire. Front. 9:2249.) A process of serial rearrangement of the κ chain locus may continue on one chromosome until all possibilities of recombination are exhausted. Recombination will then proceed on the second κ chromosome. A failure to produce a productive rearrangement on the second chromosome after multiple rounds of rearrangement will be followed by rearrangement on the λ loci (Collins and Watson (2018) Immunoglobulin light chain gene rearrangements, receptor editing and the development of a self-tolerant antibody repertoire. Front. Immunol. 9:2249.) This preferential order of light chain rearrangements is believed to give rise to a light chain repertoire in mouse that is >90% κ and <10% λ.

However, immunoglobulins in the bovine immune system are dominated by λ light chain usage, which has been estimated to be at least 90% λ to <10% κ (Arun et al. (1996) Immunohistochemical examination of light-chain expression (λ/κ ratio) in canine, feline, equine, bovine and porcine plasma cells. Zentralbl Veterinarmed A. 43(9):573-6).

The murine and bovine Ig loci are highly complex in the numbers of features they contain and in how their coding regions are diversified by V(D)J rearrangement; however, this complexity does not extend to the basic details of the structure of each variable region gene segment. The V, D and J gene segments are highly uniform in their compositions and organizations. For example, V gene segments have the following features that are arranged in essentially invariant sequential fashion in immunoglobulin loci: a short transcriptional promoter region (<600bp in length), an exon encoding the 5' UTR and the majority of the signal peptide for the antibody chain; an intron; an exon encoding a small part of the signal peptide of the antibody chain and the majority of the antibody variable domain, and a 3' recombination signal sequence necessary for V(D)J rearrangement. Similarly, D gene segments have the following necessary and invariant features: a 5' recombination signal sequence, a coding region and a 3' recombination signal sequence. The J gene segments have the following necessary and invariant features: a 5' recombination signal sequence, a coding region and a 3' splice donor sequence.

Non-bovine mammalian cells are provided that comprise an exogenously introduced, engineered, partly bovine nucleic acid sequence comprising coding sequences for bovine variable regions and non-coding sequences (e.g., promoter, 5' and 3' recombination sequences, and splice acceptor site).

Compared with humans and mice, cattle have fewer germline heavy chain V, D and J segments. Only a single V_{H} family, designated as BoV_{H}1, encoded on chromosome 21, is expressed at the cDNA level. Figure 12A provides a schematic diagram of the endogenous bovine IGH locus (1201), as well as an expanded view of the IGHC region (1202). The bovine immunoglobulin heavy chain variable region locus includes V_{H} (1203), D (1204) and J_{H} (1205) gene segments. It is thought that the bovine genome contains about 20 V_{H} gene segments, 10 D and 4 functional J_{H} gene segments.

The sequences of the bovine IGH are in Table 1.

Because of the limited number of germline heavy chain V, D and J segments, bovine immunoglobulin diversity relies on somatic hypermutation of ultralong CDR H3, rather than germline combinatorial diversity. (Stanfield et al. (2018) The unusual genetics and biochemistry of bovine immunoglobulins. Adv. Immunol. 137:135-164). Cattle of different breeds all appear to have unusually long CDR H3s, which are encoded by long bovine D segments. One bovine D (termed D2) contains 149 nucleotides, accounting for 49 amino acid codons. This long D contributes significantly to the length of the complementarity-determining region 3 (CDR3) of the bovine immunoglobulin H chain. By comparison, the average H chain CDR3 length in mice is ~11 amino acids and in humans is ~15 amino acids. Bovine antibodies are of two types, conventional antibodies, albeit with comparatively longer CDR3s (~25 amino acids), and those with ultralong CDR3s, which range from 40-67 residues, with an average of ~58 amino acids. The exceptionally long CDR3 has a unique structure that includes a supporting stalk with a projecting knob. In conventional antibodies the CDR loops of both H and L chain variable regions can contribute to antigen binding. However, in the ultralong (UL) CDR3 antibodies the CDR H1, H2, L1, and L2 loops only form the supporting stalk, and the UL CDR H3 loop forms the knob structure, which binds antigen and can be enormously diversified by changing the number of Cys residues as well as the resulting patterns and connectivities of the somatically generated disulfide bonds. The heavy chains of all UL bovine antibodies analyzed to date are encoded by a single V_{H} gene segment, VHBUL, a single D gene segment, D2, and a single J_{H} gene segment, J_{H}1.

Similar to humans and mice, two types of Ig light chains (κ and λ) are expressed in cattle, though the λ to κ ratio differs significantly among these animals. In mice, approximately 96% of light chains in the serum antibodies are the κ type, while the κ type in humans accounts for only 66% of the total population of IGL chains. In contrast, the L chain repertoire in cattle is dominated by λ chains. Cattle have 20 functional V_{λ} and 8 functional V_{κ} genes. The UL bovine antibodies use a single V_{λ}, V_{λ1x}.

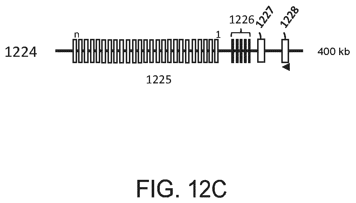

The bovine κ locus (1240) is located on chromosome 11 and is approximately 280 kb in size. The κ locus contains 22 V_{κ} genes (1225), 3 J_{κ} genes (1226) and one C_{κ} gene (1227). FIG. 12C provides a schematic diagram of the endogenous bovine IGK locus.

The bovine λ locus (1216) is located on chromosome 17 and is larger than the κ locus, and contains approximately 63 V_{λ} genes (1218), 8 J_{λ} genes and 9 C_{λ}, genes arrayed in J_{λ}-C_{λ} tandem clusters (C_{λ6} lacks a J_{λ} segment). FIG. 12B provides a schematic diagram of the endogenous bovine IGL locus.

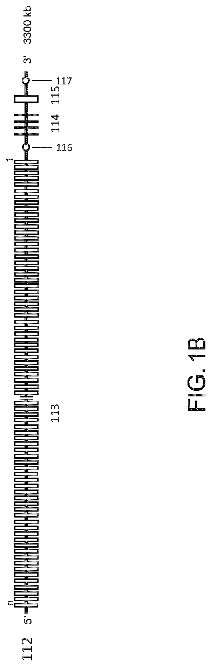

The mouse immunoglobulin κ locus is located on chromosome 6. Figure 1B provides a schematic diagram of the endogenous mouse IGK locus. The IGK locus (112) spans 3300 Kbp and includes more than 100 variable V_{κ} gene segments (113) located upstream of 5 joining (J_{K}) gene segments (114) and one constant (C_{κ}) gene (115). The mouse κ locus includes an intronic enhancer (iE_{κ}, 116) located between J_{κ} and C_{κ} that activates κ rearrangement and helps maintain the earlier or more efficient rearrangement of κ versus λ (Inlay et al. (2004) Important Roles for E Protein Binding Sites within the Immunoglobulin κ chain intronic enhancer in activating VκJκ rearrangement. J. Exp. Med. 200(9):1205-1211). Another enhancer, the 3' enhancer (3'E_{κ}, 117) is located 9.1 Kb downstream of the C_{κ} exon and is also involved in κ rearrangement and transcription; mutant mice lacking both iE_{κ} and 3'Eκ have no V_{κ}J_{κ} rearrangements in the κ locus (Inlay et al. (2002) Essential roles of the kappa light chain intronic enhancer and 3' enhancer in kappa rearrangement and demethylation. Nature Immunol. 3(5):463-468). However, disrupting the iE_{κ}, for example, by insertion of a neomycin-resistance gene is also sufficient to abolish most V_{κ}J_{κ} rearrangements (Xu et al. (1996) Deletion of the Igκ Light Chain Intronic Enhancer/Matrix Attachment Region Impairs but Does Not Abolish VκJκ Rearrangement).

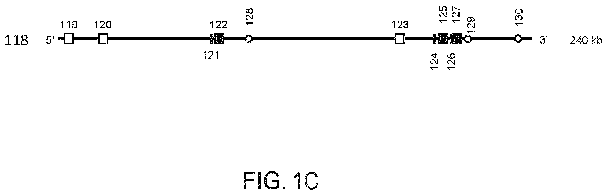

The mouse immunoglobulin λ locus is located on chromosome 16. Figure 1C provides a schematic diagram of the endogenous mouse IGL locus (118). The organization of the mouse immunoglobulin λ locus is different from the mouse immunoglobulin κ locus. The locus spans 240 kb, with two clusters comprising 3 functional variable (V_{λ}) gene segments (IGLV2, 119; IGLV3, 120 and IGLV1, 123) and 3 tandem cassettes of λ joining (J_{λ}) gene segments and constant (C_{λ}) gene segments (IGLJ2, 121; IGLC2, 122; IGLJ3, 124: IGLC3, 125; IGLJ1, 126; IGLC1, 127) in which the V_{λ} gene segments are located upstream (5') from a variable number of J-C tandem cassettes. The locus also contains three transcriptional enhancers (E_{λ2-4}, 128; E_{λ}, 129; _{Eλ3-1}, 130).

The partly bovine nucleic acid sequence described herein allows the transgenic animal to produce a heavy chain or light chain repertoire comprising bovine V_{H} or V_{L} regions, while retaining the regulatory sequences and other elements that can be found within the intervening sequences of the host genome (e.g., rodent) that help to promote efficient antibody production and antigen recognition in the host.

In one aspect, synthetic, or recombinantly produced, partly bovine nucleic acids are engineered to comprise both bovine coding sequences and non-bovine non-coding regulatory or scaffold sequences from an immunoglobulin V_{H}, V_{λ} or V_{κ} locus, or, in some aspects, a combination thereof.

In one aspect, a transgenic rodent or rodent cell that expresses immunoglobulin with a bovine variable region can be generated by inserting one or more bovine V_{H} gene segment coding sequences into a V_{H} locus of a rodent heavy chain immunoglobulin locus. In another aspect, a transgenic rodent or rodent cell that expresses immunoglobulin with bovine a variable region can be generated by inserting one or more bovine V_{L} gene segment coding sequences into a V_{L} locus of a rodent light chain immunoglobulin locus.

The existence of two light chain loci - κ and λ - means that a variety of light chain insertion combinations are possible for generating a transgenic rodent or rodent cell that expresses immunoglobulin with bovine a variable region, including but not limited to: inserting one or more bovine V_{λ} or J_{λ} gene segment coding sequences into a rodent V_{λ} locus, inserting one or more bovine V_{κ} or J_{κ} gene segment coding sequences into a rodent V_{κ} locus, inserting one or more bovine V_{λ} or J_{λ} gene segment coding sequences into a rodent V_{κ} locus and inserting one or more bovine V_{κ} or J_{κ} gene segment coding sequences into a rodent V_{λ} locus.

The selection and development of a transgenic rodent or rodent cell that expresses partly bovine immunoglobulin is complicated by the fact that more than 90% of light chains produced by mice are κ and less than 10% are λ, whereas more than 90% of light chains produced by cattle are λ and less than 10% κ.

Since mice produce mainly κ LC-containing antibodies, one reasonable method to increase production of λ LC-containing partly bovine immunoglobulin by the transgenic rodent would be to insert one or more bovine V_{λ} or J_{λ} gene segment coding sequences into a rodent κ locus. However, as shown in the Example 9 below, coupling bovine V_{λ} region exon with rodent C_{κ} region exon results in sub-optimal expression of the partly bovine immunoglobulin in vitro.

Provided herein is a transgenic rodent or rodent cell that is capable of expressing immunoglobulin comprising bovine variable domains, wherein the transgenic rodent produces more or is more likely to produce immunoglobulin comprising λ light chain than immunoglobulin comprising κ light chain. While not wishing to be bound by theory, it is believed that a transgenic rodent or rodent cell that produces more, or is more likely to produce, immunoglobulin comprising λ light chain will result in a fuller antibody repertoire for the development of therapeutics.

A transgenic rodent or rodent cell having a genome comprising an engineered partly bovine immunoglobulin light chain locus is provided herein. In one aspect, the partly bovine immunoglobulin light chain locus comprises bovine immunoglobulin λ light chain variable region gene segments. In one aspect, the engineered immunoglobulin locus is capable of expressing immunoglobulin comprising a bovine variable domain. In one aspect, the engineered immunoglobulin locus is capable of expressing immunoglobulin comprising a bovine λ variable domain. In one aspect, the engineered immunoglobulin locus is capable of expressing immunoglobulin comprising a bovine κ variable domain. In one aspect, the engineered immunoglobulin locus expresses immunoglobulin light chains comprising a bovine variable domain and a rodent constant domain. In one aspect, the engineered immunoglobulin locus expresses immunoglobulin light chains comprising a bovine λ variable domain and a rodent λ constant domain. In one aspect, the engineered immunoglobulin locus expresses immunoglobulin light chains comprising a bovine κ variable domain and a rodent κ constant domain.

In one aspect, the transgenic rodent or rodent cell produces more, or is more likely to produce, immunoglobulin comprising λ light chain than immunoglobulin comprising κ light chain. In one aspect, a transgenic rodent is provided in which more λ light chain producing cells than κ light chain producing cells are likely to be isolated from the rodent. In one aspect, a transgenic rodent is provided that produces at least about 25%, 30%, 35%, 40%, 45%, 50%, 55%, 60%, 65%, 70%, 75%, 80%, 85%, 90% or 95% and up to about 100% immunoglobulin comprising λ light chain. In one aspect, a transgenic rodent cell, or its progeny, is provided that is more likely to produce immunoglobulin with λ light chain than immunoglobulin with κ light chain. In one aspect, the transgenic rodent cell, or its progeny, has at least about a 25%, 30%, 35%, 40%, 45%, 50%, 55%, 60%, 65%, 70%, 75%, 80%, 85%, 90%, or 95% and up to about 100%, probability of producing immunoglobulin comprising λ light chain. In one aspect, a transgenic rodent or rodent cell is provided in which an endogenous rodent light chain immunoglobulin locus has been deleted and replaced with an engineered partly bovine light chain immunoglobulin locus. In one aspect, the transgenic rodent is a mouse.

### Immunoglobulin Light Chain Locus

In one aspect, a transgenic rodent or rodent cell is provided that has a genome comprising a recombinantly produced partly bovine immunoglobulin variable region locus. In one aspect, the partly bovine immunoglobulin variable region locus is a light chain variable region (V_{L}) locus. In one aspect, the partly bovine immunoglobulin variable region locus comprises one or more bovine V_{λ} gene segment coding sequences or one or more bovine J_{λ} gene segment coding sequences. In one aspect, the partly bovine immunoglobulin variable region locus comprises one or more bovine V_{κ} gene segment coding sequences or one or more bovine J_{κ} gene segment coding sequences. In one aspect, the partly bovine immunoglobulin variable region locus comprises one or more rodent constant domain genes or coding sequences. In one aspect, the partly bovine immunoglobulin variable region locus comprises one or more rodent C_{λ} genes or coding sequences. In one aspect, the partly bovine immunoglobulin variable region locus comprises one or more rodent C_{κ} genes or coding sequences. In one aspect, an endogenous rodent light chain immunoglobulin locus has been inactivated. In one aspect, an endogenous rodent light chain immunoglobulin locus has been deleted and replaced with an engineered partly bovine light chain immunoglobulin locus.

In one aspect, the engineered immunoglobulin locus expresses immunoglobulin light chains comprising a bovine λ variable domain and rodent λ constant domain. In one aspect, the engineered immunoglobulin locus expresses immunoglobulin light chains comprising a bovine κ variable domain and rodent κ constant domain.

In one aspect, the engineered partly bovine immunoglobulin variable region locus comprises a V_{L} locus comprising most or all of the V_{λ} gene segments coding sequences from a bovine genome. In one aspect, the engineered partly bovine immunoglobulin locus variable region comprises a V_{L} locus comprising at least 20, 30, 40, 50 and up to 63 bovine V_{λ} gene segment coding sequences. In one aspect the engineered partly bovine immunoglobulin variable region locus comprises a V_{L} locus comprising at least about 50%, 60%, 70%, 80%, 90% and up to 100% of the V_{λ} gene segment coding sequences from a bovine genome. In one aspect the engineered partly bovine immunoglobulin variable region locus comprises a V_{L} locus comprising at least about 50%, 60%, 70%, 80%, 90% and up to 100% of the V_{λ} gene segment coding sequences from *Bos taurus.*

In one aspect, the engineered partly bovine immunoglobulin locus variable region comprises a V_{L} locus comprising most or all of the J_{λ} gene segment coding sequences found in the bovine genome. In one aspect, the engineered partly bovine immunoglobulin locus variable region comprises a V_{L} locus comprising at least 1, 2 or, 3 bovine J_{λ} gene segment coding sequences. In one aspect the engineered partly bovine immunoglobulin variable region locus comprises a V_{L} locus comprising at least about 50%, 75%, and up to 100% of the J_{λ} gene segment coding sequences found in the bovine genome. In one aspect the engineered partly bovine immunoglobulin variable region locus comprises a V_{L} locus comprising at least about 50%, 75%, and up to 100% of the J_{λ} gene segment coding sequences from *Bos taurus.*

In one aspect, the engineered partly bovine immunoglobulin locus variable region comprises a V_{L} locus comprising most or all of the V_{λ} and J_{λ} gene segment coding sequences from the bovine genome. In one aspect the engineered partly bovine immunoglobulin variable region locus comprises a V_{L} locus comprising at least about 50%, 60%, 70%, 80%, 90% and up to 100% of the V_{λ} and J_{λ} gene segment coding sequences from the bovine genome. In one aspect the engineered partly bovine immunoglobulin variable region locus comprises a V_{L} locus comprising at least about 50%, 60%, 70%, 80%, 90% and up to 100% of the V_{λ} and J_{λ} gene segment coding sequences from the *Bos taurus.*

In one aspect, the engineered partly bovine immunoglobulin locus variable region comprises a V_{L} locus comprising most or all of the V_{κ} gene segment coding sequences from the bovine genome. In one aspect, the engineered partly bovine immunoglobulin locus variable region comprises a V_{L} locus comprising at least 1, 2, 3, 4, 5, 6, 7, 8, 9, 10, 11, 12, 13, 14, 15 and up to 22 bovine V_{κ} gene segment coding sequences. In one aspect the engineered partly bovine immunoglobulin variable region locus comprises a V_{L} locus comprising at least about 50%, 60%, 70%, 80%, 90% and up to 100% of the V_{κ} gene segment coding sequences from the bovine genome. In one aspect the engineered partly bovine immunoglobulin variable region locus comprises a V_{L} locus comprising at least about 50%, 60%, 70%, 80%, 90% and up to 100% of the V_{κ} gene segment coding sequences from *Bos taurus.*

In one aspect, the engineered partly bovine immunoglobulin locus variable region comprises a V_{L} locus comprising most or all of the J_{κ} gene segment coding sequences found in the bovine genome. In one aspect, the engineered partly bovine immunoglobulin locus variable region comprises a V_{L} locus comprising at least 1, 2, or 3 bovine J_{κ} gene segment coding sequences. In one aspect the engineered partly bovine immunoglobulin variable region locus comprises a V_{L} locus comprising at least about 50%, 75%, and up to 100% of the J_{κ} gene segment coding sequences found in the bovine genome. In one aspect the engineered partly bovine immunoglobulin variable region locus comprises a V_{L} locus comprising at least about 50%, 75%, and up to 100% of the J_{κ} gene segment coding sequences from *Bos taurus.*

In one aspect, the engineered partly bovine immunoglobulin locus variable region comprises a V_{L} locus comprising most or all of the V_{κ} and J_{κ} gene segment coding sequences from the bovine genome. In one aspect the engineered partly bovine immunoglobulin variable region locus comprises a V_{L} locus comprising at least about 50%, 60%, 70%, 80%, 90% and up to 100% of the V_{κ} and J_{κ} gene segment coding sequences from the bovine genome. In one aspect the engineered partly bovine immunoglobulin variable region locus comprises a V_{L} locus comprising at least about 50%, 60%, 70%, 80%, 90% and up to 100% of the V_{κ} and J_{κ} gene segment coding sequences from *Bos taurus.*

In one aspect, the engineered immunoglobulin locus comprises bovine V_{L} gene segment coding sequences and rodent non-coding regulatory or scaffold sequences from a rodent immunoglobulin light chain variable region gene locus. In one aspect, the engineered immunoglobulin locus comprises bovine V_{λ} or J_{λ} gene segment coding sequences and rodent non-coding regulatory or scaffold sequences from a rodent immunoglobulin light chain variable region gene locus. In one aspect, the rodent non-coding regulatory or scaffold sequences are from a rodent immunoglobulin λ light chain variable region gene locus. In one aspect, the rodent non-coding regulatory or scaffold sequences are from a rodent immunoglobulin κ light chain variable region locus. In one aspect, the engineered immunoglobulin locus comprises bovine V_{λ} and J_{λ} gene segment coding sequences and rodent non-coding regulatory or scaffold sequences from a rodent immunoglobulin λ light chain variable region gene locus. In one aspect, the partly bovine immunoglobulin locus comprises one or more rodent immunoglobulin λ constant region (C_{λ}) coding sequences. In one aspect, the partly bovine immunoglobulin locus comprises one or more bovine V_{λ} and J_{λ} gene segment coding sequences and one or more rodent immunoglobulin C_{λ} coding sequences. In one aspect, the engineered immunoglobulin locus comprises bovine V_{λ} and J_{λ} gene segment coding sequences and one or more rodent C_{λ} coding sequences embedded in rodent non-coding regulatory or scaffold sequences of a rodent immunoglobulin λ light chain variable region gene locus.

In one aspect, the engineered immunoglobulin locus comprises bovine V_{λ} or J_{λ} gene segment coding sequences and rodent non-coding regulatory or scaffold sequences from a rodent immunoglobulin κ light chain variable region gene locus. In one aspect, the engineered immunoglobulin locus comprises bovine V_{λ} or J_{λ} gene segment coding sequences embedded in rodent non-coding regulatory or scaffold sequences of a rodent immunoglobulin κ light chain variable region gene locus. In one aspect, the engineered immunoglobulin locus comprises bovine V_{λ} and J_{λ} gene segment coding sequences and one or more rodent immunoglobulin C_{λ} coding sequences and rodent non-coding regulatory or scaffold sequences from a rodent immunoglobulin κ light chain variable region gene locus. In one aspect, the engineered immunoglobulin locus comprises bovine V_{λ} and Jagene segment coding sequences and one or more rodent immunoglobulin C_{λ} coding sequences embedded in rodent non-coding regulatory or scaffold sequences of a rodent immunoglobulin κ light chain variable region gene locus.

In one aspect, one or more bovine V_{λ} gene segment coding sequences are located upstream of one or more J_{λ} gene segment coding sequences, which are located upstream of one or more rodent C_{λ} genes. In one aspect, one or more bovine V_{λ} gene segment coding sequences are located upstream and in the same transcriptional orientation as one or more J_{λ} gene segment coding sequences, which are located upstream of one or more rodent lambda C_{λ} genes.

In one aspect, the engineered immunoglobulin variable region locus comprises one or more bovine V_{λ} gene segment coding sequences, one or more bovine J_{λ} gene segment coding sequences and one or more rodent C_{λ} genes. In one aspect, the engineered immunoglobulin variable region locus comprises one or more bovine V_{λ} gene segment coding sequences, one or more bovine J_{λ} gene segment coding sequence and one or more rodent C_{λ} region genes, wherein the V_{λ} and J_{λ} gene segment coding sequences and the rodent C_{λ} region genes are inserted into a rodent immunoglobulin κ light chain locus. In one aspect, the engineered immunoglobulin variable region locus comprises one or more bovine V_{λ} gene segment coding sequences, one or more bovine J_{λ} gene segment coding sequence and one or more rodent C_{λ} genes, wherein the V_{λ} and J_{λ} gene segment coding sequences and the rodent (C₁) region genes are embedded in non-coding regulatory or scaffold sequences of a rodent immunoglobulin κ light chain locus.

In one aspect, one or more bovine V_{λ} gene segment coding sequences are located upstream of one or more J_{λ} gene segment coding sequences, which are located upstream of one or more rodent C_{λ} genes, wherein the V_{λ} and J_{λ} gene segment coding sequences and rodent C_{λ} genes are inserted into a rodent immunoglobulin κ light chain locus. In one aspect, one or more bovine V_{λ} gene segment coding sequences are located upstream of one or more J_{λ} gene segment coding sequences, which are located upstream of one or more rodent C_{λ} genes, wherein the V_{λ} and J_{λ} gene segment coding sequences and rodent Cλ genes are embedded in non-coding regulatory or scaffold sequences of a rodent immunoglobulin κ light chain locus.

In one aspect, the rodent C_{λ} coding sequence is selected from a rodent C_{λ1}, C_{λ2}, or C_{λ3} coding sequence.

In one aspect, a transgenic rodent or rodent cell is provided, wherein the engineered immunoglobulin locus comprises a rodent immunoglobulin κ locus in which one or more rodent V_{κ} gene segment coding sequences and one or more rodent J_{κ} gene segment coding sequences have been deleted and replaced by one or more bovine V_{λ} gene segment coding sequences and one or more J_{λ} gene segment coding sequences, respectively, and in which rodent C_{κ} coding sequences in the locus have been replaced by rodent C_{λ1}, C_{λ2}, or C_{λ3} coding sequence.

In one aspect, the engineered immunoglobulin variable region locus comprises one or more bovine V_{λ} gene segment coding sequences and one or more J-C units wherein each J-C unit comprises a bovine J_{λ} gene segment coding sequence and a rodent Cλ gene. In one aspect, the engineered immunoglobulin variable region locus comprises one or more bovine V_{λ} gene segment coding sequences and one or more J-C units wherein each J-C unit comprises a bovine J_{λ} gene segment coding sequence and rodent C_{λ} region coding sequence, wherein the V_{λ} gene segment coding sequences and the J-C units are inserted into a rodent immunoglobulin κ light chain locus. In one aspect, the engineered immunoglobulin variable region locus comprises one or more bovine V_{λ} gene segment coding sequences and one or more J-C units wherein each J-C unit comprises a bovine J_{λ} gene segment coding sequence and rodent C_{λ} coding sequence, wherein the V_{λ} gene segment coding sequences and the J-C units are embedded in non-coding regulatory or scaffold sequences of a rodent immunoglobulin κ light chain locus.

In one aspect, one or more bovine V_{λ} gene segment coding sequences are located upstream and in the same transcriptional orientation as one or more J-C units, wherein each J-C unit comprises a bovine J_{λ} gene segment coding sequence and a rodent C_{λ} gene. In one aspect, one or more bovine V_{λ} gene segment coding sequences are located upstream and in the same transcriptional orientation as one or more J-C units, wherein each J-C unit comprises a bovine J_{λ} gene segment coding sequence and a rodent C_{λ} coding sequence. In one aspect, the engineered immunoglobulin variable region locus comprises one or more bovine V_{λ} gene segment coding sequences located upstream of one or more J-C units wherein each J-C unit comprises a bovine J_{λ} gene segment coding sequence and rodent Cλ coding sequence, wherein the V_{λ} gene segment coding sequences and the J-C units are inserted into a rodent immunoglobulin κ light chain locus. In one aspect, the engineered immunoglobulin variable region locus comprises one or more bovine V_{λ} gene segment coding sequences upstream and in the same transcriptional orientation as one or more J-C units wherein each J-C unit comprises a bovine J_{λ} gene segment coding sequence and rodent Cλ coding sequence, wherein the V_{λ} gene segment coding sequences and the J-C units are embedded in non-coding regulatory or scaffold sequences of a rodent immunoglobulin κ light chain locus. In one aspect, the rodent C_{λ} coding sequence is selected from a rodent C_{λ1}, C_{λ2}, or C_{λ3} coding sequence.

In one aspect, the engineered immunoglobulin locus comprises bovine V_{κ} coding sequences and rodent non-coding regulatory or scaffold sequences from a rodent immunoglobulin light chain variable region gene locus. In one aspect, the engineered immunoglobulin locus comprises bovine V_{κ} or J_{κ} gene segment coding sequences and rodent non-coding regulatory or scaffold sequences from a rodent immunoglobulin light chain variable region gene locus. In one aspect, the rodent non-coding regulatory or scaffold sequences are from a rodent immunoglobulin λ light chain variable region gene locus. In one aspect, the rodent non-coding regulatory or scaffold sequences are from a rodent immunoglobulin κ light chain variable region locus. In one aspect, the engineered immunoglobulin locus comprises bovine V_{κ} and J_{κ} gene segment coding sequences and rodent non-coding regulatory or scaffold sequences from a rodent immunoglobulin κ light chain variable region gene locus. In one aspect, the engineered immunoglobulin locus comprises bovine V_{κ} and J_{κ} gene segment coding sequences and rodent non-coding regulatory or scaffold sequences from a rodent immunoglobulin λ light chain variable region gene locus. In one aspect, the partly bovine immunoglobulin locus comprises one rodent immunoglobulin C_{κ} coding sequences. In one aspect, the partly bovine immunoglobulin locus comprises one or more rodent immunoglobulin C_{λ} coding sequences. In one aspect, the partly bovine immunoglobulin locus comprises one or more bovine V_{κ} and J_{κ} gene segment coding sequences and one rodent immunoglobulin C_{κ} coding sequences. In one aspect, the engineered immunoglobulin locus comprises bovine V_{κ} and J_{κ} gene segment coding sequences and one rodent immunoglobulin C_{κ} coding sequences embedded in rodent non-coding regulatory or scaffold sequences of a rodent κ light chain variable region gene locus. In one aspect, the engineered immunoglobulin locus comprises bovine V_{κ} and J_{κ} gene segment coding sequences and one rodent immunoglobulin C_{κ} coding sequences embedded in rodent non-coding regulatory or scaffold sequences of a rodent immunoglobulin λ light chain variable region gene locus.

While not wishing to be bound by theory, it is believed that inactivating or rendering nonfunctional an endogenous rodent κ light chain locus may increase expression of λ light chain immunoglobulin from the partly bovine immunoglobulin locus. This has been shown to be the case in otherwise conventional mice in which the κ light chain locus has been inactivated in the germline (Zon, et al. (1995) Subtle differences in antibody responses and hypermutation of λ light chains in mice with a disrupted κ constant region. Eur. J. Immunol. 25:2154-2162). In one aspect, inactivating or rendering nonfunctional an endogenous rodent κ light chain locus may increase the relative amount of immunoglobulin comprising λ light chain relative to the amount of immunoglobulin comprising κ light chain produced by the transgenic rodent or rodent cell.

In one aspect, a transgenic rodent or rodent cell is provided in which an endogenous rodent immunoglobulin κ light chain locus is deleted, inactivated, or made nonfunctional. In one aspect, the endogenous rodent immunoglobulin κ light chain locus is inactivated or made nonfunctional by one or more of the following deleting or mutating all endogenous rodent V_{κ} gene segment coding sequences; deleting or mutating all endogenous rodent J_{κ} gene segment coding sequences; deleting or mutating the endogenous rodent C_{κ} coding sequence; deleting, mutating, or disrupting the endogenous intronic κ enhancer (iE_{κ}) and 3' enhancer sequence (3'E_{κ}); or a combination thereof.

In one aspect, a transgenic rodent or rodent cell is provided in which an endogenous rodent immunoglobulin λ light chain variable domain is deleted, inactivated, or made nonfunctional. In one aspect, the endogenous rodent immunoglobulin λ light chain variable domain is inactivated or made nonfunctional by one or more of the following: deleting or mutating all endogenous rodent V_{κ} gene segments; deleting or mutating all endogenous rodent J_{λ} gene segments; deleting or mutating all endogenous rodent C_{λ} coding sequences; or a combination thereof.

In one aspect, the partly bovine immunoglobulin locus comprises rodent regulatory or scaffold sequences, including, but not limited to enhancers, promoters, splice sites, introns, recombination signal sequences, and combinations thereof. In one aspect, the partly bovine immunoglobulin locus comprises rodent λ regulatory or scaffold sequences. In one aspect, the partly bovine immunoglobulin locus comprises rodent κ regulatory or scaffold sequences.

In one aspect, the partly bovine immunoglobulin locus includes a promoter to drive gene expression. In one aspect, the partly bovine immunoglobulin locus includes a κ V-region promoter. In one aspect, the partly bovine immunoglobulin locus includes a λ V-region promoter. In one aspect, the partly bovine immunoglobulin locus includes a λ V-region promoter to drive expression of one or more λ LC gene coding sequences created after V_{λ} to J_{λ} gene segment rearrangement. In one aspect, the partly bovine immunoglobulin locus includes a λ V-region promoter to drive expression of one or more κ LC gene coding sequences created after V_{κ} to J_{κ} gene segment rearrangement. In one aspect, the partly bovine immunoglobulin locus includes a κ V-region promoter to drive expression of one or more λ LC gene coding sequences created after V_{λ} to J_{λ} gene segment rearrangement. In one aspect, the partly bovine immunoglobulin locus includes a κ V-region promoter to drive expression of one or more κ LC gene coding sequences created after V_{κ} to J_{κ} gene segment rearrangement.

In one aspect, the partly bovine immunoglobulin locus includes one or more enhancers. In one aspect, the partly bovine immunoglobulin locus includes a mouse κ iEκ or 3'Eκ enhancer. In one aspect, the partly bovine immunoglobulin locus includes one or more V_{λ} or J_{λ} gene segment coding sequences and a moue κ iE_{κ} or 3'E_{κ} enhancer. In one aspect, the partly bovine immunoglobulin locus includes one or more V_{κ} or J_{κ} gene segment coding sequences and a κ iE_{κ} or 3'E_{κ} enhancer.

### Immunoglobulin Heavy Chain Locus

In one aspect, a transgenic rodent or rodent cell has a genome comprising a recombinantly produced partly bovine immunoglobulin heavy chain variable region (V_{H}) locus. In one aspect, the partly bovine immunoglobulin variable region locus comprises one or more bovine V_{H}, D or J_{H} gene segment coding sequences. In one aspect, the partly bovine immunoglobulin heavy chain variable region locus comprises one or more rodent constant domain (C_{H}) genes or coding sequences. In one aspect, an endogenous rodent heavy chain immunoglobulin locus has been inactivated. In one aspect, an endogenous rodent heavy chain immunoglobulin locus has been deleted and replaced with an engineered partly bovine heavy chain immunoglobulin locus.

In one aspect, the synthetic H chain DNA segment contains the ADAM6A or ADAM6B gene needed for male fertility, Pax-5-Activated Intergenic Repeats (PAIR) elements involved in IGH locus contraction and CTCF binding sites from the heavy chain intergenic control region 1, involved in regulating normal VDJ rearrangement ((Proudhon, et al., Adv. Immunol., 128:123-182 (2015)), or various combinations thereof. The locations of these endogenous non-coding regulatory and scaffold sequences in the mouse IGH locus are depicted in FIG 1, which illustrates from left to right: the ~100 functional heavy chain variable region gene segments (101); PAIR, Pax-5 Activated Intergenic Repeats involved in IGH locus contraction for VDJ recombination (102); ADAM6A or ADAM6B, a disintegrin and metallopeptidase domain 6A gene required for male fertility (103); Pre-D region, a 21609 bp fragment upstream of the most distal D gene segment, IGHD-5 D (104); Intergenic Control Region 1 (IGCR1) that contains CTCF insulator sites to regulate V_{H} gene segment usage (106); D, diversity gene segments (10-15 depending on the mouse strain) (105); four joining J_{H} gene segments (107); E_{µ}, the intronic enhancer involved in VDJ recombination (108); S_{µ}, the µ switch region for isotype switching (109); eight heavy chain constant region genes: C_{µ}, C_{δ}, C_{γ3}, C_{γ1}, C_{γ2b}, C2_{γa/c}, C_{ε}, and C_{α} (110); 3' Regulatory Region (3'RR) that controls isotype switching and somatic hypermutation (111). FIG. 1A is modified from a figure taken from Proudhon, et al., Adv. Immunol., 128:123-182 (2015).

In one aspect, the engineered partly bovine region to be integrated into a mammalian host cell comprises all or a substantial number of the known bovine V_{H} gene segments. In some instances, however, it may be desirable to use a subset of such V_{H} gene segments, and in specific instances even as few as one bovine V_{H} coding sequence may be introduced into the cell or the animal.

In one aspect, the engineered partly bovine immunoglobulin locus variable region comprises a V_{H} locus comprising most or all of the V_{H} gene segment coding sequences from the bovine genome. In one aspect, the engineered partly bovine immunoglobulin locus variable region comprises a V_{H} locus comprising at least 1, 2, 3, 4, 5, 6, 7, 8, 9, 10, 11 and up to 12 functional bovine V_{H} gene segment coding sequences. In one aspect the engineered partly bovine immunoglobulin variable region locus comprises a V_{H} locus comprising at least about 50%, 60%, 70%, 80%, 90% and up to 100% of the V_{H} gene segment coding sequences from the bovine genome. In one aspect the engineered partly bovine immunoglobulin variable region locus comprises a V_{H} locus comprising at least about 50%, 60%, 70%, 80%, 90% and up to 100% of the V_{H} gene segment coding sequences from *Bos taurus.*

In one aspect, the engineered partly bovine immunoglobulin locus variable region comprises a V_{H} locus comprising most or all of the V_{H} gene segment coding sequences from the bovine genome. In one aspect, the engineered partly bovine immunoglobulin locus variable region comprises a V_{H} locus comprising at least 1, 2, 3, 4, 5, 6, 7, 8, 9, 10, 11 and up to 12 bovine V_{H} gene segment coding sequences. In this aspect, the V_{H} gene segment pseudogenes are reverted to restore their functionality, e.g., by mutating an in-frame stop codon into a functional codon, using methods well known in the art. In one aspect the engineered partly bovine immunoglobulin variable region locus comprises a V_{H} locus comprising at least about 50%, 60%, 70%, 80%, 90% and up to 100% of the V_{H} gene segment coding sequences from the bovine genome.

In one aspect, the engineered partly bovine immunoglobulin locus variable region comprises a V_{H} locus comprising most or all of the D gene segment coding sequences found in the bovine genome. In one aspect, the engineered partly bovine immunoglobulin locus variable region comprises a V_{H} locus comprising at least 1, 2, 3, 4, 5, 6, 7, 8, 9, 10, 11, 12, 13, 14, 15, 16, 17, 18, 19, 20, 21, 22 and up to 23 bovine D gene segment coding sequences. In one aspect the engineered partly bovine immunoglobulin variable region locus comprises a V_{H} locus comprising at least about 50%, 60%, 70%, 80%, 90% and up to 100% of the D gene segment coding sequences found in the bovine genome. In one aspect the engineered partly bovine immunoglobulin variable region locus comprises a V_{H} locus comprising at least about 50%, 60%, 70%, 80%, 90% and up to 100% of the D gene segment coding sequences from *Bos taurus.*

In one aspect, the engineered partly bovine immunoglobulin locus variable region comprises a V_{H} locus comprising most or all of the J_{H} gene segment coding sequences found in the bovine genome. In one aspect, the engineered partly bovine immunoglobulin locus variable region comprises a V_{H} locus comprising at least 1, 2, or 3and up to 4 functional bovine J_{H} gene segment coding sequences. In one aspect the engineered partly bovine immunoglobulin variable region locus comprises a V_{H} locus comprising at least about 50%, 75%, and up to 100% of J_{H} gene segment coding sequences found in the bovine genome. In one aspect the engineered partly bovine immunoglobulin variable region locus comprises a V_{H} locus comprising at least about 50%, 75%, and up to 100% of J_{H} gene segment coding sequences from *Bos taurus.*

In one aspect, the engineered partly bovine immunoglobulin locus variable region comprises a V_{H} locus comprising most or all of the V_{H}, D and J_{H} gene segment coding sequences from the bovine genome. In one aspect the engineered partly bovine immunoglobulin variable region locus comprises a V_{H} locus comprising at least about 50%, 60%, 70%, 80%, 90% and up to 100% of the V_{H}, D and J_{H} gene segment coding sequences from the bovine genome. In one aspect the engineered partly bovine immunoglobulin variable region locus comprises a V_{H} locus comprising at least about 50%, 60%, 70%, 80%, 90% and up to 100% of the V_{H}, D and J_{H} gene segment coding sequences from *Bos taurus.*

In one aspect, a transgenic rodent or rodent cell is provided that includes an engineered partly bovine immunoglobulin heavy chain locus comprising bovine immunoglobulin heavy chain variable region gene coding sequences and non-coding regulatory or scaffold sequences of the rodent immunoglobulin heavy chain locus. In one aspect, the engineered bovine immunoglobulin heavy chain locus comprises bovine V_{H}, D or J_{H} gene segment coding sequences. In one aspect, the engineered bovine immunoglobulin heavy chain locus comprises bovine V_{H}, D or J_{H} gene segment coding sequences embedded in non-coding regulatory or scaffold sequences of a rodent immunoglobulin heavy chain locus.

In one aspect, non-bovine mammals and mammalian cells comprising an engineered partly bovine immunoglobulin locus that comprises coding sequences of bovine V_{H}, bovine D, and bovine J_{H} genes are provided that further comprise non-coding regulatory and scaffold sequences, including pre-D sequences, based on the endogenous IGH locus of the non-bovine mammalian host. In certain aspects, the exogenously introduced, engineered partly bovine region can comprise a fully recombined V(D)J exon.

In one aspect, the transgenic non-bovine mammal is a rodent, for example, a mouse, comprising an exogenously introduced, engineered partly bovine immunoglobulin locus comprising codons for multiple bovine V_{H}, bovine D, and bovine J_{H} genes with intervening sequences, including a pre-D region, based on the intervening (non-coding regulatory or scaffold) sequences in the rodent. In one aspect, the transgenic rodent further comprises partly bovine IGL loci comprising coding sequences of bovine V_{κ} or V_{λ} genes and J_{κ} or J_{λ} genes, respectively, in conjunction with their intervening (non-coding regulatory or scaffold) sequences corresponding to the immunoglobulin intervening sequences present in the IGL loci of the rodent.

In an exemplary embodiment, as set forth in more detail in the Examples section, the entire endogenous V_{H} immunoglobulin locus of the mouse genome is deleted and subsequently replaced with a partly bovine immunoglobulin locus comprising 20 bovine V_{H} gene segments containing interspersed non-coding sequences corresponding to the non-coding sequences of the J558 V_{H} locus of the mouse genome. The complete, exogenously introduced, engineered immunoglobulin locus further comprises bovine D and J_{H} gene segments, as well as the mouse pre-D region. Thus, the bovine V_{H}, D and J_{H} codon sequences are embedded in the rodent intergenic and intronic sequences.

### Preparation of a Partly Bovine Immunoglobulin Locus

In one aspect, an endogenous immunoglobulin locus variable region of a non-bovine mammal, such as a rodent, for example a rat or mouse, which contains V_{H}, D and J_{H} or V_{L} and J_{L} gene segments, is deleted using site-specific recombinases and replaced with an engineered partly bovine immunoglobulin locus. In one aspect, the partly bovine immunoglobulin locus is inserted into the genome of the host animal as a single nucleic acid or cassette. Because a cassette that includes the partly bovine immunoglobulin locus is used to replace the endogenous immunoglobulin locus variable region, the bovine coding sequences can be inserted into the host genome in a single insertion step, thus providing a rapid and straightforward process for obtaining a transgenic animal.

In one aspect, the engineered partly bovine immunoglobulin locus variable region is prepared by deleting murine V_{H}, D and J_{H} or V_{L} and J_{L} coding sequences from a mouse immunoglobulin locus variable region and replacing the murine coding sequences with bovine coding sequences. In one aspect, the non-coding flanking sequences of the murine immunoglobulin locus, which include regulatory sequences and other elements, are left intact.

In one aspect, the nucleotide sequence for the engineered partly bovine immunoglobulin locus is prepared *in silico* and the locus is synthesized using known techniques for gene synthesis. In one aspect, coding sequences from a bovine immunoglobulin variable region locus and sequences of the host animal immunoglobulin locus are identified using a search tool such as BLAST (Basic Local Alignment Search Tool). After obtaining the genomic sequences of the host immunoglobulin locus and the coding sequences of the bovine immunoglobulin variable region locus, the host coding sequences can be replaced *in silico* with the bovine coding sequences using known computational approaches to locate and delete the endogenous host animal immunoglobulin coding segments and replace the coding sequences with bovine coding sequences, leaving the endogenous regulatory and flanking sequences intact.

### Homologous Recombination

In one aspect, a combination of homologous recombination and site-specific recombination is used to create the cells and animals described herein. In some embodiments, a homology targeting vector is first used to introduce the sequence-specific recombination sites into the mammalian host cell genome at a desired location in the endogenous immunoglobulin loci. In one aspect, in the absence of a recombinase protein, the sequence-specific recombination site inserted into the genome of a mammalian host cell by homologous recombination does not affect expression and amino acid codons of any genes in the mammalian host cell. This approach maintains the proper transcription and translation of the immunoglobulin genes which produce the desired antibody after insertion of recombination sites and, optionally, any additional sequence such as a selectable marker gene. However, in some cases it is possible to insert a recombinase site and other sequences into an immunoglobulin locus sequence such that an amino acid sequence of the antibody molecule is altered by the insertion, but the antibody still retains sufficient functionality for the desired purpose. Examples of such codon-altering homologous recombination may include the introduction of polymorphisms into the endogenous locus and changing the constant region exons so that a different isotype is expressed from the endogenous locus. In one aspect, the immunoglobulin locus includes one or more of such insertions.

In one aspect, the homology targeting vector can be utilized to replace certain sequences within the endogenous genome as well as to insert certain sequence-specific recombination sites and one or more selectable marker genes into the host cell genome. It is understood by those of ordinary skill in the art that a selectable marker gene as used herein can be exploited to weed out individual cells that have not undergone homologous recombination and cells that harbor random integration of the targeting vector.

Exemplary methodologies for homologous recombination are described in U.S. Pat. Nos. 6,689,610; 6,204,061; 5,631,153; 5,627,059; 5,487,992; and 5,464,764, each of which is incorporated by reference in its entirety.

### Site/Sequence-Specific Recombination

Site/sequence-specific recombination differs from general homologous recombination in that short specific DNA sequences, which are required for recognition by a recombinase, are the only sites at which recombination occurs. Depending on the orientations of these sites on a particular DNA strand or chromosome, the specialized recombinases that recognize these specific sequences can catalyze i) DNA excision or ii) DNA inversion or rotation. Site-specific recombination can also occur between two DNA strands if these sites are not present on the same chromosome. A number of bacteriophage- and yeast-derived site-specific recombination systems, each comprising a recombinase and specific cognate sites, have been shown to work in eukaryotic cells and are therefore applicable for use in connection with the methods described herein, and these include the bacteriophage P1 Cre/lox, yeast FLP-FRT system, and the Dre system of the tyrosine family of site-specific recombinases. Such systems and methods of use are described, e.g., in U.S. Pat. Nos. 7,422,889; 7,112,715; 6,956,146; 6,774,279; 5,677,177; 5,885,836; 5,654,182; and 4,959,317, each of which is incorporated herein by reference to teach methods of using such recombinases.

Other systems of the tyrosine family of site-specific recombinases such as bacteriophage lambda integrase, HK2022 integrase, and in addition systems belonging to the separate serine family of recombinases such as bacteriophage phiC31, R4Tp901 integrases are known to work in mammalian cells using their respective recombination sites, and are also applicable for use in the methods described herein.

Since site-specific recombination can occur between two different DNA strands, site-specific recombination occurrence can be utilized as a mechanism to introduce an exogenous locus into a host cell genome by a process called recombinase-mediated cassette exchange (RMCE). The RMCE process can be exploited by the combined usage of wild-type and mutant sequence-specific recombination sites for the same recombinase protein together with negative selection. For example, a chromosomal locus to be targeted may be flanked by a wild-type LoxP site on one end and by a mutant LoxP site on the other. Likewise, an exogenous vector containing a sequence to be inserted into the host cell genome may be similarly flanked by a wild-type LoxP site on one end and by a mutant LoxP site on the other. When this exogenous vector is transfected into the host cell in the presence of Cre recombinase, Cre recombinase will catalyze RMCE between the two DNA strands, rather than the excision reaction on the same DNA strands, because the wild-type LoxP and mutant LoxP sites on each DNA strand are incompatible for recombination with each other. Thus, the LoxP site on one DNA strand will recombine with a LoxP site on the other DNA strand; similarly, the mutated LoxP site on one DNA strand will only recombine with a likewise mutated LoxP site on the other DNA strand.

In one aspect, combined variants of the sequence-specific recombination sites are used that are recognized by the same recombinase for RMCE. Examples of such sequence-specific recombination site variants include those that contain a combination of inverted repeats or those which comprise recombination sites having mutant spacer sequences. For example, two classes of variant recombinase sites are available to engineer stable Cre-loxP integrative recombination. Both exploit sequence mutations in the Cre recognition sequence, either within the 8 bp spacer region or the 13-bp inverted repeats. Spacer mutants such as lox511 (Hoess, et al., Nucleic Acids Res, 14:2287-2300 (1986)), lox5171 and lox2272 (Lee and Saito, Gene, 216:55-65 (1998)), m2, m3, m7, and m11 (Langer, et al., Nucleic Acids Res, 30:3067-3077 (2002)) recombine readily with themselves but have a markedly reduced rate of recombination with the wild-type site. This class of mutants has been exploited for DNA insertion by RMCE using non-interacting Cre-Lox recombination sites and non-interacting FLP recombination sites (Baer and Bode, Curr Opin Biotechnol, 12:473-480 (2001); Albert, et al., Plant J, 7:649-659 (1995); Seibler and Bode, Biochemistry, 36:1740-1747 (1997); Schlake and Bode, Biochemistry, 33:12746-12751 (1994)).

Inverted repeat mutants represent the second class of variant recombinase sites. For example, LoxP sites can contain altered bases in the left inverted repeat (LE mutant) or the right inverted repeat (RE mutant). An LE mutant, lox71, has 5 bp on the 5' end of the left inverted repeat that is changed from the wild type sequence to TACCG (Araki, et al, Nucleic Acids Res, 25:868-872 (1997)). Similarly, the RE mutant, lox66, has the five 3'-most bases changed to CGGTA. Inverted repeat mutants are used for integrating plasmid inserts into chromosomal DNA with the LE mutant designated as the "target" chromosomal loxP site into which the "donor" RE mutant recombines. Post-recombination, loxP sites are located in *cis,* flanking the inserted segment. The mechanism of recombination is such that post-recombination one loxP site is a double mutant (containing both the LE and RE inverted repeat mutations) and the other is wild type (Lee and Sadowski, Prog Nucleic Acid Res Mol Biol, 80:1-42 (2005); Lee and Sadowski, J Mol Biol, 326:397-412 (2003)). The double mutant is sufficiently different from the wild-type site that it is unrecognized by Cre recombinase and the inserted segment is not excised.

In certain aspects, sequence-specific recombination sites can be introduced into introns, as opposed to coding nucleic acid regions or regulatory sequences. This avoids inadvertently disrupting any regulatory sequences or coding regions necessary for proper antibody expression upon insertion of sequence-specific recombination sites into the genome of the animal cell.

Introduction of the sequence-specific recombination sites may be achieved by conventional homologous recombination techniques. Such techniques are described in references such as e.g., Sambrook and Russell (2001) (Molecular cloning: a laboratory manual 3rd ed. (Cold Spring Harbor, N.Y.: Cold Spring Harbor Laboratory Press) and Nagy, A. (2003). (Manipulating the mouse embryo: a laboratory manual, 3rd ed. (Cold Spring Harbor, N.Y.: Cold Spring Harbor Laboratory Press). Renault and Duchateau, Eds. (2013) (Site-directed insertion of transgenes. Topics in Current Genetics 23. Springer). Tsubouchi, H. Ed. (2011) (DNA recombination, Methods and Protocols. Humana Press).

Specific recombination into the genome can be facilitated using vectors designed for positive or negative selection as known in the art. In order to facilitate identification of cells that have undergone the replacement reaction, an appropriate genetic marker system may be employed and cells selected by, for example, use of a selection tissue culture medium. However, in order to ensure that the genome sequence is substantially free of extraneous nucleic acid sequences at or adjacent to the two end points of the replacement interval, desirably the marker system/gene can be removed following selection of the cells containing the replaced nucleic acid.

In one aspect, cells in which the replacement of all or part of the endogenous immunoglobulin locus has taken place are negatively selected against upon exposure to a toxin or drug. For example, cells that retain expression of HSV-TK can be selected against by using nucleoside analogues such as ganciclovir. In another aspect, cells comprising the deletion of the endogenous immunoglobulin locus may be positively selected for by use of a marker gene, which can optionally be removed from the cells following or as a result of the recombination event. A positive selection system that may be used is based on the use of two non-functional portions of a marker gene, such as HPRT, that are brought together through the recombination event. These two portions are brought into functional association upon a successful replacement reaction being carried out and wherein the functionally reconstituted marker gene is flanked on either side by further sequence-specific recombination sites (which are different from the sequence-specific recombination sites used for the replacement reaction), such that the marker gene can be excised from the genome, using an appropriate site-specific recombinase.

The recombinase may be provided as a purified protein, or as a protein expressed from a vector construct transiently transfected into the host cell or stably integrated into the host cell genome. Alternatively, the cell may be used first to generate a transgenic animal, which then may be crossed with an animal that expresses said recombinase.

Because the methods described herein can take advantage of two or more sets of sequence-specific recombination sites within the engineered genome, multiple rounds of RMCE can be exploited to insert the partly bovine immunoglobulin variable region genes into a non-bovine mammalian host cell genome.

Although not yet routine for the insertion of large DNA segments, CRISPR-Cas technology is another method to introduce the chimeric bovine Ig locus.

### Generation of Transgenic Animals

In one aspect, methods for the creation of transgenic animals, for example rodents, such as mice, are provided that comprise the introduced partly bovine immunoglobulin locus.

In one aspect, the host cell utilized for replacement of the endogenous immunoglobulin genes is an embryonic stem (ES) cell, which can then be utilized to create a transgenic mammal. In one aspect, the host cell is a cell of an early stage embryo. In one aspect, the host cell is a pronuclear stage embryo or zygote. Thus, in accordance with one aspect, the methods described herein further comprise: isolating an embryonic stem cell or a cell of an early stage embryo such as a pronuclear stage embryo or zygote, which comprises the introduced partly bovine immunoglobulin locus and using said ES cell to generate a transgenic animal that contains the replaced partly bovine immunoglobulin locus.

### Methods of Use

In one aspect, a method of producing antibodies comprising bovine variable regions is provided. In one aspect, the method includes providing a transgenic rodent or rodent cell described herein and isolating antibodies comprising bovine variable regions expressed by the transgenic rodent. In one aspect, a method of producing monoclonal antibodies comprising bovine variable regions is provided. In one aspect, the method includes providing B-cells from a transgenic rodent or cell described herein, immortalizing the B-cells; and isolating antibodies comprising bovine variable domains expressed by the immortalized B-cells.

In one aspect, the antibodies expressed by the transgenic rodent or rodent cell comprise bovine HC variable domains. In one aspect, the antibodies expressed by the transgenic rodent or rodent cell comprise mouse HC constant domains. These can be of any isotype, IgM, IgD, IgG1, IgG2a/c, IgG2b, IgG3, IgE or IgA.

In one aspect, the antibodies expressed by the transgenic rodent or rodent cell comprise bovine HC variable domains and mouse HC constant domains. In one aspect, the antibodies expressed by the transgenic rodent or rodent cell comprise bovine LC variable domains and mouse LC constant domains. In one aspect, the antibodies expressed by the transgenic rodent or rodent cell comprise bovine HC variable domains and bovine LC variable domains and mouse HC constant domains and mouse LC constant domains.

In one aspect, the antibodies expressed by the transgenic rodent or rodent cell comprise bovine λ LC variable domains. In one aspect, the antibodies expressed by the transgenic rodent or rodent cell comprise mouse λ constant domains. In one aspect, the antibodies expressed by the transgenic rodent or rodent cell comprise bovine λ LC variable domains and mouse λ constant domains. In one aspect, the antibodies expressed by the transgenic rodent or rodent cell comprise bovine κ LC variable domains. In one aspect, the antibodies expressed by the transgenic rodent or rodent cell comprise mouse κ constant domains. In one aspect, the antibodies expressed by the transgenic rodent or rodent cell comprise bovine κ LC variable domains and mouse κ constant domains.

In one aspect, a method of producing antibodies or antigen binding fragments comprising bovine variable regions is provided. In one aspect, the method includes providing a transgenic rodent or cell described herein and isolating antibodies comprising bovine variable regions expressed by the transgenic rodent or rodent cell. In one aspect, the variable regions of the antibody expressed by the transgenic rodent or rodent cell are sequenced. Antibodies comprising bovine variable regions obtained from the antibodies expressed by the transgenic rodent or rodent cell can be recombinantly produced using known methods.

In one aspect, a method of producing an immunoglobulin specific to an antigen of interest is provided. In one aspect, the method includes immunizing a transgenic rodent as described herein with the antigen and isolating immunoglobulin specific to the antigen expressed by the transgenic rodent or rodent cell. In one aspect, the variable domains of the antibody expressed by the rodent or rodent cell are sequenced and antibodies comprising bovine variable regions that specifically bind the antigen of interest are recombinantly produced using known methods. In one aspect, the recombinantly produced antibody or antigen binding fragment comprises bovine HC and LC, κ or λ, constant domains.

### Incorporation by Reference

All references cited herein, including patents, patent applications, papers, textbooks and the like, and the references cited therein, to the extent that they are not already, are hereby incorporated herein by reference in their entirety for all purposes.

The invention is further described by one or more of the following items.
1. A transgenic rodent or rodent cell comprising a genome comprising an engineered partly bovine immunoglobulin light chain locus comprising bovine immunoglobulin λ light chain variable region gene segments, wherein the engineered immunoglobulin locus is capable of expressing immunoglobulin comprising bovine variable domains and wherein the transgenic rodent produces more, or is more likely to produce, immunoglobulin comprising λ light chain than immunoglobulin comprising κ light chain.
2. The transgenic rodent according to item 1, wherein more λ light chain producing cells than κ light chain producing cells are likely to be isolated from said rodent.
3. The transgenic rodent according to item 1, wherein the transgenic rodent produces at least about 25%, 30%, 35%, 40%, 45%, 50%, 55%, 60%, 65%, 70%, 75%, 80%, 85%, 90% or 95% and up to about 100% immunoglobulin comprising λ light chain.
4. The transgenic rodent cell according to item 1, wherein the transgenic rodent cell, or its progeny, has at least about a 25%, 30%, 35%, 40%, 45%, 50%, 55%, 60%, 65%, 70%, 75%, 80%, 85%, 90%, or 95% and up to about 100%, probability of producing immunoglobulin comprising λ light chain.
5. The transgenic rodent or rodent cell according to any of the preceding items, wherein the engineered immunoglobulin locus comprises bovine V_{λ} gene segment coding sequences and J_{λ} gene segment coding sequences and rodent non-coding regulatory or scaffold sequences of a rodent immunoglobulin light chain variable region gene locus.
6. The transgenic rodent or rodent cell according to any of the preceding items, wherein the engineered immunoglobulin locus comprises bovine V_{λ} and J_{λ} gene segment coding sequences embedded in rodent non-coding regulatory or scaffold sequences of a rodent immunoglobulin λ light chain variable region gene locus.
7. The transgenic rodent or rodent cell according to any of the preceding items, wherein the partly bovine immunoglobulin locus comprises one or more bovine V_{λ} and J_{λ} gene segment coding sequences and one or more rodent immunoglobulin λ constant region coding sequences.
8. The transgenic rodent or rodent cell according to any of items 1 to 4, wherein the engineered immunoglobulin locus comprises bovine V_{λ} and J_{λ} gene segment coding sequences embedded in rodent non-coding regulatory or scaffold sequences of a rodent immunoglobulin κ light chain variable region gene locus.
9. The transgenic rodent or rodent cell according to item 8, wherein the engineered immunoglobulin variable region locus comprises one or more bovine V_{λ} gene segment coding sequences and one or more J-C units wherein each J-C unit comprises a bovine J_{λ} gene segment coding sequence and a rodent λ constant region coding sequence.
10. The transgenic rodent or rodent cell according to item 9, wherein the rodent λ constant region coding sequence comprises a rodent C_{λ1}, C_{λ2}, C_{λ3} coding sequence, or a combination thereof.
11. The transgenic rodent or rodent cell according to item 9 to 10, comprising one or more bovine V_{λ} gene segment coding sequences located upstream of one or more J-C units, wherein each J-C unit comprises a bovine J_{λ} gene segment coding sequence and a rodent C_{λ} gene segment coding sequence.
12. The transgenic rodent or rodent cell according to item 9 or 10, comprising one or more bovine V_{λ} gene segment coding sequences located upstream of one or more J-C units, wherein each J-C unit comprises a bovine J_{λ} gene segment coding sequence and a rodent C_{λ} gene segment coding sequence and rodent C_{λ} non-coding sequences.
13. The transgenic rodent or rodent cell according to any of items 9 to 12, wherein the J-C units comprise bovine J_{λ} gene segment coding sequences and rodent λ constant region coding sequences embedded in non-coding regulatory or scaffold sequences of a rodent immunoglobulin κ light chain locus.
14. The transgenic rodent or rodent cell according to item 8, wherein the engineered immunoglobulin locus comprises a rodent immunoglobulin κ locus in which one or more rodent V_{κ} gene segment coding sequences and one or more rodent J_{κ} gene segment coding sequences have been deleted and replaced by one or more bovine V_{λ} gene segment coding sequences and one or more J_{λ} gene segment coding sequences, respectively, and in which rodent C_{κ} coding sequences in the locus have been replaced by rodent C_{λ1}, C_{λ2}, C_{λ3} coding sequence, or a combination thereof.
15. The transgenic rodent or rodent cell according to item 14, wherein the engineered immunoglobulin locus comprises one or more bovine V_{λ} gene segment coding sequences upstream of one or more bovine J_{λ} gene segment coding sequences which are upstream of one or more rodent C_{λ} coding sequences.
16. The transgenic rodent or rodent cell according any of the preceding items wherein an endogenous rodent immunoglobulin κ light chain locus is deleted, inactivated, or made nonfunctional one or more of:
   a. deleting or mutating all endogenous rodent V_{κ} gene segment coding sequences;
   b. deleting or mutating all endogenous rodent J_{κ} gene segment coding sequences;
   c. deleting or mutating all endogenous rodent C_{κ} coding sequence;
   d. deleting or mutating a 5' splice site and adjacent polypyrimidine tract of a rodent C_{κ} coding sequence;
   e. deleting, mutating, or disrupting an endogenous intronic κ enhancer (iE_{κ}) and 3' enhancer sequence.
17. The transgenic rodent or rodent cell according to any of the preceding items wherein expression of an endogenous rodent immunoglobulin λ light chain variable domain is suppressed or inactivated by one or more of:
   a. deleting or mutating all endogenous rodent V_{λ} gene segments
   b. deleting or mutating all endogenous rodent J_{λ} gene segments; and
   c. deleting or mutating all endogenous rodent C_{λ} coding sequences.
18. The transgenic rodent or rodent cell according to any of the preceding items, wherein the engineered immunoglobulin locus expresses immunoglobulin light chains comprising a bovine λ variable domain and rodent λ constant domain.
19. The transgenic rodent or rodent cell according to any of items 1 to 4, wherein the genome of the transgenic rodent or rodent cell comprises an engineered immunoglobulin locus comprising bovine V_{κ} and J_{κ} gene segment coding sequences.
20. The transgenic rodent or rodent cell according to claim 19, wherein the bovine V_{κ} and J_{κ} gene segment coding sequences are inserted into a rodent immunoglobulin κ light chain locus.
21. The transgenic rodent or rodent cell according to item 19 or 20, wherein the bovine V_{κ} and J_{κ} gene segment coding sequences are embedded in rodent non-coding regulatory or scaffold sequences of the rodent immunoglobulin κ light chain variable region gene locus.
22. The transgenic rodent or rodent cell according to any of items 19 to 21, wherein the bovine V_{κ} and J_{κ} coding sequences are inserted upstream of a rodent immunoglobulin κ light chain constant region coding sequence.
23. The transgenic rodent or rodent cell according to any of items 1 to 4, wherein the genome of the transgenic rodent or rodent cell comprises an engineered immunoglobulin locus comprising bovine V_{κ} and J_{κ} gene segment coding sequences inserted into a rodent immunoglobulin λ light chain locus.
24. The transgenic rodent or rodent cell according to item 23, wherein the bovine V_{κ} and J_{κ} gene segment coding sequences are embedded in rodent non-coding regulatory or scaffold sequences of the rodent immunoglobulin λ light chain variable region gene locus.
25. The transgenic rodent or rodent cell according to items 23 or 24, comprising a rodent immunoglobulin κ light chain constant region coding sequence inserted downstream of the bovine V_{κ} and J_{κ} gene segment coding sequences.
26. The transgenic rodent or rodent cell according to item 25, wherein the rodent immunoglobulin κ light chain constant region is inserted upstream of an endogenous rodent C_{λ2} coding sequence.
27. The transgenic rodent or rodent cell according to any of items 23 to 26, wherein expression of an endogenous rodent immunoglobulin λ light chain variable domain is suppressed or inactivated by one or more of:
   a. deleting or mutating all endogenous rodent V_{λ} gene segment coding sequences.
   b. deleting or mutating all endogenous rodent J_{λ} gene segment coding sequences; and
   c. deleting or mutating all endogenous C_{λ} coding sequences or splice sites.
28. The transgenic rodent or rodent cell according to any of the preceding items, wherein the engineered bovine immunoglobulin light chain locus comprises a rodent intronic κ enhancer (iE_{κ}) and 3'E_{κ} regulatory sequences.
29. The transgenic rodent or rodent cell according to any of the preceding items, wherein the transgenic rodent or rodent cell comprises an engineered partly bovine immunoglobulin heavy chain locus comprising bovine immunoglobulin heavy chain variable region gene coding sequences and rodent non-coding regulatory or scaffold sequences of the rodent immunoglobulin heavy chain locus.
30. The transgenic rodent or rodent cell according to item 29, wherein the engineered bovine immunoglobulin heavy chain locus comprises bovine V_{H}, D and J_{H} gene segments.
31. The transgenic rodent or rodent cell according to item 30, wherein each bovine V_{H}, D or J_{H} coding gene segment comprises V_{H}, D or J_{H} coding sequence embedded in rodent non-coding regulatory or scaffold sequences of the rodent immunoglobulin heavy chain locus.
32. The transgenic rodent or rodent cell according to item 31, wherein the heavy chain rodent non-coding regulatory or scaffold sequences are interspersed by functional ADAM6A genes, ADAM6B genes, or a combination thereof.
33. The transgenic rodent or rodent cell according to any of the preceding items, wherein the rodent regulatory or scaffold sequences comprise enhancer, promoters, splice sites, introns, recombination signal sequences, or combinations thereof.
34. The transgenic rodent or rodent cell according to any of the preceding items, wherein an endogenous rodent immunoglobulin locus has been deleted and replaced with the engineered partly bovine immunoglobulin locus.
35. The transgenic rodent or rodent cell according to any of the preceding items, wherein the rodent is a mouse or a rat.
36. The transgenic rodent or rodent cell according to any of the preceding items, wherein the rodent cell is an embryonic stem (ES) cell or a cell of an early stage embryo.
37. The transgenic rodent or rodent cell according to any of the preceding items, wherein the rodent cell is a mouse or rat embryonic stem (ES) cell, or mouse or rat cell of an early stage embryo.
38. A cell of B lymphocyte lineage obtained from the transgenic rodent of any of the preceding items, wherein the engineered immunoglobulin locus expresses a chimeric immunoglobulin heavy chain or light chain comprising a bovine variable region and a rodent immunoglobulin constant region.
39. A hybridoma cell or immortalized cell line derived from a cell of B lymphocyte lineage according to item 38.
40. Antibodies or antigen binding portions thereof produced by the cell of items 38 or 39.
41. A nucleic acid sequence of a V_{H}, D, or J _{H}, or a V_{L} or J_{L} gene segment coding sequence derived from an immunoglobulin produced by the cell of items 38 or 39.

### EXAMPLES

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed. It will be appreciated by persons skilled in the art that numerous variations or modifications may be made to the invention as shown in the specific embodiments without departing from the spirit or scope of the invention as broadly described. The present embodiments are, therefore, to be considered in all respects as illustrative and not restrictive.

Efforts have been made to ensure accuracy with respect to terms and numbers used (e.g., vectors, amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

The examples illustrate targeting by both a 5' vector and a 3' vector that flank a site of recombination and introduction of synthetic DNA. It will be apparent to one skilled in the art upon reading the specification that the 5' vector targeting can take place first followed by the 3', or the 3' vector targeting can take place first followed by the 5' vector. In some circumstances, targeting can be carried out simultaneously with dual detection mechanisms.

### Example 1: Introduction of an Engineered Partly Bovine Immunoglobulin Variable Region Gene Locus into the Immunoglobulin H Chain Variable Region Gene Locus of a Non-Bovine Mammalian Host Cell Genome

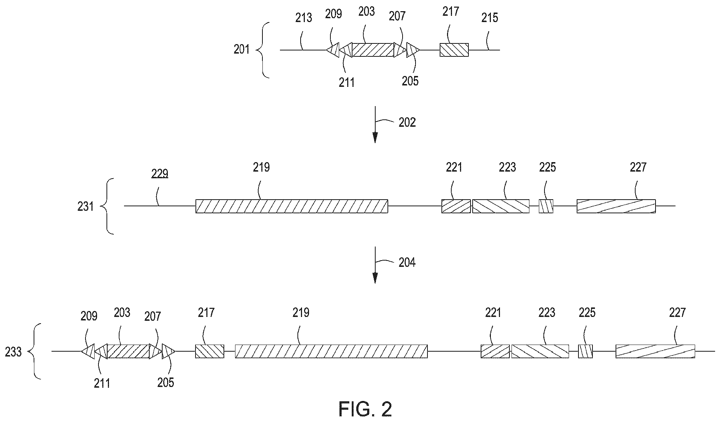

An exemplary method illustrating the introduction of an engineered partly bovine immunoglobulin locus into the genomic locus of a non-mammalian ES cell is illustrated in more detail in FIGS. 2-6. In FIG. 2, a homology targeting vector (201) is provided comprising a puromycin phosphotransferase-thymidine kinase fusion protein (puro-TK) (203) flanked by two different recombinase recognition sites (e.g., FRT (207) and loxP (205) for Flp and Cre, respectively) and two different mutant sites (e.g., modified mutant FRT (209) and mutant loxP (211)) that lack the ability to recombine with their respective wild-type counterparts/sites (i.e., wild-type FRT (207) and wild-type loxP (205)). The targeting vector comprises a diphtheria toxin receptor (DTR) cDNA (217) for use in negative selection of cells containing the introduced construct in future steps. The targeting vector also optionally comprises a visual marker such as a green fluorescent protein (GFP) (not shown). The regions 213 and 215 are homologous to the 5' and 3' portions, respectively, of a contiguous region (229) in the endogenous non-bovine locus that is 5' of the genomic region comprising the endogenous, non-bovine V_{H} gene segments (219). The homology targeting vector (201) is introduced (202) into the ES cell, which has an immunoglobulin locus (231) comprising endogenous V_{H} gene segments (219), the pre-D region (221), the D gene segments (223), J_{H} gene segments (225), and the immunoglobulin constant gene region genes (227). The site-specific recombination sequences and the DTR cDNA from the homology targeting vector (201) are integrated (204) into the non-bovine genome at a site 5' of the endogenous mouse V_{H} gene locus, resulting in the genomic structure illustrated at 233. The ES cells that do not have the exogenous vector (201) integrated into their genome can be selected against (killed) by including puromycin in the culture medium; only the ES cells that have stably integrated the exogenous vector (201) into their genome and constitutively express the puro-TK gene are resistant to puromycin.

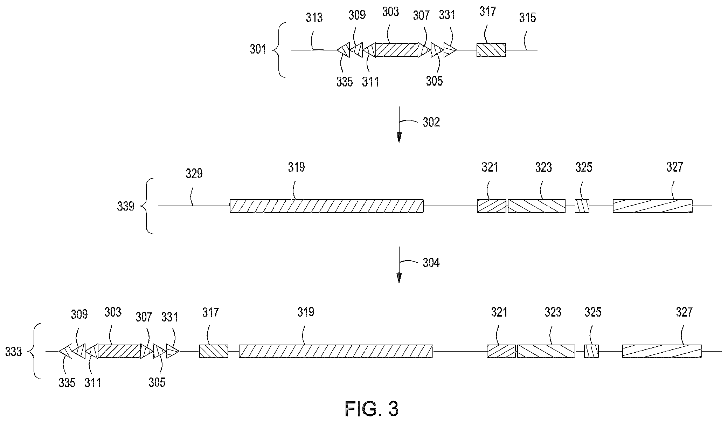

FIG. 3 illustrates effectively the same approach as FIG. 2, except that an additional set of sequence-specific recombination sites is added, e.g., a Rox site (331) and a modified Rox site (335) for use with the Dre recombinase. In FIG. 3, a homology targeting vector (301) is provided comprising a puro-TK fusion protein (303) flanked by wild type recombinase recognition sites for FRT (307), loxP (305), and Rox (331) and mutant sites for Flp (309), Cre (311), and Dre (335) recombinases that lack the ability to recombine with the wild-type sites 307, 305 and 331, respectively. The targeting vector also comprises a diphtheria toxin receptor (DTR) cDNA (317). The regions 313 and 315 are homologous to the 5' and 3' portions, respectively, of a contiguous region (329) in the endogenous non-bovine locus (339) that is 5' of the genomic region comprising the endogenous mouse V_{H} gene segments (319). The homology targeting vector is introduced (302) into the mouse immunoglobulin locus (339), which comprises the endogenous V_{H} gene segments (319), the pre-D region (321), the D gene segments (323), J_{H} (325) gene segments, and the constant region genes (327) of the IGH locus. The site-specific recombination sequences and the DTR cDNA (317) in the homology targeting vector (301) are integrated (304) into the mouse genome at a site 5' of the endogenous mouse V_{H} gene locus, resulting in the genomic structure illustrated at 333.

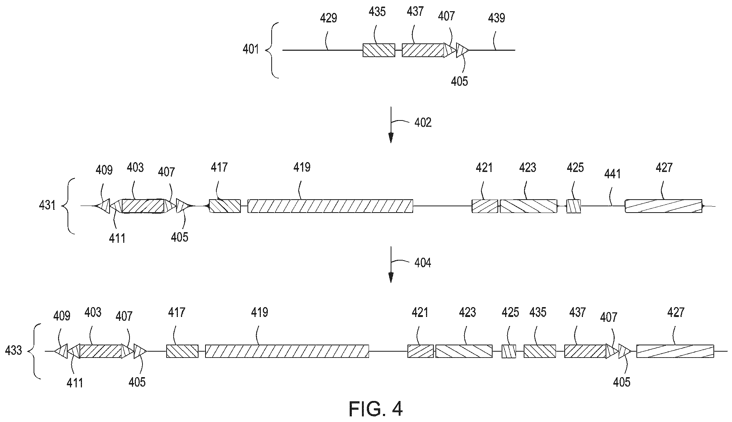

As illustrated in FIG. 4, a second homology targeting vector (401) is provided comprising an optional hypoxanthine-guanine phosphoribosyltransferase (HPRT) gene (435) that can be used for positive selection in HPRT-deficient ES cells; a neomycin resistance gene (437); recombinase recognition sites FRT (407) and loxP (405), for Flp and Cre, respectively, which have the ability to recombine with FRT (407) and loxP (405) sites previously integrated into the mouse genome from the first homology targeting vector. The previous homology targeting vector also includes mutant FRT site (409), mutant loxP site (411), a puro-TK fusion protein (403), and a DTR cDNA at a site 5' of the endogenous mouse V_{H} gene locus (419). The regions 429 and 439 are homologous to the 5' and 3' portions, respectively, of a contiguous region (441) in the endogenous non-bovine locus that is downstream of the endogenous J_{H} gene segments (425) and upstream of the constant region genes (427). The homology targeting vector is introduced (402) into the modified mouse immunoglobulin locus (431), which comprises the endogenous V_{H} gene segments (419), the pre-D region (421), the D gene segments (423) the J_{H} gene segments (425), and the constant region genes (427). The site-specific recombination sequences (407, 405), the HPRT gene (435) and a neomycin resistance gene (437) of the homology targeting vector are integrated (404) into the mouse genome upstream of the endogenous mouse constant region genes (427), resulting in the genomic structure illustrated at 433.

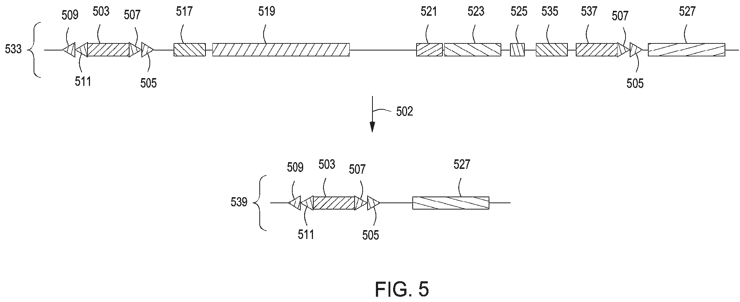

Once the recombination sites are integrated into the mammalian host cell genome, the endogenous region of the immunoglobulin domain is then subjected to recombination by introducing one of the recombinases corresponding to the sequence-specific recombination sites integrated into the genome, e.g., either Flp or Cre. Illustrated in FIG. 5 is a modified IGH locus of the mammalian host cell genome comprising two integrated DNA fragments. One fragment comprising mutant FRT site (509), mutant LoxP site (511), puro-TK gene (503), wild-type FRT site (507), and wild-type LoxP site (505), and DTR cDNA (517) is integrated upstream of the V_{H} gene locus (519). The other DNA fragment comprising HPRT gene (535), neomycin resistance gene (537), wild-type FRT site (507), and wild-type LoxP site (505) is integrated downstream of the pre-D (521), D (523) and J_{H} (525) gene loci, but upstream of the constant region genes (527). In the presence of Flp or Cre (502), all the intervening sequences between the wild-type FRT or wild-type LoxP sites including the DTR gene (517), the endogenous IGH variable region gene loci (519, 521, 525), and the HPRT (535) and neomycin resistance (537) genes are deleted, resulting in a genomic structure illustrated at 539. The procedure depends on the second targeting having occurred on the same chromosome rather than on its homolog (i.e., in *cis* rather than in *trans*)*.* If the targeting occurs in *cis* as intended, the cells are not sensitive to negative selection after Cre- or Flp-mediated recombination by diphtheria toxin introduced into the media, because the DTR gene which causes sensitivity to diphtheria toxin in rodents should be absent (deleted) from the host cell genome. Likewise, ES cells that harbor random integration of the first or second targeting vector(s) are rendered sensitive to diphtheria toxin by presence of the undeleted DTR gene.

ES cells that are insensitive to diphtheria toxin are then screened for the deletion of the endogenous variable region gene loci. The primary screening method for the deleted endogenous immunoglobulin locus can be carried out by Southern blotting, or by polymerase chain reaction (PCR) followed by confirmation with a secondary screening technique such as Southern blotting.

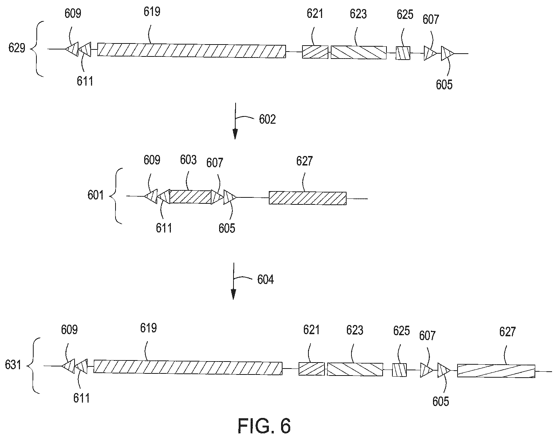

FIG. 6 illustrates introduction of the engineered partly bovine sequence into a non-bovine genome previously modified to delete part of the endogenous IGH locus (V_{H}, D and J_{H}) that encodes the heavy chain variable region domains as well as all the intervening sequences between the V_{H} and J_{H} gene locus. A site-specific targeting vector (629) comprising partly bovine V_{H} gene locus (619), endogenous non-bovine pre-D gene region (621), partly bovine D gene locus (623), partly bovine J_{H} gene locus (625), as well as flanking mutant FRT (609), mutant LoxP (611), wild-type FRT (607), and wild-type LoxP (605) sites is introduced (602) into the host cell. Specifically, the partly bovine V_{H} locus (619) comprises 20 bovine V_{H} coding sequences in conjunction with the intervening sequences based on the endogenous non-bovine genome sequences; the pre-D region (621) comprises a 21.6 kb mouse sequence with significant homology to the corresponding region of the endogenous bovine IGH locus; the D gene locus (623) comprises codons of 10 D gene segments embedded in the intervening sequences surrounding the endogenous non-bovine D gene segments; and the J_{H} gene locus (625) comprises codons of 4 bovine J_{H} gene segments embedded in the intervening sequences based on the endogenous non-bovine genome. The IGH locus (601) of the host cell genome has been previously modified to delete all the V_{H}, D, and J_{H} gene segments including the intervening sequences as described in FIG. 5. As a consequence of this modification, the endogenous non-bovine host cell IGH locus (601) is left with a puro-TK fusion gene (603), which is flanked by a mutant FRT site (609) and a mutant LoxP site (611) upstream as well as a wild-type FRT (607) and a wild-type LoxP (605) downstream. Upon introduction of the appropriate recombinase (604), the partly bovine immunoglobulin locus is integrated into the genome upstream of the endogenous non-bovine constant region genes (627), resulting in the genomic structure illustrated at 631.

The sequences of the bovine V_{H}, D and J_{H} gene segment coding regions are in Table 1.

Primary screening procedure for the introduction of the partly bovine immunoglobulin locus can be carried out by Southern blotting, or by PCR followed by confirmation with a secondary screening method such as Southern blotting. The screening methods are designed to detect the presence of the inserted V_{H}, D and J_{H} gene loci, as well as all the intervening sequences.

### Example 2: Introduction of an Engineered, Partly Bovine Immunoglobulin Variable Region Gene Locus Comprising Additional Non-Coding Regulatory or Scaffold Sequences into the Immunoglobulin H Chain Variable Region Gene Locus of a Non-Bovine Mammalian Host Cell Genome

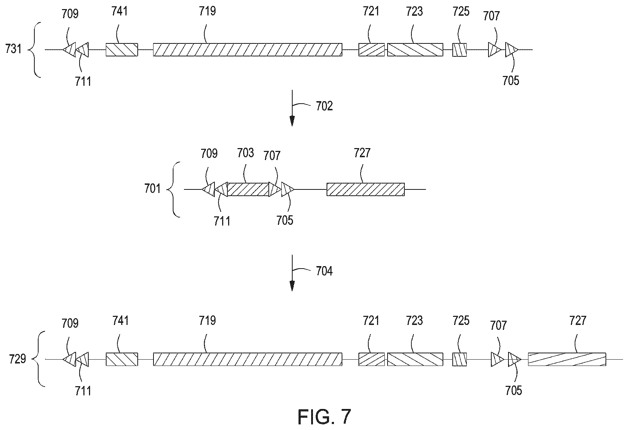

In certain aspects, the partly bovine immunoglobulin locus comprises the elements as described in Example 1, but with additional non-coding regulatory or scaffold sequences e.g., sequences strategically added to introduce additional regulatory sequences, to ensure the desired spacing within the introduced immunoglobulin locus, to ensure that certain coding sequences are in adequate juxtaposition with other sequences adjacent to the replaced immunoglobulin locus, and the like. FIG. 7 illustrates the introduction of a second exemplary engineered partly bovine sequence into the modified non-bovine genome as produced in FIGS. 2-5 and described in Example 1 above.

FIG. 7 illustrates introduction of the engineered partly bovine sequence into the mouse genome previously modified to delete part of the endogenous non-bovine IGH locus (V_{H}, D and J_{H}) that encodes the heavy chain variable region domains as well as all the intervening sequences between the endogenous V_{H} and J_{H} gene loci. A site-specific targeting vector (731) comprising an engineered partly bovine immunoglobulin locus to be inserted into the non-bovine host genome is introduced (702) into the genomic region (701). The site-specific targeting vector (731) comprising a partly bovine V_{H} gene locus (719), mouse pre-D region (721), partly bovine D gene locus (723), partly bovine J_{H} gene locus (725), PAIR elements (741), as well as flanking mutant FRT (709), mutant LoxP (711) wild-type FRT (707) and wild-type LoxP (705) sites is introduced (702) into the host cell. Specifically, the engineered partly bovine V_{H} gene locus (719) comprises 20 bovine V_{H} gene segment coding regions in conjunction with intervening sequences based on the endogenous non-bovine genome sequences; the pre-D region (721) comprises a 21.6 kb non-bovine sequence present upstream of the endogenous non-bovine genome; the D region (723) comprises codons of 10 bovine D gene segments embedded in the intervening sequences surrounding the endogenous non-bovine D gene segments; and the J_{H} gene locus (725) comprises codons of 4 bovine J_{H} gene segments embedded in the intervening sequences based on the endogenous non-bovine genome sequences. The IGH locus (701) of the host cell genome has been previously modified to delete all the V_{H}, D and J_{H} gene segments including the intervening sequences as described in relation to FIG. 5. As a consequence of this modification, the endogenous non-bovine IGH locus (701) is left with a puro-TK fusion gene (703), which is flanked by a mutant FRT site (709) and a mutant LoxP site (711) upstream as well as a wild-type FRT (707) and a wild-type LoxP (705) downstream. Upon introduction of the appropriate recombinase (704), the engineered partly bovine immunoglobulin locus is integrated into the genome upstream of the endogenous mouse constant region genes (727), resulting in the genomic structure illustrated at 729.

The primary screening procedure for the introduction of the engineered partly bovine immunoglobulin region can be carried out by Southern blotting, or by PCR with confirmation by a secondary screening method such as Southern blotting. The screening methods are designed to detect the presence of the inserted PAIR elements, the V_{H}, D and J_{H} gene loci, as well as all the intervening sequences.

### Example 3: Introduction of an Engineered Partly Bovine Immunoglobulin Locus into the Immunoglobulin Heavy Chain Gene Locus of a Mouse Genome

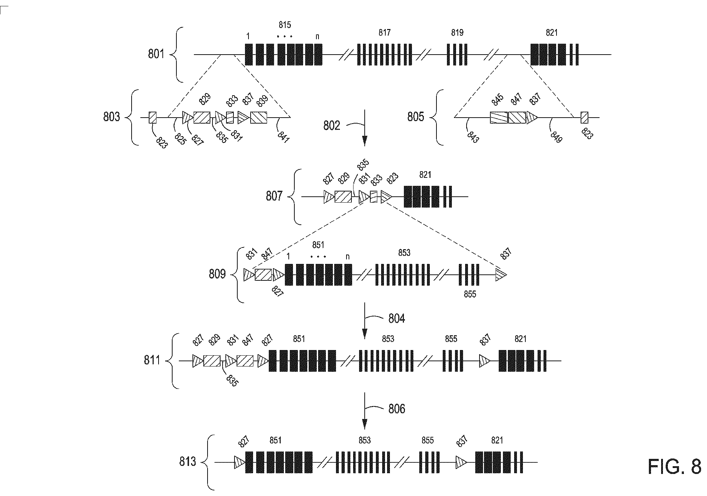

A method for replacing a portion of a mouse genome with an engineered partly bovine immunoglobulin locus is illustrated in FIG. 8. This method uses introduction of a first site-specific recombinase recognition sequence into the mouse genome followed by the introduction of a second site-specific recombinase recognition sequence into the mouse genome. The two sites flank the entire clusters of endogenous mouse V_{H}, D and J_{H} region gene segments. The flanked region is deleted using the relevant site-specific recombinase, as described herein.

The targeting vectors (803, 805) employed for introducing the site-specific recombinase sequences on either side of the V_{H} (815), D (817) and J_{H} (819) gene segment clusters and upstream of the constant region genes (821) in the wild-type mouse immunoglobulin locus (801) include an additional site-specific recombination sequence that has been modified so that it is still recognized efficiently by the recombinase, but does not recombine with unmodified sites. This mutant modified site (e.g., lox5171) is positioned in the targeting vector such that after deletion of the endogenous V_{H}, D and J_{H} gene segments (802) it can be used for a second site-specific recombination event in which a non-native piece of DNA is moved into the modified IGH locus by RMCE. In this example, the non-native DNA is a synthetic nucleic acid comprising both bovine and non-bovine sequences (809).

Two gene targeting vectors are constructed to accomplish the process just outlined. One of the vectors (803) comprises mouse genomic DNA taken from the 5' end of the IGH locus, upstream of the most distal V_{H} gene segment. The other vector (805) comprises mouse genomic DNA taken from within the locus downstream of the J_{H} gene segments.

The key features of the 5' vector (803) in order from 5' to 3' are as follows: a gene encoding the diphtheria toxin A (DTA) subunit under transcriptional control of a modified herpes simplex virus type I thymidine kinase gene promoter coupled to two mutant transcriptional enhancers from the polyoma virus (823); 4.5 Kb of mouse genomic DNA mapping upstream of the most distal V_{H} gene segment in the IGH locus (825); a FRT recognition sequence for the Flp recombinase (827); a piece of genomic DNA containing the mouse Polr2a gene promoter (829); a translation initiation sequence (methionine codon embedded in a "Kozak" consensus sequence, 835)); a mutated loxP recognition sequence (lox5171) for the Cre recombinase (831); a transcription termination/polyadenylation sequence (pA. 833); a loxP recognition sequence for the Cre recombinase (837); a gene encoding a fusion protein with a protein conferring resistance to puromycin fused to a truncated form of the thymidine kinase (pu-TK) under transcriptional control of the promoter from the mouse phosphoglycerate kinase 1 gene (839); and 3 Kb of mouse genomic DNA (841) mapping close to the 4.5 Kb mouse genomic DNA sequence present near the 5' end of the vector and arranged in the native relative orientation.

The key features of the 3' vector (805) in order from 5' to 3' are as follows; 3.7 Kb of mouse genomic DNA mapping within the intron between the J_{H} and C_{H} gene loci (843); an HPRT gene under transcriptional control of the mouse Polr2a gene promoter (845); a neomycin resistance gene under the control of the mouse phosphoglycerate kinase 1 gene promoter (847); a loxP recognition sequence for the Cre recombinase (837); 2.1 Kb of mouse genomic DNA (849) that maps immediately downstream of the 3.7 Kb mouse genomic DNA fragment present near the 5' end of the vector and arranged in the native relative orientation; and a gene encoding the DTA subunit under transcriptional control of a modified herpes simplex virus type I thymidine kinase gene promoter coupled to two mutant transcriptional enhancers from the polyoma virus (823).

Mouse embryonic stem (ES) cells (derived from C57B1/6NTac mice) are transfected by electroporation with the 3' vector (805) according to widely used procedures. Prior to electroporation, the vector DNA is linearized with a rare-cutting restriction enzyme that cuts only in the prokaryotic plasmid sequence or the polylinker associated with it. The transfected cells are plated and after ~24 hours they are placed under positive selection for cells that have integrated the 3' vector into their DNA by using the neomycin analogue drug G418. There is also negative selection for cells that have integrated the vector into their DNA but not by homologous recombination. Non-homologous recombination results in retention of the DTA gene (823), which kills the cells when the gene is expressed, whereas the DTA gene is deleted by homologous recombination since it lies outside of the region of vector homology with the mouse IGH locus. Colonies of drug-resistant ES cells are physically extracted from their plates after they became visible to the naked eye about a week later. These picked colonies are disaggregated, re-plated in micro-well plates, and cultured for several days. Thereafter, each of the clones of cells is divided such that some of the cells can be frozen as an archive, and the rest used for isolation of DNA for analytical purposes.

DNA from the ES cell clones is screened by PCR using a widely practiced gene-targeting assay design. For this assay, one of the PCR oligonucleotide primer sequences maps outside the region of identity shared between the 3' vector (805) and the genomic DNA, while the other maps within the novel DNA between the two arms of genomic identity in the vector, i.e., in the HPRT (845) or neomycin resistance (847) genes. According to the standard design, these assays detect pieces of DNA that would only be present in clones of ES cells derived from transfected cells that undergo fully legitimate homologous recombination between the 3' targeting vector and the endogenous mouse IGH locus. Two separate transfections are performed with the 3' vector (805). PCR-positive clones from the two transfections are selected for expansion followed by further analysis using Southern blot assays.

The Southern blot assays are performed according to widely used procedures using three probes and genomic DNA digested with multiple restriction enzymes chosen so that the combination of probes and digests allow the structure of the targeted locus in the clones to be identified as properly modified by homologous recombination. One of the probes maps to DNA sequence flanking the 5' side of the region of identity shared between the 3' targeting vector and the genomic DNA; a second probe maps outside the region of identity but on the 3' side; and the third probe maps within the novel DNA between the two arms of genomic identity in the vector, i.e., in the HPRT (845) or neomycin resistance (847) genes. The Southern blot identifies the presence of the expected restriction enzyme-generated fragment of DNA corresponding to the correctly mutated, i.e., by homologous recombination with the 3' IGH targeting vector, part of the IGH locus as detected by one of the external probes and by the neomycin or HPRT probe. The external probe detects the mutant fragment and also a wild-type fragment from the non-mutant copy of the immunoglobulin IGH locus on the homologous chromosome.

Karyotypes of PCR- and Southern blot-positive clones of ES cells are analyzed using an *in situ* fluorescence hybridization procedure designed to distinguish the most commonly arising chromosomal aberrations that arise in mouse ES cells. Clones with such aberrations are excluded from further use. ES cell clones that are judged to have the expected correct genomic structure based on the Southern blot data-and that also do not have detectable chromosomal aberrations based on the karyotype analysis-are selected for further use.

Acceptable clones are then modified with the 5' vector (803) using procedures and screening assays that are similar in design to those used with the 3' vector (805) except that puromycin selection is used instead of G418/neomycin for selection. The PCR assays, probes and digests are also tailored to match the genomic region being modified by the 5' vector (805).

Clones of ES cells that have been mutated in the expected fashion by both the 3' and the 5' vectors, i.e., doubly targeted cells carrying both engineered mutations, are isolated following vector targeting and analysis. The clones must have undergone gene targeting on the same chromosome, as opposed to homologous chromosomes (i.e., the engineered mutations created by the targeting vectors must be in *cis* on the same DNA strand rather than in *trans* on separate homologous DNA strands). Clones with the *cis* arrangement are distinguished from those with the *trans* arrangement by analytical procedures such as fluorescence *in situ* hybridization of metaphase spreads using probes that hybridize to the novel DNA present in the two gene targeting vectors (803 and 805) between their arms of genomic identity. The two types of clones can also be distinguished from one another by transfecting them with a vector expressing the Cre recombinase, which deletes the pu-TK (839), HPRT (845) and neomycin resistance (847) genes if the targeting vectors have been integrated in *cis,* and then comparing the number of colonies that survive ganciclovir selection against the thymidine kinase gene introduced by the 5' vector (803) and by analyzing the drug resistance phenotype of the surviving clones by a "sibling selection" screening procedure in which some of the cells from the clone are tested for resistance to puromycin or G418/neomycin. Cells with the *cis* arrangement of mutations are expected to yield approximately 10³ more ganciclovir-resistant clones than cells with the *trans* arrangement. The majority of the resulting cis-derived ganciclovir-resistant clones are also sensitive to both puromycin and G418/neomycin, in contrast to the trans-derived ganciclovir-resistant clones, which should retain resistance to both drugs. Doubly targeted clones of cells with the cis-arrangement of engineered mutations in the heavy chain locus are selected for further use.

The doubly targeted clones of cells are transiently transfected with a vector expressing the Cre recombinase and the transfected cells subsequently are placed under ganciclovir selection, as in the analytical experiment summarized above. Ganciclovir-resistant clones of cells are isolated and analyzed by PCR and Southern blot for the presence of the expected deletion between the two engineered mutations created by the 5' (803) and the 3' (805) targeting vectors. In these clones, the Cre recombinase causes a recombination (802) to occur between the loxP sites (837) introduced into the heavy chain locus by the two vectors to create the genomic DNA configuration shown at 807. Because the loxP sites are arranged in the same relative orientations in the two vectors, recombination results in excision of a circle of DNA comprising the entire genomic interval between the two loxP sites. The circle does not contain an origin of replication and thus is not replicated during mitosis and therefore is lost from the cells as they undergo proliferation. The resulting clones carry a deletion of the DNA that was originally between the two loxP sites. Clones that have the expected deletion are selected for further use.

ES cell clones carrying the deletion of sequence in one of the two homologous copies of their immunoglobulin heavy chain locus are retransfected (804) with a Cre recombinase expression vector together with a piece of DNA (809) comprising a partly bovine immunoglobulin heavy chain locus containing bovine V_{H}, D and J_{H} region gene coding region sequences flanked by mouse regulatory and flanking sequences. The key features of this piece of synthetic DNA (809) are the following: a lox5171 site (831); a neomycin resistance gene open reading frame (847) lacking the initiator methionine codon, but in-frame and contiguous with an uninterrupted open reading frame in the lox5171 site a FRT site (827); an array of 20 bovine V_{H} heavy chain variable region genes (851), each with bovine coding sequences embedded in mouse noncoding sequences; optionally a 10 kb pre-D region from the mouse heavy chain locus (not shown); a 58 Kb piece of DNA containing the 10 bovine D gene segments (853) and 4 bovine J_{H} gene segments (855) where the bovine V_{H}, D and J_{H} coding sequences are embedded in mouse noncoding sequences; a loxP site (837) in opposite relative orientation to the lox5171 site (831).

The transfected clones are placed under G418 selection, which enriches for clones of cells that have undergone RMCE in which the engineered partly bovine donor immunoglobulin locus (809) is integrated in its entirety into the deleted endogenous immunoglobulin heavy chain locus between the lox5171 (831) and loxP (837) sites to create the DNA region illustrated at 811. Only cells that have properly undergone RMCE have the capability to express the neomycin resistance gene (847) because the promoter (829) as well as the initiator methionine codon (835) required for its expression are not present in the vector (809) but are already pre-existing in the host cell IGH locus (807). The remaining elements from the 5' vector (803) are removed via Flp-mediated recombination (806) *in vitro* or *in vivo,* resulting in the final bovine-based locus as shown at 813.

G418-resistant ES cell clones are analyzed by PCR and Southern blot to determine if they have undergone the expected RMCE process without unwanted rearrangements or deletions. Clones that have the expected genomic structure are selected for further use.

ES cell clones carrying the partly bovine immunoglobulin heavy chain DNA (813) in the mouse heavy chain locus are microinjected into mouse blastocysts from strain DBA/2 to create partially ES cell-derived chimeric mice according to standard procedures. Male chimeric mice with the highest levels of ES cell-derived contribution to their coats are selected for mating to female mice. The female mice of choice here are of C57B1/6NTac strain, and also carry a transgene encoding the Flp recombinase that is expressed in their germline. Offspring from these matings are analyzed for the presence of the partly bovine immunoglobulin heavy chain locus, and for loss of the FRT-flanked neomycin resistance gene that was created in the RMCE step. Mice that carry the partly bovine locus are used to establish a colony of mice.

### Example 4: Introduction of an Engineered Partly Bovine Immunoglobulin Locus into the Immunoglobulin κ Chain Gene Locus of a Mouse Genome

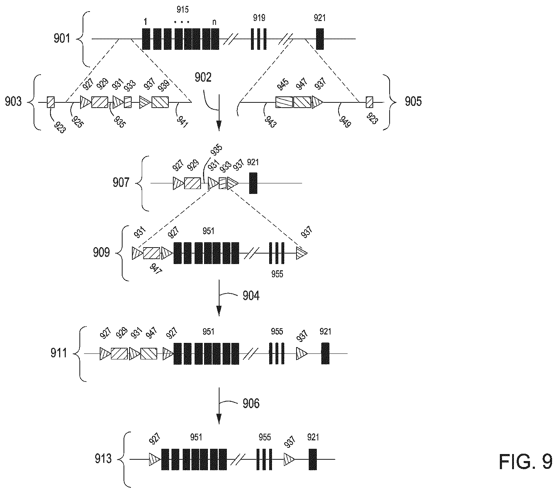

Another method for replacing a portion of a mouse genome with partly bovine immunoglobulin locus is illustrated in FIG. 9. This method includes introducing a first site-specific recombinase recognition sequence into the mouse genome, which may be introduced either 5' or 3' of the cluster of endogenous V_{κ} (915) and J_{κ} (919) region gene segments of the mouse genome, followed by the introduction of a second site-specific recombinase recognition sequence into the mouse genome, which in combination with the first sequence-specific recombination site flanks the entire locus comprising clusters of V_{κ} and J_{κ} gene segments upstream of the constant region gene (921). The flanked region is deleted and then replaced with a partly bovine immunoglobulin locus using the relevant site-specific recombinase, as described herein.

The targeting vectors employed for introducing the site-specific recombination sequences on either side of the V_{κ} (915) and J_{κ} (919) gene segments also include an additional site-specific recombination sequence that has been modified so that it is still recognized efficiently by the recombinase, but does not recombine with unmodified sites. This site is positioned in the targeting vector such that after deletion of the V_{κ} and J_{κ} gene segment clusters it can be used for a second site specific recombination event in which a non-native piece of DNA is moved into the modified V_{κ} locus via RMCE. In this example, the non-native DNA is a synthetic nucleic acid comprising bovine V_{κ} and J_{κ} gene segment coding sequences embedded in mouse regulatory and flanking sequences.

Two gene targeting vectors are constructed to accomplish the process just outlined. One of the vectors (903) comprises mouse genomic DNA taken from the 5' end of the locus, upstream of the most distal V_{κ} gene segment. The other vector (905) comprises mouse genomic DNA taken from within the locus downstream (3') of the J_{κ} gene segments (919) and upstream of the constant region genes (921).

The key features of the 5' vector (903) are as follows: a gene encoding the diphtheria toxin A (DTA) subunit under transcriptional control of a modified herpes simplex virus type I thymidine kinase gene promoter coupled to two mutant transcriptional enhancers from the polyoma virus (923); 6 Kb of mouse genomic DNA (925) mapping upstream of the most distal variable region gene in the κ chain locus; a FRT recognition sequence for the Flp recombinase (927); a piece of genomic DNA containing the mouse Polr2a gene promoter (929); a translation initiation sequence (935, methionine codon embedded in a "Kozak" consensus sequence); a mutated loxP recognition sequence (lox5171) for the Cre recombinase (931); a transcription termination/polyadenylation sequence (933); a loxP recognition sequence for the Cre recombinase (937); a gene encoding a fusion protein with a protein conferring resistance to puromycin fused to a truncated form of the thymidine kinase (pu-TK) under transcriptional control of the promoter from the mouse phosphoglycerate kinase 1 gene (939); 2.5 Kb of mouse genomic DNA (941) mapping close to the 6 Kb sequence at the 5' end in the vector and arranged in the native relative orientation.

The key features of the 3' vector (905) are as follows: 6 Kb of mouse genomic DNA (943) mapping within the intron between the J_{κ} (919) and C_{κ} (921) gene loci; a gene encoding the human hypoxanthine-guanine phosphoribosyl transferase (HPRT) under transcriptional control of the mouse Polr2a gene promoter (945); a neomycin resistance gene under the control of the mouse phosphoglycerate kinase 1 gene promoter (947); a loxP recognition sequence for the Cre recombinase (937); 3.6 Kb of mouse genomic DNA (949) that maps immediately downstream in the genome of the 6 Kb DNA fragment included at the 5' end in the vector, with the two fragments oriented in the same transcriptional orientation as in the mouse genome; a gene encoding the diphtheria toxin A (DTA) subunit under transcriptional control of a modified herpes simplex virus type I thymidine kinase gene promoter coupled to two mutant transcriptional enhancers from the polyoma virus (923).

Mouse embryonic stem (ES) cells derived from C57B1/6NTac mice are transfected by electroporation with the 3' vector (905) according to widely used procedures. Prior to electroporation, the vector DNA is linearized with a rare-cutting restriction enzyme that cuts only in the prokaryotic plasmid sequence or the polylinker associated with it. The transfected cells are plated and after ~24 hours they are placed under positive selection for cells that have integrated the 3' vector into their DNA by using the neomycin analogue drug G418. There is also negative selection for cells that have integrated the vector into their DNA but not by homologous recombination. Non-homologous recombination results in retention of the DTA gene, which kills the cells when the gene is expressed, whereas the DTA gene is deleted by homologous recombination since it lies outside of the region of vector homology with the mouse IGK locus. Colonies of drug-resistant ES cells are physically extracted from their plates after they became visible to the naked eye about a week later. These picked colonies are disaggregated, re-plated in micro-well plates, and cultured for several days. Thereafter, each of the clones of cells is divided such that some of the cells could be frozen as an archive, and the rest used for isolation of DNA for analytical purposes.

DNA from the ES cell clones is screened by PCR using a widely used gene-targeting assay design. For this assay, one of the PCR oligonucleotide primer sequences maps outside the region of identity shared between the 3' vector (905) and the genomic DNA (901), while the other maps within the novel DNA between the two arms of genomic identity in the vector, i.e., in the HPRT (945) or neomycin resistance (947) genes. According to the standard design, these assays detect pieces of DNA that are only present in clones of ES cells derived from transfected cells that had undergone fully legitimate homologous recombination between the 3' vector (905) and the endogenous mouse IGK locus. Two separate transfections are performed with the 3' vector (905). PCR-positive clones from the two transfections are selected for expansion followed by further analysis using Southern blot assays.

The Southern blot assays are performed according to widely used procedures; they involve three probes and genomic DNA digested with multiple restriction enzymes chosen so that the combination of probes and digests allowed for conclusions to be drawn about the structure of the targeted locus in the clones and whether it is properly modified by homologous recombination. One of the probes maps to DNA sequence flanking the 5' side of the region of identity shared between the 3' κ targeting vector (905) and the genomic DNA; a second probe also maps outside the region of identity but on the 3' side; the third probe maps within the novel DNA between the two arms of genomic identity in the vector, i.e., in the HPRT (945) or neomycin resistance (947) genes. The Southern blot identifies the presence of the expected restriction enzyme-generated fragment of DNA corresponding to the correctly mutated, i.e., by homologous recombination with the 3' κ targeting vector (905) part of the κ locus, as detected by one of the external probes and by the neomycin resistance or HPRT gene probe. The external probe detects the mutant fragment and also a wild-type fragment from the non-mutant copy of the immunoglobulin κ locus on the homologous chromosome.

Karyotypes of PCR- and Southern blot-positive clones of ES cells are analyzed using an *in situ* fluorescence hybridization procedure designed to distinguish the most commonly arising chromosomal aberrations that arise in mouse ES cells. Clones with such aberrations are excluded from further use. Karyotypically normal clones that are judged to have the expected correct genomic structure based on the Southern blot data are selected for further use.

Acceptable clones are then modified with the 5' vector (903) using procedures and screening assays that are similar in design to those used with the 3' vector (905), except that puromycin selection is used instead of G418/neomycin selection, and the protocols are tailored to match the genomic region modified by the 5' vector (903). The goal of the 5' vector (903) transfection experiments is to isolate clones of ES cells that have been mutated in the expected fashion by both the 3' vector (905) and the 5' vector (903), i.e., doubly targeted cells carrying both engineered mutations. In these clones, the Cre recombinase causes a recombination (902) to occur between the loxP sites introduced into the κ locus by the two vectors, resulting in the genomic DNA configuration shown at 907.

Further, the clones must have undergone gene targeting on the same chromosome, as opposed to homologous chromosomes; i.e., the engineered mutations created by the targeting vectors must be in *cis* on the same DNA strand rather than in *trans* on separate homologous DNA strands. Clones with the *cis* arrangement are distinguished from those with the *trans* arrangement by analytical procedures such as fluorescence *in situ* hybridization of metaphase spreads using probes that hybridize to the novel DNA present in the two gene targeting vectors (903 and 905) between their arms of genomic identity. The two types of clones can also be distinguished from one another by transfecting them with a vector expressing the Cre recombinase, which deletes the pu-Tk (939), HPRT (945) and neomycin resistance (947) genes if the targeting vectors have been integrated in *cis,* and comparing the number of colonies that survive ganciclovir selection against the thymidine kinase gene introduced by the 5' vector (903) and by analyzing the drug resistance phenotype of the surviving clones by a "sibling selection" screening procedure in which some of the cells from the clone are tested for resistance to puromycin or G418/neomycin. Cells with the *cis* arrangement of mutations are expected to yield approximately 10³ more ganciclovir-resistant clones than cells with the *trans* arrangement. The majority of the resulting *cis*-derived ganciclovir-resistant clones should also be sensitive to both puromycin and G418/neomycin, in contrast to the *trans-*derived ganciclovir-resistant clones, which should retain resistance to both drugs. Clones of cells with the cis-arrangement of engineered mutations in the κ chain locus are selected for further use.

The doubly targeted clones of cells are transiently transfected with a vector expressing the Cre recombinase (902) and the transfected cells are subsequently placed under ganciclovir selection, as in the analytical experiment summarized above. Ganciclovir-resistant clones of cells are isolated and analyzed by PCR and Southern blot for the presence of the expected deletion (907) between the two engineered mutations created by the 5' vector (903) and the 3' vector (905). In these clones, the Cre recombinase has caused a recombination to occur between the loxP sites (937) introduced into the κ chain locus by the two vectors. Because the loxP sites are arranged in the same relative orientations in the two vectors, recombination results in excision of a circle of DNA comprising the entire genomic interval between the two loxP sites. The circle does not contain an origin of replication and thus is not replicated during mitosis and is therefore lost from the clones of cells as they undergo clonal expansion. The resulting clones carry a deletion of the DNA that was originally between the two loxP sites. Clones that have the expected deletion are selected for further use.

The ES cell clones carrying the deletion of sequence in one of the two homologous copies of their immunoglobulin κ chain locus are retransfected (904) with a Cre recombinase expression vector together with a piece of DNA (909) comprising a partly bovine immunoglobulin κ chain locus containing V_{κ} (951) and J_{κ} (955) gene segment coding sequences. The key features of this piece of DNA (referred to as "K-K") are the following: a lox5171 site (931); a neomycin resistance gene open reading frame (947, lacking the initiator methionine codon, but in-frame and contiguous with an uninterrupted open reading frame in the lox5171 site (931)); a FRT site (927); an array of 8 bovine V_{κ} gene segments (951), each with bovine coding sequences embedded in mouse noncoding sequences; optionally a 13.5 Kb piece of genomic DNA from immediately upstream of the cluster of J_{κ} region gene segments in the mouse κ chain locus (not shown); a 2 Kb piece of DNA containing the 3 bovine J_{κ} region gene segments (955) embedded in mouse noncoding DNA; a loxP site (937) in opposite relative orientation to the lox5171 site (931).

The sequences of the bovine V_{κ} and J_{κ} gene coding regions are in Table 2.

In a second independent experiment, an alternative piece of partly bovine DNA (909) is used in place of the K-K DNA. The key features of this DNA (referred to as "L-K") are the following: a lox5171 site (931); a neomycin resistance gene open reading frame (947) lacking the initiator methionine codon, but in-frame and contiguous with an uninterrupted open reading frame in the lox5171 site (931); a FRT site (927); an array of 25 functional bovine V_{λ} variable region gene segments (951), each with bovine coding sequences embedded in mouse noncoding regulatory or scaffold sequences; optionally, a 13.5 Kb piece of genomic DNA from immediately upstream of the cluster of the J_{κ} region gene segments in the mouse κ chain locus (not shown); a 2 Kb piece of DNA containing bovine J_{λ} region gene segments embedded in mouse noncoding DNA (955); a loxP site (937) in opposite relative orientation to the lox5171 site (931).

The transfected clones from the K-K and L-K transfection experiments are placed under G418 selection, which enriches for clones of cells that have undergone RMCE, in which the partly bovine donor DNA (909) is integrated in its entirety into the deleted immunoglobulin κ chain locus between the lox5171 (931) and loxP (937) sites that were placed there by 5' (903) and 3' (905) vectors, respectively. Only cells that have properly undergone RMCE have the capability to express the neomycin resistance gene (947) because the promoter (929) as well as the initiator methionine codon (935) required for its expression are not present in the vector (909) and are already pre-existing in the host cell IGH locus (907). The DNA region created using the K-K sequence is illustrated at 911. The remaining elements from the 5' vector (903) are removed via Flp-mediated recombination (906) *in vitro* or *in vivo,* resulting in the final bovine-based light chain locus as shown at 913.

G418-resistant ES cell clones are analyzed by PCR and Southern blotting to determine if they have undergone the expected RMCE process without unwanted rearrangements or deletions. Both K-K and L-K clones that have the expected genomic structure are selected for further use.

The K-K ES cell clones and the L-K ES cell clones carrying the partly bovine immunoglobulin DNA in the mouse κ chain locus (913) are microinjected into mouse blastocysts from strain DBA/2 to create partly ES cell-derived chimeric mice according to standard procedures. Male chimeric mice with the highest levels of ES cell-derived contribution to their coats are selected for mating to female mice. The female mice of choice for use in the mating are of the C57B1/6NTac strain, and also carry a transgene encoding the Flp recombinase that is expressed in their germline. Offspring from these matings are analyzed for the presence of the partly bovine immunoglobulin κ or λ light chain locus, and for loss of the FRT-flanked neomycin resistance gene that was created in the RMCE step. Mice that carry the partly bovine locus are used to establish colonies of K-K and L-K mice.

Mice carrying the partly bovine heavy chain locus, produced as described in Example 3, can be bred with mice carrying a bovine-based κchain locus. Their offspring are in turn bred together in a scheme that ultimately produces mice that are homozygous for both bovine-based loci, i.e., bovine-based for heavy chain and κ. Such mice produce partly bovine heavy chains with bovine variable domains and mouse constant domains. They also produce partly bovine κ proteins with bovine κ variable domains and the mouse κ constant domain from their κ loci. Monoclonal antibodies recovered from these mice have bovine heavy chain variable domains paired with bovine κ variable domains.

A variation on the breeding scheme involves generating mice that are homozygous for the bovine-based heavy chain locus, but heterozygous at the κ locus such that on one chromosome they have the K-K bovine-based locus and on the other chromosome they have the L-K bovine-based locus. Such mice produce partly bovine heavy chains with bovine variable domains and mouse constant domains. They also produce partly bovine κ proteins with bovine κ variable domains and the mouse κ constant domain from one of their κ loci. From the other κ locus, they produce partly bovine λ proteins with bovine λ variable domains the mouse κ constant domain. Monoclonal antibodies recovered from these mice have bovine variable domains paired in some cases with bovine κ variable domains and in other cases with bovine λ variable domains.

### Example 5: Introduction of an Engineered Partly Bovine Immunoglobulin Locus into the Immunoglobulin λ Chain Gene Locus of a Mouse Genome

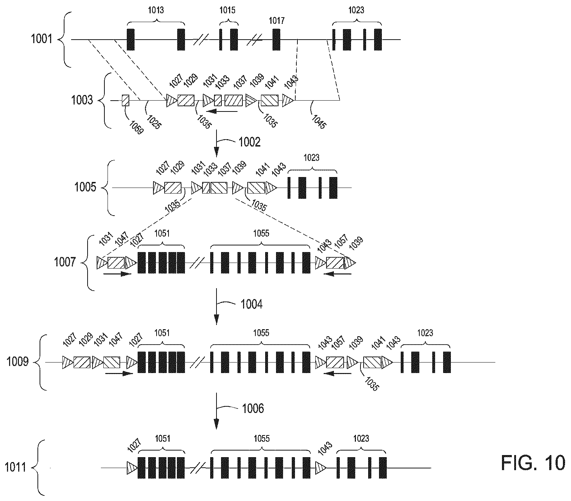

Another method for replacing a portion of a mouse genome with an engineered partly bovine immunoglobulin locus is illustrated in FIG. 10. This method comprises deleting approximately 194 Kb of DNA from the wild-type mouse immunoglobulin λ locus (1001)-comprising V_{λx}/V_{λ2} gene segments (1013), J_{λ2}/C_{λ2} gene cluster (1015), and V_{λ1} gene segment (1017)-by a homologous recombination process involving a targeting vector (1003) that shares identity with the locus both upstream of the V_{λx}/V_{λ2} gene segments (1013) and downstream of the V_{λ1} gene segment (1017) in the immediate vicinity of the J_{λ3}, C_{λ3}, J_{λ1} and C_{λ1} λ gene cluster (1023). The vector replaces the 194 Kb of DNA with elements designed to permit a subsequent site-specific recombination in which a non-native piece of DNA is moved into the modified V_{λ} locus via RMCE (1004). In this example, the non-native DNA is a synthetic nucleic acid comprising both bovine and mouse sequences.

The key features of the gene targeting vector (1003) for accomplishing the 194 Kb deletion are as follows: a negative selection gene such as a gene encoding the A subunit of the diphtheria toxin (DTA, 1059) or a herpes simplex virus thymidine kinase gene (not shown); 4 Kb of genomic DNA from 5' of the mouse V_{λx}/V_{λ2} variable region gene segments in the λ locus (1025); a FRT site (1027); a piece of genomic DNA containing the mouse Polr2a gene promoter (1029); a translation initiation sequence (methionine codon embedded in a "Kozak" consensus sequence) (1035); a mutated loxP recognition sequence (lox5171) for the Cre recombinase (1031); a transcription termination/polyadenylation sequence (1033); an open reading frame encoding a protein that confers resistance to puromycin (1037), whereas this open reading frame is on the antisense strand relative to the Polr2a promoter and the translation initiation sequence next to it and is followed by its own transcription termination/polyadenylation sequence (1033); a loxP recognition sequence for the Cre recombinase (1039); a translation initiation sequence (a methionine codon embedded in a "Kozak" consensus sequence) (1035) on the same, antisense strand as the puromycin resistance gene open reading frame; a chicken beta actin promoter and cytomegalovirus early enhancer element (1041) oriented such that it directs transcription of the puromycin resistance open reading frame, with translation initiating at the initiation codon downstream of the loxP site and continuing back through the loxP site into the puromycin open reading frame all on the antisense strand relative to the Polr2a promoter and the translation initiation sequence next to it; a mutated recognition site for the Flp recombinase known as an "F3" site (1043); a piece of genomic DNA upstream of the J_{λ3}, C_{λ3}, J_{λ1} and C_{λ1} gene segments (1045).

Mouse embryonic stem (ES) cells derived from C57B1/6NTac mice are transfected (1002) by electroporation with the targeting vector (1003) according to widely used procedures. Homologous recombination replaces the native DNA with the sequences from the targeting vector (1003) in the 196 Kb region resulting in the genomic DNA configuration depicted at 1005.

Prior to electroporation, the vector DNA is linearized with a rare-cutting restriction enzyme that cuts only in the prokaryotic plasmid sequence or the polylinker associated with it. The transfected cells are plated and after ~24 hours placed under positive drug selection using puromycin. There is also negative selection for cells that have integrated the vector into their DNA but not by homologous recombination. Non-homologous recombination results in retention of the DTA gene, which kills the cells when the gene is expressed, whereas the DTA gene is deleted by homologous recombination since it lies outside of the region of vector homology with the mouse IGL locus. Colonies of drug-resistant ES cells are physically extracted from their plates after they became visible to the naked eye approximately a week later. These picked colonies are disaggregated, re-plated in micro-well plates, and cultured for several days. Thereafter, each of the clones of cells are divided such that some of the cells are frozen as an archive, and the rest used for isolation of DNA for analytical purposes.

DNA from the ES cell clones is screened by PCR using a widely used gene-targeting assay design. For these assays, one of the PCR oligonucleotide primer sequences maps outside the regions of identity shared between the targeting vector and the genomic DNA, while the other maps within the novel DNA between the two arms of genomic identity in the vector, e.g., in the puro gene (1037). According to the standard design, these assays detect pieces of DNA that would only be present in clones of cells derived from transfected cells that had undergone fully legitimate homologous recombination between the targeting vector (1003) and the native DNA (1001).

Six PCR-positive clones from the transfection (1002) are selected for expansion followed by further analysis using Southern blot assays. The Southern blots involve three probes and genomic DNA from the clones that has been digested with multiple restriction enzymes chosen so that the combination of probes and digests allow identification of whether the ES cell DNA has been properly modified by homologous recombination.

Karyotypes of the six PCR- and Southern blot-positive clones of ES cells are analyzed using an *in situ* fluorescence hybridization procedure designed to distinguish the most common chromosomal aberrations that arise in mouse ES cells. Clones that show evidence of aberrations are excluded from further use. Karyotypically normal clones that are judged to have the expected correct genomic structure based on the Southern blot data are selected for further use.

The ES cell clones carrying the deletion in one of the two homologous copies of their immunoglobulin λ chain locus are retransfected (1004) with a Cre recombinase expression vector together with a piece of DNA (1007) comprising a partly bovine immunoglobulin λ chain locus containing V_{λ}, J_{λ} and C_{λ} region gene segments. The key features of this piece of DNA (1007) are as follows: a lox5171 site (1031); a neomycin resistance gene open reading frame lacking the initiator methionine codon, but in-frame and contiguous with an uninterrupted open reading frame in the lox5171 site (1047); a FRT site 1027); an array of 25 functional bovine λ region gene segments, each with bovine λ coding sequences embedded in mouse λ noncoding sequences (1051); an array of J-C units where each unit has a bovine J_{λ} gene segment and a mouse λ constant domain gene segment embedded within noncoding sequences from the mouse λ locus (1055) (the bovine J_{λ} gene segments are those encoding J_{λ1}, J_{λ2}, J_{λ3}, J_{λ4}, J_{λ5}, J_{λ6}, and J_{λ7}, while the mouse λ constant domain gene segments are C_{λ1} or C_{λ2} or C_{λ3}); a mutated recognition site for the Flp recombinase known as an "F3" site (1043); an open reading frame conferring hygromycin resistance (1057), which is located on the antisense strand relative to the immunoglobulin gene segment coding information in the construct; a loxP site (1039) in opposite relative orientation to the lox5171 site.

The sequences of the bovine V_{λ} and J_{λ} gene coding regions are in Table 3.

The transfected clones are placed under G418 or hygromycin selection, which enriches for clones of cells that have undergone a RMCE process, in which the partly bovine donor DNA is integrated in its entirety into the deleted immunoglobulin λ chain locus between the lox5171 and loxP sites that were placed there by the gene targeting vector. The remaining elements from the targeting vector (1003) are removed via FLP-mediated recombination (1006) *in vitro* or *in vivo* resulting in the final bovinized locus as shown at 1011.

G418/hygromycin-resistant ES cell clones are analyzed by PCR and Southern blotting to determine if they have undergone the expected recombinase-mediated cassette exchange process without unwanted rearrangements or deletions. Clones that have the expected genomic structure are selected for further use.

The ES cell clones carrying the partly bovine immunoglobulin DNA (1011) in the mouse λ chain locus are microinjected into mouse blastocysts from strain DBA/2 to create partially ES cell-derived chimeric mice according to standard procedures. Male chimeric mice with the highest levels of ES cell-derived contribution to their coats are selected for mating to female mice. The female mice of choice here are of the C57B1/6NTac strain, which carry a transgene encoding the Flp recombinase expressed in their germline. Offspring from these matings are analyzed for the presence of the partly bovine immunoglobulin λ chain locus, and for loss of the FRT-flanked neomycin resistance gene and the F3-flanked hygromycin resistance gene that were created in the RMCE step. Mice that carry the partly bovine locus are used to establish a colony of mice.

In some aspects, the mice comprising the bovine-based heavy chain and κ locus (as described in Examples 3 and 4) are bred to mice that carry the bovine-based λ locus. Mice generated from this type of breeding scheme are homozygous for the bovine-based heavy chain locus and can be homozygous for the K-K bovine-based locus or the L-K bovine-based locus. Alternatively, they can be heterozygous at the κ locus carrying the K-K locus on one chromosome and the L-K locus on the other chromosome. Each of these mouse strains is homozygous for the bovine-based λ locus. Monoclonal antibodies recovered from these mice has bovine heavy chain variable domains paired in some cases with bovine κ variable domains and in other cases with bovine λ variable domains. The λ variable domains are derived from either the bovine-based L-K locus or the bovine-based λ locus.

### Example 6: Introduction of an Engineered Partly Bovine Immunoglobulin Minilocus into a Mouse Genome

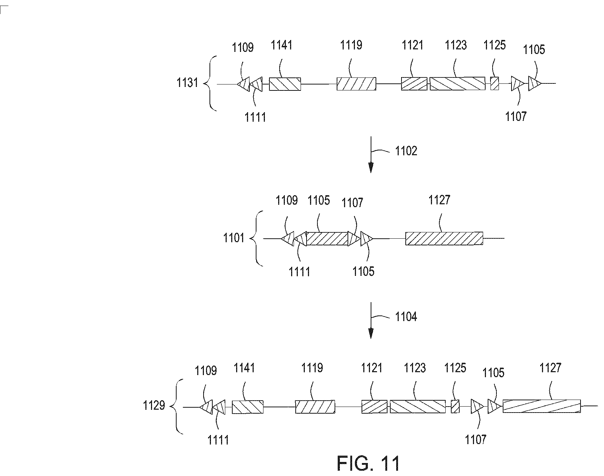

In certain other aspects, the partly bovine immunoglobulin locus comprises a bovine variable domain minilocus such as the one illustrated in FIG. 11. Here instead of a partly bovine immunoglobulin locus comprising all or substantially all of the bovine V_{H} gene segment coding sequences, the mouse immunoglobulin locus is replaced with a minilocus (1119) comprising fewer chimeric bovine V_{H} gene segments, e.g. 1-20 bovine V_{H} gene segments determined to be functional; that is, not pseudogenes.

A site-specific targeting vector (1131) comprising the partly bovine immunoglobulin locus to be integrated into the mammalian host genome is introduced (1102) into the genomic region (1101) with the deleted endogenous immunoglobulin locus comprising the puro-TK gene (1105) and the following flanking sequence-specific recombination sites: mutant FRT site (1109), mutant LoxP site (1111), wild-type FRT site (1107), and wild-type LoxP site (1105). The site-specific targeting vector comprises i) an array of optional PAIR elements (1141); ii) a V_{H} locus (1119) comprising, e.g., 1-20 functional bovine V_{H} coding regions and intervening sequences based on the mouse genome endogenous sequences; iii) a 21.6 kb pre-D region (1121) comprising mouse sequence; iv) a D locus (1123) and a J_{H} locus (1125) comprising 10 D and J_{H} bovine coding sequences and intervening sequences based on the mouse genome endogenous sequences. The partly bovine immunoglobulin locus is flanked by recombination sites-mutant FRT (1109), mutant LoxP (1111), wild-type FRT (1107), and wild-type LoxP (1105)-that allow recombination with the modified endogenous locus. Upon introduction of the appropriate recombinase, e.g., Cre) (1104), the partly bovine immunoglobulin locus is integrated into the genome upstream of the constant gene region (1127) as shown at 1129.

As described in Example 1, the primary screening for introduction of the partly bovine immunoglobulin variable region locus is carried out by primary PCR screens supported by secondary Southern blotting assays. The deletion of the puro-TK gene (1105) as part of the recombination event allows identification of the cells that did not undergo the recombination event using ganciclovir negative selection.

### Example 7: Introduction of an Engineered Partly Bovine Immunoglobulin Locus with Bovine λ Variable Region Coding Sequences with Mouse λ Constant Region Sequences embedded in κ Immunoglobulin Non-coding Sequences

Cattle antibodies mostly contain λ light chains, whereas mouse antibodies mostly contain κ light chains. To increase production of antibodies containing a λ LC, the endogenous mouse V_{κ} and J_{κ} are replaced with a partly bovine locus containing V_{λ} and J_{λ} gene segment coding sequences embedded in mouse V_{κ} region flanking and regulatory sequences, the L-K mouse of Example 4. In such a mouse, the endogenous regulatory sequences promoting high level κ locus rearrangement and expression are predicted to have an equivalent effect on the ectopic λ locus. However, *in vitro* studies demonstrated that bovine V_{λ} domains do not function well with mouse C_{κ} (see Example 9). Thus, the expected increase in λ LC-containing antibodies in the L-K mouse might not occur. As an alternate strategy, the endogenous mouse V_{κ} and J_{κ} are replaced with a partly bovine locus containing V_{λ} and J_{λ} gene segment coding sequences embedded in mouse V_{κ} region flanking and regulatory sequences and mouse C_{κ} is replaced with mouse C_{λ}.

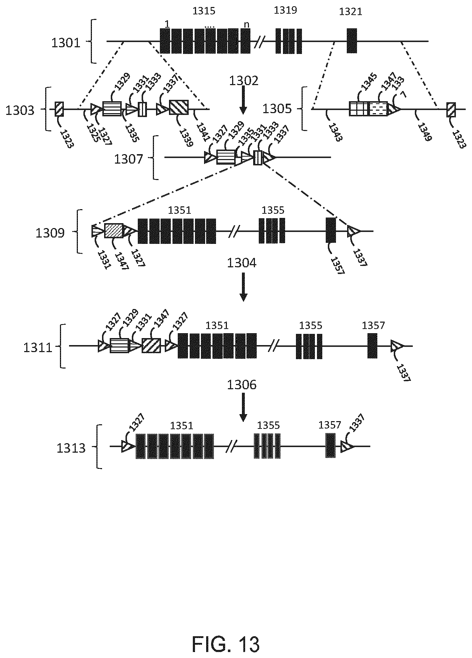

FIG. 13 is a schematic diagram illustrating the introduction of an engineered partly bovine light chain variable region locus in which one or more bovine V_{λ} gene segment coding sequences are inserted into a rodent immunoglobulin κ light chain locus upstream of one or more bovine J_{λ} gene segment coding sequences, which are upstream of one or more rodent C_{λ} region coding sequences.

The method for replacing a portion of a mouse genome with a partly bovine immunoglobulin locus is illustrated in FIG. 13. This method includes introducing a first site-specific recombinase recognition sequence into the mouse genome, which may be introduced either 5' or 3' of the cluster of endogenous V_{κ} (1315) and J_{κ} (1319) region gene segments and the C_{κ} (1321) exon of the mouse genome, followed by the introduction of a second site-specific recombinase recognition sequence into the mouse genome, which in combination with the first sequence-specific recombination site flanks the entire locus comprising clusters of V_{κ} and J_{κ} gene segments and the C_{κ} exon. The flanked region is deleted and then replaced with a partly bovine immunoglobulin locus using the relevant site-specific recombinase, as described herein.

The targeting vectors employed for introducing the site-specific recombination sequences on either side of the V_{κ} (1315) gene segments and the C_{κ} exon (1321) also include an additional site-specific recombination sequence that has been modified so that it is still recognized efficiently by the recombinase, but does not recombine with unmodified sites. This site is positioned in the targeting vector such that after deletion of the V_{κ} and J_{κ} gene segment clusters and the C_{κ} exon it can be used for a second site specific recombination event in which a non-native piece of DNA is moved into the modified V_{κ} locus via RMCE. In this example, the non-native DNA is a synthetic nucleic acid comprises bovine V_{λ} and J_{λ} gene segment coding sequences and mouse C_{λ} exon(s) embedded in mouse IGK regulatory and flanking sequences.

Two gene targeting vectors are constructed to accomplish the process just outlined. One of the vectors (1303) comprises mouse genomic DNA taken from the 5' end of the locus, upstream of the most distal V_{κ} gene segment. The other vector (1305) comprises mouse genomic DNA taken from within the locus in a region spanning upstream (5') and downstream (3') of the C_{κ} exon (1321).

The key features of the 5' vector (1303) are as follows: a gene encoding the diphtheria toxin A (DTA) subunit under transcriptional control of a modified herpes simplex virus type I thymidine kinase gene promoter coupled to two mutant transcriptional enhancers from the polyoma virus (1323); 6 Kb of mouse genomic DNA (1325) mapping upstream of the most distal variable region gene in the κ chain locus; a FRT recognition sequence for the Flp recombinase (1327); a piece of genomic DNA containing the mouse Polr2a gene promoter (1329); a translation initiation sequence (1335, methionine codon embedded in a "Kozak" consensus sequence); a mutated loxP recognition sequence (lox5171) for the Cre recombinase (1331); a transcription termination/polyadenylation sequence (1333); a loxP recognition sequence for the Cre recombinase (1337); a gene encoding a fusion protein with a protein conferring resistance to puromycin fused to a truncated form of the thymidine kinase (pu-TK) under transcriptional control of the promoter from the mouse phosphoglycerate kinase 1 gene (1339); 2.5 Kb of mouse genomic DNA (1341) mapping close to the 6 Kb sequence at the 5' end in the vector and arranged in the native relative orientation.

The key features of the 3' vector (1305) are as follows: 6 Kb of mouse genomic DNA (1343) mapping within the locus in a region spanning upstream (5') and downstream (3') of the C_{κ} exon (1321); a gene encoding the human hypoxanthine-guanine phosphoribosyl transferase (HPRT) under transcriptional control of the mouse Polr2a gene promoter (1345); a neomycin resistance gene under the control of the mouse phosphoglycerate kinase 1 gene promoter (1347); a loxP recognition sequence for the Cre recombinase (1337); 3.6 Kb of mouse genomic DNA (1349) that maps immediately downstream in the genome of the 6 Kb DNA fragment included at the 5' end in the vector, with the two fragments oriented in the same transcriptional orientation as in the mouse genome; a gene encoding the diphtheria toxin A (DTA) subunit under transcriptional control of a modified herpes simplex virus type I thymidine kinase gene promoter coupled to two mutant transcriptional enhancers from the polyoma virus (1323).

One strategy to delete the endogenous mouse IGK locus is to insert the 3' vector (1305) in the flanking region downstream of the mouse C_{κ} exon (1321). However, the 3'κ enhancer, which needs to be retained in the modified locus, is located 9.1 Kb downstream of the C_{κ} exon, which is too short to accommodate the upstream and downstream homology arms of the 3' vector, which total 9.6 Kb. Therefore, the upstream region of homology was extended.

Mouse embryonic stem (ES) cells derived from C57B1/6NTac mice are transfected by electroporation with the 3' vector (1305) according to widely used procedures. Prior to electroporation, the vector DNA is linearized with a rare-cutting restriction enzyme that cuts only in the prokaryotic plasmid sequence or the polylinker associated with it. The transfected cells are plated and after ~24 hours they are placed under positive selection for cells that have integrated the 3' vector into their DNA using the neomycin analogue drug G418. There is also negative selection for cells that have integrated the vector into their DNA but not by homologous recombination. Non-homologous recombination retains the DTA gene, which kills the cells when the gene is expressed, but the DTA gene is deleted by homologous recombination since it lies outside of the region of vector homology with the mouse IGK locus. Colonies of drug-resistant ES cells are physically extracted from their plates after they are visible to the naked eye about a week later. These colonies are disaggregated, re-plated in micro-well plates, and cultured for several days. Thereafter, each of the clones of cells is divided - some of the cells are frozen as an archive, and the rest are used to isolate DNA for analytical purposes.

DNA from the ES cell clones is screened by PCR using a widely used gene-targeting assay design. For this assay, one of the PCR oligonucleotide primer sequences maps outside the region of identity shared between the 3' vector (1305) and the genomic DNA (1301), while the other maps within the novel DNA between the two arms of genomic identity in the vector, i.e., in the HPRT (1345) or neomycin resistance (1347) genes. According to the standard design, these assays detect pieces of DNA that are only present in clones of ES cells derived from transfected cells that had undergone fully legitimate homologous recombination between the 3' vector (1305) and the endogenous mouse IGK locus. Two separate transfections are performed with the 3' vector (1305). PCR-positive clones from the two transfections are selected for expansion followed by further analysis using Southern blot assays.

Southern blot assays are performed according to widely used procedures using three probes and genomic DNA digested with multiple restriction enzymes chosen so that the combination of probes and digests allowed for conclusions to be drawn about the structure of the targeted locus in the clones and whether it is properly modified by homologous recombination. A first probe maps to DNA sequence flanking the 5' side of the region of identity shared between the 3' κ targeting vector (1305) and the genomic DNA; a second probe also maps outside the region of identity but on the 3' side; a third probe maps within the novel DNA between the two arms of genomic identity in the vector, i.e., in the HPRT (1345) or neomycin resistance (1347) genes. The Southern blot identifies the presence of the expected restriction enzyme-generated fragment of DNA corresponding to the correctly mutated, i.e., by homologous recombination with the 3' κ targeting vector (1305) part of the κ locus, as detected by one of the external probes and by the neomycin resistance or HPRT gene probe. The external probe detects the mutant fragment and also a wild-type fragment from the non-mutant copy of the immunoglobulin κ locus on the homologous chromosome.

Karyotypes of PCR- and Southern blot-positive clones of ES cells are analyzed using an *in situ* fluorescence hybridization procedure designed to distinguish the most commonly arising chromosomal aberrations that arise in mouse ES cells. Clones with such aberrations are excluded from further use. Karyotypically normal clones that are judged to have the expected correct genomic structure based on the Southern blot data are selected for further use.

Acceptable clones are then modified with the 5' vector (1303) using procedures and screening assays that are similar in design to those used with the 3' vector (1305), except that puromycin selection is used instead of G418/neomycin selection, and the protocols are tailored to match the genomic region modified by the 5' vector (1303). The goal of the 5' vector (1303) transfection experiments is to isolate clones of ES cells that have been mutated in the expected fashion by both the 3' vector (1305) and the 5' vector (1303), i.e., doubly targeted cells carrying both engineered mutations. In these clones, the Cre recombinase causes a recombination (1302) to occur between the loxP sites introduced into the κ locus by the two vectors, resulting in the genomic DNA configuration shown at 1307.

Further, the clones must have undergone gene targeting on the same chromosome, as opposed to homologous chromosomes; i.e., the engineered mutations created by the targeting vectors must be in *cis* on the same DNA strand rather than in *trans* on separate homologous DNA strands. Clones with the *cis* arrangement are distinguished from those with the *trans* arrangement by analytical procedures such as fluorescence *in situ* hybridization of metaphase spreads using probes that hybridize to the novel DNA present in the two gene targeting vectors (1303 and 1305) between their arms of genomic identity. The two types of clones can also be distinguished from one another by transfecting them with a vector expressing the Cre recombinase, which deletes the pu-Tk (1339), HPRT (1345) and neomycin resistance (1347) genes if the targeting vectors have been integrated in *cis,* and comparing the number of colonies that survive ganciclovir selection against the thymidine kinase gene introduced by the 5' vector (1303) and by analyzing the drug resistance phenotype of the surviving clones by a "sibling selection" screening procedure in which some of the cells from the clone are tested for resistance to puromycin or G418/neomycin. Cells with the *cis* arrangement of mutations are expected to yield approximately 10³ more ganciclovir-resistant clones than cells with the *trans* arrangement. The majority of the resulting cis-derived ganciclovir-resistant clones should also be sensitive to both puromycin and G418/neomycin, in contrast to the *trans-derived* ganciclovir-resistant clones, which should retain resistance to both drugs. Clones of cells with the cis-arrangement of engineered mutations in the κ chain locus are selected for further use.

The doubly targeted clones of cells are transiently transfected with a vector expressing the Cre recombinase (1302) and the transfected cells are subsequently placed under ganciclovir selection, as in the analytical experiment summarized above. Ganciclovir-resistant clones of cells are isolated and analyzed by PCR and Southern blot for the presence of the expected deletion (1307) between the two engineered mutations created by the 5' vector (1303) and the 3' vector (1305). In these clones, the Cre recombinase causes a recombination to occur between the loxP sites (1337) introduced into the κ chain locus by the two vectors. Because the loxP sites are arranged in the same relative orientations in the two vectors, recombination results in excision of a circle of DNA comprising the entire genomic interval between the two loxP sites. The circle does not contain an origin of replication and thus is not replicated during mitosis and is therefore lost from the clones of cells as they undergo clonal expansion. The resulting clones carry a deletion of the DNA that was originally between the two loxP sites and have the genomic structure show at 1307. Clones that have the expected deletion are selected for further use.

The ES cell clones carrying the sequence deletion in one of the two homologous copies of their immunoglobulin κ chain locus are retransfected (1304) with a Cre recombinase expression vector together with a piece of DNA (1309) comprising a partly bovine immunoglobulin λ chain locus containing V_{λ} (1351) and J_{λ} (1355) gene segment coding sequences and mouse C_{λ} exon(s) (1357). The key features of this piece of DNA are the following: a lox5171 site (1331); a neomycin resistance gene open reading frame (1347, lacking the initiator methionine codon, but in-frame and contiguous with an uninterrupted open reading frame in the lox5171 site (1331); a FRT site (1327); an array of 1-24 functional bovine V_{λ} variable region gene segments (1351), each with bovine coding sequences embedded in mouse noncoding regulatory or scaffold sequences; optionally, a 13.5 Kb piece of genomic DNA from immediately upstream of the cluster of the J_{κ} region gene segments in the mouse κ chain locus (not shown); a 2 Kb piece of DNA containing 1-5 functional bovine J_{λ} region gene segments embedded in mouse noncoding DNA (1355) and mouse C_{λ} exon(s) (1357); a loxP site (1337) in opposite relative orientation to the lox5171 site (1331). The piece of DNA also contains the deleted iEκ (not shown).

The sequences of the bovine V_{λ} and J_{λ} gene coding regions are in Table 3.

The transfected cells are placed under G418 selection, which enriches for clones of cells that have undergone RMCE, in which the partly bovine donor DNA (1309) is integrated in its entirety into the deleted immunoglobulin κ chain locus between the lox5171 (1331) and loxP (1337) sites that were placed there by 5' (1303) and 3' (1305) vectors, respectively. Only cells that have properly undergone RMCE have the capability to express the neomycin resistance gene (1347) because the promoter (1329) as well as the initiator methionine codon (1335) required for its expression are not present in the vector (1309) and are already pre-existing in the host cell IGK locus (1307). The DNA region created by RMCE is illustrated at 1311. The remaining elements from the 5' vector (1303) are removed via Flp-mediated recombination (1306) *in vitro* or *in vivo,* resulting in the final bovine-based light chain locus as shown at 1313.

G418-resistant ES cell clones are analyzed by PCR and Southern blotting to determine if they have undergone the expected RMCE process without unwanted rearrangements or deletions. Clones that have the expected genomic structure are selected for further use.

Clones carrying the partly bovine immunoglobulin DNA in the mouse κ chain locus (1313) are microinjected into mouse blastocysts from strain DBA/2 to create partly ES cell-derived chimeric mice according to standard procedures. Male chimeric mice with the highest levels of ES cell-derived contribution to their coats are selected for mating to female mice. The female mice of choice for use in the mating are of the C57B1/6NTac strain, and also carry a transgene encoding the Flp recombinase that is expressed in their germline. Offspring from these matings are analyzed for the presence of the partly bovine immunoglobulin λ light chain locus, and for loss of the FRT-flanked neomycin resistance gene that was created in the RMCE step. Mice that carry the partly bovine locus are used to establish colonies of mice.

Mice carrying the partly bovine heavy chain locus, produced as described in Example 3, can be bred with mice carrying a bovine λ-based κ chain locus. Their offspring are in turn bred together in a scheme that ultimately produces mice that are homozygous for both bovine-based loci, i.e., bovine-based for heavy chain and λ-based λ. Such mice produce partly bovine heavy chains with bovine variable domains and mouse constant domains. They also produce partly bovine λ proteins with bovine λ variable domains and the mouse λ constant domain from their κ loci. Monoclonal antibodies recovered from these mice have bovine heavy chain variable domains paired with bovine λ variable domains.

A variation on the breeding scheme involves generating mice that are homozygous for the bovine-based heavy chain locus, but heterozygous at the κ locus such that on one chromosome they have the K-K bovine-based locus described in Example 4 and on the other chromosome they have the partly bovine λ-based κ locus described in this example. Such mice produce partly bovine heavy chains with bovine variable domains and mouse constant domains. They also produce partly bovine κ proteins with bovine κ variable domains and the mouse κ constant domain from one of their κ loci. From the other κ locus, partly bovine λ proteins comprising bovine λ variable domains and the mouse λ constant domain is produced. Monoclonal antibodies recovered from these mice include bovine variable domains paired in some cases with bovine κ variable domains and in other cases with bovine λ variable domains.

### Example 8. Introduction of an Engineered Partly Bovine Immunoglobulin Locus with Bovine λ Variable Region Coding Sequences with Mouse λ Constant Region Sequences embedded in Mouse κ Immunoglobulin Non-coding Sequences

This example describes an alternate strategy to Example 7 in which the endogenous mouse V_{κ} and J_{κ} are replaced with a partly bovine locus containing bovine V_{λ} and J_{λ} gene segment coding sequences embedded in mouse V_{κ} region flanking and regulatory sequences and mouse C_{κ} is replaced with mouse C_{λ}. However, in this example the structure of the targeting vector containing the partly bovine locus is different. The bovine V gene locus coding sequences include an array of anywhere from 1 to 24 functional V_{λ} gene segment coding sequences, followed by an array of J_{λ}-C_{λ} tandem cassettes in which the J_{λ} is of bovine origin and the C_{λ} is of mouse origin, for example, C_{λ1}, C_{λ2} or C_{λ3}. The number of cassettes ranges from one to five, the number of unique functional bovine J_{λ} gene segments. The overall structure of the partly bovine λ locus in this example is similar to the endogenous mouse λ locus, whereas the structure of the locus in Example 7 is similar to the endogenous mouse κ locus, which is being replaced by the partly bovine λ locus in that example.

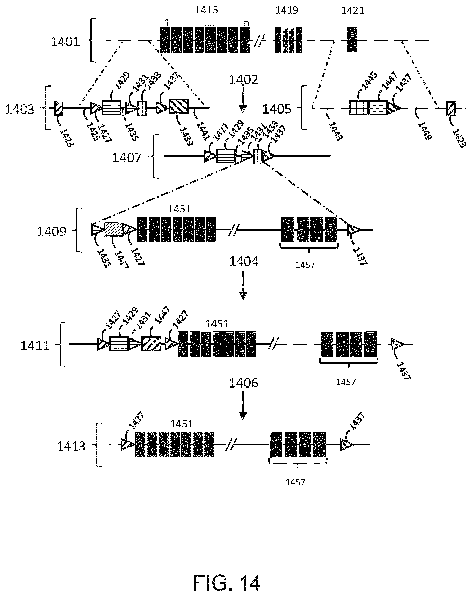

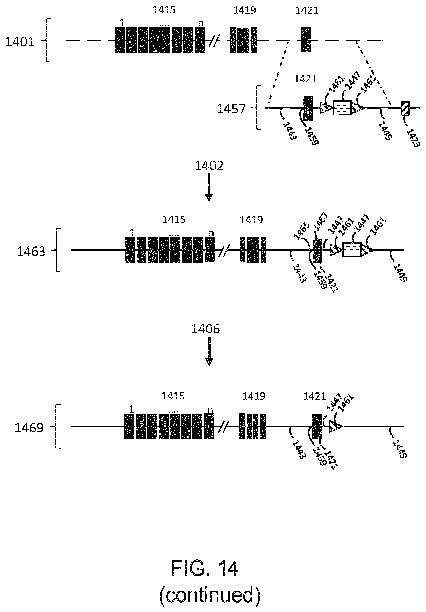

FIG. 14 is a schematic diagram illustrating the introduction of an engineered partly bovine light chain variable region locus in which one or more bovine V_{λ} gene segment coding sequences are inserted into a rodent immunoglobulin κ light chain locus upstream of an array of J_{λ}-C_{λ} tandem cassettes in which the J_{λ} is of bovine origin and the C_{λ} is of mouse origin, for example, C_{λ1}, C_{λ2} or C_{λ3}.

The method for replacing a portion of a mouse genome with a partly bovine immunoglobulin locus is illustrated in FIG. 14. This method provides introducing a first site-specific recombinase recognition sequence into the mouse genome, which may be introduced either 5' or 3' of the cluster of endogenous V_{κ} (1415) and J_{κ} (1419) region gene segments and the C_{κ} (1421) exon of the mouse genome, followed by the introduction of a second site-specific recombinase recognition sequence into the mouse genome, which in combination with the first sequence-specific recombination site flanks the entire locus comprising clusters of V_{κ} and J_{κ} gene segments and the C_{κ} exon. The flanked region is deleted and then replaced with a partly bovine immunoglobulin locus using the relevant site-specific recombinase, as described herein.

The targeting vectors employed for introducing the site-specific recombination sequences on either side of the V_{κ} (1415) gene segments and the C_{κ} exon (1421) also include an additional site-specific recombination sequence that has been modified so that it is still recognized efficiently by the recombinase, but does not recombine with unmodified sites. This site is positioned in the targeting vector such that after deletion of the V_{κ} and J_{κ} gene segment clusters and the C_{κ} exon it can be used for a second site specific recombination event in which a non-native piece of DNA is moved into the modified V_{κ} locus via RMCE. In this example, the non-native DNA is a synthetic nucleic acid comprising an array of bovine V_{λ} gene segment coding sequences and an array of J_{λ}-C_{λ} tandem cassettes in which the J_{λ} is of bovine origin and the C_{λ} is of mouse origin, for example, C_{λ1}, C_{λ2} or C_{λ3} embedded in mouse IGK regulatory and flanking sequences.

Two gene targeting vectors are constructed to accomplish the process just outlined. One of the vectors (1403) comprises mouse genomic DNA taken from the 5' end of the locus, upstream of the most distal V_{κ} gene segment. The other vector (1405) comprises mouse genomic DNA taken from within the locus in a region spanning upstream (5') and downstream (3') of the C_{κ} exon (1321).

The key features of the 5' vector (1403) and the 3' vector (1405) are described in Example 7.

Mouse embryonic stem (ES) cells derived from C57B1/6NTac mice are transfected by electroporation with the 3' vector (1405) according to widely used procedures as described in Example 7. DNA from the ES cell clones is screened by PCR using a widely used gene-targeting assay as described in Example 7. The Southern blot assays are performed according to widely used procedures as described in Example 7.

Karyotypes of PCR- and Southern blot-positive clones of ES cells are analyzed using an *in situ* fluorescence hybridization procedure designed to distinguish the most commonly arising chromosomal aberrations that arise in mouse ES cells. Clones with such aberrations are excluded from further use. Karyotypically normal clones that are judged to have the expected correct genomic structure based on the Southern blot data are selected for further use.

Acceptable clones are modified with the 5' vector (1403) using procedures and screening assays as described in Example 7. The resulting correctly targeted ES clones have the genomic DNA configuration of the endogenous κ locus in which the 5' vector (1403) is inserted upstream of endogenous V_{κ} gene segments and the 3' vector (1405) is inserted downstream of the endogenous C_{κ}. In these clones, the Cre recombinase causes recombination (1402) to occur between the loxP sites introduced into the κ locus by the two vectors, resulting in the genomic DNA configuration shown at 1407.

Acceptable clones undergo gene targeting on the same chromosome, as opposed to homologous chromosomes; such that the engineered mutations created by the targeting vectors are in *cis* on the same DNA strand rather than in *trans* on separate homologous DNA strands. Clones with the *cis* arrangement are distinguished from those with the *trans* arrangement by analytical procedures as described in Example 7.

The doubly targeted clones of cells are transiently transfected with a vector expressing the Cre recombinase (1402) and the transfected cells are subsequently placed under ganciclovir selection and analyses using procedures described in Example 7. In selected clones, the Cre recombinase has caused a recombination to occur between the loxP sites (1437) introduced into the κ chain locus by the two vectors. Because the loxP sites are arranged in the same relative orientations in the two vectors, recombination results in excision of a circle of DNA comprising the entire genomic interval between the two loxP sites. The circle does not contain an origin of replication and thus is not replicated during mitosis and is therefore lost from the clones of cells as they undergo clonal expansion. The resulting clones carry a deletion of the DNA that was originally between the two loxP sites and have the genomic structure show at 1407. Clones that have the expected deletion are selected for further use.

The ES cell clones carrying the deletion of sequence in one of the two homologous copies of their immunoglobulin κ chain locus are retransfected (1404) with a Cre recombinase expression vector together with a piece of DNA (1409) comprising a partly bovine immunoglobulin λ chain locus containing V_{λ} (1451) segment coding sequences and a tandem array of cassettes containing bovine J_{λ} gene segment coding sequences and mouse C_{λ} exon(s) embedded in mouse IGK flanking and regulatory DNA sequences (1457). The key features of this piece of DNA are the following: a lox5171 site (1431); a neomycin resistance gene open reading frame (1447, lacking the initiator methionine codon, but in-frame and contiguous with an uninterrupted open reading frame in the lox5171 site (1431); a FRT site (1427); an array of 1-24 functional bovine V_{λ} variable region gene segments (1451), each containing bovine coding sequences embedded in mouse noncoding regulatory or scaffold sequences; optionally, a 13.5 Kb piece of genomic DNA from immediately upstream of the cluster of the J_{κ} region gene segments in the mouse κ chain locus (not shown); DNA containing a tandem array of cassettes containing bovine J_{λ} gene segment coding sequences and mouse C_{λ} exon(s) embedded in mouse IGK flanking and regulatory DNA sequences (1457); a loxP site (1437) in opposite relative orientation to the lox5171 site (1431).

The sequences of the bovine V_{λ} and J_{λ} gene coding regions are in Table 3.

The transfected cells are placed under G418 selection, which enriches for clones of cells that have undergone RMCE, in which the partly bovine donor DNA (1409) is integrated in its entirety into the deleted immunoglobulin κ chain locus between the lox5171 (1431) and loxP (1437) sites placed there by the 5' (1403) and 3' (1405) vectors, respectively. Only cells that properly undergo RMCE have the capability to express the neomycin resistance gene (1447) because the promoter (1429) as well as the initiator methionine codon (1435) required for its expression are not present in the vector (1409) and are already pre-existing in the host cell IGK locus (1407). The DNA region created by RMCE is illustrated at 1411. The remaining elements from the 5' vector (1403) are removed via Flp-mediated recombination (1406) *in vitro* or *in vivo,* resulting in the final bovine-based light chain locus as shown at 1413.

G418-resistant ES cell clones are analyzed by PCR and Southern blotting to determine if they have undergone the expected RMCE process without unwanted rearrangements or deletions. Clones that have the expected genomic structure are selected for further use.

Clones carrying the partly bovine immunoglobulin DNA in the mouse κ chain locus (1413) are microinjected into mouse blastocysts from strain DBA/2 to create partly ES cell-derived chimeric mice according to standard procedures. Male chimeric mice with the highest levels of ES cell-derived contribution to their coats are selected for mating to female mice. The female mice of choice for use in the mating are of the C57B1/6NTac strain, and also carry a transgene encoding the Flp recombinase that is expressed in their germline. Offspring from these matings are analyzed for the presence of the partly bovine immunoglobulin λ light chain locus, and for loss of the FRT-flanked neomycin resistance gene that was created in the RMCE step. Mice that carry the partly bovine locus are used to establish colonies of mice.

Mice carrying the partly bovine heavy chain locus, produced as described in Example 3, can be bred with mice carrying a bovine λ-based κ chain locus. Their offspring are in turn bred together in a scheme that ultimately produces mice that are homozygous for both bovine-based loci, i.e., bovine-based for heavy chain and λ-based κ. Such mice produce partly bovine heavy chains with bovine variable domains and mouse constant domains. They also produce partly bovine λ proteins with bovine λ variable domains and the mouse λ constant domain from their κ loci. Monoclonal antibodies recovered from these mice have bovine heavy chain variable domains paired with bovine λ variable domains.

A variation on the breeding scheme involves generating mice that are homozygous for the bovine-based heavy chain locus, but heterozygous at the κ locus such that on one chromosome they have the K-K bovine-based locus described in Example 4 and on the other chromosome they have the partly bovine λ-based κ locus described in this example. Such mice produce partly bovine heavy chains with bovine variable domains and mouse constant domains. They also produce partly bovine κ proteins with bovine κ variable domains and the mouse κ constant domain from one of their κ loci. From the other κ locus, they produce partly bovine λ proteins with bovine λ variable domains and the mouse λ constant domain. Monoclonal antibodies recovered from these mice have bovine variable domains paired in some cases with bovine κ variable domains and in other cases with bovine λ variable domains.

The method described above for introducing an engineered partly bovine immunoglobulin locus with bovine λ variable region coding sequences and mouse λ constant region sequences embedded in mouse κ immunoglobulin non-coding sequences involve deletion of the mouse C_{κ} exon. An alternate method involves inactivating the C_{κ} exon by mutating its splice acceptor site. Introns must be removed from primary mRNA transcripts by a process known as RNA splicing in which the spliceosome, a large molecular machine located in the nucleus, recognizes sequences at the 5' (splice donor) and 3' (splice acceptor) ends of the intron, as well as other features of the intron including a polypyrimidine tract located just upstream of the splice acceptor. The splice donor sequence in the DNA is NGT, where "N" is any deoxynucleotide and the splice acceptor is AGN (Cech TR, Seitz JA and Atkins JF Eds. (2019) (RNA Worlds: New Tools for Deep Exploration, CSHL Press) ISBN 978-1-621822-24-0).

The mouse C_{κ} exon is inactivated by mutating its splice acceptor sequence and the polypyrimidine tract. The wild type sequence upstream of the C_{κ} exon is CTTCCTTCCTCAG (SEQ ID NO: 294) (the splice acceptor site is underlined). It is mutated to AAATTAATTAACC (SEQ ID NO: 295), resulting in a non-functional splice acceptor site and thus a non-functional C_{κ} exon. The mutant sequence also introduces a PacI restriction enzyme site (underlined). As an eight base pair recognition sequence, this restriction site is expected to be present only rarely in the mouse genome (~ every 65,000 bp), making it simple to detect whether the mutant sequence has been inserted into the IGK locus by Southern blot analysis of the ES cell DNA that has been digested with PacI and another, more frequently cutting restriction enzyme. The wild type sequence is replaced with the mutant sequence by homologous recombination, a technique widely known in the art, as to insert the 3' RMCE vector. The key features of the homologous recombination vector (MSA, 1457) to mutate the C_{κ} exon splice acceptor sequence and the polypyrimidine tract are as follows: 6 Kb of mouse genomic DNA (1443) mapping within the κ locus in a region spanning upstream (5') and downstream (3') of the C_{κ} exon (1421) and containing the mutant AAATTAATTAACC (SEQ ID NO: 295) (1459) sequence instead of the wild type CTTCCTTCCTCAG (SEQ ID NO: 294) sequence in its natural position just upstream of the C_{κ} exon; a neomycin resistance gene under the control of the mouse phosphoglycerate kinase 1 gene promoter (1447) and flanked by mutant FRT sites (1461); 3.6 Kb of mouse genomic DNA (1449) that maps immediately downstream in the genome of the 6 Kb DNA fragment included at the 5' end in the vector, with the two fragments oriented in the same transcriptional orientation as in the mouse genome; a gene encoding the diphtheria toxin A (DTA) subunit under transcriptional control of a modified herpes simplex virus type I thymidine kinase gene promoter coupled to two mutant transcriptional enhancers from the polyoma virus (1423). Mutant FRT sites (1461), e.g., FRT F3 or FRT F5 (Schlake and Bode (1994) Use of mutated FLP recognition target (FRT) sites for the exchange of expression cassettes at defined chromosomal loci. Biochemistry 33:12746-12751 PMID: 7947678 DOI: 10.1021/bi00209a003), are being used here because, once the spicing mutation is introduced and the Neo gene is deleted by transient transfection of a FLP recombinase expression vector (1406), the ES cells are subjected to further genetic manipulation. This process requires wild type FRT sites to delete another Neo selection gene (1447 at 1403). If the FRT site (1461) remaining in the IGK locus (1469) after introduction of the splicing mutation is wild type, attempted FRT-mediated deletion of this second Neo gene (1406 at 1413) may inadvertently result in deletion of the entire newly-introduced partly bovine locus and the inactivated mouse C_{κ} exon.

Mouse embryonic stem (ES) cells derived from C57B1/6NTac mice are transfected by electroporation with the MSA vector (1457) according to widely used procedures. Prior to electroporation, the vector DNA is linearized with a rare-cutting restriction enzyme that cuts only in the prokaryotic plasmid sequence or the polylinker associated with it. The transfected cells are plated and after ~24 hours they are placed under positive selection for cells that have integrated the MSA vector into their DNA by using the neomycin analogue drug G418. There is also negative selection for cells that have integrated the vector into their DNA but not by homologous recombination. Non-homologous recombination results in retention of the DTA gene, which kills the cells when the gene is expressed, whereas the DTA gene is deleted by homologous recombination since it lies outside of the region of vector homology with the mouse IGK locus. Colonies of drug-resistant ES cells are physically extracted from their plates after they became visible to the naked eye about a week later. These picked colonies are disaggregated, re-plated in micro-well plates, and cultured for several days. Thereafter, each of the clones of cells is divided such that some of the cells are frozen as an archive, and the rest used to isolate DNA for analytical purposes.

The IGK locus in ES cells that are correctly targeted by homologous recombination has the configuration depicted at 1463.

DNA from the ES cell clones is screened by PCR using a widely used gene-targeting assay design. For this assay, one of the PCR oligonucleotide primer sequences maps outside the region of identity shared between the MSA vector (1457) and the genomic DNA (1401), while the other maps within the novel DNA between the two arms of genomic identity in the vector, i.e., the neomycin resistance (1447) gene. According to the standard design, these assays detect pieces of DNA that are only present in clones of ES cells derived from transfected cells that had undergone fully legitimate homologous recombination between the MSA vector (1457) and the endogenous mouse IGK locus. Two separate transfections are performed with the MSA vector (1457). PCR-positive clones from the two transfections are selected for expansion followed by further analysis using Southern blot assays.

The Southern blot assays are performed according to widely used procedure using three probes and genomic DNA digested with multiple restriction enzymes chosen so that the combination of probes and digests allowed for conclusions to be drawn about the structure of the targeted locus in the clones and whether it is properly modified by homologous recombination. In in this particular example, the DNA is double digested with Pac1 and another restriction enzyme such as EcoRI or HindIII, as only cells with the integrated MSA vector contains the PacI site. A first probe maps to DNA sequence flanking the 5' side of the region of identity shared between the MSA vector (1457) and the genomic DNA; a second probe also maps outside the region of identity but on the 3' side; a third probe maps within the novel DNA between the two arms of genomic identity in the vector, i.e., in the neomycin resistance (1447) gene. The Southern blot identifies the presence of the expected restriction enzyme-generated fragment of DNA corresponding to the correctly mutated, i.e., by homologous recombination with the MSA κ targeting vector (1457) part of the κ locus, as detected by one of the external probes and by the neomycin resistance gene probe. The external probe detects the mutant fragment and also a wild-type fragment from the non-mutant copy of the immunoglobulin κ locus on the homologous chromosome. The Southern blot assays are performed according to widely used procedures described in Example 7.

Karyotypes of PCR- and Southern blot-positive clones of ES cells are analyzed using an *in situ* fluorescence hybridization procedure designed to distinguish the most commonly arising chromosomal aberrations that arise in mouse ES cells. Clones with such aberrations are excluded from further use. Karyotypically normal clones that are judged to have the expected correct genomic structure based on the Southern blot data are selected for further use.

Although the ability of the ES cell DNA to be digested by PacI in the mutated IGK allele confirms the presence of the TTAATTAA sequence, DNA sequencing focusing on the region upstream of the C_{κ} exon is performed to confirm the presence of the complete expected splicing mutation. The region is amplified by genomic PCR using primers that flank the mutation [1465 and 1467 (Table 5: SEQ ID NO: 275 and SEQ ID NO:276)]. An alternate primer pair is shown in SEQ ID NO: 277 and SEQ ID NO: 278. These primers are designed using NCBI Primer-Blast and verified *in silico* to lack any predicted off-target binding sites in the mouse genome.

Sequence-verified ES cell clones are transiently transfected (1406) with a FLP recombinase expression vector to delete the neomycin resistance gene (1427). The cells are then subcloned and the deletion is confirmed by PCR. The IGK locus in the ES cells have the genomic configuration depicted at 1469.

The ES cells are electroporated with the 5' and 3' RMCE vectors, as described above. The only differences are that the 3' vector (1405) is inserted upstream of the mutant C_{κ} exon at the position shown in FIG. 9 at 901 and upstream and downstream homology arms of the 3' vector (1405) is replaced by the sequences 943 and 949, respectively of the 3' vector (905) shown in FIG. 9. As a result, PCR primers and Southern blot probes used to test for correct integration of the 3' vector (1405) are derived from sequences 943 and 949 instead of 1443 and 1449. The iEκ enhancer is not included in the targeting vector (1409), since this sequence was not deleted.

### Example 9: Bovine Vλ domains do not function well with mouse Cκ domains and bovine Vκ domains do not function well with mouse Cλ domains.

For the proposed L-K mouse (Example 4), bovine V_{λ} and J_{λ} gene segment coding sequences flanked by mouse non-coding and regulatory sequences are embedded in the mouse IGK locus from which endogenous V_{κ} and J_{κ} gene segments have been deleted. After productive V_{λ} J_{λ} gene rearrangement, the resulting Ig gene encodes a LC with a bovine λ variable domain and a mouse κ constant domain. To test whether such a hybrid LC was properly expressed and forms an intact Ig molecule, a series of transient transfection assays were performed with different combinations of Vs, both V_{κ} and V_{λ}, and C light chain exons, both C_{κ} and C_{λ}, together with an Ig HC and tested for cell surface and intracellular expression and secretion of the encoded Ig.

For these experiments, bovine sequences encoding two bovine IGH variable region domains were tested, both of which were cloned from heterohybridomas and are somatically hypermutated. One contained an ultra-long HCDR3 (clone BLV1H12, Accession # AF015506.1) and the other contained a normal-length HCDR3 (clone B4, Accession #U11628.1) linked to a mouse IgM^{b} allotype HC. Each V_{H}-encoding DNA contains the endogenous bovine L1-intron-L2. Each was individually cloned into a pCMV vector. Cow Vλ (AF023843.1) or Vκ (BC122795) V_{L} exon was linked to the constant region of mouse C_{κ}, C_{λ1}, C_{λ2}, or C_{λ3} and cloned into a pFUSE vector. The L1-intron-L2 sequences in each V_{L} are all of cow origin. These sequences contain somatically hypermutated residues that were reverted back to germline sequence.

To examine cell surface IgM expression, 293T/17 cells were co-transfected with a human CD4 (hCD4) expression vector as a transfection control plus one of the HC and LC constructs and a CD79a/b expression vector. The CD79a/b heterodimer was required for cell surface expression of the IgM. Approximately 24hr later, the transfected cells were subjected to cell surface or intracellular staining by flow cytometry. For analysis of Ig secretion, the same V_{H} genes as above were cloned into a pFUSE vector containing mouse IgG2a Fc. 293T/17 cells were co-transfected with a human CD4 (hCD4) expression vector as a transfection control plus one of the HC and LC constructs described above. Approximately 48hr later, the transfected cells and their corresponding supernatants were harvested and analyzed for HC/LC expression/secretion by western blotting.

To summarize the data obtained from these experiments, when bovine VH domains containing either ultra-long or normal-length HCDR3 and linked to a mouse IgM backbone were co-expressed with a LC having V_{κ} or V_{λ} with a mismatched C_{L} region, i.e., V_{λ} with C_{κ} or V_{κ} with C_{λ}, IgM expression on the cell surface as assessed by flow cytometry was at least two times less than when the same bovine V_{H} was co-expressed with a LC having a matched V_{L}/C_{L}, i.e., when V_{λ} was linked to C_{λ1}, C_{λ2} or C_{λ3}, or when V_{κ} was linked to C_{κ}. These results were confirmed by staining for cell surface CD79b, an obligate component of the BCR. The extent of the expression defect was dependent of the particular V_{H} gene being used; some V_{H} genes allowed for some cell surface expression of the hybrid light chains, but others were more stringent. The same trends were seen with Ig secretion.

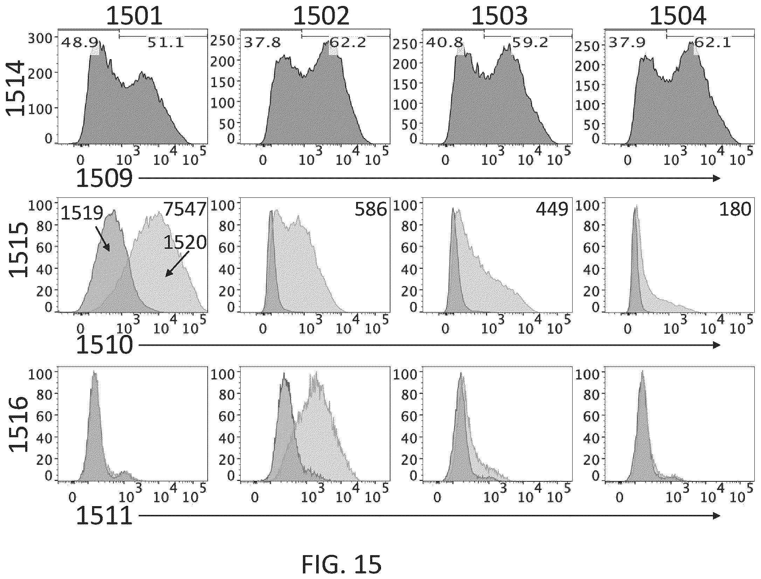

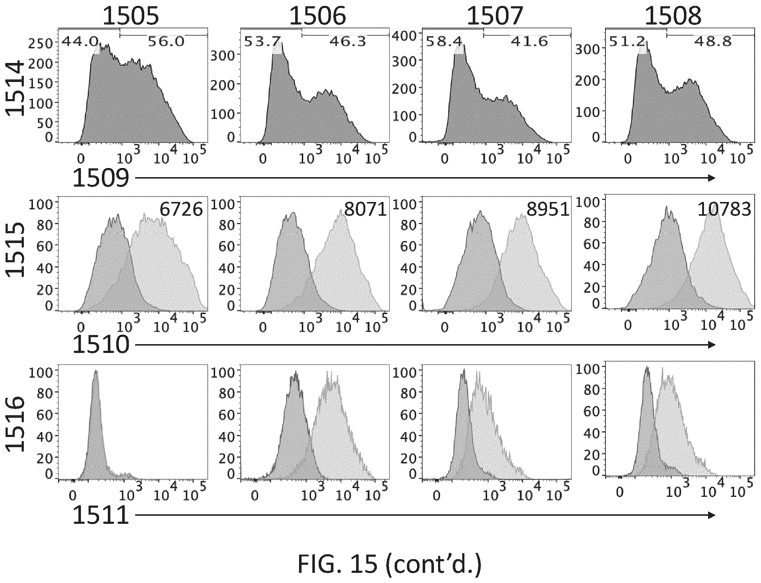

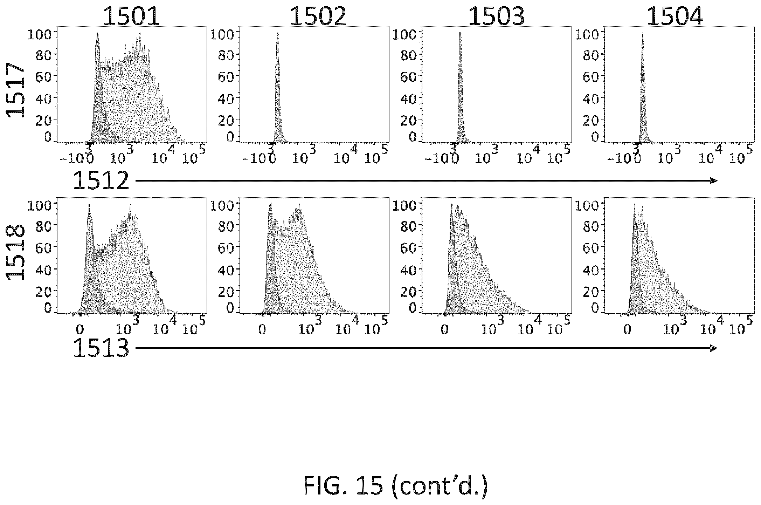

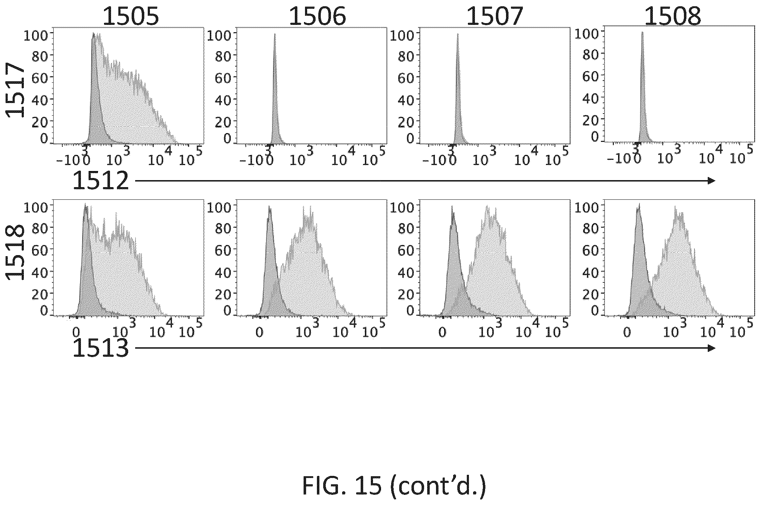

FIG. 15 shows the results of flow cytometry analysis of cells expressing bovine BLV1H12, the VH domain with an ultra-long HCDR3 with a mouse IgM backbone paired with bovine (b) V_{λ} (AF023843.1) or V_{κ} (BC122795) linked to the constant region of mouse (m) C_{κ}, C_{λ1}, C_{λ2}, or C_{λ3}. The identity of each column of flow cytometry profiles is: 1501, bV_{κ}/mC_{κ}; 1502, bV_{κ}/mC_{λ1}; 1503, bV_{κ}mC_{λ2}; 1504, bV_{κ}/mC_{λ3}; 1505, bV_{λ}/mC_{κ}; 1506, bV_{λ}/mC_{λ1}; 1507, bV_{λ}/mC_{λ2}; 1508, bV_{λ}/mC_{λ3}. The panels in row 1509 are transfection controls stained for hCD4, in row 1510 were stained for mouse IgM^{b} allotype, in row 1511 were stained for λLC, in row 1512 were stained for κLC, and in row 1513 were stained for CD79b. The X axis (1514) of all panels in row one indicates cell count. The X axis of all panels in the remaining rows (1515-1518) indicates % of maximum. The frequency of non-transfected, hCD4- cells is indicated by the number in the upper left of each panel in row 1514 and the frequency of transfected, hCD4+ cells is indicated by the number in the upper right of each panel in row 1514. Transfection efficiency was similar in all cases. The different shaded histograms in all panels in rows 1515-1518 indicated negative (1519) and positive (1520) staining by the particular antibody being used in each row, gated on the transfected hCD4+ cells. (Shown as an example in column 1501, row 1515). The numbers in the upper right of each panel in row 1515 indicate the mean fluorescence intensity (MFI) of the cell surface IgM^{b} staining on the transfected, i.e., hCD4+, cells, which is a quantitative indication of the level of expression. When bovine V_{κ} was linked to mouse C_{λ1}, C_{λ1} or C_{λ2} (row 1515, panel 1502-1504), IgM expression on the cell surface was less (MFI 586, 449, 180, respectively) than when the same bovine V_{κ} was linked to mouse C_{κ} (row 1515, panel 1501, MFI 7547) Similarly, the bovine IgM with V_{λ} was expressed better when linked to C_{λ1}, C_{λ1} or C_{λ2} (row 1515, panel 1506-1508, MFI 8017, 8951, 10783, respectively) than to C_{κ} (row 1515, panel 1505, MFI 6726), although in this case the expression differences in this case were not so dramatic.

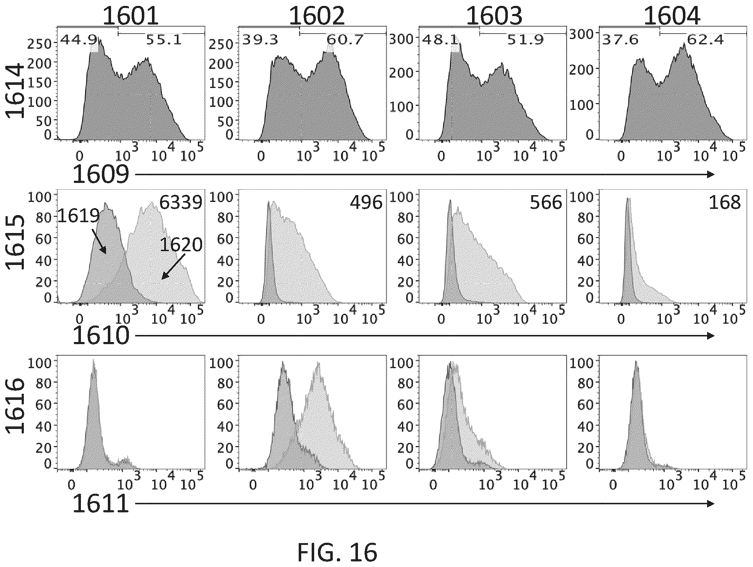

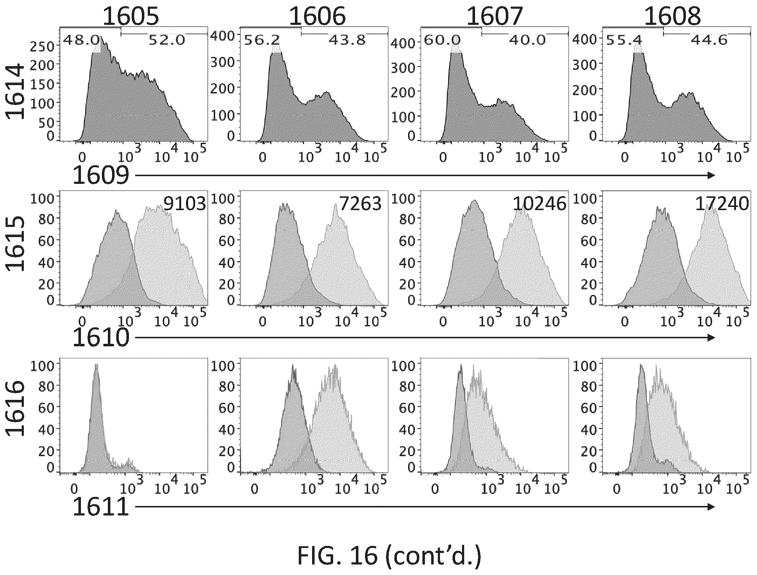

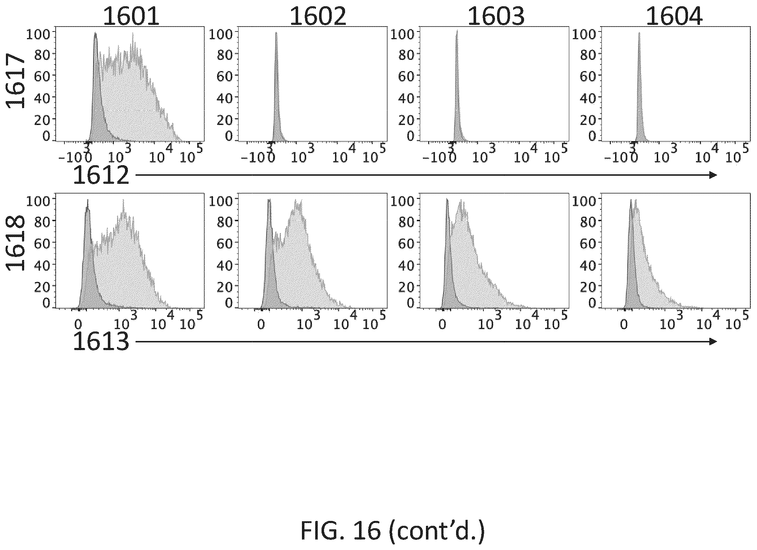

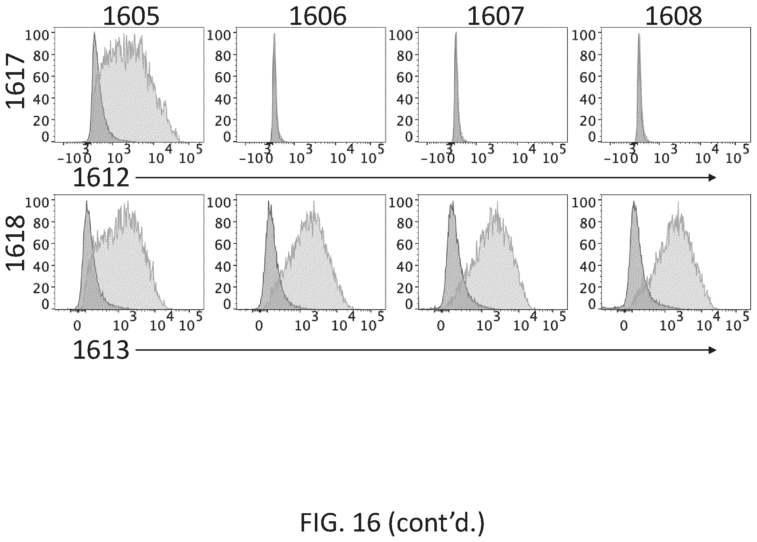

FIG. 16 shows the results of flow cytometry analysis of cells expressing bovine IGHV B4, the VH domain with a normal-length HCDR3 with a mouse IgM backbone paired with bovine (b) V_{λ} (AF023843.1) or V_{κ} (BC122795) linked to the constant region of mouse (m) C_{κ}, C_{λ1}, C_{λ2}, or C_{λ3}. The identity of each column of flow cytometry profiles is: 1601, bV_{κ}/mC_{κ}; 1602, bV_{κ}/mC_{λ1}; 1603, bV_{κ}mC_{λ2}; 1604, bV_{κ}/mC_{λ3}; 1605, bV_{λ}/mC_{κ}; 1606, bV_{λ}/mC_{λ1}; 1607, bV_{λ}/mC_{λ2}; 1608, bV_{λ}/mC_{λ3}. The panels in row (1609) are transfection controls stained for hCD4, in row (1610) were stained for mouse IgM^{b} allotype, in row (1611) were stained for λLC, in row (1612) were stained for κLC, and in row (1613) were stained for CD79b. The X axis (1614) of all panels in row one indicates cell count. The X axis of all panels in the remaining rows (1615-1618) indicates % of maximum. The frequency of non-transfected, hCD4- cells is indicated by the number in the upper left of each panel in row 1614 and the frequency of transfected, hCD4+ cells is indicated by the number in the upper right of each panel in row 1614. Transfection efficiency was similar in all cases. The different shaded histograms in all panels in rows 1615-1618 indicated negative (1619) and positive (1620) staining by the particular antibody being used in each row, gated on the transfected hCD4+ cells. (Shown as an example in column 1601, row 1615). The numbers in the upper right of each panel in row 1615 indicate the mean fluorescence intensity (MFI) of the cell surface IgM^{b} staining on the transfected, i.e., hCD4+, cells, which is a quantitative indication of the level of expression. When bovine V_{κ} was linked to mouse C_{λ1}, C_{λ1} or C_{λ2} (row 1615, panel 1602-1604), IgM expression on the cell surface was less (MFI 496, 566, 168, respectively) than when the same bovine V_{κ} was linked to mouse C_{κ} (row 1615, panel 1601, MFI 6339) Similarly, the bovine IgM with V_{λ} was expressed better when linked to C_{λ2} or C_{λ3} (row 1615, panel 1607, 1608, MFI 10246, 17240, respectively) than to C_{κ} (row 1615, panel 1605, 9103). However in this case the MFI of V_{λ}-C_{κ} (9108) was actually higher than for V_{λ}- C_{λ1} (7263).

The results of this analysis indicate that hybrid light chains composed of bovine V_{λ} and mouse C_{κ} or bovine V_{κ} and mouse C_{λ1}, C_{λ2} or C_{λ3} were relatively poorly expressed on the cell surface with µHC. Since B cell survival depends on IgM BCR expression, it is clear that pairing of bovine V_{λ} and mouse C_{κ} would result in a major reduction in the development of λLC-expressing B cells. Similarly, pairing of bovine V_{κ} with mouse C_{λ1}, C_{λ2} or C_{λ3} would reduce the development of κ-LC expressing B cells.

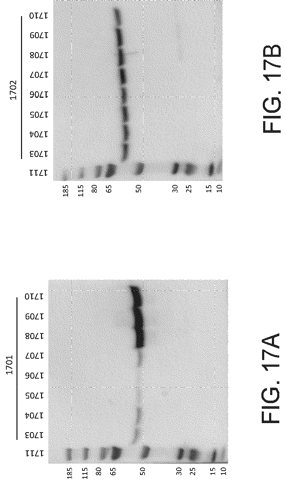

Expression and secretion of the Ig with hybrid or homologous LC. Supernatants and cell lysates of the transiently transfected cells were analyzed by western blotting. FIG. 17 shows the results of supernatants (1701) and cell lysates (1702) of cells expressing bovine BLV1H12, the VH domain with an ultra-long HCDR3, with a mouse IgG2a HC backbone paired with bovine (b) V_{λ} (AF023843.1) or V_{κ} (BC122795) linked to the constant region of mouse (m) C_{κ}, C_{λ1}, C_{λ2}, or C_{λ3}. The blots were electrophoresed under reducing conditions and probed with antibody to the mouse γ2a HC. The contents of each lane: 1703, bV_{κ}/mC_{κ}; 1704, bV_{κ}/mC_{λ1}; 1705, bV_{κ}mC_{λ2}; 1706, bV_{κ}/mC_{λ3}; 1707, bV_{λ}/mC_{κ}; 1708, bV_{λ}/mC_{λ1}; 1709, bV_{λ}/mC_{λ2}; 1710, bV_{λ}/mC_{λ3}

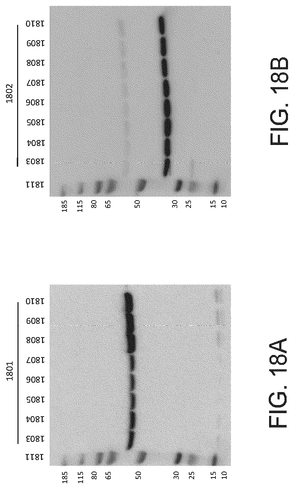

Molecular weight standards are in lane 1711. This particular bV_{κ}/MC_{L} combination was not particularly well secreted, no matter what the LC, but clearly bV_{κ}/mC_{κ} (1703) was secreted better than bV_{κ}/mC_{λ2} (1705) or bV_{κ}/mC_{λ3} (1706). On the other hand, bV_{λ} was secreted very poorly with mC_{κ}(1707) but was secreted well with all three mouse C_{λ} subclasses (1708-1710). The intracellular HC levels were similar with all eight constructs (blot 1702, lanes 1703-1710), indicating that differential intracellular protein stability was not responsible for the observed differences in secretion. FIG. 18 shows loading controls using Myc (1802) and GAPDH (1803). The lane designations in this figure correspond to those in FIG. 17

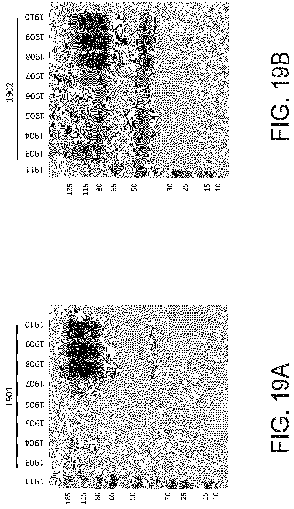

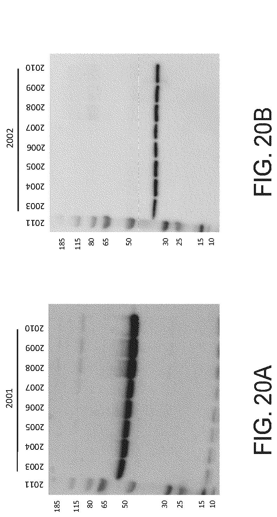

FIG. 19 shows the results of supernatants (1901) and cell lysates (1902) of cells expressing bovine BLV1H12, the VH domain with an average length HCDR3, with a mouse IgG2a HC backbone paired with bovine (b) V_{λ} (AF023843.1) or V_{κ} (BC122795) linked to the constant region of mouse (m) C_{κ}, C_{λ1}, C_{λ2}, or C_{λ3}. In this case, the blots were electrophoresed under non-reducing conditions and probed with antibody to the mouse γ2a HC. The contents of each lane: 1903, bV_{κ}/mC_{κ}; 1904, bV_{κ}/mC_{λ1}; 1905, bV_{κ}mC_{λ2}; 1906, bV_{κ}/mC_{λ3}; 1907, bV_{λ}/mC_{κ}; 1908, bV_{λ}/mC_{λ1}; 1909, bV_{λ}/mC_{λ2}; 1910, bV_{λ}/mC_{λ3}. Molecular weight standards are in lane 1911. This particular bV_{κ}/MC_{L} combination was not particularly well secreted, no matter what the LC, but clearly bV_{κ}/mC_{K} (1903) was secreted better than bV_{κ}/mC_{λ2} (1905) or bV_{κ}/mC_{λ3} (1906). On the other hand, bV_{λ} (1907) was secreted very poorly with mC_{κ} but secreted well with all three mouse C_{λ} subclasses (1908-1910). The intracellular HC levels were similar with all eight constructs (blot 1902, lanes 1903-1910), indicating that differential intracellular protein stability was not responsible for the observed differences in secretion. FIG. 20 shows loading controls using Myc (2001) and GAPDH (2002). The lane designations in this figure correspond to those in FIG. 19.

### Example 10: Expression of Partly Bovine Immunoglobulin with Mouse IgD

IgD is co-expressed with IgM on mature B cells in most mammals. However, the issue of whether cows have a functional constant region gene to encode the δHC has been quite controversial. Early studies found no evidence for a Cδ gene or IgD protein in cows (Butler, et al. ibid; Naessens J ibid). Subsequently, evidence for the existence of bovine Cδ genes (Zhao, et al. ibid) and expression of bovine IgD (Xu, et al. ibid) have been reported, although the reported frequency of bovine IgD⁺ cells was much lower than in mice. The current annotation of the bovine IGH locus by the **International ImMunoGeneTics** information system^{®} http://www.imgt.org, (**IMGT**) lists four Cδ genes, IGHDD1P, IGHDD2P, IGHDD39, and IGHD, none of which is functional. There are two IGHD alleles, one is a pseudogene due to a frameshift in M1 and the other is an ORF due to a non-canonical splice donor, NGC instead of NGT. The low frequency of IgD⁺ cells observed by Xu et al. (ibid) may be due to occasional leaky splicing from the ORF allele. In any case, one concern was that the bovine V_{H} domains might not fold properly when linked to mouse Cδ, since the bovine V_{H} gene region has apparently been evolving with a partial or completely non-functional C_{δ} gene. A problem with partial or absent assembly of the partly bovine IgD could disturb normal B cell development.

To test whether bovine V_{H} domains with a Cδ backbone can assemble into an IgD molecule expressible on the cell membrane, transient transfection and flow cytometry analysis were conducted using methods similar to Example 9.

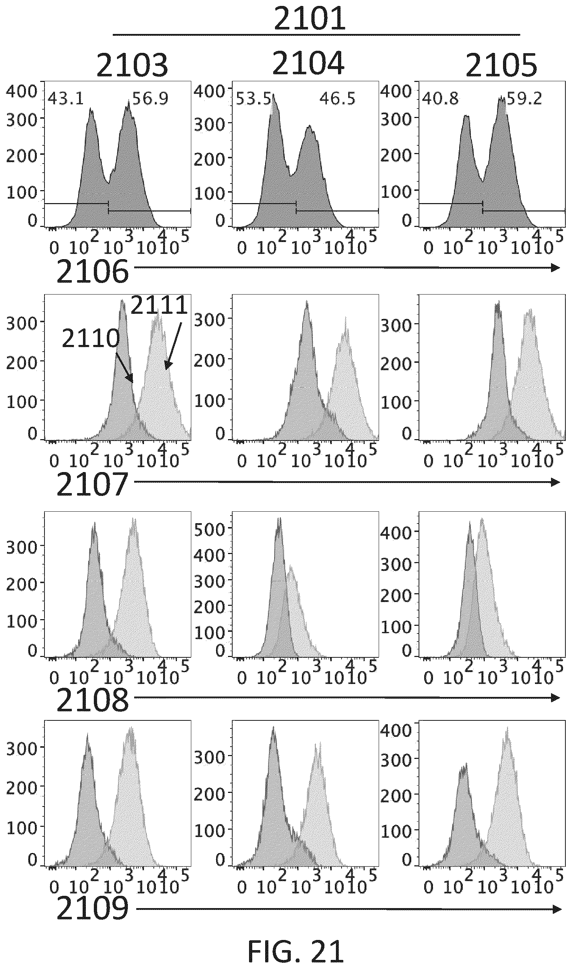

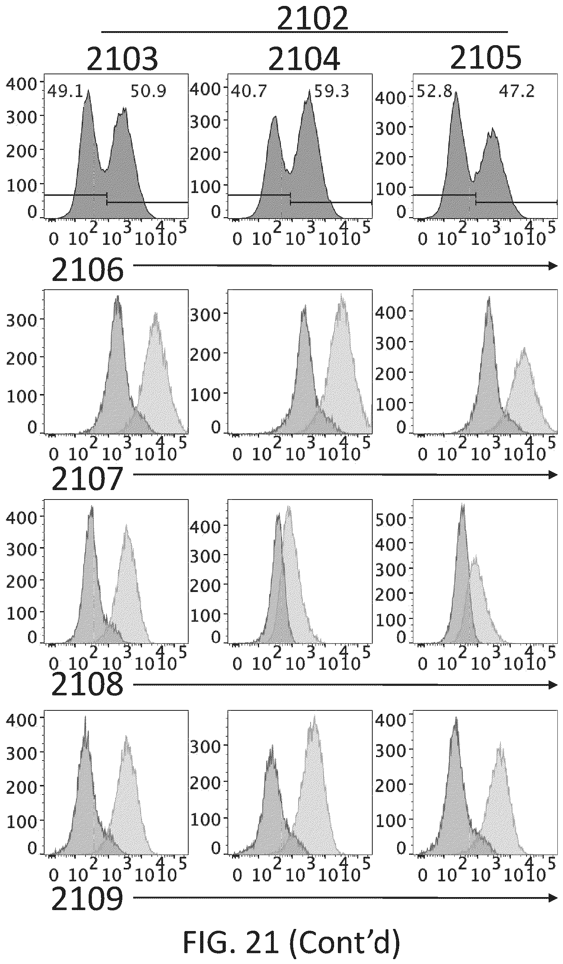

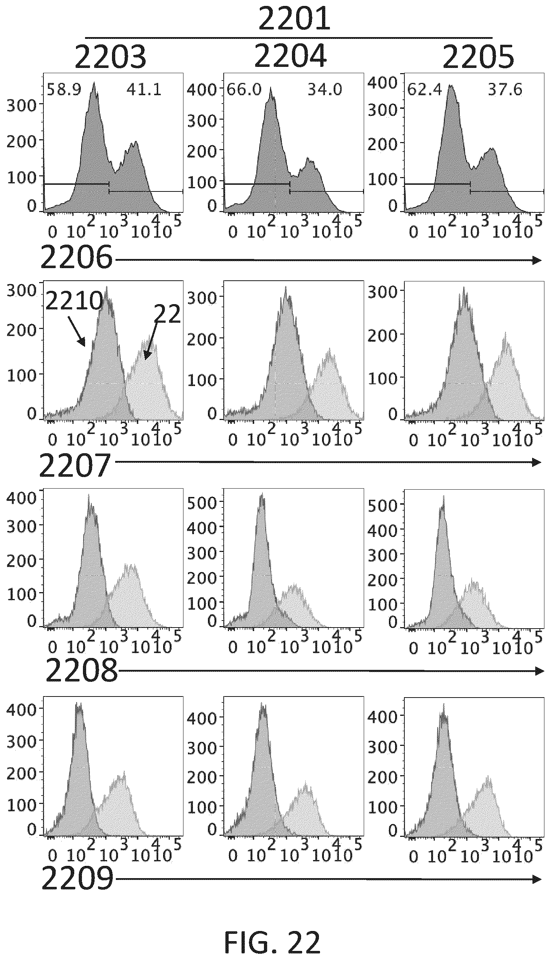

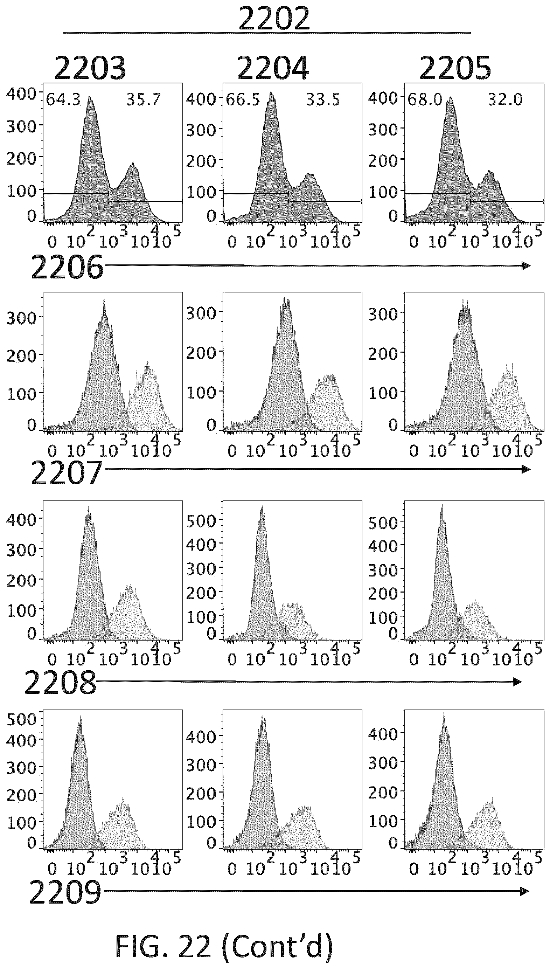

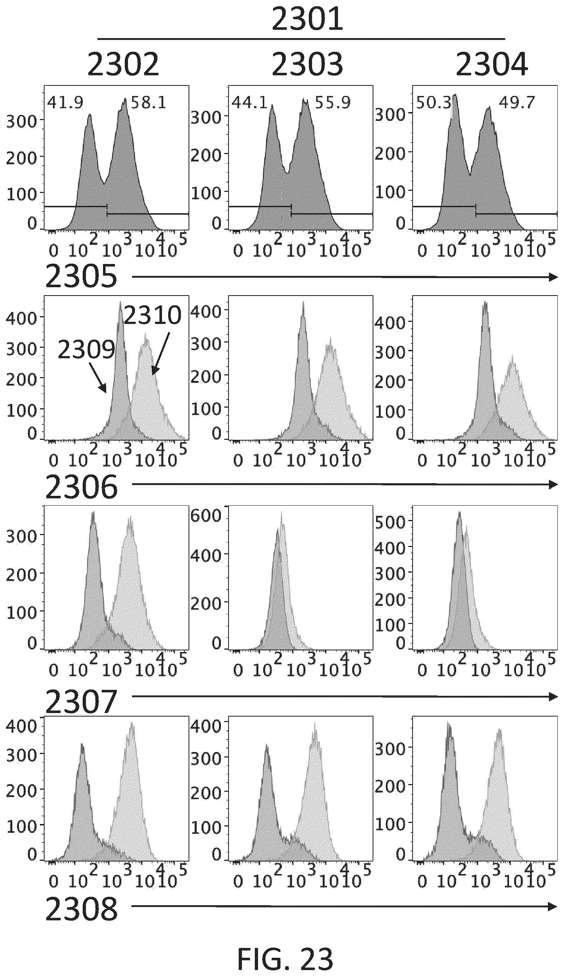

293T/17 cells were co-transfected with a human CD4 (hCD4) expression vector as a transfection control plus one of the HC constructs from Example 9 (IGHV BLV1H12 or B4), except that Cµ was replaced with Cδ, as well as an additional IGHV BL5B8, also linked to Cδ, Both IGHV BLV1H12 B4 have ultra-long HCDR3 regions, 61 and 56 amino acids, respectively. Each heavy chain construct was co-transfected one of the λ LC constructs described in Example 9, along with a CD79a/b expression vector. As can be seen if FIGS. 21-24, the HC with bovine VH domains with a moue IgD backbone were expressed intracellularly as well as on the cell surface when paired with a bovine V_{λ}-mouse C_{λ1, 2 or 3} LC. FIG. 21 shows expression of intracellular bovine IGHV BLV1H12 (2101) and bovine IGHV BLV5B8 (2102) with a mouse IgD backbone and bovine V_{λ}-mouse C_{λ1} (2103), C_{λ2} (2104) or C_{λ3} (2105). In these studies, row 2106 shows staining for intracellular hCD4, the control for transfection efficiency. The frequency of non-transfected, hCD4- cells is indicated by the number in the lower left of each panel in row 2106 and the frequency of transfected, hCD4+ cells is indicated by the number in the lower right of each panel in row 2106. Transfection efficiency was similar in all cases. The different shaded histograms in all panels in rows 2107-2109 indicated negative (2110) and positive (2111) staining by the particular antibody being used in each row, gated on the transfected hCD4+ cells. (Shown as an example in column 2103, row 2107). Row 2107 shows staining for intracellular IgD, row 2108 shows staining for intracellular λLC and row 2109 shows staining for intracellular CD79b. These particular bovine bV_{H}-mC_{δ}/bV_{λ}-mC_{λ} combinations were expressed well intracellularly in the transfected cells. FIG. 22 shows expression of cell surface of the same bovine constructs as in FIG.21, stained with the same antibodies. All were well expressed based on cell surface IgD, λLC or CD79b staining. (The cell surface staining data is arranged the same as in FIG. 21.) FIG. 23 shows expression of intracellular bovine IGHV B4, which has an average length HCDR3, with a mouse IgD backbone and bovine Vλ attached to mouse C_{λ1} (2302), C_{λ2} (2303) or C_{λ3} (2304). In these studies, row 2305 shows staining for intracellular hCD4, the control for transfection efficiency. The frequency of non-transfected, hCD4- cells is indicated by the number in the lower left of each panel in row 2305 and the frequency of transfected, hCD4+ cells is indicated by the number in the lower right of each panel in row 2305.Transfection efficiency was similar in all cases. The different shaded histograms in all panels in rows 2306-2308 indicated negative (2309) and positive (2310) staining by the particular antibody being used in each row, gated on the transfected hCD4+ cells. (Shown as an example in column 2302, row 2306). Row 2306 shows staining for intracellular IgD, row 2307 shows staining for intracellular λLC and row 2308 shows staining for intracellular CD79b. These particular bovine bV_{H}-mC_{δ}/bV_{λ}-mC_{λ} combinations were expressed well intracellularly in the transfected cells.

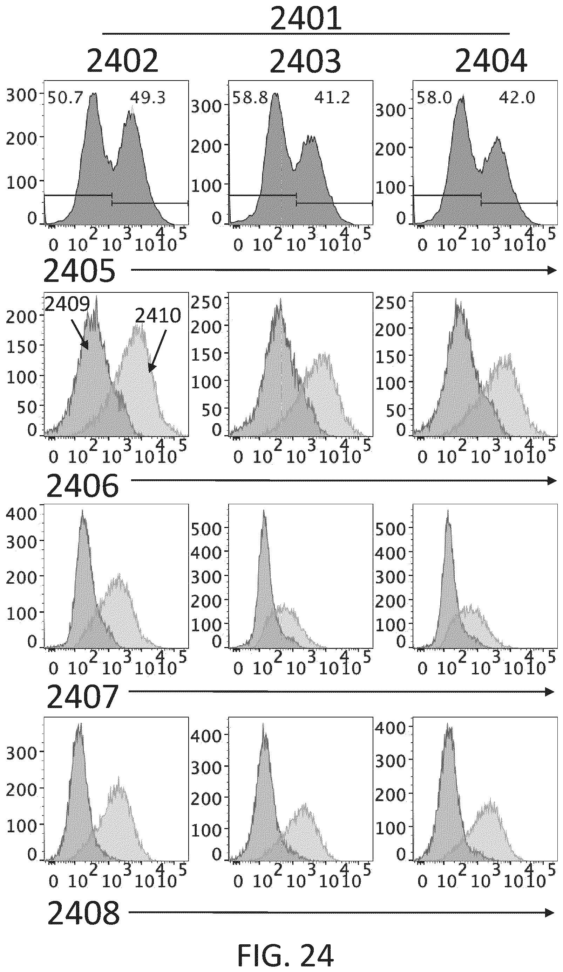

FIG. 24 again shows expression bovine IGHV B4 with a mouse IgD backbone and bovine Vλ attached to mouse C_{λ1} (2402), C_{λ2} (2403) or C_{λ3} (2404), but this figure shows cell surface staining. (The cell surface staining data is arranged the same as in FIG. 23.) These particular bovine bV_{H}-mC_{δ}/bV_{λ}-mC_{λ} combinations were expressed well on the surface of the transfected cells.

Thus, the three tested bovine V_{H} domains were expressed with a mouse IgD backbone, although there was some variability in the level of cell surface expression depending on the particular HC/LC combination. It is believed that HC/LC combinations that can be expressed as IgD on the cell surface are selected into the follicular B cell compartment during B cell development, generating a diverse BCR repertoire

The preceding merely illustrates the principles of the methods described herein. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims. In the claims that follow, unless the term "means" is used, none of the features or elements recited therein should be construed as means-plus-function limitations pursuant to 35 U.S.C. §112 ¶6. All references cited herein are incorporated by reference in their entirety for all purposes.

### SEQUENCE TABLES

### Bovine Ig

(NB, the sequence and annotation of the bovine genome is still incomplete. These tables do not necessarily describe the complete bovine V_{H}, D and J_{H}, V_{κ} AND J_{κ}, or V_{λ} and J_{λ} gene segment repertoire.)

(**F** = Functional, **ORF** = open reading frame, **P** = pseudogene, *01 indicates the IMGT allele number)

**Table 1. Bovine IGH locus**

| **Germline V_{H} sequences** |
|---|
| Functionality is shown between brackets, [F] and [P], when the accession number (underlined, if known) refers to rearranged genomic DNA or cDNA and the corresponding germline gene has not yet been isolated and the chromosomal location is unknown. |
| SEQ ID NO. 1 IGHV1-7 (**F**) |
| >IGHV1-7*01\|Bos taurus_Holstein\|F\|V-REGION\| |
| caggtgcagctgcgggagtcgggccccagcctggtgaagccgtcacagaccctctccctc acctgcacggtctctggattctcattgagcgacaaggctgtaggctgggtccgccaggct ccagggaaggcgctggagtggctcggtggtatagacactggtggaagcacaggctataac ccaggcctgaaatcccggctcagcatcaccaaggacaactccaagagccaagtctctctg tcagtgagcagcgtgacaactgaggactcggccacatactactgtactactgtgcaccag |
| SEQ ID NO. 2 IGHV1-10 (**F**) |
| >IGHV1-10*01\|Bos taurus_Holstein\|F\|V-REGION\| |
| caggtgcagctgcgggagtcgggccccagcctggtgaagccctcacagaccctctccctc acctgcacggtctctggattctcattgagcagctatggtgtaggctgggtccgccaggct ccagggaaggcgctggagtgtcttggtggtataagtagtgctggaagcacaggctataac ccagccctgaaataccggctcagcatcaccaaggacaactccaagagccaagtctctctg tcactgagcagcgtgacaactgaggacacggccacatactactgtgcgaagga |
| SEQ ID NO. 3 IGHV1-14 (**F**) |
| >IGHV1-14*01\|Bos taurus_Holstein\|F\|V-REGION\| |
| caggtgcagctgcgggagtcgggccccagcctggtgaagccctcacagaccctgtccctc acctgcacggtctctggattctcattaagcgataatagtgtaggctgggtccgccaggct ccaggaaaggcgctggagtggctcggtgtcatatatagtggtggaagcacaggctataac ccagccctgaaatcccggctcagcatcaccaaggacaactccaagagccaagtctctcta tcactgagcagcgtgacaactgaggacacggccacatactactgtgcaagaga |
| SEQ ID NO. 4 IGHV1-17 (**F**) |
| >IGHV1-17*01\|Bos taurus_Holstein\|F\|V-REGION\| |
| caggtgcagctgcgcgagtcgggccccagcctggtgaagccctcacagaccctctccctc acctgcacggtctctggattctcattgagcagctatgctgtaagctgggtccgccaggct ccagggaaggctctggagtggcttggtgatataagcagtggtggaagcacaggctataac ccagccctgaaatcccggctcagcatcaccaaggacaactccaagagccaagtctctctg tcagtgagcagcgtgacacctgaggacacggccacatactactgtgcgaagga |
| SEQ ID NO. 5 IGHV1-20 (**F**) |
| >IGHV1-20*01\|Bos taurus_Holstein\|F\|V-REGION\| |
| caggtgcagctgcgggagtcgggccccagcctggtgaagccctcacagaccctctccctc acctgcacggtctctggattctcactgagcagctatgctgtaggctgggtccgccaggct ccagggaaggcgctggagtggctcggtggtataagcagtggtggaagcacatactataac ccagccctgaaatcccggctcagcatcaccaaggacaactccaagagccaagtctctctg |
| tcagtgagcagcgtgacacctgaggacacggccacatactactgtgcgaagga |
| SEQ ID NO. 6 IGHV1-121 (**F**) |
| >IGHV1-21*01\|Bos taurus_Holstein\|F\|V-REGION\| |
| caggtgcagctgcgggagtcgggccccagcctggtgaagccctcacagaccctctccctc acctgcacgatctctggattctcattgagcagctatgctgtaggctgggtccgccaggct ccggggaaggcgctggagtgggttggtggtataagtagtggtggaagcacatgccttaac ccagccctgaaatcccggctcagcatcaccaaggacaactccaagagccaagtctctctg tcagtgagcagcgtgacaactgaggacacggccacatactactgtgcgaagga |
| SEQ ID NO. 7 IGHVI-25 (**F**) |
| >IGHV1-25*01\|Bos taurus_Holstein\|F\|V-REGION\| |
| caggtgcagctgcaggagtcgggccccagcctggtaaagacctcacagaccctctccctc acctgcacggcctctggattatcattaaccagatatggtatacactgggtccgccaggct ccaggaaaggcgctggagtggcttggtgatataagcagtggtggaagcacaggctataac ccaggcctgaaatcccggctcagcatcaccaaggacaactccaagagccaagtctctctg tcactgagcagcctgacacctgaggactcagccacatactactgtgcaagaga |
| SEQ ID NO. 8 IGHVI-27 (**F**) |
| >IGHV1-27*01\|Bos taurus_Holstein\|F\|V-REGION\| |
| caggtgcagctgcgggagtcgggccccagcctggtgaagccctcacagaccctctccctc acctgcacggtctctggattctcattgagcagcaatggtgtaggctgggtccgccaggct ccagggaaggcgctggagtgggttggtggtatagataatgatggagacacatactataac ccagccctgaaatcccggctcagcatcaccaaggacaactccaagagccaagtctctctg tcagtgagcagcgtgacacctgaggacacggccacatactactgtgcaaaaga |
| SEQ ID NO. 9 IGHV1-30 (**F**) |
| >IGHV1-30*01\|Bos taurus_Holstein\|F\|V-REGION\| |
| caggtgcagctgcgggagtcgggccccagcctggtgaagccctcacagaccctctccctc acctgcacggtctctggattctcattgagcagcaatggtgtagtctgggtccgccaggct ccagggaaggcgctggagtggctcggtggtatatgcagtggtggaagcacaagctttaac ccagccctgaaatcccggctcagcatcaccaaggacaactccaagagccaagtctctctg tcagtgagcagcgtgacacctgaggacacggccacatactactgtgcaagaga |
| SEQ ID NO. 10 IGHV1-32 (**P**) |
| >IGHV1-32*01\|Bos taurus_Holstein\|P\|V-REGION\| |
| caggtgcagctgcaggagtcgggccccagcctggtgaagccctcacagaccctgtccctc acctgcacggtctctggattctcattaagcgacaatgctgtaggctgggtccgccaggct ccaggaaaggcgctggagtagcttggtggtatagataatgatggatacacaaggtataac ccagccctgaaatcccggctcagcattaccaaggacaactccaagagctaagtctctgtg tcagtgagcagcgtgacaactgaggactcggccgtgtactactgtgcgaagga |
| SEQ ID NO. 11 IGHV1-33 (**F**) |
| >IGHV1-33*01\|Bos taurus_Holstein\|F\|V-REGION\| |
| caggtgcagctgcgggagtcgggccccagcctggtgaagccctcacagaccctctccctc acctgcacgatctctggattctcattgagcagctatgctgtaggctgggtccgccaggct ccggggaaggcgctggagtgggttggtggtataagtagtggtggaagcacatgccttaac ccagccctgaaatcccggctcagcatcaccaaggacaactccaagagccaagtctctctg tcagtgagcagcgtgacaactgaggacacggccacatactactgtgcgaagga |
| SEQ ID NO. 12 IGHV1-37 (**F**) |
| >IGHV1-37*01\|Bos taurus_Holstein\|F\|V-REGION\| |
| caggtgcagctgcaggagtcgggccccagcctggtgaagacctcacagacactctccctc acttgcacggcctctggattatcattaaccagatatggtatacactgggtccgccaggct ccaggaaaggcgctggagtggcttggtgatataagcagtggtggaagcacaggctataac ccaggcctgaaatcccggctcagcatcaccaaggacaactccaagagccaagtctctctg tcactgagcagcctgacgcctgaggactcagccacatactactgtgcaagaga |
| SEQ ID NO. 13 IGHV1-39 (**F**) |
| >IGHV1-39*01\|Bos taurus_Holstein\|F\|V-REGION\| |
| aaggtgcagctgcaggagtcgggtcccagcctggtgaagccctcacagaccctctccctc acctgcacgacctctggattctcattgaccagctatggtgtaagctgggtccgccaggct ccagggaaggcgctggagtggctcggtggtatagatagtggtggaagcacaggctataac ccaggcctgaaatccaggctcagcatcaccagggacaactccaagagccaagtctctctg tcagtgagcagcgtgacacctgaggacacggccacatactactgtgcgaagga |
| SEQ ID NO. 14 IGHV1-43 (**F**) |
| >IGHV1-43*01\|Bos taurus_Holstein\|P\|V-REGION\| |
| gaggtgcagctgcgggagtggggccccagcctggtgaagccctcacagaccctctccctc accttcatggtctctggattctcattgaccagctatggtgtagattgggtccgccaggct ccagggaaggtgccggaatgggttggtggtataagcagtggtggaagtacatactataac ccagccctgaaattcccggctcagcatcacgagggaaacctccaagagccaagtctctct gagcagcgtgacaactgaggacacggccgtgcactactgtgtgaagga |
| SEQ ID NO. 15 IGHV1-46 (**P**) |
| >IGHV1-46*01\|Bos taurus_Holstein\|P\|V-REGION\| |
| aaggtgcagctgcgggagtggggccccagcctggtgaagccctcacagaccctctccctc accttcatggtctctggattctcattgaccagctatggtgtagattgggtccgccaggct ccagggaaggtgccggaatgggttggtggtataagcagtggtggaagtacatactataac ccagccctgaaattcctggctcagcatcacgagggaaacctccaagagccaagtctctct gagcagcgtgacaactgaggacacggccgtgcactactgtgtgaagga |
| SEQ ID NO. 16 IGHVIS1 [**F**] |
| >U55165\|IGHV1S1*01\|Bos taurus\|F\|V-REGION\| |
| aaggtgcagctgcaggagtcgggtcccagcctggtgaagccctcacagaccctctccctc acctgcacggtctctggattctcattgagcagctatggtatacactgggtccgccaggct ccaggaaaggcgctggagtggcttggtgatataagcagtggtggaagcacaggctataac ccaggcctgaaatcccggctcagcatcaccaaggacaactccaagagccaagtctctctg tcactgagcagcctgacgcctgaggacacagccacatactactgt |
| SEQ ID NO. 17 IGHV1S2 [**P**] |
| >IGHV1S2*01\|Bos taurus_Hereford\|P\|V-REGION\| |
| ccgatgcagctgcaggagtcaggccccagcctggtgaagccctcacagaccctttccttc acctgcactgtgtctggattctcattaaccaacaatggtgtaggctgggtccgccaggct ccacaaaagggattggagttggttggtatcatatgtacaatatgatatatatatgatatg gaagcacatactaccacccagcccttaagtccaggctcagcatcaccagcgacatctcca agagcctagtctcttgtattactgagcagcgtgacaactgaggacacggccctgtagtac tgtgcaaaaga |
| SEQ ID NO. 18 IGHV2-5 (**P**) |
| >IGHV2-5*01\|Bos taurus_Holstein\|P\|V-REGION\| |
| cagatgagctgcagcagtcgggccagaactggtgaaccctcactcaccctctcctgacgt gcactgtctcttcttactccatcacgtggttattgtcggaactggattcgccaggcccta gggaaggggctagagcagatgtcataaataagctatgatggtgacacttactacagcccc tgcatcaagatcacacctccatctgcagagacatccaagaatcagttctctctgcagctg agctctgtgaccactgaggacacggcagtgtgctgctgtgcaagaga |
| SEQ ID NO. 19 IGHV2-6 (**P**) |
| >IGHV2-6*01\|Bos taurus_Holstein\|P\|V-REGION\| |
| caggtgaaggtgcaggagtcgggcccagacctggtgaagccctcgcagatcgtctccctc acatgtgctgtctctggttactccatcacgtggttatggtgagagctgggtccaccaggc cccagcgaaggggctagagtggatgggaagcatatattataatggtgacacttaccacag cccctccatcaagagccacacctccatctccaggggcacgtctaagaaccagtcctccct gcagctgagctccgtgaccactgtggacacagctgtgtattactgtgcaagaga |
| SEQ ID NO. 20 IGHV2-9 (**P**) |
| >IGHV2-9*01\|Bos taurus_Holstein\|P\|V-REGION\| |
| ccagtgcagctgcaggagcagggtccagaactgcggaagctctcacctgtgccatctctg gttgctccatcaccaatggttactggtaaccagtactccctgcagctaagctctgtgaca accaaggacaccgcagtatgctgctgtgcaagaga |
| SEQ ID NO. 21 IGHV2-12 (**P**) |
| >IGHV2-12*01\|Bos taurus_Holstein\|P\|V-REGION\| |
| ccagatgcactgcagcagtcgggcccagaactagtgagccctcactgaccctctcctcac gtgcactatctctggttactccatcaagtggttattgagggaactggttttgccaggccc cagggaaggggctagagcagatggcatgcataaactaagatggtgacacttactacatc ccctccatcaagagccacacctccaatcagttctccctgcagctgaactctgtgaccattg aggacacggccatgtgttgctgggcaagaga |
| SEQ ID NO. 22 IGHV2-13 (**P**) |
| >IGHV2-13*01\|Bos taurus_Holstein\|P\|V-REGION\| |
| caggtgaaggtgcaggagtcgggcccagagacctggtgaagccctcgcagatcgtctccc tcacatgtgctgtctctggttactccatcacgtggttatggtgggagctggatccaccag gccccagcgaaggggctagagtagatgggaagcatatattataatggtgacacttaccac agcccctccttcaagagccacacgtccatctccaggggcacgtctaagaaccagtcctcc ctgcagctgagctccgtgaccactgtggacacagctgtgtgttactgtgcaagaga |
| SEQ ID NO. 23 IGHV2-16 (**P**) |
| >IGHV2-16*01\|Bos taurus_Holstein\|P\|V-REGION\| |
| ccagtgcagctccaggagcagggtccagaactgcggaagctctcacctgtgccatctctg gttgctccatcaccagtggttactggtaaccagtactccctgcagctaagctctgtgaca accaaggacaccgcagtatgctgctgtgcaagaga |
| SEQ ID NO. 24 IGHV2-19 (**P**) |
| >IGHV2-19*01\|Bos taurus_Holstein\|P\|V-REGION\| |
| ccagtgcagctgcaggagcagggtccagaactgcggaagctctcacctgtgccatctctg gttgctccatcaccagtggttactggtaaccagtactccctgcagctaagctctgtgatg accaaggacacggcagtatgctgctgtgcaagaga |
| SEQ ID NO. 25 IGHV23 (**P**) |
| >IGHV2-23*01\|Bos taurus_Holstein\|P\|V-REGION\| |
| cccagatgcactgctgcagtcgggccagaactggtgaagccctcactgactgtctcctca cttgcactgtctctgcttactccatcacgtggttattgtgggaactggattcgccaggcc ccagggaaggggctagagcagatgacatgcataaactatgatggtgacacttactacagc ccctccatcaagagccacatctccatctgcagggacatccaagaatcagttctccctgca gctgagctctgtgaccactgaggacacggccatgtgttgctgtgcaagaga |
| SEQ ID NO. 26 IGHV2-24 (**P**) |
| >IGHV2-24*01\|Bos taurus_Holstein\|P\|V-REGION\| |
| caggtgaaggtgcaggagtcgggcccagaactggtgaagccctcgcagatcgtctccctc acatgtgctgtctctggttactccatcacgtggttatggtgggagctggatccaccaggc ccgagggaaggggctagagtagatgggaagcatatattataatggtgacacttaccacag cccctccatcaagagccacacgtccatctccaggggcacgtctaagaaccagtcctccct gcagctgagctctgtgaccactgaggacacagctgtgtattactgtgcaagaga |
| SEQ ID NO. 27 IGHV2-26 (**P**) |
| >IGHV2-26*01\|Bos taurus_Holstein\|P\|V-REGION\| |
| ccagtgcagctccaggagcagggtccagaactgcggaagctctcacctgtgccatctctg gttgctccatcaccagtggttactggtaaccagtactccctgcagctaagctctgtgacg accaaggacaccgcagtatactgctgtgcaagaga |
| SEQ ID NO. 28 IGHV2-29 (**P**) |
| >IGHV2-29*01\|Bos taurus_Holstein\|P\|V-REGION\| |
| ccagtgcagctgcaggagcagggtccagaactgcggaagctctcacctgtgccatctctg gttgctccatcaccagtggttactggtaaccagtactccctgcagctaagctctgtgatg accaaggacaccgcagtatactgctgtgcaagaga |
| SEQ ID NO. 29 IGHV2-31 (**P**) |
| >IGHV2-31*01\|Bos taurus_Holstein\|P\|V-REGION\| |
| caggtgaaggtgcaggagtcgggcccagaactggtgaagccctcgcagatcatctccctc |
| acatgtgctgtctctggttactccatcacgtggttatggtgggagctggatccaccaggc cccagggaaggggttagagtggatgggaagcatatattataatggtgacacttaccacag cccctccatcaagagccacacctccatctgcagagacatccaacaatcagttctccctgt agctgagctctgtgaccactgaggacacagctctgtgttgctgtgcaagaga |
| SEQ ID NO. 30 IGHV2-35 (**P**) |
| >IGHV2-35*01\|Bos taurus_Holstein\|P\|V-REGION\| |
| cccagatgcactgctgcagtcgggccagaactggtgaagccctcactgactgtctcctca cttgcactgtctctgcttactccatcacgtggttattgtgggaactggattcgccaggcc ccagggaaggggctagagcagatgacatgcataaactatgatggtgacacttactacagc ccctccatcaagagccacatctccatctgcagggacatccaagaatcagttctccctgca gctgagctctgtgaccactgaggacacggccatgtgttgctgtgcaagaga |
| SEQ ID NO. 31 IGHV2-36 (**P**) |
| >IGHV2-36*01\|Bos taurus_Holstein\|P\|V-REGION\| |
| caggtgaaggtgcaggagtcgggcccagaactggtgaagccctcgcagatcgtctccctc acatgtgctgtctctggttactccatcacgtggttatggtgggagctggatccaccaggc cccagggaaggggctagagtggatgggaagcatatattataaaggtgacacttaccacag cccttccatcaagagccacacgtccatctccaggggcacgtctaagaaccagtcctccct gcagctgagctctgtgaccactgaggacacagctgtgtattactgtgcaagaga |
| SEQ ID NO. 32 IGHV2-38 (**P**) |
| >IGHV2-38*01\|Bos taurus_Holstein\|P\|V-REGION\| |
| ccagtgcagctgcaggagtagggcccagaactgctgaagctctcactgaccctctccctc acctgtgccatctctggttgctccatcaccagtgcttactggtaaccagtactccctgca gctaagctctgtgatgaccaaggacacggcagtgtgttgctgtgcaagaga |
| SEQ ID NO. 33 IGHV2-42 (**P**) |
| >IGHV2-42*01\|Bos taurus_Holstein\|P\|V-REGION\| |
| caggtgtcctgcagcagttgggcccagaactggtgagccctcagagaccctctcctcaca tgtgctatctctgcttacaccatcatgtggttatagtcagaagtggattcgccaggccgc agggaaggggctagagcagatggcatgcataaactatgatggtgacacttactacagccc ctccattaagagccacacctccatctcagagacatccaagaatcagttctccctgctact gagctctgtgaccactgaggacatggccgtgtgttgctgtgcaagaga |
| SEQ ID NO. 34 IGHV2-45 (**P**) |
| >IGHV2-45*01\|Bos taurus_Holstein\|P\|V-REGION\| |
| caggtgtcctgcagcagttgggcccagaactggtgagccctcagagaccctctcctcaca tgtgctatctctgcttacaccatcatgtggttatagtcagaagtggattcgccaggccgc agggaaggggctagagcagatggcatgcataaactatgatggtgacacttactacagccc ctccattaagagccacacctccatctcagagacatccaagaatcagttctccctgctact gagctctgtgaccactgaggacatggccgtgtgttgctgtgcaagaga |
| SEQ ID NO. 35 IGHV2S1 [**P**] |
| >IGHV2S1*01\|Bos taurus_Hereford\|P\|V-REGION\| |
| caggtgcagctgcaggaatcaggcccggaactgataaagtcttcacagacactctccctc acctgtgctggttctggttactccatcaccagtgattatggtaggaactggatccgccag gccccagggaagggtctagagcagatgggatgcatgtattataatggtgacacttattac agcccctccatcaagagtcacacctccatctgcagagacacacccaagaaccagttctcc ctgcagctgagctctgtgcccacagaggacacagccatgtgttacttggcaagaaa |
| SEQ ID NO. 36 IGHV3-1 (**P**) |
| >IGHV3-1*01\|Bos taurus_Holstein\|P\|V-REGION\| |
| caggtgcagctggtgcagtgggggctgagctgaggaagcctggggcctcagtgaaggtgt cctgcaaggcttctggatataccttcaccgactacatgcactgggtgtgacaggcccctc agcaagagcttgaacggatggacagattgacagcaaagatggtggagcaaagtacgcaca gaagttccagggcagagtcacgttggctgcagacacgtccaccagcatcacctacgtgga gctgagcagtctgaggtctgacgacacggctgtttattactgtgtgaga |
| SEQ ID NO. 37 IGHV3-2 (**P**) |
| >IGHV3-2*01\|Bos taurus _Holstein\|P\|V-REGION\| |
| caggtgcagctggtgcagtgggggctgagctgaggaagcctggggcctcagtgaaggtgt cctgcaaggcttctggatataccttcaccgactacatgcactgggtgtgacaggcccctc agcaagagcttgaacggatggacagattgacagcaaagatggtggagcaaagtacgcaca gaagttccagggcagagtcacgttggctgcagacacgtccaccagcatcacctacgtgga gctgagcagtctgaggtctgacgacacggctgtttattactgtgtgaga |
| SEQ ID NO. 38 IGHV3-3 (**P**) |
| >IGHV3-3*01\|Bos taurus_Holstein\|P\|V-REGION\| |
| caggtgcagctggtgcagtgggggctgagctgaggaagcctggggcctcagtgaaggtgt cctgcaaggcttctggatataccttcaccgactacatgcactgggtgtgacaggcccctc agcaagagcttgaacggatggacagattgacagcaaagatggtggagcaaagtacgcaca gaagttccagggcagagtcacgttggctgcagacacgtccaccagcatcacctacgtgga gctgagcagtctgaggtctgacgacacggctgtttattactgtgtgaga |
| SEQ ID NO. 39 IGHV3-4 (**P**) |
| >IGHV3-4*01\|Bos taurus_Holstein\|P\|V-REGION\| |
| caggtgcagctggtgctgttggggctgagctgaggaagcctggggcctcagtgaaggtgt cctgcaaggcttctggatacaccttcaccgactactacacgcactaggtgtgacaggccc ctcagcaagggcttgaacagatggacagattgacagcaaagatggtggagcaaagtacgc acagaagttccagggcagagtcacgttggctgcagacacgtccaccagcaccgccgacat ggagctgagcagtctgaggtctgaggacacggctgtttattactgtgtgagaca |
| SEQ ID NO. 40 IGHV3-8 (**P**) |
| >IGHV3-8*01\|Bos taurus_Holstein\|P\|V-REGION\| |
| caggtgcagctggtgccgttggggctgagctgaggaagcctggggcctcagtgaaggtgt cctgcaaggcttctggatacaccttcaccgactacatgcactgcgtgtgacaggcccctc agcaagggcttgaacggatggatggactgaccctcaacatggtggaactaggtacaccta gaagttccagagcagagtgatcttggttgcacacacgcccaccaacaccgcctacatgga gcagagcagtctgagtctgaggacacggctgtttattactgtgtgaggta |
| SEQ ID NO. 41 IGHV3-11 (**P**) |
| >IGHV3-11*01\|Bos taurus_Holstein\|P\|V-REGION\| |
| cagttgcagctggtgccattggggctgagctgaggaagcctggggcctcagtgaaggtgt cctgcaaggcttctggatacaccttcaccgactacatgcactgggtgtgacaggcccctc aacaagggcttgaaaggatggacagattgacagcaaagatggtggagcaaagtatgcaca gaagttccagggcagagtcacgttggctgcagacacgtccaccagcaccgcctacatgga gctgagcagtctgaggtctgaggacacggctgtttattactgtgtgagaca |
| SEQ ID NO. 42 IGHV3-15 (**P**) |
| >IGHV3-15*01\|Bos taurus_Holstein\|P\|V-REGION\| |
| caggtgcagctggtgctgttgtggctgagctgaggaagcctggggcctcagtgaaggtgt cctgcaaggcttctggatacaccttcaccgactacatgcactgggtgtgacaggcccctc agcaagggcttgaagggatggacagattgacagcaaagatggtggagcaaagtatgcaca gaagttccagggcagagtcacgttggctgcagacacgtccaccagcactgcctacatgga gctgagcagtctgaggtctgaggacatggctgtttattactgtgtgagaca |
| SEQ ID NO. 43 IGHV3-18 (**P**) |
| >IGHV3-18*01\|Bos taurus_Holstein\|P\|V-REGION\| |
| caggtgcagctggtgcagtgggggctgagctgaggaagcctggggcctcagtgaaggtgt cctgcaaggcttctggatacaccttcaccgactacatgcactgcgtgtgacaggcccctc agcaagggcttgaacggatggatggactgaccctcaacatggtggaactaggtacaccta gaagttccagagcagagtgatcttggttgcacacacgtccaccaacaccgcctacatgga gcagagcagtctgaggtctgaggacacggctgtttattactgtgtgagg |
| SEQ ID NO. 44 IGHV3-22 (**P**) |
| >IGHV3-22*01\|Bos taurus_Holstein\|P\|V-REGION\| |
| caggtgcagctggtgccattggggctgagctgaggaagcctggggcctcagtgaaggtgt cctgcaaggcttctggatacaccttcaccgactacatgcactgcatgtgacaggcccctc agcaagggcttgaaaggatggacagattgacagcaaagatggtggagcaaagtatgcaca |
| gaagttccaggccagagtcaccttggttgcagacccgtgcacctgcactgcctacatgga gctgagcagtctgaggtctgaggacacggctgtttattactgtgtgagaca |
| SEQ ID NO. 45 IGHV3-28 (**P**) |
| >IGHV3-28*01\|Bos taurus_Holstein\|P\|V-REGION\| |
| caggtgcagctggtgcagtgggggctgagctgaggaagcctggggcctcagtgaaggtgt cctgcaaggcttctggatacaccttcaccgactacatgcactgcgtgtgacaggcccctc agcaagggcttgaacggatggatggactgaccctcaacatggtggaacaaggtacaccta gaagttccagagcagagtgatcttggttgcacacacgcccaccaacaccgcctacatgga gcagagcagtctgagtctgaggacacggctgtttattactgtgtgaggta |
| SEQ ID NO. 46 IGHV3-34 (**P**) |
| >IGHV3-34*01\|Bos taurus_Holstein\|P\|V-REGION\| |
| caggtgcagctggtgccattggggctgagctgaggaagcctggggcctcagtgaaggtgt cctgcaaggcttctggatacaccttcaccgactacatgcactgcatgtgacaggcccctc agcaagggcttgaaaggatggacagattgacagcaaagatggtggagcaaagtatgcaca gaagttccagggcagagtcaccttggttgcagacccgtgcacctgcactgcctacatgga gctgagcagtctgaggtctgaggacacggctgtttattactgtgtgagaca |
| SEQ ID NO. 47 IGHV3-40 (**P**) |
| >IGHV3-40*01\|Bos taurus_Holstein\|P\|V-REGION\| |
| caggtgcagctggtgccgttggggctgagctgaggaagcctggggcctcagtgaaggtgt cttgcaaggcttctggatacaccttcaccgactacatgcactgcatgtgacaggcccctc agcaagggcttgaacggatggatagattgacagcaaagatggtggagcaaagtatgcaca gaagttccagggcagagtcacgttgctgcagacacgtccaccagcaccgcctacatggag ctgagcagtctgaggtctgaggacacggctgtttattattgtgtgagaca |
| SEQ ID NO. 48 IGHV3-41 (**P**) |
| >IGHV3-41*01\|Bos taurus_Holstein\|P\|V-REGION\| |
| caggtgcagctggtgcagtgggggctgagctgaggaagcctggggcctcagtgaaggtgt cctgcaaggcttctggatacaccttcaccgactactacacgcactaggtgtgacaggccc ctcagcaagggcttgaacagatggacagattgacagcaaagatggtggagcaaagtacgc acagaagttccagggcagagtcacgttggctgcagacacgtccaccagcaccgccgacat ggagctgagcagtctgaggtctgaggacacggctgtttattactgtgtgagaca |
| SEQ ID NO. 49 IGHV3-44 (**P**) |
| >IGHV3-44*01\|Bos taurus_Holstein\|P\|V-REGION\| |
| caggtgcagctggtgcagtgggggctgagctgaggaagcctggggcctcagtgaaggtgt cctgcaaggcttctggatacaccttcaccgactacatgcactgcgtgtgacaggcccctc agcaagggcttgaacagatggacagattgacagcaaagatggtggagcaaagtatgcaca gaagttccagggcagagtcacgttggctgcagacacgtccaccagcaccgccgacatgga gctgagcagtctgaggtctgaggacacggctgtttattactgtgtgagaca |
| SEQ ID NO. 50 IGHV3-47 (**P**) |
| >IGHV3-47*01\|Bos taurus_Holstein\|P\|V-REGION\| |
| caggtacagttggggctgagctaaggaagcctggggcctcagtgaaggtgtcctgcaagg cttctggatacaccttcaccaactactacatgcactgggtgtgacaggaccctcagcaat ggcttgaatggatggacagattgacagcaaagatggtggagcaaagtatgcacagaagtt ccagggcagagtcacgttggctgcagacacgtccaccagcaccgcctacatggagctgag cagtctgaggtctgaggacacggctgttattactgtgtgaaaca |

| **Germline D sequences** |
|---|
| SEQ ID NO. 51 IGHD1-1 (**F**) |
| >IGHD1-1*01\|Bos taurus_Holstein\|F\|D-REGION\| |
| agaataccgtgatgatggttactgctacacc |
| SEQ ID NO. 52 IGHD2-1 (**F**) |
| >KT723008\|IGHD2-1*01\|Bos taurus_Holstein\|F\|D-REGION\|286128..286143\|16 nt\|1\| \| \| \| 116+0=161 \| \| |
| ttactatagtgaccac |
| SEQ ID NO. 53 IGHD3-1 (**F**) |
| >IGHD3-1*01\|Bos taurus_Holstein\|F\|D-REGION\| |
| gtattgtggtagctattgtggtagttattatggtac |
| SEQ ID NO. 54 IGHD4-1 (**F**) |
| >IGHD4-1*01\|Bos taurus_Holstein\|F\|D-REGION\| |
| gtagttatagtggttatggttatggttatagttatggttatac |
| SEQ ID NO. 55 IGHD5-2 (**F**) |
| >IGHD5-2*01\|Bos taurus_Holstein\|F\|D-REGION\| |
| atgatacgataggtgtggttgtagttattgtagtgttgctac |
| SEQ ID NO. 56 IGHD6-2 (**F**) |
| >IGHD6-2*01\|Bos taurus_Holstein\|F\|D-REGION\| |
| gtagttgttatagtggttatggttatggttgtggttatggttatggttatgattatac |
| SEQ ID NO. 57 IGHD7-3 (**F**) |
| >IGHD7-3*01\|Bos taurus_Holstein\|F\|D-REGION\| |
| gtagttatggtcgttatggttatggtggttatggttgttatggttatggttatggttatg gttatac |
| SEQ ID NO. 58 IGHD8-2 (**F**) |
| >IGHD8-2*01\|Bos taurus_Holstein\|F\|D-REGION\| |
| gtagttgtcctgatcgttatagttatggttatggttgtggttatggttatggttgtagtg gttatgattgttatcgttatggtggttatggtggttatggtggttatggttatagtagtt atagttatagttatacttacgaatatac |
| SEQ ID NO. 59 IGHD9-1 (**F**) |
| >IGHD9-1*01\|Bos taurus_Holstein\|F\|D-REGION\| |
| gaactcggtggggc |

| **Germline J_{H} sequences** |
|---|
| SEQ ID NO. 60 IGJ1-1 (**ORF**) |
| >IGHJ1-1*01\|Bos taurus_Holstein\|ORF\|J-REGION\| |
| actatgcagacttccatctctggagccaggctgccctgggcaccgtctcctcag |
| SEQ ID NO. 61 IGJ1-2 (**ORF**) |
| >IGHJ1-2*01\|Bos taurus_Holstein\|ORF\|J-REGION\|\| |
| ctgctgggacttggatctctggggccagcgcaccccggtcaccatgtccttgggga |
| SEQ ID NO. 62 IGJ1-3 (**ORF**) |
| >IGHJ1-3*01\|Bos taurus_Holstein\|ORF\|J-REGION\| |
| caatgcttttgactcctggggccagcgcacccccatctccatctcctcag |
| SEQ ID NO. 63 IGJ1-4 (**F**) |
| >IGHJ1-4*01\|Bos taurus_Holstein\|F\|J-REGION\| |
| actattcgacaactggggcccaggaatccaaaacaccgtctcctcag |
| SEQ ID NO. 64 IGJ1-5 **(P)** |
| >IGHJ1-5*01\|Bos taurus_Holstein\|P\|J-REGION\| |
| taacaactggctcaagcactggggtcaggaagcctgggcactgtctgctc |
| SEQ ID NO. 65 IGJ1-6 (**F**) |
| >IGHJ1-6*01\|Bos taurus_Holstein\|F\|J-REGION\| |
| tttactatggtatagacgcctggggccgagggctcagggtcaccgtctcctcag |
| SEQ ID NO. 66 IGJ2-1 (**ORF**) |
| >IGHJ2-1*01\|Bos taurus_Holstein\|ORF\|J-REGION\| |
| actatgctgacttccatctctgtggccaggctgccctgggcaccgtctcctcagctgaat ctg |
| SEQ ID NO. 67 IGJ2-2 (**ORF**) |
| >IGHJ2-2*01IBos taurus_Holstein\|ORF\|J-REGION\| |
| ctgctgggacatggatctctggggccagcgcaccccggtcaccgtgtccttgggga |
| SEQ ID NO. 68 IGJ2-3 (**ORF**) |
| >IGHJ2-3*01\|Bos taurus_Holstein\|ORF\|J-REGION\| |
| caatgcttttgactcctggggccagcgcgccccggtctccatctcctcag |
| SEQ ID NO. 69 IGJ2-4 (**F**) |
| >IGHJ2-4*01\|Bos taurus_Holstein\|F\|J-REGION\| |
| actacgtcgatgcctggggccaaggactcctggtcaccgtctcctcag |
| SEQ ID NO. 70 IGJ2-5 (**P**) |
| >IGHJ2-5*01\|Bos taurus_Holstein\|P\|J-REGION\| |
| taacaactggctcaagcactggggtcgggaagcctgggcactgtctgctc |
| SEQ ID NO. 71 IGJ2-6 (**ORF**) |
| >IGHJ2-6*01\|Bos taurus_Holstein\|ORF\|J-REGION\| |
| attactatagtatatatgtttgcggccgagggatcgaggtcaccgtctcctcag |

**Table 2. Bovine IGK locus**

| **Germline V_{K} sequences** |
|---|
| SEQ ID NO. 72 IGKV1-1 **(ORF)** |
| >IGKV1-1*01\|Bos taurus_Hereford\|ORF\|V-REGION\| |
| gacatccaggtaacccagtctccatcctccttgtctgcatctctaggagacagagtctcc atcacttgccaggccagtcagagcattgacactaaattagcctggtatcaacagaaacca gggaaagctcctaagctcctcatctatgcaatacccaggtcgccttcctggttcccttcc cagttcagtggcagtggatttggggcagatttcaccctcaccatcagcagtctgaaggct gatgatattgcaacttactactgtcagcaggatcacggtttacctac |
| SEQ ID NO. 73 IGKVI-4 (**F**) |
| >IGKV1-4*01\|Bos taurus_Hereford\|F\|V-REGION\| |
| gacatccaggtgacccagtctccctcctacctgtctgcttctctaggagacagagtctcc atcacttgccaggccaatcagagcgttagccactacttaaactggtatcaacagaaaccag gggaagctcctaagctcctgatctattatgcaaccagccggtacaccagagtcccatcc cgattcagtggcagtggatctgggacagatttcaccctcaccatcagcagcctggaggct gacgatgctgcaaattattactgtcaacaggattatagtacacctcc |
| SEO ID NO. 74 IGKV1=5 (**P**) |
| >IGKV1-5*01\|Bos taurus_Hereford\|P\|V-REGION\| |
| ggcatccagatgactcagtctccatcctccttgtctgcctctctaggagacagagcatcc atcatttgctgggccaattaaagcattagcaaatggttagacagatatgagcagaaacca gggcaagttcctaagctcctgttctatgcagcatccaatttgggaactggggtcccatcc cggttcagtggcagtagatctgggacagatttcactgtcaccactctcacctggaggctg aagatgctgcaacttattactgtctacaaactgatagtacacctcc |
| SEQ ID NO. 75 IGKVI-21 (**P**) |
| >IGKV1-21*01\|Bos taurus_Hereford\|P\|V-REGION\| |
| gacctccagatgattcagtctctatcttccctgtctgcatctctaggagacagagtctcc atcacttgtcgggccagtcaaagcgttagaaataatttacagtggtatcaagagaaacca gggaaagctcctaagttcctcatctatgacagaaccagtgtgtacacaggggtcccatcc catttcagtggcagtggatctgggacagattataccttcaccaccagcagcctgggctga tgattttgcaacttattactgtaaacaggataacagtagacctcc |
| SEQ ID NO. 76 IGKVI-25 (**P**) |
| >IGKV1-25*01\|Bos taurus_Hereford\|P\|V-REGION\| |
| gacctccagatgacccagtctccatcctccctgtctgcatctctaggagacagaatctcc atcacttgccgggccagagcgttagcaactacttagcctggtatcaacagaaaccaggga aagctcctaagctcctgatctatcacgaatccagattgcacacaggggtcccatcccggt tcagtggcagtggatgtgggacagattacaccctcaccatcagcaggctggagtctgacg atgctgcaaattattactgtcagcagtataatagtacacctcc |
| SEQ ID NO. 77 IGKV2-6 (**F**) |
| >IGKV2-6*01\|Bos taurus_Hereford\|F\|V-REGION\| |
| gatgttgtgctgacccagactccactctccctgtctatcatccctggagagatggcctcc atctcctgcaagtctagtcagagcctggtacacagtgatggaaaaacctatttgaattgg attcaatataaaccaggccaatcaccacagggtctgatctatcaggtttccaaccgttac tctggggtctcagacaggttcactggcagtgggtcagggacagatttcacacttacaatc agcagagtgcaggctgaggatgctggagtctattactgttaccaaggtacagaagatcct cc |
| SEQ ID NO. 78 IGKV2-7 (**P**) |
| >IGKV2-7*01\|Bos taurus_Hereford\|P\|V-REGION\| |
| gctactatgcagacccagactctacgctccctgtctgtcatcccgggagagatggcctcc atctcctgcagagccagtcagagcgttcaaaatagatatggagacaattttttgcactgg tatgtgcagaagcccagccagtctccacagctcctgatttatagggcttctaactgggag tcttgggtcccagacaggttcacaagcagtgggttgggaacacatttcatcctcataatc agcagggtggagtctgaggatgctggagtttattactgccagcagagtttacaagcacct cc |
| SEQ ID NO. 79 IGKV2-9 (**F**) |
| >IGKV2-9*01\|Bos taurus_Hereford\|F\|V-REGION\| |
| gatgttgtgctgacccagactcccctctccctgtctgtcatccctggagagacggtcacc atctcctgcaagtctactcagagtctgaaatatagtgatggaaaaacgtatttgcaatgg tttcaacataaaccaggccagtctccacggctattgatctatcagatttccaaccgttac actggggtcccagacaggttcactggcagtgggtcagagacagatttcacacttacaatc agcagtgtgcaggctgaggatgctggagtctattactgtcttcaaagatcatatgctcct cg |
| SEQ ID NO. 80 IGKV2-10 **(P)** |
| >IGKV2-10*01\|Bos taurus_Hereford\|P\|V-REGION\| |
| ggctattatgcagaaccaactatacgctccctgtctgtcatccctggagagactgccttc atctcctgcagagccagtcagagcattcaatatagatagggagacaatttttttgcactg gtatgtgcagaagcccagccagtctccacagctcctgatttatagggcttctaactggga gtcttgggtcccagacaggttcacaagcagtgggttgcgaacagatttcatcctcataat cagcagggtggaggctgaggatgctggagtttattactgccagcagagtttacaagcacc tcc |
| SEQ ID NO. 81 IGKV2-12 (**ORF**) |
| >IGKV2-12*01\|Bos taurus_Hereford\|ORF\|V-REGION\| |
| gatgttgtgctgacccagactcccctctccctgtctgtcatccctggagagacggtctcc atctcctgcaagtctactcagagtctgaaatacagtgatggaaaaacctatttgtaccgg cttcaacataaaccaggccaatcaccacagagtttgatctattatgtttccaaccgttac actggggtcccagacaggttcactggcagtgggtcagagacagatttcacacttacgatc agcagtgtgcaggctgaggatgctggagtctattactgttttcaaggcacacaggttcct cc |
| SEQ ID NO. 82 IGKV2-13 (**P**) |
| >IGKV2-13*01IBos taurus_Hereford\|P\|V-REGION\| |
| gctataaggcagacccagactctacgctccctgtctgtcatccctggagagacggcctcc gtctcctgcagagccagtcagagcgttcaaaatagatatggagacaatttttttgcattg gtatgtgcagaagcccagccagtctccacagctcctgatctatggagcttctaaccaggc ctctggagtctgagacaggttcactggcagtgggtcagggacagacttcaccctcaaaag cagcagggtcggggctgaggatgctggagtttattattgccaccaaagtaaagaaactcc tcc |
| SEQ ID NO. 83 IGKV2-15 (**F**) |
| >IGKV2-15*01\|Bos taurus_Hereford\|F\|V-REGION\| |
| gatgttgtgctgacccagactcccctctccctgtctgtcatccctggagagacggtctcc atctcctgcaagtctactcagagtctgaaatatagtgatggaaaaacctatttgcgttgg gttcaacataaaccaggccaatcaccacagggtgtgatctatcaggtttccaaccgtaat actggggtcccagacaggttcactggcagtgggtcagaaacagatttcacacttacaatc agcagtgtgcaggctgaggatgctggagtctattactgttttcaaggtacatatgaacct cc |
| SEQ ID NO. 84 IGKV2-16 (**P**) |
| >IGKV2-16*01\|Bos taurus_Hereford\|P\|V-REGION\| |
| gctataatgcagacccagactctacgctccctgtctgtcatccctggagagacggcctcc atctcctgcagagccagtcagagcgttcaaaatagatatggagacaatttttttgcattg gtatgtgcagaagcccagccagtctccacagctcctgatctatggagcttttaaccaggc ctctggagtctgagacaggttcaccggcagtgggtcagggacagatttcaccttcaaaac cagcagggtgggggctgaggatgctggagtttattattgccaccaaagtaaagaaactcc tcc |
| SEQ ID NO. 85 IGKV2-18 (**F**) |
| >IGKV2-18*01\|Bos taurus_Hereford\|F\|V-REGION\| |
| gatgttgtgctgacccagactcccctctccctgtctgtcatccctggagagacggtctcc atctcctgcaagtctactcagagtctgaaatatagtggaaaaacctatttgcgttggctt caacataaaccaggccaatcaccacagagtctgatctatcaggtttccaaccgttacact ggggtcccagacaggttcactggcagtgggtcagagacagatttcacacttacgatcagc agtgtgcaggctgaggatgctggagtctattactgtgttcaagagacacatgatcctcg |
| SEQ ID NO. 86 IGKV2-19 (**P**) |
| >IGKV2-19*01\|Bos taurus_Hereford\|P\|V-REGION\| |
| gctattgttctgacccagactccacgctccctgtctgtcatccctggagagacggcctcc atctcctgcagatccagtcagagcattcaaaatagatatggagacaatcttttgcactgg tatgtgcagaagcccagccagtctccacagctcctgatctatggaacttctaaccaggcc tctggagtctgagacaggttcaccggcagtgggtcagggacagatttcaccctcaaaacc agcagggtggaggctgaggttgctggagtttattattgcaggaaagtaaagaaactcctt c |
| SEQ ID NO. 87 IGKV2-22 (**P**) |
| >IGKV2-22*01IBos taurus_Hereford\|P\|V-REGION\| |
| gatgtagtgctgacccagactccactctccttgtctgtcatccctggtgagacggcctcc atctcctgcaagtcaagtcagagcctcctgcatagtgatggaaaaacctatttgaactgg tttcaacataaaccaggccagtctccacagtgactgatctatcaggcttccaaccgtgac actggggtctcagagaggttcactggcagtgggtcagggacagatttcacacttaaaatc agcagagtgcagattgagtatgctggcatctattactgttttcagcatacatatgatcct cc |
| SEQ ID NO. 88 IGKV2-23 (**P**) |
| >IGKV2-23*01\|Bos taurus_Hereford\|P\|V-REGION\| |
| gacattgtgctgacccagactccactctccttgtctgtcttccctggagagacggcgtcc atctcctgcagaaccagtcagagccttgaagatagttatggagacacttatttgagttgg cacttgcagaagcctagccagtctccacagcttctgatctatttggtttccaatagagcc tctggggtcccagacaggttcactggcagtgggtcaggaacagatttcacctttaaaatc agcagggtggaggctgaggatgctggagtttattactgccaacagagtatacaagcacct cc |
| SEQ ID NO. 89 IGKV3-24 (**P**) |
| >IGKV3-24*01\|Bos taurus_Hereford\|P\|V-REGION\| |
| gaaattatgttaacgcagtctccagcctccctgtctttgactccaggggaaatagccacc ctcacctgcagggccagtcaaagtattagtagctacttaagactggtaccagcagaagcc tgggaaggcccccagattcttcatctatgctgcctccagcagggcctctggcatcccagc ccggttcggtgacagttgggtcagggacagactttactctcaccatcagcagcctggagc ctgaagatgctgcagttcattcctagtgctagcataatagcgggaa |
| SEQ ID NO. 90 IGKV8-3 (**F**) |
| >IGKV8-3*01\|Bos taurus_Hereford\|F\|V-REGION\| |
| gaggctgtgctctaccagactccagcctacatcgctgcatccctaggagagagcatctcc atcacttgcagagccaatcaaagcattagtgattacttaagctggtataagcagaaacct ggccaggctcctatgattctcatctatgatgctgataatcgttataatggtgtcccagag aggttcactgcgactcaatctgagacagaatttgttttcacaatcagccaagtagaggct gatgatgctgccatgtattactgccagcaggattatgcacttcctcc |
| SEQ ID NO**.** 91 IGKV(II)-2 (**P**) |
| >IGKV(II)-2*01\|Bos taurus _Hereford\|P\|V-REGION\| |
| gacatcatgatcacccagtctcgaagttccttggctgagtctgcaggagaggaaggtcat catcatctacagatccagccagaatcttctatacttcaaccagaaaacctagtttgcctg gtaccagcagaaactgtg |
| SEQ ID NO**.** 92 IGKV(II)-8 (**P**) |
| >IGKV(II)-8*01\|Bos taurus_Hereford\|P\|V-REGION\| |
| gctgctgtactgactccatctcctccctctggcctgtggccatggagggagcgtcgccat ctcctgaaggccatctcttcccatccacagtaatggacacacctgcccgccgtgattcgg gcacaaaccacacactctcctcaaccactgatcatagggattcccaggctcctgaggtcc cagcctggctcagtgattgtgggtctggatggatttcacacttacactcaccagggtggc gtcccttcatgtgacttctgtgtacagaaccctcactgggaccac |
| SEQ ID NO. 93 IGKV(II)-11 (**P**) |
| >IGKV(II)-11*01\|Bos taurus_Hereford\|P\|V-REGION\| |
| actgctgtgctgactccatctctccctctggcctgtggccatggagggagcgtcgccatc tcctgaaggccatctcttctcatccacagtaatggacacaccttcccgccatgcttctgg cacaaaccacacactctcctcaaccactgaccatagagggtcaccaggctcttgaggtcc cagcctggctcagtgattgtgggtctggatagatttcacactcacactcaccagggtggg gtccctacatgtgacttctgtgcacagaaccctcacttggaccac |
| SEQ ID NO. 94 IGKV(II)-14 (**P**) |
| >IGKV(II)-14*01\|Bos taurus _Hereford\|P\|V-REGION\| |
| gctgctgtgctgacttcatctctccctctggcctgtggccatggagggagcatcgccatc tcctgaaggccatctcttctcatccacagtaatggacacacctgcccgccatgcttctgg caaaaaccacacactctcctcaaccactgatcataggtggtccccaggcttcaggggtcc aagcctggctcagtgactgtgggtctgggacggatttcacacttacacttaccagggtgg ggtccctacatgtgacttctgtgcacagaaccctcactgggaccac |
| SEQ ID NO. 95 IGKV(II)-17 (**F**) |
| >IGKV(II)-17*01\|Bos taurus_Hereford\|P\|V-REGION\| |
| gctgaggagactccatctcctccctctggcctgtggccatggagggagcgccgccatctc ctgaaggccatctcttctcatccacagtaatggacacacctgcccaccatgtttccggca caaaccacacactctcctcaaccactgatcatagggcttccccaggcttcaggggtccca gcctggctcagtgactgtgggtctgggatggatttcacacttacactcaccagggtgggg tctctacatgtgacttctgtgcacagaaccctcactgggaccac |
| SEQ ID NO. 96 IGKV(II)-20 (**P**) |
| >IGKV(II)-20*01\|Bos taurus_Hereford\|P\|V-REGION\| |
| gctgctgtgctgactccgtcccctccctctggtctgcagtcgtggaaagaggcatctcca tctcctgcaaggccatcccatctcatccatagaaatgaatacacctgtcctccctccttc cagaacaaaccacacactctcctcacccattgacataggggtcccccaggctcctggggt |
| cccatcctggctcagtggccatggatctgggacggatttcacactcacactcaccactgt ggagactggggaccctgaatgtgacttctgtgcacagagccttcaatgggaact |

| **Germline J_{K} sequences** |
|---|
| SEQ ID NO. 97 IGKJI (**ORF**) |
| >IGKJ1*01\|Bos taurus_Hereford\|ORF\|J-REGION |
| atggacgttaggtcaaggaaccaagctggaagtcaaac |
| SEQ ID NO. 98 IGKJ2 (**F**) |
| >IGKJ2*01\|Bos taurus_Hereford\|F\|J-REGION |
| taaatactttcggccaaggaaccaaggtagagatcaaaa |
| SEQ ID NO. 99 IGKJ3 (**F**) |
| >IGKJ3*01\|Bos taurus_Hereford\|ORF\|J-REGION |
| gttcactttcgggccaaggaccagagtggagatcaaat |
| SEQ ID NO. 100 IGKJ4 (**ORF**) |
| >IGKJ4*01\|Bos taurus_Hereford\|ORF\|J-REGION\| \| |
| aattacgttcggcggcgggaccaaggtggaaatcaatc |
| SEQ ID NO. 101 IGKJ5 (**ORF**) |
| >IGKJ5*01\|Bos taurus_Hereford\|ORF\|J-REGION\| |
| gatcatctttggccaagggacacgtctggagattagac |

**Table 3. Bovine IGL locus**

| **Germline V_{λ} sequences** |
|---|
| SEQ ID NO. 102 IGLVI-12 (**F**) |
| >IGLV1-12*01\|Bos taurus_Hereford\|F\|V-REGION\| |
| caggctgtgctgactcagccgccctccgtgtccgggtccctgggccagacggtcaccatc tcctgcaccggaagcagcaacaacatcgggattttgggtgtgagctggtaccaacagatc ccaggatcggcccccagaaccctgatctataatagtaacaaacgaccctcgggggtcccc gaccgattctctggcaccaagtccggcaacacaggcaccctgaccatcgcttcgctccag gctgaggacgaggctgattattactgtgcgtctgctgacctcagtcttactagtcc |
| SEQ ID NO. 103 IGLV1-14 (**P**) |
| >IGLV1-14*01\|Bos taurus_Hereford\|P\|V-REGION\| |
| caggctgtgctgactcagcagccctccgtttccgggtccctgggccagagggtctccatc tcctgctctggaagcagcagcaacattgggcatggttatggtacctggtaccaacagatc ccaggatcggcccccagaatgctcatctatggtgcgaccagtcgagcctccggtccccga ccgattctccggctccaggtctggcaacacagcgactctgaccatcagctcgctccaggc tgaggatgaggcggattatttctgtgcagcttatgacagtagcagtggtcc |
| SEQ ID NO. 104 IGLV1-16 (**P**) |
| >IGLV1-16*01\|Bos taurus_Hereford\|P\|V-REGION\| |
| caggatgtgctgactcagccgtcctccgtgtccgggtccctgggtcagaaggtctccatc acctgctctggaagcagcagcaacgttggatatgccaattatgtgagctggcaccaacag aaaccaggatcggcccccagaaccctcatctatggtgcgaccagtcgagcctcgggggtc cctgaccaattctccggctccaagtctggcaacacagcgactctgaccatcagctcgctc cagcctgaggacgaggctgattattactgttcatcttatgacagtagcagcaatattgg |
| SEQ ID NO. 105 IGLV1-17 (**P**) |
| >IGLV1-17*01\|Bos taurus_Hereford\|P\|V-REGION\| |
| caggctgtgctgactcagccgccctccgtgtctgggactttgggtcagagggtcaccctc tcctgcactggaagcagcagcaacactggggtctttatgtgagctggtaccaacagctcc |
| caggaaggcccccagagtcctgacctatgaaaatagcaaacgaacctcaggactcccaga ttgattctctgcctccaagtctggcagctcggccgcactgaccacctcagttcagtctga ggacgacactgattattactgtttctcatgggctgatggattgaaagtttg |
| SEQ ID NO. 106 IGLV1-18 (**P**) |
| >IGLV1-18*01\|Bos taurus_Hereford\|P\|V-REGION\| |
| caggctgtgctgactcagccgccctccgtgtccaggactttgggtcagagggtcaccctc tcctgcactggaagcagcaacaaccctggggttattctgtgagctggtaccaacagctcc caggaaaggcccccacacttctcatctatgaaaatagcacacgaccctcagcactcccag attgattctctggctccaagtctggcaagtcggtctctctgaccacctcagttcagtctg aggacgacactgattattactgtttctcatgggctgacggattgaaagtttg |
| SEQ ID NO. 107 IGLV1-21 (**F**) |
| >IGLV1-21*01\|Bos taurus_Hereford\|F\|V-REGIONI |
| caggctgtgctgactcagccgtcctccgtgtccggctccctgggccagagggtctccatc acctgctctggaagcagcaacaacatcggtagttatggtgtgggctggtaccaacaggtc ccaggatcgggcctcagaaccatcatctatggtagtagcagtcgaccctcgggggtcccc gaccgattctccggctccaagtctggcaacacagccaccctgaccatcagctcgctccag gctgaggacgaggcggattatttctgtgcaactgttgactacagtagcagtactgt |
| SEQ ID NO. 108 IGLV1-22 (**P**) |
| >IGLV1-22*01\|Bos taurus_Hereford\|P\|V-REGION\| |
| caggctatgctgactcagctgccctccatgtccgggactttgggtcagagggtcaccctc tcctgcactgaaagcagcagcaaccctggggtctttatgtgagctggtaccaacagctcc caggaaaggcccccagagtcctgacctgtgaaaatagcaaacgaccctcaggggtcccag attgattctctggctccaagtctggcaacagcctctcttaccacctcagttcatgctgag gatactgattactactgtttctcatgggctgacggcttgaaagtttg |
| SEQ ID NO. 109 IGLV1-26 (**F**) |
| >IGLV1-26*01\|Bos taurus\|F\|V-REGION\| |
| caggctgtgctgactcagccgtcctccgtgtccgggtccctgggccagagggtctccatc acgtgctctggaagcagcagcaacgttggatatggcaattatgtgagctggttccaagac atcccaggatcggcccccagaaccctcatctatggtgacaccagtcgagcctcgggggtc cccgaccgattctccggctccaggtctgggaacacagccaccctgaccatcagctcgctc caggctgaggacgaggcagattatttctgtgcatcttatcagagtggtaacacag |
| SEQ ID NO**.** 110 IGLV1-28 (**P**) |
| >IGLV1-28*01\|Bos taurus_Hereford\|P\|V-REGION\| |
| caggctgtgctgactcagccatcatccgtgtccgggtccctgggccagagggtctccatc acctgctctggaagcagcagcaatgttggaaatggatatgtgagctggtaccaactgatc ccaggatcggcccccagaaccctcatctatggtgacaccagtcgagcctcgggggtcccc gaccgattctccggctccaggtctggaacacagccaccctgaccatcagctcgctccagg ctgaggacgaggcagattatttctgtgcatctgctgaggatagtagcagtaatgc |
| SEQ ID NO. 111 IGLV1-29 (**P**) |
| >IGLV1-29*01\|Bos taurus_Hereford\|P\|V-REGION\| |
| caggctatgctgactcagccgccctccatgttcgggactttgggtcagagggttaccctc tccttcactggaaggagcagcaacactgggggtctttatgtgagctggtaccaacagctc ccaggaaaggcccctagactcctgacatatgaaaatagcaaacgaccctcaggggtccca gattgattctctggctccaagcctgccaactcagcctctctgaccacctcagttcatgct gaacgatactgattattactgtttctcatgggctgatggcttgaaagtttg |
| SEQ ID NO. 112 IGLV1-31 (**F**) |
| >IGLV1-31*01\|Bos taurus\|F\|V-REGION\| |
| caggctgtgctgactcagccgtcctccgtgtccgggtccctgggccagagggtctccatc acctgctctggaagcagcagcaacgttggaactggcaattatgtgagctggttccaacag atcccaggatcggcccccagaaccctcatctatggtgcgaccagtcgagcctcgggggtc cccgaccgattctccggctccaggtctgggaacacagccaccctgaccatcagctcgctc caggctgaggacgaggcagattatttctgtgcatcttatcagagtggtaacacag |
| SEQ ID NO. 113 IGLV1-26 (**ORF**) |
| >IGLV1-36*01\|Bos taurus\|ORF\|V-REGION\| |
| caggctgtgctgactcagccgtcctccatgtccgggtccctgggccagagggtctccatc accagctctggaagcagcagcaacgttggatatggcatttatgtgaaccagtaccaaaaa atcccaggatcggcccccagaaccctcatctatggtgccaccagtcgagcctcgggggtc cccgaccgattctccggctccaggtctgggaacacagccaccctgaccatcagctcgctc caggctgagaacgaggcagattatttctgtgcagcttatgacagcagtagcagtgatgg |
| SEQ ID NO. 114 IGLV1- 40 (**F**) |
| >IGLV1-40*01\|Bos taurus\|F\|V-REGION\| |
| caggctgtgctgactcagccgtcctccgtgtccgggtccctgggccagagggtctccatc acctgctctggaagcagcagcaacgttgggcttggtaattatgtgagctggttccaacag atcccaggatcggcccccagaaccctcatctatggtgcgaccagtcgagcctcgggggtc cccgaccgattctccggctccaggtctgggaacacagccaccctgaccatcagctcgctc caggccgaggacgaggcggattatttctgtgcatctcctgacagtagtagcagtagtta |
| SEQ ID NO. 115 IGLV1-41 (**P**) |
| >IGLV1-41*01\|Bos taurus_Hereford\|P\|V-REGION\| |
| caggctatgctgactcagccgccctccatgttcgggactttgggtcagagggttaccctc tccttcactggaaggagcagcaacactgggggtctttatgtcagctggtaccaacagctc ccaggaaaggcccctagactcctgacatatgaaaatagcaaacgaccctcaggggtccca gattgattctctggctccaagcctggcaactcagcctctctgaccacctcagttcatgct gaacgatactgattattactgtttctcatgggctgatggcttgaaagtttg |
| SEQ ID NO. 116 IGLV1-43 (**F**) |
| >IGLV1-43*01\|Bos taurus\|F\|V-REGION\| |
| caggctgtgctgactcagccgtcctccgtgtccgggtccctgggccagagggtctccatc acgtgctctggaagcagcagcaacgttggatatggcaattatgtgagctggttccaagag atcccaggatcggcccccagaaccctcatctatggtgacaccagtcgagcctcgggggtc cccgaccgattctccggctccaggtctgggaacacagccaccctgaccatcagctcgctc caggctgaggacgaggcagattatttctgtgcatcttatcagagtggtaacacag |
| SEQ ID NO. 117 IGLV1-46 (**P**) |
| >IGLV1-46*01\|Bos taurus\|P\|V-REGION\| |
| caagctgtgctgactcaaccgccctccatgtctgggtccctgggccagagggtctccatc acctgctctggaagcagcagcaacgttggaactggcaattatgttggctggtaccaaatg atcccaggatcggcccccagaaccctcatctaccgtgctaccagtcgactctcggggtc cccgactgattctccgtctccaggtctgggaacacagccaccctgaccatcagctcgcac caggctgaggacgaggctgattattactgtgtatcttaggacagcagtatcagtggtgc |
| SEQ ID NO. 118 IGLV1-47 (**F**) |
| >IGLV1-47*01\|Bos taurus\|F\|V-REGION\| |
| caggctgtgctgactcagccatcatccgtgtccgggtccctgggccagagggtctccatc acctgctctggaagcagcagcaatgttggaaatggatatgtgagctggtaccaactgatc ccaggatcggcccccagaaccctcatctatggtgacaccagtcgagcctcgggggtcccc gaccgattctccggctccaggtctgggaacacagccaccctgaccatcagctcgctccag gctgaggacgaggcagattatttctgtgcatctgctgaggatagtagcagtaatgc |
| SEQ ID NO. 119 IGLV1-48 (**P**) |
| >IGLV1-48*01\|Bos taurus_Hereford\|P\|V-REGION\| |
| caggctatgctgactcagccgccctccatgttcgggactttgggtcagagggttaccctc tccttcactggaaggagcagcaacactgggggtctttatgtgagctggtaccaacagctc ccaggaaaggcccctagactcctgacatatgaaaatagcaaacgaccctcaggggtccca gattgattctctggctccaagcctgccaactcagcctctctgaccacctcagttcatgct gaacgatactgattattactgtttctcatgggctgatggcttgaaagtttg |
| SEQ ID NO. 120 IGLV1- 51 (**P**) |
| >IGLV1-51*01\|Bos taurus\|P\|V-REGION\| |
| caagctgtgctgactcaaccgccctccatgtctgggtccctgggccagagggtctccatc acctgctctggaagcagcagcaacgttggaactggcaattatgttggctggtaccaaatg atcccaggatcggcccccagaaccctcattgaccgtgctaccagtcgactctcaggggtc cccgactgattctccgtctccaggtctgggaacacagccaccctgaccatcagctcgcac caggctgaggacgaggctgattattactgtgtatcttacgacagcagtatcagtggtgc |
| SEQ ID NO. 121 IGLV1-52 (**F**) |
| >IMGT000046\|IGLV1-52*01\|Bos taurus\|F\|V-REGION\| |
| caggctgtgctgactcagccatcatccgtgtccgggtccctgggccagagggtctccatc acctgctctggaagcagcagcaatgttggaaatggatatgtgagctggtaccaactgatc ccaggatcggcccccagaaccctcatctatggtgacaccagtcgagcctcgggggtcccc gaccgattctccggctccaggtctgggaacacagccaccctgaccatcagctcgctccag gctgaggacgaggcagattatttctgtgcatctgctgaggatagtagcagtaatgc |
| SEQ ID NO. 122 IGLV1-53 (**P**) |
| >IGLV1-53*01\|Bos taurus_Hereford\|P\|V-REGION\| \| |
| caggctatgctgactcagccgccctccatgttcgggactttgggtcagagggttaccctc tccttcactggaaggagcagcaacactgggggtctttatgtgagctggtaccaacagctc ccaggaaaggcccctagactcctgacatatgaaaatagcaaacgaccctcaggggtccca gattgattctctggctccaagcctgccaactcagcctctctgaccacctcagttcatgct gaacgatactgattattactgtttctcatgggctgatggcttgaaagtttg |
| SEQ ID NO. 123 IGLV1-55 (**F**) |
| >IGLV1-55*01\|Bos taurus\|F\|V-REGION\| |
| caggctgtgctgactcagccgtcctccgtgtccgggtccctgggccagagggtctccatc acctgctctggaagcagcagcaacgttggaactggcaattatgttggctggttccaacag atcccaggatcagcccccagaaccctcatctatggtgcgaccagtcgagcctcgggggtc cccgaccgattctccggctccaggtctgggaacacagccaccctgaccatcagctcgctc caggctgaggacgaggcagattatttctgtgcatcttatcagagtggtaacacag |
| SEQ ID NO. 124 IGLV1- 57 (**P**) |
| >IGLV1-57*01\|Bos taurus_Hereford\|P\|V-REGION\| |
| caggctgtgctgactcagccgccctccgtgtctgggactttgggtcagagggtcaccctc tcctgcactggaagcagcagcaaccctggggtctttatgtgagctggtaccaacagctcc caggaaggcccccagagtcctgacctatgaaaatagcaaacgaccctcaggactcccaga ttgattctctggctccaagtctggcagctcggccgcgctgaccacctcagttcagtctga ggacgacactgattattactgtttctcatgggctgatggcttgaaagtttg |
| SEQ ID NO. 125 IGLV1- 58 (**F**) |
| >IGLV1-58*01\|Bos taurus\|F\|V-REGION\| |
| caggctgtgctgactcagccatcatccgtgtccgggtccctgggccagagggtctccatc acctgcaccggaagcagcagcaatgttggaaatggatatgtgagctggttccaacagatc ccaggatcggcccccagaaccctcatctatggtgacaccagtcgagcctcgggggtcccc gaccgattctccggctccaggtctgggaacacagccaccctgaccatcagctcgctccag gctgaggacgaggcagattatttctgtgcagctggtgacagcagtagcagtaatgc |
| SEQ ID NO. 126 IGLV1- 59 (**P**) |
| >IGLV1-59*01\|Bos taurus_Hereford\|P\|V-REGION\| |
| caggctatgctgactcagccgccctccgtgtttgggactttgggtcagagggttaccctc tccttcactggaaggagcagcaacactgggggtctttatgtgagctggtaccaacagctc ccaggaaaggcccctagactcctgacatatgaaaatagcaaacgaccctcaggggtccca gattgattctctggctccaagcctggcaactcagcctctctgaccacctcagttcatgct gaatgatactgattattactgtttctcatgggctgatggcttgaaagtttg |
| SEQ ID NO. 127 IGLV1- 60 (**P**) |
| >IGLV1-60*01\|Bos taurus_Hereford\|P\|V-REGION\| |
| caggctgtgctgactcagccatcatccgtgtccgggtccctgggccagagggtctccatc acctgctctggaagcagcagcaatgttggaaatggatatgtgagctggtaccaactgatc ccaggatcggcccccagaacccttcatctatggtgacaccagtcgagcctcgggggtccc cgaccgattctccggctccaggtctgggaacacagccacccctgaccatcagctcgctcc |
| aggctgaggacgaggcagattatttctgtgcatctgctgaggatagtagcagtaatgc |
| SEQ ID NO. 128 IGLV1-61 (**P**) |
| >IGLV1-61*01\|Bos taurus_Hereford\|P\|V-REGION\| |
| caagctgtgctgactcaaccgccctccatgtctgggtccctgggccagagggtctccatc acctgctctggaagcagcagcaacgttggaactggcaattatgttggctggtaccaaatg atcccaggatcggcccccagaaccctcatctaccgtgctaccagtcgactctcggggtcc ccgactgattctccgtctccaggtctgggaacacagccaccctgaccatcagctcgcacc aggctgaggacgaggctgattattactgtgtatcttaggacagcagtatcagtggtgc |
| SEQ ID NO. 129 IGLV1- 63 (**F**) |
| >IGLV1-63*01\|Bos taurus\|F\|V-REGION\| |
| caggctgggctaactcagccggcctccgtgttcaggactttgggtcagagggtcaccatc tcttgtactggaagcagcaacaacattgggggttattatgtgagctggtaccaacagctc ccaggaaaggcccccagactcctgacctatgaaattagcaaacgacccccaggggtccca gatcgagtctctggctccaagtctggcaactcagcctctctgaccagctcagttcatgct gaggacgacactgattattactgtttctcatgggctgacggcttgaaagtttg |
| SEQ ID NO. 130 IGLV1- 64 (**F**) |
| >IGLV1-64*01\|Bos taurus\|F\|V-REGION\| |
| caggctgtgctgacccagccgccctccgtgtccgggtccctgggccagagggtgaccatc tcctgcactggaagcagcagcaacatcggtggtggtaattgtgtgggctggtaccaacag atcccaggatcggcccccaaaaccctcatctataggagtaccagtcgaccctcgggggtc ccagaccgattctccggctccaggtctggcaacacggccaccttgaccatcagctcgctc caggccgaagacgaggcggattactactgtgcaacttacgaaagtagtagttatcatgg |
| SEQ ID NO. 131 IGLV1- 66 (**P**) |
| >IGLV1-66*01\|Bos taurus_Hereford\|P\|V-REGION\| |
| caggctatgctgactcagccgccctccgtgttcgggactttgggtcagagggttaccctc tccttcactggaaggagcagcaacactgggggtctttatgtcagctggtaccaacagctc ccaggaaaggcccctagactcctgacatatgaaaatagcaaacaaccctcaggactccca gattgactctctggctccaagcctggcaactcagcctctctgaccacctcagttcatgct gaacgatactgattattactgtttctcatgggctgacggcttgaaagtttg |
| SEQ ID NO. 132 IGLV1- 67 (**F**) |
| >IGLV1-67*01\|Bos taurus\|F\|V-REGION\| |
| acctgctctggaagcagcaacaacatcggtagatatggtgtgggctggtaccaacaggtc ccaggatcgggcctcagaaccatcatctatggtagtagcagtcgaccctcgggggtcccc gaccgattctccggctccaagtctggcaacacagccaccctgaccatcagctcgctccag gctgaggacgaggcggattatttctgtgcaactggtgactacagtagcagtactgt |
| SEQ ID NO. 133 IGLV1- 70 (**F**) |
| >IGLV1-70*01\|Bos taurus\|F\|V-REGION\| |
| caggctgtgctgacccagccgccctccgtgtccgggtccctgggacagagggtgaccatc tcctgcactggaagcagcagcaacatcggtggtggtaattatgtgggctggtaccaacag atcccaggatcggcccccaaaaccctcatctataggagtaccagtcgaccctcgggggtc ccagaccgattctccggctccaggtctggcaacacggccaccttgaccatcagctcgctc caggccgaagacgaggcggattactactgtgcaacttacgaaagtagtagttatcatgg |
| SEQ ID NO. 134 IGLV1-71 (**F**) |
| >IGLV1-71*01\|Bos taurus\|F\|V-REGION\| |
| caggctgtgctgactcagctgccctccgtgttcaggactttgggtcagagggtcaccatc tcttgtactggaagcagcaacaacattgggggttattatgtgagctggtaccaacagctc ccaggaaaggcccccagactcctgacctatgaaattagcaaacgacccccaggggtccca gatcgagtctctggctccaagtctggcaactcagcctctctgaccagctcagttcatgct gaggacgacactgattattactgtttctcatgggctgacggcttgaaagtttg |
| SEQ ID NO. 135 IGLV1- 73 (**F**) |
| >IGLV1-73*01\|Bos taurus\|F\|V-REGION\| |
| caggctgtgctgactcagccgccctccgtgtccgggtccctgggccagagggtgaccatc acctgcaccggaagcagcagctacgtttcgcgtggcaatcatgtgagctggtaccaactg atcccaggattggcccccaaaaccctgatctataatagtaacaaacgaccctcgggggtc cctgaccgattctctggcaccaagtccggcaacacaggcaccctgaccatcgcttcgctc caggctgaggacgaggctgattattactgtgcgtctgctgacctcagtcttactggtcc |
| SEQ ID NO. 136 IGLV2-6 (**F**) |
| >IGLV2-6*01\|Bos taurus_Hereford\|F\|V-REGION\| |
| cagtctggcctgactcagccttcctcagtgtctgggaatctgggacagacggtcaccatc tcctgtgctgggaccagcagtgatgtaggggcttataatggtgtgggctggtaccaacag ctcccgggctcagcccccaaaactctgatctataatctcaacaaacggtcctcagggatc cctgctcggttctctggctccaagtctgggaacacagccaccctgaccatctctgggctc caggctgaggacgaggccgactattactgtagctcatataaaagtggtggtagtgtg |
| SEQ ID NO. 137 IGLV2-7 (**ORF**) |
| >IGLV2-7*01\|Bos taurus_Hereford\|ORF\|V-REGION\| |
| cagtctagcctgactcagccttcctcagtgtctgggaatctgggacagacggtcaccatc tcctgtgctgggaccagctgtgacgtagggagttataatggtgcgggctggtaccaacag ctcccgggctcagcccccaaaactctgatatgtaatgtcagcaaacggccctcagggatc cctgactggttctctggctccaagtccggaaacacagccaccctgaccatctctgggctc caggctgaggacgaggctgattactattggagctctcctagaagtgatagcactgtg |
| SEQ ID NO. 138 IGLV2-8 (**P**) |
| >IGLV2-8*01\|Bos taurus_Hereford\|P\|V-REGION\| |
| cagtctggcctgactcagcctccctcagtgtctgggaatctgggacagacggtcaccagc tcctgttctgagaccagcagtgacatcatggttataactctgtagattggtactggcagc acttgggcacggccccacactactgatttatgctgtcagtaaacgaccctcagggatccc tgatcgcttctctggctccaagcctggacacacagcctccctgactgtctctgggctcca ggctgaggacgaggctgattatcactgtggctcagacagaagcagcaaccacttg |
| SEQ ID NO. 139IGLV2-9 (**F**) |
| >IGLV2-9*01\|Bos taurus_Hereford\|F\|V-REGION\| |
| cagtctggcctgactcagccttcctcagtgtctgggaatctgggacagactgtcatcacc tcctgtgctgggaccagcagttatgtagggagttataatggtgtgggctggtaccaacag ctcccgggctcagcccccaaaactctaatatataatgtcagcaaacggccctcaggaatc cctgaccgcttctctggctccaagtccgggaacacagccaccctgaccgtctctgggctc caggctgaggacgaggccgactactactgtagctcatataaaagtggtggtagtgtg |
| SEQ ID NO. 140 IGLV3-1 (**P**) |
| >IGLV3-1*01\|Bos taurus_Hereford\|P\|V-REGION\| |
| tcctacgagctgactcagtcacccccggcatcgatgtccccaggacagacggccaggatc acgtgtggggggcccagcgttggaggtgaaaatgttgagtggcaccagcagaagccaggc caggcccgtgcgctggtcacctatggtgacgataaccgacccacgggggtccctgaccag ttctctggcgccaactcagggaacatggccaccctgaccatcagcggggcccgggccaag gatgaggccgactattactgtcagctgtgggacagcagcagtaacaatcct |
| SEQ ID NO. 141 IGLV3- 2 (**F**) |
| >IGLV3-2*01\|Bos taurus_Hereford\|F\|V-REGION\| |
| tcttctcagctgactcagccgcctgcggtgtccgtgtccttgggacagacagccagcatc acctgccagggagacgacttagaattgcttagtgcccactggtaccagcagaagccgggc caggcccctgtgctggtcatttatgcagatgacaacctggcctcagggatccctgaccgg ttctctggctccaaatcagacaccacggccaccctgaccattcgcggggcccaggccgaa gacgaggccgactattattgtcagtcagctgatatcagtggtgtt |
| SEQ ID NO. 142 IGLV3-3 (**F**) |
| >IGLV3-3*01\|Bos taurus_Hereford\|F\|V-REGION\| |
| tcctatgaactgacacagttgacttcagtgtcagtggccttgggacagacggccaagatc acctgctcgggggagctgctggatgaacaatatactcagtggtaccagcagaagccgggc caggcccctaagctggtcatttataaagacagtaagcggcgctcagggatccctgaccag ttctctggctccagctccggcaaaacagccatcttgaccatcagtggggtccgtgccgag |
| gatgaagccgactattactgtctgtcgtgggacagtggcagttacaatgtt |
| SEQ ID NO. 143 IGLV3-4 (**F**) |
| >IGLV3-4*01\|Bos taurus_Hereford\|F\|V-REGION\| |
| tcctatgaactgacccagccgacttcagtgtcagtggccttgggacagacggccaagatc acctgctcgggggacctgctggatgaacaatatactcagtggtaccagcagaagccgggc cagggccctgtgagggtcatttataaagatagtgagcggccctcagggatctctgaccgg ttctctggctccagctcaggcaaaacagccacgctgaccatcagcggggcccagaccgag gacgaggccgactattactgtcagtcagctgacagcagtgataatcct |
| SEQ ID NO. 144 IGLV3- 5 (**F**) |
| > IGLV3-5*01\|Bos taurus_Hereford\|F\|V-REGION\| |
| tcttctcagctgactcagccgcctgcggtgtccgtgtccttgggacagacggccagcatc acctgccagggagacgacttagaaagctattatgctcactggtaccagcagaagccaagc caggcccctgtgctggtcatttatgagtctagtgagagaccctcagggatccctgaccgg ttctctggctccagctcagggaacacggccaccctgaccatcagcggggcccagactgag gacgaggccgactattactgtcagtcatatgacagcagcggtgatcct |
| SEQ ID NO. 145 IGLV5- 11 (**P**) |
| >IGLV5-11*01\|Bos taurus_Hereford\|P\|V-REGION\| |
| cagcctgtgctgactcagccagccaccctctctgcgtctctgggagcatcagccaaactc accggcaccctgagcagtggctacaatgttggcagctactacataaactggtaccagcag aagccagggagtcctccctggtacccctgtattgttactcagactccaataagcaccagg gttccagggtccccagccacttctttggatccaaagacacctcggtgaaggcagggctcc tgctaatctcggctacaggctgaggacaaggctgactattactctgctacatattgcagt agtactaa |
| SEQ ID NO. 146 IGL5-13 (**P**) |
| >IGLV5-13*01\|Bos taurus_Hereford\|P\|V-REGION\| |
| cagcgtgtgctgactcagccggcctccccctctgcgtctccgggagcatcagccagactc ccagactgcaccctgaacagtgggtctatcttggtggctagcaaatgtcctgtttccagc agaagccagggagccctccccaggacctcctgatgttcaagtcagactccgataagcccc agggctccggggtccccagtcacttctctggatccaaggctgcctcagccaatgcagggc ccccgttcatctctgggctgcagcctgaggctgaggctgattataactgctactgtcccc aaaacactgcattca |
| SEQ ID NO. 147 IGLV5- 45 (**ORF**) |
| >IGLV5-45*01\|Bos taurus\|ORF\|V-REGION\| |
| cagcctgtgctgactcagtcagactccctgtctgcatctctgggagcatcagccagactc tcctgcaccctgagcaatggctataacattggcagcttgtctataacctggtgccagcag aagccagggagtcctccccggtacctcctgtcctacaactcagattcccaaaagctccag ggctccggggtccccagacacttctctggatccaaagacacctcgtccaacgcggggctc ctgctcatctctgggctgcagacgggggacgaggctgtaggttcc |
| SEQ ID NO. 148 IGLV5-50 (**ORF**) |
| >IGLV5-50*01\|Bos taurus\|ORF\|V-REGION\| |
| cagcctgtgctgactcagtcagactccctgtctgcatctctgggagcatcagccagactc tcctgcaccctgagcaatggctataacattggcagcttgtctataacctggtgccagcag aagccagggagtcctccccggtacctcctgtcctacaactcagattcccaaaagctccag ggctccggggtccccagacacttctctggacccaaagacacctcgtccaacgcggggctc ctgctcatctctgggctgcagacggaggacgaggctgtaggttcc |
| SEQ ID NO. 149 IGLV5-62 (**P**) |
| >IGLV5-62*01\|Bos taurus_Hereford\|P\|V-REGION\| |
| cagcctgtgctgactcagccgtcgccgtctctgcgtctctgggagcatcagccagactct cctgcaccctgagcagtggctacaatgttagcaactatagcaatatactggtatcagcag aaggcagggaaccctctccggtacctcctgaggttcaaatcagactcggataaacaccag ggctccggggtccccagccgcttctctggatccaaagatgcctcgaccaatgcagggctc ctgctcatctctgggctgcagcccgaggacgaggccgactactactgtgctgtgtggcat ggcgacactaacgctta |
| SEQ ID NO. 150 IGLV5- 72 (**F**) |
| >IGLV5-72*01\|Bos taurus\|F\|V-REGION\| |
| cagcctgtgctgactcagccagtcaccgtctctgcgtctctgggagcatcagccagactc tcctgcaccctgagcagtggctacaatgttagcaactatagcatatactggtatcagcag aaggcagggaaccctctccggtacctcctgaggttcaaatcagactcggataaacaccag ggctccggggtccccagccgcttctctggatccaaagatgcctcgaccaatgcagggctc ctgctcatctctgggctgcagcccgaggacgaggccgactactactgtgctgtgtggcat ggcgacactaacgctta |
| SEQ ID NO. 151 IGLV5-74 (**P**) |
| >IGLV5-74*01\|Bos taurus\|P\|V-REGION\| |
| cagcctgtgctgactgagccggcatccccctctgcatatccaggagcatcagccagactc acctgcaccctgagcagtgggtaccgtgttggtggctaccaaatgtcctgtttccagcag aaggcagggagccctccctggtacctcctgatgttcaagtcagactcggataagccccag ggctctggggtccccagtcacttctctggatccaaggctgcctcagccaacacagggctc ctgctcatctccgggctgcagactcaggatgaggctgattataactgctactgtccccga aacactggtattta |
| SEQ ID NO. 152 IGLV8-19 (**P**) |
| >IGLV8-19*01\|Bos taurus_Hereford\|P\|V-REGION\| |
| cagactgtggtccaggagccagcgctgtcagtgtgtccagtagggacggtcaccctcacc ggtggactgagctctgggtcagtcactaccagtaactgcccagctggtaccagcagaccc cgggccaggctccccgactgcttatctacagcacaagcagccgatattctggggtccctg ctcacttctctggctccatctctggaaacaaatccaccctcaccatctcgggggcccagt ccgaggtagaagccgactattactgtggtctgggaactggtagttacaatgc |
| SEQ ID NO. 153 IGLV8-19 (**P**) |
| >IGLV8-19*01\|Bos taurus_Hereford\|P\|V-REGION\| |
| cagactgtggtccaggagccagcgctgtcagtgtgtccagtagggacggtcaccctcacc ggtggactgagctctgggtcagtcactaccagtaactgcccagctggtaccagcagaccc cgggccaggctccccgactgcttatctacagcacaagcagccgatattctggggtccctg ctcacttctctggctccatctctggaaacaaatccaccctcaccatctcgggggcccagt ccgaggtagaagccgactattactgtggtctgggaactggtagttacaatgc |
| SEQ ID NO. 154 IGLV8-27 (**P**) |
| >IGLV8-27*01\|Bos taurus\|P\|V-REGION\| |
| tagactgtggcccagaagccgtcacggccagtgtctccaggagagacggtcaccctcacc tgtggactgagctctgggtcagtcacaaccagtaactaccctagctggttgcagcagacc ccaggccaggctccctgactgcttttctatagcacaaacagtcgcccgtctggggtctct aattgcatctctggttccatctctgggaacaaagccaccctcaccatcacgggggcccag tccgaggacgaagccgattatcactgtggtctgggaactggtagttacaatgc |
| SEQ ID NO. 155 IGLV8-32 (**P**) |
| >IGLV8-32*01\|Bos taurus_Hereford\|P\|V-REGION\| |
| cagactgtggtccaggagccagcactgtcagtgtgtccaggagggacggtcaccctcacc tgtggactgagctctgggtcagtcactaccagtaactgcccagctggtaccagcagaccc caggccaggctccccgactgcttatctacagcgcaagcagctgatattctggggtccctg cttacttctctggctccatctcgggaaacaaatccaccctcaccatctcgggggcccagt ccgaggtagaagccgactattactgtggtctgggaactggtagttacaatgc |
| SEQ ID NO. 156 IGLV8- 37 (**P**) |
| >IGLV8-37*01\|Bos taurus_Hereford\|P\|V-REGION\| |
| gcagcagtggcccagaagccgtcacggtcagtgtgtccaggaggcacggtcaccctcacc tgtggactgagctctgggtcggtcactacaggtaaccacccagctggttccagcagaccc cagtgcagcctcccggaactgtcaactacaacaccaacacccgtccctctggggtccctg gtaacttctctggctcctcctctgggagcagatctgccctcaccatcacaggagaccagt ccgaggacaaagccaactatcactgtgctctgggagctagtagttaaaatcc |
| SEQ ID NO. 157 IGLV8-38 (**F**) |
| >IGLV8-38*01\|Bos taurus\|F\|V-REGION\| |
| cagattgtgatccaggaaccgtcactgtcagtgtctccaggagggacggtcaccctcacc tgtggactgagctctgggtcagtcactacttacaacgaacccagctggtaccagcagacc ccaggccaggctcccagaaatgttatctacaacacaaacacccgcacctctggggtccct gatcgcttctctgcctccatctctgggaacaaagccaccctcaccatcacgggggcccag cccaaggacgaggctgactatcactgcttgctgtaccagggcagtggtagttacagctg |
| SEQ ID NO. 158 IGLV8-44 (**P**) |
| >IGLV8-44*01\|Bos taurus\|P\|V-REGION\| |
| tagactgtggcccagaagccgtcacggccagtgtctccaggagagacggtcaccctcacc tgtggactgagctctgggtcagtcacaaccagtaactaccctagctggttgcagcagacc ccaggccaggctccctgactgcttttctatagcacaaacagtcgcccgtctggggtctct aattgcatctctggttccatctctgggaacaaagccaccctcaccatcacgggggcccag tccgaggacgaagccgattatcactgtggtctgggaactggtagttacaatgc |
| SEQ ID NO. 159 IGLV8-49 (**P**) |
| >IGLV8-49*01\|Bos taurus\|P\|V-REGION\| |
| tagactgtggcccagaagccgtcacggccagtgtctccaggagagaaggtcaccctcacc tgtggactgagctctgggtcagtcacaaccagtaactaccccagctggttgcagcagacc ccaggccaggctccctgactgcttttctatagcacaaacagtcgcccgtctggggtctct aattgcatctctggttccatctctgggaacaaagccaccctcaccatcacgagggcccag cccaaggacgaagccgattatcactgtggtctgggaagtggtagttacaatgc |
| SEQ ID NO. 160 IGLV8-54 (**P**) |
| >IGLV8-54*01\|Bos taurus_Hereford\|P\|V-REGION\| |
| tagaactgtggcccagaagccgtcacggccagtgtctccaggagagaaggtcaccctcac ctgtggactgacctctgggtcactcacaaccagtaactaccccagctggttccagcagac cccaggccaggctccctgattgctttcctatagcacaaacagtcgtccgtctggggtttc taattgcatctctggttccatctctgggaacaaaaccaccctcaccatcaaggtggccca gcccaaggacgaagccgattatcactgtggtctgggaactggtagttacaatgc |
| SEQ ID NO. 161 IGLV13-23 (**ORF**) |
| >\|IGLV13-23*01\|Bos taurus\|ORF\|V-REGION\| |
| caatccgggctgactcaggaggcctccgtgtcacggtctctgggtcagtcggtcaccctc acctgcactgggagcagcaacaacgttggattctatggcgcgggctggtcccaacaccgc ccaggtgctgcccccagaaccgtggtgctgggaagttctcgaccctcaggcctcccagcc cggctctcgggctcctgttctggctccaccacctcactgagcatctctgggctccaggct gcggacgaggctgactattactgttccctgtgggcccgcagcctcagtgctcc |
| SEQ ID NO. 162 IGLV(I)-10 (**P**) |
| >IGLV(I)-10*01\|Bos taurus_Hereford\|P\|V-REGION\| |
| gtgtctttgtatcaggagaacccaggctctgccctaaaatctctgatttatgatgtcagt aaaaggtcctcaggggtcctgtttgcttctcaggctcaaactctgggaacaccctggcca cctctgggctccaggctgactcttattgtggctcacatgaaagtggacgcaatttg |
| SEQ ID NO. 163 IGLV(I)-35 (**P**) |
| >IGLV(I)-35*01\|Bos taurus _Hereford\|P\|V-REGION\| |
| tatggacgctcagccacactcgcctgcctgggaacagcaacactgttggcaccaaggggg cgacttggctgccgtctcagtcgtgctgagtcccaagctctgagcacaggagtcacaacc cacccgcagggtctcagcgaggcctgagggctccgggtcaggtggggccacctgaccatc tctgggctccagcctgacgccaaggctgattgttcctgctcagctgggactcagccctgc tgtt |
| SEQ ID NO. 164 IGLV (IV)-15 (**P**) |
| >\|IGLV(IV)-15*01\|Bos taurus_Hereford\|P\|V-REGION\| |
| tgcaccctgagcagtgacatcagtgtcggctgctctgctgttactggaacaagcagcagc cagggagccctcttcggtcctctggtcctaccactcagactccagtaagaaccaggctct ggggtccccgtgggttttctggatccaaagatgccttggccaacgtggggctcctgctcg tctctgggctgcagcctgaggatgaggctgactctcaccatgcaggctggtgcaacaggg |
| cttct |
| SEQ ID NO. 165 IGLV (IV)-20 (**P**) |
| >IGLV(IV)-20*01\|Bos taurus_Hereford\|P\|V-REGION\| |
| tgtaccctgagcagtgacatcagtgtcggccactctgctgttactggagccggcagaagc cagcgagccctcctcggtcctccggtcctaccgctcagactccagtaaggaccaggctcc agggtccccgtgggttttctggatccaaagatacctcggccaacgtgggcctcctgctca tctctgggctgcagcctgagcatgaagctgactctgaccatgcaggctggtgcaacaggg cttct |
| SEQ ID NO. 166 IGLV(IV)-25 (**P**) |
| >IGLV(IV)-25*01\|Bos taurus_Hereford\|P\|V-REGION\| |
| tgcaccccgagcagtgacatcagtgttggctgctctgctgttactggaaccagcagaagc cagcgagccctccttagtcctccagtcctaccactcagactccagtaagaaccagtcttt ggggtccccgcgggttttctggatccaaagatgcctcagccaacgcggggctcctgctcg tctctgggctgcagcctgaggatgaggctatctctaaccatgcaggctggtgcaacaggg cttct |
| SEQ ID NO. 167 IGLV(IV)- 30 (**P**) |
| > IGLV(IV)-30*01\|Bos taurus_Hereford\|P\|V-REGION\| |
| tgcaccctgagcagtgacatcaatgttggctgctctgctgttactggaaccagcagaagc cagcgagccctccttagtcctccggtcctaccactcagactccagtaagaaccagtcttt ggggtccccgcgggttttctggatccaaagatgcctcagccaacgcggggctcctgctcg tctctgggctgcagcctgaggatgaggctatctctaaccatgcaggctggtgcaacaggg cttct |
| SEQ ID NO. 168 IGLV(IV)-33 (**P**) |
| >IGLV(IV)-33*01\|Bos taurus _Hereford\|P\|V-REGION\| |
| tgcaccctgagcagtgacatcagtgtcggccgctctgctgttactggaaccagcagaagc cagcgagccctcctcggtcctccggtcctaccactcagattccagtaagaaccaggctcc ggggtccccatgggttttctggatccaaagatgcctcggccaatgcggggctcctgctcg tctctgggctacagcttgaggatgaggctgactctgaccatgcaggctggtgcaacaggg ctcct |
| SEQ ID NO. 169 IGLV(IV)-34 (**P**) |
| >IGLV(IV)-34*01\|Bos taurus _Hereford\|P\|V-REGION\| |
| tgcacccggagcagtgacatcagtgtcggccgctctgctgttactagaaccagcagaagc cagtgagccctccttggtcctccagttctaccactcagactccagtatgaaccgggctcc aggatcccccgcgggttttctggatccaaagatgcctcagccaacgcagggctccttctt gtctctgggctgcagcctgagcacgaggctgactctgaccgtgcaggctggtgcaacagg gcttct |
| SEQ ID NO. 170 IGLV(IV)-39 (**P**) |
| >IGLV(IV)-39*01\|Bos taurus_Hereford\|P\|V-REGION\| \| |
| tgcaccctgcgcagtgacatcagtgtcggccactctgctcttactggaaccagcagaagc cagcgagccctcctcggtcctctggtcctaccactgagactccagtaagaatcaggctcc ggggtcccccgtgggttttctggatccaaagatgcctcggccaatgtagggctcctgctc ttctctgggctgcagcccgaggacgaggctgactctgaccgtgcaggctggtgcaacagg gcttct |
| SEQ ID NO. 171 IGLV (IV)-42 (**P**) |
| >IGLV(IV)-42*01\|Bos taurus _Hereford\|P\|V-REGION\| |
| tgcaccctgagcagtgacatcaatgttggctgctctgctgttactggaaccagcagaagc cagcgagccctccttagtcctccggtcctaccactcagactccagtaagaaccagtcttt ggggtccccgcgggttttctggatccaaagatgcctcagccaacgcggggctcctgctcg tctctgggctgcagcctgaggatgaggctatctctaaccatgcaggctggtgcaacaggg cttct |
| SEQ ID NO. 172 IGLV (IV)- 56 (**P**) |
| >IGLV(IV)-56*01\|Bos taurus_Hereford\|P\|V-REGION\| |
| tgcaccccgagcagtgacatcagtgtcagtcgctctgctgttactagaaccagcagaagc cagcgagccctccttagtcctccggtcctaccactcagaccccagtaagaaccagtcttt ggggtccccgcgggttttctggatccaaagatgcctcagccaacgcggggctcctgctcg tctctgggctgcagcctgaggatgaggctgactctgaccatgcaggctggtgcaacaggg cttct |
| SEQ ID NO. 173 IGLV (IV)- 65 (**P**) |
| >IGLV(IV)-65*01\|Bos taurus_Hereford\|P\|V-REGION\| |
| gttactggaaccagcagaagccagtgagccctcctccgccctccgatcctaccactcaga ctccagtaagaaccaggctccggggtcctcgcaggttttctggatccaaagatgcctcgg ccaatgcagggctcctgctcgtctccgggctgcagcctgaggatgaggctgactctgacc gtgcaggctggtgcaacagggcttct |
| SEQ ID NO. 174 IGLV (IV)-68 (**P**) |
| >IGLV(IV)-68*01\|Bos taurus_Hereford\|P\|V-REGION\| |
| tgttactggaaccagcagaagccagcgagccctccttagtcctccggtcctaccactcag actccagtaagaaccagtcggggtccccgcgggttttctggatccaaagatgcctcagcc aaggggctcctgctcgtctctgggctgcagcctgaggatgaggctactctaaccatgcag gctggtgcaacagggctt |
| SEQ ID NO. 175 IGLV (IV)- 69 (**P**) |
| >IGLV(IV)-69*01\|Bos taurus_Hereford\|P\|V-REGION\| |
| gttactggaaccagcagaagccagtgagcctcctccgccctccgatcctaccactcagac tccagtacagaaccaggctccggggtcctcgcaggttttctggatccaaagatgcctcgg ccaatgcagggctcctgctcgtctccgggctgccacctgaggataggctggactctgacc gtgcaggctggtgcaacagggcttct |

| **Germline J_{λ} sequences** |
|---|
| SEQ ID NO. 176 IGLJ1 (**ORF**) |
| >IGLJ1*01\|Bos taurus_Hereford\|ORF\|J-REGION\| |
| ttttgtcttaggcggcgggacctgggtcaccgtcctgg |
| SEQ ID NO. 177 IGLJ2 (**F**) |
| >IGLJ2*01\|Bos taurus_Hereford\|F\|J-REGION\| |
| tgatcttttcggcggcgggaccagagtgaccgtcctgg |
| SEQ ID NO. 178 IGLJ3 (**F**) |
| >IGLJ3*01\|Bos taurus_Hereford\|F\|J-REGION\| |
| tgatcttttcggcggcgggaccacagtgaccgtcctgg |
| SEQ ID NO. 179 IGLJ4 (**F**) |
| >IGLJ4*01\|Bos taurus _Hereford\|F\|J-REGION\| |
| tgctgttttcggcagcgggaccacactgaccgtcctgg |
| SEQ ID NO. 180 IGLJ5 (**ORF**) |
| >IGLJ5*01\|Bos taurus_Hereford\|ORF\|J-REGION\| |
| tcctattttcattggcaggaccaggctgactgtcctgg |
| SEQ ID NO. 181 IGLJ7 (**F**) |
| >IGLJ7*01\|Bos taurus _Hereford\|F\|J-REGION\| |
| tgctgttttcggcagcgggaccacactgaccgtcctgg |
| SEQ ID NO. 182 IGLJ8 (**F**) |
| >IGLJ8*01\|Bos taurus _Hereford\|F\|J-REGION\| |
| tgctgttttcggcagcgggaccacactgaccgtcctgg |
| SEQ ID NO. 183 IGLJ9 (**ORF**) |
| >IGLJ9*01\|Bos taurus_Hereford\|ORF\|J-REGION\| |
| tcctattttcattggcaggaccaggctgactgtcctgg |

**Table 4. Bovine Constant Region Genes**

| **IGHC** |
|---|
| GenBank Accession Numbers, if available, are underlined. |

| **IGHA (F)** |
|---|
| SEQ ID NO. 184 |
| >IGHA*01\|Bos taurus_Holstein\|F\|**CH1**\| |
| nagagtgaaaccagccccagcatcttcccgctgagccttgggaacaacgacccagccggg caggtggtcatcggctgcctggtccagggcttcttcccgtcggcacccctgagcgtgacc tggaaccagaatggcgacagcgtgtccgtcaggaacttccctgccgtgctggccgggagc ctgtacaccatgagcagccagctgaccttgccggccagcctgtgcccaaaaggccagtcc gtgacctgccaagtgcagcacctctccaaagccagcaagaccgtggccgtgccctgcata attcaa |
| SEQ ID NO. 185 |
| >IGHA*01\|Bos taurus_Holstein\|F\|**H-CH2**\| |
| gactcaagttcatgttgcgtgccgaactgcgagcccagcctgtccgtgcagccaccagcc ctcgaggacctgctcctgggctccaacgccagcctcacgtgcacactgagtggcctgaaa agcgccgagggcgccagcttcacctggaacccgacaggtgggaagaccgccgtccagggg tcgcccaagcgtgactcctgtggctgctacagcgtgtccagcgtcctgccgggctgtgcc gatccctggaacagtggacagactttctcctgctctgtcacccaccccgagtccaagagt tcactgaccgccaccatcaagaaagactta |
| SEQ ID NO. 186 |
| >IGHA*01\|Bos taurus_Holstein\|F\|**CH3-CHS**\| |
| gggaacacgttccggcctcaggtccacctgctgccgccgtcggaggagctggccctc aacgagctggtgacgctgacgtgcctggtgcggggattcagccccaaggaggtgctggtg cgttggctgcagggcaatcaagagctgccccgcgagaagtatctgacctggggccccctg cccgaggctggccagagcgtcaccaccttcgccgtgaccagcgtgctacgcgtggacgcc gaggtctggaagcagggggacaccttctcctgcatggtgggccacgaggccctgcccctg gccttcacccagaagaccatcgaccgcctggcgggtaaacccacccacgtgaacgtgtcc gtggtcatgtcagaggtggatggggtctgctac |
| SEQ ID NO. 187 |
| >IGHA*01\|Bos taurus_Holstein\|F\|**M**\| |
| gaacatcagccctggctggtgctggacctgatgcagagcagccccgaggaggacagccca gaggccagcctgtggcccacgaccgtcaccctgctcaccctcttcctgctgagcctcttc tacagcacagcactgactgtgacgagcatccggaccacgccggacagccgggaggtcccc cagtac |

| **IGHD** (**P**) |
|---|
| SEQ ID NO. 188 |
| >AF411244\|IGHD*01\|Bos taurus\|P\|**CH1**\| |
| aaggtgaatcacacctgagagtcttccccctggtgtcctgcgtgagctccccatccgatg agagcacggtggccctgggctgcctggcccgggacttcgtgcccaattcagtcagcttct cctggaagttcaacaacagcacagtcagcagcgagagattctggaccttccccgaagtcc tgagggacggcttgtggtcggcctcctctcaggtggtcctgccctcctcaagcgcctttc aagggccggatgactacctggtgtgtgaagtccagcaccccaagggaggaaagaccgtcg gaaccgtgagggtggtccccagag |
| SEQ ID NO. 189 |
| >\|IGHD*01\|Bos taurus\|P\|**H1**\| |
| catcgactccaaccccgaccactccattgccttctctgatatccgggtcagagggctcca acaaggcggtcagcacgcagagcagcccag |
| SEQ ID NO. 190 |
| >IGHD*01\|Bos taurus\|P\|**H2**\| |
| cactgaccaccagccacagacagacagaagcccagacactggcgtgtccaaaggaaccct gcagag |
| SEQ ID NO. 191 |
| >IGHD*01\|Bos taurus\|P\|**CH2**\| |
| agtgtcagaaccacacccaggcccccagagtccacctgctgcccccaaccccccagggcc tttggctcctggacaaggccgagttcacctgcctggccacgggggaggccccgctggatg cccacttctcctgggaggtgtacgggcagccccacggcggggccttggaggagggaccca ccaggcacataaacagctcctggagccagagcagccgcctggccctgcccaggtccctgt gggcctcgggctccaacgtcacctgcacactgagcagccctggcctgcagtcgccggtga ccctgacggctcagagagaacatg |
| SEQ ID NO. 192 |
| >IGHD*01\|Bos taurus\|P\|**CH3**\| |
| ccgcctcagtgcctggcaatctgaccctccgcactgtgaccgcgcctggccccttctccc ctgcctggctcctgtgcgaggtgtccggcttctcacccgtggacatcctcctcacgtggc tggagagccagcaagaagtggagccttcccagtttgccacggcgcacaccacggcccagg ctgggcgtgcctcatcccacacctggagtgtcctgcgtgtctccagccccctggaccacg cgggggccacctacacctgtgtggtcagccacgaggcctcccggacgctcctcaatggca gctgcagcctggacactggtg |
| SEQ ID NO. 193 |
| >IGHD*01\|Bos taurus\|P\|**M1**\| |
| gtctggccacctggccaccggtggagcccaggacgagaagcagcgattgacggcacggat gtggaggatgccagcccactctggctcaccttcctggccctcttcctcgtcactgtggtc tacggcggcttcgtcaccttcatcaag |
| SEQ ID NO. 194 |
| >IGHD*01\|Bos taurus\|P\|**M2**\| |
| gtgaag |

| **IGHDD1P** (**P**) |
|---|
| SEQ ID NO. 195 |
| >IGHDD1P*01\|Bos taurus_Holstein\|P\|**CH1**\| |
| aaggtgaatcgctcccgagagtcttccccttggtgtcctgcatgagctccccgtctgatg agagaatggtggccctgggctgcctggcccggatcttcatacccaattcgttcagcttct cctggaagttaaacaacagcacagtcagcagcgagagattctggaccttccccgcagtcc tgagggacggcttgtggtcggcctcctctcaggtggccctgtcctcctcgagcgcctttc aagggccggatgactacctggtgtgtgaagtccagcaccccaagggaggaaagaccctcg gccccgtgagggtgggccccagag |
| SEQ ID NO. 196 |
| >IGHDD1P*01\|Bos taurus_Holstein\|P\|**CHS**\| |
| ctcctgcctgtcattcacaggagctctcctgtgcgtctgagaagacctccctgacctcca tggctgggccatggcgcagctgggacccctggcctttacccgaacggagctcatccaggg ggtcctgtcatcgcatttagccgcctcaccggccccagaccactcacgcctgcattccat gtgtc |
| SEQ ID NO. 197 |
| >IGHDD1P*01\|Bos taurus_Holstein\|P\|**H1**\| |
| catcgactccaaccccgaccactccattgccttctctgatatccgggtcagagggctcca acaaggcggtcagcacgcagagcagcccag |
| SEQ ID NO. 198 |
| >IGHDD1P*01\|Bos taurus_Holstein\|P\|**H2**\| |
| cgccggccaccagccacagacagacagaagcccggacactggcatgtccaaaggacccct gcagag |
| SEQ ID NO. 199 |
| >IGHDD1P*01\|Bos taurus_Holstein\|P\|**CH2**\| |
| agtgtcagaaccacacccaggcccccagcgtccacctactgcccccaaaacccgcagggc ctctggctcctggacaaggccgagttcacctgcctggccatgggggaggccccaccggat gtccacttctcctgggaggtgaacgggcagccccacggcgcggccttggaggagggaccc acctggcacatgaacggctcctggagccagagcagccgcctggccctgcccaggtccctg tgggcctcaggctccaacgtcacctgcacactgagtggccccggcctgcggtcgccggtg accctgatggctcagagagaacatg |
| SEQ ID NO. 200 |
| >IGHDD1P*01\|Bos taurus_Holstein\|P\|**CH3**\| |
| ccgcctcagtgcctggcaatctgaccctccgcactgtgaccgcgcctggccccttctccc ctgcctggctcctgtgcgaggtgtccggcttctcacccgtggacatcctcctcacgtggc tggagggccagcaagaagtggagccttcccagtttgccacggcacacaccacagcccagg ctgggcacgcctcatcccacacctggagtgtcctgcgtgtctccagccccctggaccacg tgggggccacctacacctgtgtggtcagccacgaggcctcccggacgctcctcaatggca gctgcagcctggacactggtg |
| SEQ ID NO. 201 |
| >IGHDD1P*01\|Bos taurus_Holstein\|P\|**M1**\| |
| gtctggccacctggccgccctggagccaggatgagagcagcgacgacggcacggatgtgg aggatgccagcccactctggctcaccttcctggccctcgtcctcatcactgtggtctacg gcggctttgtcaccttcatcaag |
| SEQ ID NO. 202 |
| >IGHDD1P*01\|Bos taurus_Holstein\|P\|**M2**\|344061..344066\|6 nt\|1\| \| \| \| \|6+0=6\| \| \| |
| gtgaag |

| **IGHDD2P (P)** |
|---|
| SEQ ID NO. 203 |
| >IGHDD2P*01\|Bos taurus_Holstein\|P\|**CH1**\| |
| ttctcaagcacctttcaagggccggatgactacctggtgtgcgaagtccagcaccccaag ggaggaaagaccgttggaaccgtgagggtggtccccagag |
| SEQ ID NO. 204 |
| >IGHDD2P*01\|Bos taurus Holstein\|P\|**CHS**\| |
| ctcctgcctgtcattcacacgagctctcctgtgcgtctgagaagacctccctgactccat ggctgggccatggggcagccgggacccctggcctttacccgaacggagctcatccagggg gtcctgtcatcgcatttagccgcctcaccggccccagaccactcacccctgcattccatg tgtc |
| SEQ ID NO. 205 |
| >IGHDD2P*01\|Bos taurus_Holstein\|P\|**H1**\| |
| catcgactccaaccccgaccacttcagtgccttctctgatatccgggtcagagggctcca tcaaggccgtcagcacgcagagcagcccag |
| SEQ ID NO. 206 |
| >IGHDD2P*01\|Bos taurus_Holstein\|P\|**CH2**\| |
| agtgtcagaaccacacccaggcccccagcgtccacctgctgcccccaaccccccagggcc tctggctcctggacaaggccgagttcacctgcctggccacgggggaggccccgctggatg cccgcttctcctgggaggtgaatgggcagccccacggcggggccttggaggagggaccca cctggcacatgaacagctcctggagccagagcagccgcctggccctgcccaggtccctgt gggcctcaggctccaacgtcacctgcacactgagtggcccctgcctgcggtcaccggtga ccctgatggctcagagagaacatg |
| SEQ ID NO. 207 |
| >IGHDD2P*01\|Bos taurus_Holstein\|P\|**CH3**\| |
| ccgcctcagtgcccggcaatctgaccctccgcactgtgaccgcgcctggccccttctccc atgcctggctcctgtgcaaggtttccggcttctcacccgtggacatcctcctcacgtggc tggagagccagcaagaagtggagccttcccagtttgccacggcgcacaccacggcccagg ctgggcatgcctcatcccacacctggagtgtcctgcgtgtctccagccccctggaccacg cgggggccacctacacctgtgtggtcagccacgaggcctcccggacgctcctcaatggca |
| gctgcagccctggacactggtg |
| SEQ ID NO. 208 |
| >IGHDD2P*01\|Bos taurus_Holstein\|P\|**M1**\| |
| gtctggccacctggccaccgtggagccaggacgagagcagcgacgacggcacggatgtgg aggatgccagcccactctggctcaccttcctggccctcttcctcgtcactgtggtctacg gcggcttcgtcaccttcatcaag |
| SEQ ID NO. 209 |
| >IGHDD2P*01\|Bos taurus_Holstein\|P\|**M2**\| |
| gtgaag |

| **IGHDD3P (P)** |
|---|
| SEQ ID NO. 210 |
| >IGHDD3P*01\|Bos taurus_Holstein\|P\|**CH1**\| |
| ttctcaagcacctttcaagggccggatgactacctggtgtgcgaagtccagcaccccaag ggaggaaagaccgttggaaccgtgagggtggtccccagag |
| SEQ ID NO. 211 |
| >IGHDD3P*01\|Bos taurus_Holstein\|P\|**CHS**\| |
| ctcctgcctgtcattcacacgagctctcctgtgcgtctgagaagacctccctgactccat ggctgggccatcgggcagccgggacccctggcctttacccgaacggagctcatccagggg gtcctgtcatcgcatttagccgcctcaccggccccagaccactcacccctgcattccatg tgtc |
| SEQ ID NO. 212 |
| >KT723GHDD3P*01\|Bos taurus_Holstein\|P\|**H1**\|4534 |
| catcgactccaaccccgaccacttcagtgccttctctgatatccgggtcagagggctcca tcaaggccgtcagcacgcagagcagcccag |
| SEQ ID NO. 213 |
| >IGHDD3P*01\|Bos taurus_Holstein\|P\|**CH2**\| |
| agtgtcagaaccacacccaggcccccagcgtccacctgctgcccccaaccccccagggcc tctggctcctggacaaggccgagttcacctgcctggccacgggggaggccccgctggatg cccgcttctcctgggaggtaaatgggcagccccacggcggggccttggaggagggaccca cctggcacatgaacagctcctggagccagagcagccgcctggccctgcccaggtccctgt gggcctcaggctccaacgtcacctgcacactgagtggcccctgcctgcggtcaccggtga ccctgatggctcagagagaacatg |
| SEQ ID NO. 214 |
| >IGHDD3P*01\|Bos taurus_Holstein\|P\|**CH3**\| |
| ccgcctcagtgcccggcaatctgaccctccgcactgtgaccgtgcctggccccttctccc atgcctggctcctgtgcgaggtgtccggcttctcacccgtggacatcctcctcacgtggc tggagagccagcaagaagtggagccttcccagtttgccacggcgcacaccacggcccagg ctgggcgtgcctcatcccacacctggagtgtcctgcgtgtctccagccccctggaccacg cgggggccacctacacctgtgtggtcagccacgaggcctcccggacgctcctcaatggca gctgcagcctggacactggtg |
| SEQ ID NO. 215 |
| >IGHDD3P*01\|Bos taurus_Holstein\|P\|**M1**\| |
| gtctggccacctggccaccgtggagccaggacgagagcagcgacgacggcacggatgtgg aggatgccagcccactctggctcaccttcctggccctcttcctcgtcactgtggtctacg gcggcttcgtcaccttcatcaag |
| SEQ ID NO. 216 |
| >IGHDD3P*01\|Bos taurus_Holstein\|P\|**M2**\| |
| gtgaag |

| **IGHE (F)** |
|---|
| SEQ ID NO. 217 |
| >IGHE*02\|Bos taurus_Holstein\|F\|**CH1**\| |
| ncctccatccaggctccatccatctaccccttgaggctctgttgcacagaagaagcccgt gtaaggctgggctgcctggtcaaggactacttgccagggtcagtgactgtgacctgggac acagtgcccctggatggaagcaccctgaccttccctagcatccaaatggcgagctccagt ctctacattaccaccagccagctgaccatctcgggcgagcagcccaaggagttcacctgc agagttttacacgctgagaccaactccaacttgaataagaccatcagcact |
| SEQ ID NO. 218 |
| >IGHE*02\|Bos taurus_Holstein\|F\|**CH2**\| |
| gagtgcgccaagaacttcagcgacccctcagtgaagctcttctactcctcctgcaacccc aatggcgacacccagaccaccatccacctcctgtgccggatctccggctacaccccgggc aaaatcaaggtcacctggctggtggatgggctcaagtccgaagagctgtatgcacgatcc ggccctgagatacaggagggcaatctgacctccacctacagtgaagtcaacatcacacag ggccagtgggtgtccgagaaaacctacacctgccgggtcaactattacggctacaacttc gaaagccacgcccacaggtgcaca |
| SEQ ID NO. 219 |
| >IGHE*02\|Bos taurus_Holstein\|F\|**CH3**\| |
| gccgagtccgagccccgcggcgtgagcgcctacctgagcccacccacccccctcgagctg tacgtcaacaagtcacccaagatcacctgcctggttgtggacctggccaacgagaagaac ttgagcctgacctggtcgagggcaaacgggaagcctgtgcacgcaggcccccctgagatc aagcgtcagttcaacgggacggtcaccgtcacgtccaccctgccggtggacgtcactgac tgggtagaaggcgagacctactactgcaaagtgagccacagcgacctgcccacggacatc cagcgatccatctccaaggatgtt |
| SEQ ID NO. 220 |
| >IGHE*02\|Bos taurus_Holstein\|F\|**CH4-CHS**\| \| \| |
| ggcaagcgtctggcccccaaggcctacgtgttcttgccagacggaaaggagctggagaac gaggaggagctcaccctcacctgcatgatccagaaattcttccccagggacatctttgtg cggtggctgcataacaaggaactgatgcgggcagaccagcacaccaccacacagccccac agggccgacaacaacaccccagccttcttcgcctacagccgcctggcggtccctagggcc aactggaagaggggcgatgagttcacctgccaagtgatccatgaggcactgcccaggact aggaccctggagaaatcggtgttcattaacagcggtaaa |
| SEQ ID NO. 221 |
| >IGHE*02\|Bos taurus_Holstein\|F\|**M1**\| |
| gagctggccctcgaggacctgtgtgcagaggaagctgagagcgaggagctggaggagacc tggaccagcctccttgtcttcatcgtgctcttcctgctcagcatgagctatggcgccgcc gtcagcctctgcaag |
| SEQ ID NO. 222 |
| >IGHE*02\|Bos taurus_Holstein\|F\|**M2**\| |
| gtgaagtggatcctggccgccatcctccaggggcagccccagggctcccaagactacacc aacgtcacacagcgcccactgcctgctggtgtc |

| **IGHG1** (**F**) |
|---|
| SEQ ID NO. 223 |
| >IGHG1*04\|Bos taurus_Holstein\|F\|**CH1**\| |
| ncctccaccacagccccgaaagtctaccctctgagttcttgctgcggggacaagtccagc tccaccgtgaccctgggctgcctggtctccagctacatgcccgagccggtgaccgtgacc tggaactcgggtgccctgaagagcggcgtgcacaccttcccggctgtccttcagtcctcc gggctctactctctcagcagcatggtgaccgtgcccggcagcagctcaggacagaccttc acctgcaacgtagcccacccggccagcagcaccaaggtggacaaggctgtt |
| SEQ ID NO. 224 |
| >IGHG1*04\|Bos taurus_Holstein\|F\|**H**\|g |
| gatcccagatgcaaaacaacctgtgactgttgccca |
| SEQ ID NO. 225 |
| >IGHG1*04\|Bos taurus_Holstein\|F\|**CH2**\| |
| ccgcctgagctccctggaggaccctctgtcttcatcttcccaccgaaacccaaggacacc ctcacaatctcgggaacgcccgaggtcacgtgtgtggtggtggacgtgggccacgatgac |
| cccgaggtgaagttctcctggttcgtggacgacgtggaggtaaacacagccacgacgaag ccgagagaggagcagttcaacagcacctaccgcgtggtcagcgccctgcgcatccagcac caggactggactggaggaaaggagttcaagtgcaaggtccacaacgaaggcctcccggcc cccatcgtgaggaccatctccaggaccaaa |
| SEQ ID NO. 226 |
| >IGHG1*04\|Bos taurus_Holstein\|F\|**CH3-CHS**\| \| |
| gggccggcccgcgagccgcaggtgtatgtcctggccccaccccaggaagagctcagcaaa agcacggtcagcctcacctgcatggtcaccagcttctacccagactacatcgccgtggag tggcagagaaatgggcagcctgagtcagaggacaagtacggcacgacccctccccagctg gacgccgacggctcctacttcctgtacagcaggctcagggtggacaggaacagctggcag gaaggagacacctacacgtgtgtggtgatgcacgaggccctgcacaatcactacacgcag aagtccacctctaagtctgcgggtaaa |
| SEQ ID NO. 227 |
| >IGHG1*04\|Bos taurus_Holstein\|F\|**M1**\| |
| gacctgctgctggaggaggagatctgtgcggacgcccaggacggggagctggacgggctc tggacgactatctccatcttcatcacactcttcctgctcagcgtctgctacagcgccacc gtgaccctcttcaag |
| SEQ ID NO. 228 |
| >\|IGHG1*04\|Bos taurus_Holstein\|F\|**M2**\| |
| gtgaagtggatcttctcatcagtggtggagctgaagaggacgatcgtccccgactacaga aatatgatcgggcagggagcc |

| **IGHG2** (**F**) |
|---|
| SEQ ID NO. 229 |
| >IGHG2*04\|Bos taurus_Holstein\|F\|**CH1**\| |
| ncctccaccacagccccgaaagtctaccctctggcatcccgctgcggagacacatccagc tccaccgtgaccctgggctgcctggtctccagctacatgcccgagccggtgaccgtgacc tggaactcgggtgccctgaagagcggcgtgcacaccttcccggccgtccttcagtcctcc gggctctactctctcagcagcatggtgaccgtgcccggcagcagctcaggacagaccttc acctgcaacgtagcccacccggccagcagcaccaaggtggacaaggctgtt |
| SEQ ID NO. 230 |
| >IGHG2*04\|Bos taurus_Holstein\|F\|**H**\| |
| ggggtctccattgactgctccaagtgtcataaccagcct |
| SEQ ID NO. 231 |
| >IGHG2*04\|Bos taurus_Holstein\|F\|**CH2**\| |
| tgcgtgagggaaccatctgtcttcatcttcccaccgaaacccaaagacaccctgatgatc acaggaacgcccgaggtcacgtgtgtggtggtgaacgtgggccacgataaccccgaggtg cagttctcctggttcgtggatgacgtggaggtgcacacggccaggtcgaagccaagagag gagcagttcaacagcacgtaccgcgtggtcagcgccctgcccatccagcaccaggactgg actggaggaaaggagttcaagtgcaaggtcaacaacaaaggcctctcggcccccatcgtg aggatcatctccaggagcaaa |
| SEQ ID NO. 232 |
| >IGHG2*04\|Bos taurus Holstein\|F\|**CH3-CHS**\| \| |
| gggccggcccgcgagccgcaggtgtatgtcctggacccacccaaggaagagctcagcaaa agcacgctcagcgtcacctgcatggtcaccggcttctacccagaagatgtagccgtggag tggcagagaaaccggcagactgagtcggaggacaagtaccgcacgaccccgccccagctg gacaccgaccgctcctacttcctgtacagcaagctcagggtggacaggaacagctggcag gaaggagacacctacacgtgtgtggtgatgcacgaggccctgcacaatcactacatgcag aagtccacctctaagtctgcgggtaaa |
| SEQ ID NO. 233 |
| >IGHG2*04\|Bos taurus_Holstein\|F\|**M1**\| |
| gacctgctgctggaggaggagatctgtgcggacgcccaggacggggagctggacgggctc tggacgactatctccatcttcatcacactctttctgctcagcgtctgctacagcgccacc gtgaccctcttcaag |
| SEQ ID NO. 234 |
| >IGHG2*04\|Bos taurus_Holstein\|F\|**M2**\| |
| gtgaagtggatcctctcatccgtggtggagctgaagcagtcgatcactcccaactacaga aacatgatcggacagggcgcc |

| **IGHG3** (**F**) |
|---|
| SEQ ID NO. 235 |
| >IGHG3*03\|Bos taurus_Holstein\|F\|**CH1**\| |
| ncctccaccacagccccgaaagtctaccctctggcatccagctgcggagacacatccagc tccaccgtgaccctgggctgcctggtctccagctacatgcccgagccggtgaccgtgacc tggaactcgggtgccctgaagagcggcgtgcacaccttcccggccgtccggcagtcctct gggctgtactctctcagcagcatggtgaccgtgcccgccagcacctcaggaacccagacc ttcacctgcaacgtagcccacccggccagcagcaccaaggtggacaaggctgtc |
| SEQ ID NO. 236 |
| >IGHG3*03\|Bos taurus_Holstein\|F\|**H1**\| |
| actgcaaggcgtccagtcccgacgacgccaaagacaaccatccctcctggaaaacccaca acccag |
| SEQ ID NO. 237 |
| >IGHG3*03\|Bos taurus_Holstein\|F\|**H2**\| |
| gagcctgaagttgaaaagacaccctgccagtgttccaaatgccca |
| SEQ ID NO. 238 |
| >IGHG3*03\|Bos taurus_Holstein\|F\|**CH2**\| |
| gaacctccgggaggactgtctgtcttcatcttcccaccgaaacccaaggacaccctcaca atctcaggaacgcccgaggtcacgtgtgtggtggtggacgtgggccaggatgaccccgag gtgcagttctcctggttcgtggacgacgtggaggtgcacacggccaggacgaagccgaga gaggagcagttcaacagcacctaccgcgtggtcagcgccctgcgcatccagcaccaggac tggctgcagggaaaggagttcaagtgcaaggtcaacaacaaaggcctcccggcccccatt gtgaggaccatctccaggaccaaa |
| SEQ ID NO. 239 |
| >IGHG3*03\|Bos taurus_Holstein\|F\|**CH3-CHS**\| |
| gggcaggcccgcgagccgctggtgtatgtcctggccccaccccgggaagagctcagcaaa agcacgctcagcctcacctgcctgatcaccggtttctacccagaagagatagacgtggag tggcagagaaatgggcagcctgagtcagaggacaagtaccacacgaccgcaccccagctg gatgctgacggctcctacttcctgtacagcaggctcagggtgaacaagagcagctggcag gaaggagaccactacacgtgtgcagtgatgcatgaagctttacggaatcactacaaagag aagtccatctccaggtctccgggtaaa |
| SEQ ID NO. 240 |
| >IGHG3*03\|Bos taurus Holstein\|F\|**M1**\| |
| gacttgctgctggaggaggagatctgtgcggacgacctggatggggagctggacgggctc tggacgactatctccatcttcatcacactcttcctgctcagcgtctgctacagcgccact gtgaccctcttcaag |
| SEQ ID NO. 241 |
| >IGHG3*03\|Bos taurus Holstein\|F\|M2\| |
| gtgaaatggatcttctcctcggcggtggagctgaagaggacgatcgtccccgactacaga aacatgctcgggcagggcgcc |

| **IGHG3** (**F**) |
|---|
| SEQ ID NO. 242 |
| >IGHG3*03\|Bos taurus_Holstein\|F\|**CH1**\| |
| ncctccaccacagccccgaaagtctaccctctggcatccagctgcggagacacatccagc tccaccgtgaccctgggctgcctggtctccagctacatgcccgagccggtgaccgtgacc tggaactcgggtgccctgaagagcggcgtgcacaccttcccggccgtccggcagtcctct gggctgtactctctcagcagcatggtgaccgtgcccgccagcacctcaggaacccagacc ttcacctgcaacgtagcccacccggccagcagcaccaaggtggacaaggctgtc |
| SEQ ID NO. 243 |
| >IGHG3*03\|Bos taurus_Holstein\|F\|**H1**\| |
| actgcaaggcgtccagtcccgacgacgccaaagacaaccatccctcctggaaaacccaca acccag |
| SEQ ID NO. 244 |
| >IGHG3*03\|Bos taurus_Holstein\|F\|**H2**\| |
| gagcctgaagttgaaaagacaccctgccagtgttccaaatgccca |
| SEQ ID NO. 245 |
| >IGHG3*03\|Bos taurus_Holstein\|F\|**CH2**\| |
| gaacctccgggaggactgtctgtcttcatcttcccaccgaaacccaaggacaccctcaca atctcaggaacgcccgaggtcacgtgtgtggtggtggacgtgggccaggatgaccccgag gtgcagttctcctggttcgtggacgacgtggaggtgcacacggccaggacgaagccgaga gaggagcagttcaacagcacctaccgcgtggtcagcgccctgcgcatccagcaccaggac tggctgcagggaaaggagttcaagtgcaaggtcaacaacaaaggcctcccggcccccatt gtgaggaccatctccaggaccaaa |
| SEQ ID NO. 246 |
| >IGHG3*03\|Bos taurus_Holstein\|F\|**CH3-CHS**\| \| |
| gggcaggcccgggagccgctggtgtatgtcctggccccaccccgggaagagctcagcaaa agcacgctcagcctcacctgcctgatcaccggtttctacccagaagagatagacgtggag tggcagagaaatgggcagcctgagtcagaggacaagtaccacacgaccgcaccccagctg gatgctgacggctcctacttcctgtacagcaggctcagggtgaacaagagcagctggcag gaaggagaccactacacgtgtgcagtgatgcatgaagctttacggaatcactacaaagag aagtccatctcgaggtctccgggtaaa |
| SEQ ID NO. 247 |
| >IGHG3*03\|Bos taurus_Holstein\|F\|**M1**\| |
| gacttgctgctggaggaggagatctgtgcggacgacctggatggggagctggacgggctc tggacgactatctccatcttcatcacactcttcctgctcagcgtctgctacagcgccact gtgaccctcttcaag |
| SEQ ID NO. 248 |
| >IGHG3*03\|Bos taurus_Holstein\|F\|**M2**\| |
| gtgaaatggatcttctcctcggcggtggagctgaagaggacgatcgtccccgactacaga aacatgctcgggcagggcgcc |

| **IGHGP** (**P**) |
|---|
| SEQ ID NO. 249 |
| >IGHGP*01\|Bos taurus\|P\|**CH1**\| |
| cctccaccacagccccgaaagtctaccctctggcatccagctgcggagacacatccagct ccaccgtgaccctgggctgcctggtctccagctacatgcccgagccggtgaccgtgacct ggaactcgggtgccctgaagagcggcgtgcacaccttcccggccgtccggcagtcctctg ggctgtactctctcagcagcatggtgaccgtgcccgccagcagctcagaaacccagacct tcacctgcaacctagcccacccggccagcagcaccaaggtggacaagg |
| SEQ ID NO. 250 |
| >IGHGP*01\|Bos taurus\|P\|**H**\| |
| agtctgaagttgaaaagacaccctgccagtgttccaaatgcccag |
| SEQ ID NO. 251 |
| >IGHGP*01\|Bos taurus\|P\|**CH2**\| |
| aacctctggacgactgtctgtcttcatcttcccaccgaaacccaaggacaacctacaca tctcgggaacgcccgaggttacgtgtgtggtggtggacgtgggccaggatgaccccgagg tgcagttctcctggttcgtggacgacgtggaggtgcacacggccaggacgaagccgagag aggagcagttcaacagcacctaccgcgtggtcagcgccctgcgcatccagcaccaggact ggctgcagggaaaggagttcaagtgcaaggtcaacaacaaaggcctcccggcccccattg tgaggaccatctccaggaccaaag |
| SEQ ID NO. 252 |
| >IGHGP*01\|Bos taurus\|P\|**CH3-CHS**\| |
| ggcaggcccggcagccgcaggtgtatgtcctggccccacccgggaagagctcagcaaaag cacgctcagcctcacctgcctgatcaccggtttctacccagaagagatagacgtggagtg gcagagaaatgcgcagcctgagtcggaggacaagtaccacacgaccgcaccccagctgga tgctgacggctcctacttcctgtacagcaggctcagggtgaacaagagcagctggcagga aggagaccactacacgtgtgcagtgatgcatgaagctttacggaatcactacaaagagaa gtccatctcgacgtctccgggtaaa |

| **IGHM1** (**F**) |
|---|
| SEQ ID NO. 253 |
| >U63637\|IGHM1*01\|Bos taurus_Brown Swiss\|F\|**CH1**\| |
| naaggtgaatcacacccgaaagtcttccccctggtgtcctgcgtgagctcgccatccgat gagagcacggtggccctgggctgcctggcccgggacttcgtgcccaattcagtcagcttc tcctggaagttcaacaacagcacagtcagcagcgagagattctggaccttccccgaagtc ctgagggacggcttgtggtcggcctcctctcaggtggtcctgccctcctcaagcgccttt caagggccggatgactacctggtgtgcgaagtccagcaccccaagggaggaaagaccgtc ggcaccgtgagggtgatcgctaca |
| SEQ ID NO. 254 |
| >IGHM1*01\|Bos taurus_Brown Swiss\|F\|**CH2**\| |
| aaggcggaagtgctgtccccagtcgtgagtgtctttgtcccgcctcgcaacagcctctct ggtgacggcaatagcaagtccagcctcatctgccaggccacggacttcagccccaaacag atctcgttgtcctggtttcgtgatggaaagcggatagtgtctggaatttctgaaggccag gtggagactgtgcagtcctcacccataaccttcagggcctacagcatgctgaccatcacg gagagagactggctcagccagaacgcgtacacctgccaggtagaacacaacaaggaaacc ttccagaagaacgtgtcctcctcatgtgatgtt |
| SEQ ID NO. 255 |
| >IGHM1*01\|Bos taurus_Brown Swiss\|F\|**CH3**\| |
| gcaccaccatctccgatcggggtcttcaccatccccccatccttcgccgacatcttcctc acgaagtcagccaagctgtcctgtctggtcacaaacctggcctcctatgatggcctgaac atcagctggtcccgtcagaacgccaaggccctggagacccacacgtattttgagcgacac ctcaacgacaccttcagcgcccggggtgaggcctcggtctgctcggaggactgggagtcc ggagaggagttcacgtgcacagtggcccactcggacctgcccttcccagaaaagaacgcc gtctccaagcccaaa |
| SEQ ID NO. 256 |
| >IGHM1*01\|Bos taurus_Brown Swiss\|F\|**CH4-CHS\|** |
| gacgtcgccatgaaaccgccgtccgtgtacctgctgcctccaacgcgggaacagctgagc ctgcgcgagtcggcctccgtcacctgcctggtgaaggcgttcgcgcccgcggacgtgttc gtgcagtggctgcagaggggggagcccgtgaccaagagcaagtacgtgaccagcgcccgc gcgcccgagccccaggaccccagcgtggtgtactttgtgcacagcatcctgacggtggcc gaggaggactggagcaaaggggagacctacacctgcgtcgtgcacgaagccctgccccac atggtcaccgagcggaccgtggacaagtccaccggtaaacccaccctgtacaacgtgtcc ctggtcctgtctgacacagccagcacctgctac |
| SEQ ID NO. 257 |
| >IGHM1*01\|Bos taurus_Brown Swiss\|F\|**M1**\| |
| gaggggaggtgagcgccgaggaagaaggctttgaaaacctcaacaccatggcctccacc ttcatcgtgctcttcctcctgagcctcttctacagcaccacggtcaccctgttcaag |
| SEQ ID NO. 258 |
| >IGHM1*01\|Bos taurus_Brown Swiss\|F\|**M2**\| |
| gtgaag |

| **IGHM2** (**F**) |
|---|
| SEQ ID NO. 259 |
| >IGEM2*02\|Bos taurus_Holstein\|F\|**CH1**\| |
| naaggtgaatcacacccgagagtcttccccctggtgtcctgcgtgagctccccatccgat gagagcacggtggccctgggctgcctggcccgggacttcgtgcccaattcagtcagcttc tcctggaagttcaacaacagcacagtcagcagcgagagattctggaccttccccgaagtc ctgagggacggcttgtggtcggcctcctctcaggtggtcctgccctcctcaagcgccttt |
| caagggccggatgactacctggtgtgcgaagtccagcaccccaagggaggaaagaccgtc ggcaccgtgagggtggtcgctaca |
| SEQ ID NO. 260 |
| >IGHM2*02\|Bos taurus_Holstein\|F\|**CH2**\| |
| aaggcggaagtgctgtccccagtcgtgagtgtctttgtcccgcctcgcaacagcctctct ggtgacggcaatagcaagtccagcctcatctgccaggccacggacttcagccccaaacag atctccttgtcctggtttcgtgatggaaagcggatagtgtctggcatttctgaaggccag gtggagactgtgcagtcctcacccgtaactttcagggcctacagcatgctgaccatcacg gagagagactggctcagccagaacgtgtacacctgccaggtagaacacaacaaggaaacc ttccagaagaacgtgtcctcctcatgtgatgtt |
| SEQ ID NO. 261 |
| >IGHM2*02\|Bos taurus_Holstein\|F\|**CH3**\| |
| gcaccaccatctcccatcggggtcttcaccatccccccatccttcgccgacatcttcctc acgaagtcagccaagctgtcctgtctggtcacaaacctggcctcctatgatggcctgaac atcagctggtcccgtcagaacggcaaggccctggagacccacacgtattttgagcgacac ctcaacgacaccttcagcgcccggggtgaggcctcggtctgctcggaggactgggagtcc ggagaggagttcacgtgcacagtggcccactcggacctgcccttcccagaaaagaacacc gtctccaagcccaaa |
| SEQ ID NO. 262 |
| >IGHM2*02\|Bos taurus_Holstein\|F\|**CH4-CHS**\| |
| gatgtcgccatgaaaccgccgtccgtgtacctgctgcctccaacgcgggaacagctgagc ctgcgggagtcggcctccgtcacctgcctggtgaagggcttcgcgcccgcggacgtgttc gtgcagtggctgcagaggggggagcccgtgaccaagagcaagtacgtgaccagcagcccg gcgcccgagccccaggaccccagcgtgtactttgtgcacagcatcctgacggtggccgag gaggactggagcaaaggggagacctacacctgcgtcgtgggccacgaggccctgccccac atggtcaccgagcggaccgtggacaagtccaccggtaaacccaccctgtacaacgtgtcc ctggtcctgtctgacacagccagcacctgctac |
| SEQ ID NO. 263 |
| >IGHM2*02\|Bos taurus_Holstein\|F\|**M1**\| |
| gaggggaggtgagcgccgaggaggaaggctttgagaacctcaacaccatggcctccacc ttcatcgtgctcttcctcctgagcctcttctacagcaccacggtcaccctgttcaag |
| SEQ ID NO. 264 |
| >IGHM2*02\|Bos taurus_Holstein\|F\|**M2**\| |
| gtgaag |

| **IGKC** |
|---|
| **IGKC** (**F**) |
| SEQ ID NO. 265 |
| >IGKC*01\|Bos taurus_Hereford\|F\|C-REGION\| |
| nggtctgatgctgagccatccgtcttcctcttcaaaccatctgatgagcagctgaagacc ggaactgtctctgtcgtgtgcttggtgaatgatttctaccccaaagatatcaatgtcaag tggaaagtggatggggttactcagagcagcagcaacttccaaaacagtttcacagaccag gacagcaagaaaagcacctacagcctcagcagcatcctgacactgcccagctcagagtac caaagccatgacgcctatacgtgtgaggtcagccacaagagcctgactaccaccctcgtc aagagcttcagtaagaacgagtgt |

| **IGLC** |
|---|
| SEQ ID NO. 266 **IGLC1** (**P**) |
| >IGLC1*01\|Bos taurus_Hereford\|P\|C-REGION |
| gtcagcccaagtccgcaccctcggtcaccctgttcccgccccccaccgaggagctcagcg ccaacaaggccaccctggtgtgtctcatcagtgacttctacccgggtagcgtgaccgtgg cctggaaggcagacggcagcaccatcacccgcaacgtggagaccacccgggcctccaaac agagcaacagcaagtacgcggccagcagctactgagcctgacgggcagcgactggaaatc gaaaggcagttacagctgcgaggtcacgcacgaggggagcaccgtgacgaagacagtgaa |
| gaccctcagcgtgttct |
| SEQ ID NO. 267 **IGLC2** (**F**) |
| >IGLC2*01\|Bos taurus_Hereford\|F\|C-REGION\| |
| ggtcagcccaagtccccaccctcggtcaccctgttcccgccctccaaggaggagctcagc gccaacaaggccaccctggtgtgtctcatcagcgacttctacccgggtagcgtgaccgtg gcctggaaggcagacggcagcaccatcacccgcaacgtgaagaccacccgggcctccaaa cagagcaacagcaagtacgcggccagcagctacctgagcctgacagacagcgactggaaa tcgaaaggcagttacagctgcgaggtcacgcacgaggggagcaccgtgacgaagacagtg aagacctcagcgtgttct |
| SEQ ID NO. 268 **IGLC3** (**F**) |
| >IGLC3*01\|Bos taurus_Hereford\|F\|C-REGION\| |
| ggtcagcccaagtccccaccctcggtcaccctgttcccgccctccaaggaggagctcagc gccaacaaggccaccctggtgtgtctcatcagcgacttctacccgggtagcgtgaccgtg gcctggaaggcagacggcagcaccatcacccgcaacgtggagaccacccgggcctccaaa cagagcaacagcaagtacgcggccagcagctacctgagcctgacgagcagcgactggaaa tcgaaaggcagttacagctgcgaggtcacgcacgaggggagcaccgtgacgaagacagtg aagacctcagcgtgttct |
| SEQ ID NO. 269 **IGLC4** (**F**) |
| >IGLC4*01\|Bos taurus_Hereford\|F\|C-REGION\| |
| ggtcagcccaagtccccaccctcggtcaccctgttcccgccctccacggaggagctcaac ggcaacaaggccaccctggtgtgtctcatcagcgacttctacccgggtagcgtgaccgtg gtctggaaggcagacggcagcaccatcacccgcaacgtggagaccacccgggcctccaaa cagagcaacagcaagtacgcggccagcagctacctgagcctgacgagcagcgactggaaa tcgaaaggcagttacagctgcgaggtcacgcacgaggggagcaccgtgacgaagacagtg aagccctcagagtgttct |
| SEQ ID NO. 270 **IGLC5** (**P**) |
| >IGLC5*01\|Bos taurus_Hereford\|P\|C-REGION |
| gtcagcccaagtccacacctcggtcaccctgttcccgccctccaaggaggagctcagcac caacaaggccaccctggtgtgtctcatcagcgacttctacccgggtagcgtgaccgtggt ctagaaggcagacggcagcaccatcacccacaacgtggagaccacccgggcctccaaaca gagcaacagcaagtaccgcggccagcagctacctgagcctgatgggcagcgactggaaat cgaaaggcagttacagctgcgaggtcacgcacgagggagcaccgtgacgaagacagtgaa gcctcagagtgttct |
| SEQ ID NO. 271 **IGLC6** (**P**) |
| >IGLC6*01\|Bos taurus_Hereford\|P\|C-REGION\| |
| gtcagcccaagttcacaccctcgtcactgtttccgcccacctccaaggagagccagcacc aacaaggccaacccctgtgtgtctcatcagcgacttctaccccggtagtcgtacccgggt cagaaggcagaacggcaaccatctacccacaacgtggagaccacccggccccaaacagag caccagcaagtaacgcggccgagcactacccacctgagcctgatggcagcgacggaaaat cgaaaggctagttacagcgcgagttcagcacgaaggggagcacgtgacgaaacatgaagc ctcagagtgttcct |
| SEQ ID NO. 272 **IGLC7** (**P**) |
| >IGLC7*01\|Bos taurus_Hereford\|P\|C-REGION |
| gtcagcccaagtccccaccctcgtcaccctgttcccgccctccacggaggagctcaacgg caacaaggccaccctggtgtgtctcatcagcgacttctacccgggtagcgtgaccgtggt ctggaaggcagacggcagcaccatcacccgcaacgtggagaccacccgggcctccaaaca gagcaacagcaagtacgcggccagcagctacctgagcctgacgagcagcgactggaaatc gaaaggcagttacagctgcgaggtcacgcacgaggggagcaccgtgacgaagacagtgaa gccctcagagtgttct |
| SEQ ID NO. 273 **IGLC8** (**F**) |
| >IGLC8*01\|Bos taurus_Hereford\|F\|C-REGION\| |
| ggtcagcccaagtccccaccctcggtcaccctgttcccgccctccacggaggagctcaac ggcaacaaggccaccctggtgtgtctcatcagcgacttctacccgggtagcgtgaccgtg gtctggaaggcagacggcagcaccatcacccgcaacgtggagaccacccgggcctccaaa cagagcaacagcaagtacgcggccagcagctacctgagcctgacgagcagcgactggaaa |
| tcgaaaggcagttacagctgcgaggtcacgcacgaggggagcaccgtgacgaagacagtg aagccctcagagtgttct |
| SEQ ID NO. 274 **IGLC9** (**P**) |
| >IGLC9*01\|Bos taurus_Hereford\|P\|C-REGION\| |
| gtcagcccaagtccacaccctcggtcaccctgttcccgccctccaaggaggagctcagca ccaacaaggccaccctggtgtgtctcatcagcgacttctacccgggtagcgtgaccgtgg tctagaaggcagacggcagcaccatcacccacaacgtggagaccacccgggcctccaaac agagcaacagcaagtacgcggccagcagctacctgagcctgatgggcagcgactggaaat cgaaaggcagttacagctgcgaggtcacgcacgaggggagcaccgtgacgaagacagtga agcctcagagtgttct |

**Table 5. PCR Primers**

| | |
|---|---|
| SEQ ID NO. 275 | 1F: ACATAATACACTGAAATGGAGCCC |
| SEQ ID NO. 276 | 1R: GTCCTTGGTCAACGTGAGGG |
| SEQ ID NO. 277 | 2F: CATAATACACTGAAATGGAGCCCT |
| SEQ ID NO. 278 | 2R: GCAACAGTGGTAGGTCGCTT |

**Table 6. Miscellaneous sequence data**

| **Pre-DJ** |
|---|
| This is a 21609 bp fragment upstream of the Ighd-5 D gene segment. The pre-DJ sequence can be found in Mus musculus strain C57BL/6J chromosome 12, Assembly: GRCm38.p4, Annotation release 106, Sequence ID: NC_000078.6 |
| The entire sequence lies between the two 100 bp sequences shown below: |
| Upstream of the Ighd-5 D gene segment, corresponding to positions 113526905-113527004 in NC_000078.6: |
| ATTTCTGTACCTGATCTATGTCAATATCTGTACCATGGCTCTAGCAGAGATGAAATATG AGACAGTCTGATGTCATGTGGCCATGCCTGGTCCAGACTTG (SEQ ID NO. 279) |
| 2 kb upstream of the Adam6a gene corresponding to positions 113548415 - 113548514 in NC_000078.6: |
| GTCAATCAGCAGAAATCCATCATACATGAGACAAAGTTATAATCAAGAAATGTTGCCCA TAGGAAACAGAGGATATCTCTAGCACTCAGAGACTGAGCAC (SEQ ID NO. 280) |

| **ADAM6A** |
|---|
| ADAM6A (a disintegrin and metallopeptidase domain 6A) is a gene involved in male fertility. The ADAM6A sequence can be found in Mus musculus strain C57BL/6J chromosome 12, Assembly: GRCm38.p4, Annotation release 106, Sequence ID: NC_000078.6 at position 113543908-113546414. |
| ADAM6A sequence ID: OTTMUSG00000051592 (VEGA) |

**Table 7. Chimeric bovine/mouse Ig gene sequences.**

| **IGK Version A** |
|---|
| Sequence upstream of mouse Igkv 1-133 (SEQ ID NO. 281) |
| GCATTGAATAAACCAGTATAAACAAGCAAGCAAAGATAGATAGATAGATAGATAGATAGATAGATAGATAC ATAGATAGATAGATAGATAGATAGATGATAGATAGATAGATAGATAGATAGATTTTTACGTATAATACAAT AAAAACATTCATTGTCCCTCTATTGGTGACTACTCAAGGAAAAAAATGTTCATATGCAAGAAAAAATGTTA TCATTACCAGATGATCCAGCAATCTAGCAATATATATATTGTTTATTCACAAAACATGAATGAACCTTTTA AGAAGCTGTTACAGTGTAAAAATTAAGTTAAATCACTGAAGAACATATACTGTGTGATTTCATTCAAATGA AATTTGAGAAGTAAATATATATGTATATATATATATATGTAAAAAATATAAGTCTGAACTACAAAAATTCA ATTTGTTTGATATGTAAGAATAAGAAAAATTGACCCCCAAAATTTGTTAATAATTAGGTATGTGTATTTTT ATGAATATATAAGTATAATAATGCTTATAGTATACACTATTCTGAATCACATTTATTCCCTAAGTGTGTTC CCTTGATTATAATTAAAAGTATATTTTTTAAATACAGAGTCAGAGTACAGTCAATAAGGCGAAAATATAGT TGAATGATTTGCTTCAGCTTTTGTAATGTACTAGAGATTGTGAGTACAAAGTCTCAGAGCTCATTTTATCC CTGACAATAACCAGCTCTGTGCTTCAAGTACATTTCCATCTTTCTCTGAAATTTAGTCTTATATAGATAGA CAAAATTTAAGTAAATTTCAAACTACACAGAACAACTAAGTTGTTGTTTCATATTGATAATGGATTTGAAC TGCATTAACAGAACTTTAACATCCTGCTTATTCTCCCTTCAGCCATCATATTTTGCTTTATTATTTTCACT TTTTGAGTTATTTTTCACATTCAGAAAGCTCACATAATTGTCACTTCTTTGTATACTGGTATACAGACCAG AACATTTGCATATTGTTCCCTGGGGAGGTCTTTGCCCTGTTGGCCTGAGATAAAACCTCAAGTGTCCTCTT GCCTCCACTGATCACTCTCCTATGTTTATTTCCTCAAA |
| Cow exon 1 (leader) from LOC100294952 (SEQ ID NO. 282) |
| Atgagattctctgctcagctcctggggctcctcctgctctgggtcccag |
| Cow intron 1 from LOC100294952 (SEQ ID NO. 283) |
| Gtaagtacagagagggatgagaaggaggatgggggtgagttctggggcagcactgctctccacatgtgttc tctgttagatgtgtatgacttgtcctgcagatgagcatgggaaccttagatcaatgatagtgaggaatgtt ccagaaggaagaaggtcctgtgctctggtcaggactgtgacaggggaagtggggatgatgtaggggatgtt tagaggtctctttatacttcacagatatcaagttcattattgtgattgtacaattttgctgtatgatcaca gacaatgtgagtaatacaaagtagtattaatgttttagctaaaataaatcagaaaatggaaacaataaaaa tggttgctaatatttgtagctttctaattctctgtcattcctttag |
| Cow Vκ from LOC100294952 (SEQ ID NO. 284) |
| Gatccagtggggatgttgtgctgacccagactccactctccctgtctatcatccctggagagatggcctcc atctcctgcaagtctagtcagagcctggtacacagtgatggaaaaacctatttgaattggattcaatataa accaggccaatcaccacagggtctgatctatcaggtttccaaccgttactctggggtctcagacaggttca ctggcagtgggtcagggacagatttcacacttacaatcagcagagtgcaggctgaggatgctggagtctat tactgttaccaaggtacagaagat |
| Mouse RSS heptamer (SEQ ID NO. 285) |
| CACAGTG |
| Mouse sequence downstream of RSS heptamer (SEQ ID NO. 286) |
| ATACAGACTCTATCAAAAACTTCCTTGCCTGGGGCAGCCCAGCTGACAATGTGCAATCTGAAGAGGAGCAG AGAGCATCTTGTGTCTGTGTGAGAAGGAGGGGCTGGGATACATGAGTAATTCTTTGCAGCTGTGAGCTCTG |

| **IGK Version B** |
|---|
| Sequence upstream of mouse Igkv 1-133 (SEQ ID NO. 287) |
| GCATTGAATAAACCAGTATAAACAAGCAAGCAAAGATAGATAGATAGATAGATAGATAGATAGATAGATAC ATAGATAGATAGATAGATAGATAGATGATAGATAGATAGATAGATAGATAGATTTTTACGTATAATACAAT AAAAACATTCATTGTCCCTCTATTGGTGACTACTCAAGGAAAAAAATGTTCATATGCAAGAAAAAATGTTA TCATTACCAGATGATCCAGCAATCTAGCAATATATATATTGTTTATTCACAAAACATGAATGAACCTTTTA AGAAGCTGTTACAGTGTAAAAATTAAGTTAAATCACTGAAGAACATATACTGTGTGATTTCATTCAAATGA AATTTGAGAAGTAAATATATATGTATATATATATATATGTAAAAAATATAAGTCTGAACTACAAAAATTCA |
| ATTTGTTTGATATGTAAGAATAAGAAAAATTGACCCCCAAAATTTGTTAATAATTAGGTATGTGTATTTTT ATGAATATATAAGTATAATAATGCTTATAGTATACACTATTCTGAATCACATTTATTCCCTAAGTGTGTTC CCTTGATTATAATTAAAAGTATATTTTTTAAATACAGAGTCAGAGTACAGTCAATAAGGCGAAAATATAGT TGAATGATTTGCTTCAGCTTTTGTAATGTACTAGAGATTGTGAGTACAAAGTCTCAGAGCTCATTTTATCC CTGACAATAACCAGCTCTGTGCTTCAAGTACATTTCCATCTTTCTCTGAAATTTAGTCTTATATAGATAGA CAAAATTTAAGTAAATTTCAAACTACACAGAACAACTAAGTTGTTGTTTCATATTGATAATGGATTTGAAC TGCATTAACAGAACTTTAACATCCTGCTTATTCTCCCTTCAGCCATCATATTTTGCTTTATTATTTTCACT TTTTGAGTTATTTTTCACATTCAGAAAGCTCACATAATTGTCACTTCTTTGTATACTGGTATACAGACCAG AACATTTGCATATTGTTCCCTGGGGAGGTCTTTGCCCTGTTGGCCTGAGATAAAACCTCAAGTGTCCTCTT GCCTCCACTGATCACTCTCCTATGTTTATTTCCTCAAA |
| Mouse Igkv 1-133 exon 1 (leader) (SEQ ID NO. 288) |
| ATGATGAGTCCTGCCCAGTTCCTGTTTCTGTTAGTGCTCTGGATTCAGG |
| Mouse Igkv 1-133 intron 1 (SEQ ID NO. 289) |
| GTAAGGAGTTTTGGAATGTGAGGGATGAGAATGGGGATGGAGGGTGATCTCTGGATGCCTATGTGTGCTGT TTATTTGTGGTGGGGCAGGTCATATCTTCCAGAATGTGAGGTTTTGTTACATCCTAATGAGATATTCCACA TGGAACAGTATCTGTACTAAGATCAGTATTCTGACATAGATTGGATGGAGTGGTATAGACTCCATCTATAA TGGATGATGTTTAGAAACTTCAACACTTGTTTTATGACAAAGCATTTGATATATAATATTTTTAAATCTGA AAAACTGCTAGGATCTTACTTGAAAGGAATAGCATAAAAGATTTCACAAAGGTTGCTCAGGATCTTTGCAC ATGATTTTCCACTATTGTATTGTAATTTCAG |
| Mouse Igkv 1-133 5' part of exon 2 (leader) (SEQ ID NO. 290) |
| AAACCAACGGT |
| Cow Vκ from LOC100294952 (SEQ ID No. 291): |
| Gatccagtggggatgttgtgctgacccagactccactctccctgtctatcatccctggagagatggcctcc atctcctgcaagtctagtcagagcctggtacacagtgatggaaaaacctatttgaattggattcaatataa accaggccaatcaccacagggtctgatctatcaggtttccaaccgttactctggggtctcagacaggttca ctggcagtgggtcagggacagatttcacacttacaatcagcagagtgcaggctgaggatgctggagtctat tactgttaccaaggtacagaagat |
| Mouse RSS heptamer (SEQ ID NO. 292) |
| CACAGTG |
| Mouse sequence downstream of RSS heptamer (SEQ ID NO. 293) |
| ATACAGACTCTATCAAAAACTTCCTTGCCTGGGGCAGCCCAGCTGACAATGTGCAATCTGAAGAGGAGCAG AGAGCATCTTGTGTCTGTGTGAGAAGGAGGGGCTGGGATACATGAGTAATTCTTTGCAGCTGTGAGCTCTG |

## Claims

1. A transgenic rodent or rodent cell comprising a genome comprising an engineered partly bovine immunoglobulin light chain locus comprising bovine immunoglobulin λ light chain variable region gene segments, wherein the engineered immunoglobulin locus is capable of expressing immunoglobulin comprising bovine variable domains and wherein the transgenic rodent produces more, or is more likely to produce, immunoglobulin comprising λ light chain than immunoglobulin comprising κ light chain.

2. The transgenic rodent or rodent cell according to claim 1, wherein the engineered immunoglobulin locus comprises bovine V_{λ} and J_{λ} gene segment coding sequences embedded in rodent non-coding regulatory or scaffold sequences of a rodent immunoglobulin λ light chain variable region gene locus.

3. The transgenic rodent or rodent cell according to claim 1 or claim 2, wherein the partly bovine immunoglobulin locus comprises one or more bovine V_{λ} and J_{λ} gene segment coding sequences and one or more rodent immunoglobulin λ constant region coding sequences.

4. The transgenic rodent or rodent cell according to any of claims 1 to 3, wherein the engineered immunoglobulin locus comprises bovine V_{λ} and J_{λ} gene segment coding sequences embedded in rodent non-coding regulatory or scaffold sequences of a rodent immunoglobulin κ light chain variable region gene locus.

5. The transgenic rodent or rodent cell according to claim 4, wherein the engineered immunoglobulin variable region locus comprises one or more bovine V_{λ} gene segment coding sequences and one or more J-C units wherein each J-C unit comprises a bovine J_{λ} gene segment coding sequence and a rodent λ constant region coding sequence, preferably comprising a rodent C_{λ1}, C_{λ1}, C_{λ3} coding sequence, or a combination thereof.

6. The transgenic rodent or rodent cell according to claim 5, comprising one or more bovine V_{λ} gene segment coding sequences located upstream of one or more J-C units, wherein each J-C unit comprises a bovine J_{λ} gene segment coding sequence and a rodent C_{λ} gene segment coding sequence and rodent C_{λ} non-coding sequences.

7. The transgenic rodent or rodent cell according to any of claims 5 or 6, wherein the JC units comprise bovine J_{λ} gene segment coding sequences and rodent λ constant region coding sequences embedded in non-coding regulatory or scaffold sequences of a rodent immunoglobulin κ light chain locus.

8. The transgenic rodent or rodent cell according to claim 4, wherein the engineered immunoglobulin locus comprises a rodent immunoglobulin κ locus in which one or more rodent V_{κ} gene segment coding sequences and one or more rodent J κ gene segment coding sequences have been deleted and replaced by one or more bovine V_{λ} gene segment coding sequences and one or more J_{λ} gene segment coding sequences, respectively, and in which rodent C κ coding sequences in the locus have been replaced by rodent C_{λ1}, C_{λ1}, C_{λ3} coding sequence, or a combination thereof.

9. The transgenic rodent or rodent cell according to claim 8, wherein the engineered immunoglobulin locus comprises one or more bovine V_{λ} gene segment coding sequences upstream of one or more bovine J_{λ} gene segment coding sequences which are upstream of one or more rodent C_{λ} coding sequences.

10. The transgenic rodent or rodent cell according to claim 1, wherein the genome of the transgenic rodent or rodent cell comprises an engineered immunoglobulin locus comprising bovine V_{K} and J _{K} gene segment coding sequences.

11. The transgenic rodent or rodent cell according to claim 10, wherein the bovine V_{κ} and J_{κ} gene segment coding sequences are inserted into a rodent immunoglobulin κ light chain locus.

12. The transgenic rodent or rodent cell according to claim 10 or 11, wherein the bovine V_{K} and J_{K} gene segment coding sequences are embedded in rodent non-**coding regulatory** or scaffold sequences of the rodent immunoglobulin κ light chain variable region gene locus.

13. The transgenic rodent or rodent cell according to any of claims 10 to 12, wherein the bovine V_{K} and J_{K} coding sequences are inserted upstream of a rodent immunoglobulin κ light chain constant region coding sequence.

14. The transgenic rodent or rodent cell according to claim 1, wherein the genome of the transgenic rodent or rodent cell comprises an engineered immunoglobulin locus comprising bovine V_{K} and J_{K} gene segment coding sequences inserted into a rodent immunoglobulin λ light chain locus.

15. The transgenic rodent or rodent cell according to claim 14, wherein the bovine V_{K} and J_{K} gene segment coding sequences are embedded in rodent non-coding regulatory or scaffold sequences of the rodent immunoglobulin λ light chain variable region gene locus.

16. The transgenic rodent or rodent cell according to claim 14 or 15, comprising a rodent immunoglobulin κ light chain constant region coding sequence inserted downstream of the bovine V_{K} and J_{K} gene segment coding sequences.

17. The transgenic rodent or rodent cell according to claim 16, wherein the rodent immunoglobulin κ light chain constant region is inserted upstream of an endogenous rodent C_{λ2} coding sequence.

18. The transgenic rodent or rodent cell according to any of claims 1 to 9 or 14 to17, wherein expression of an endogenous rodent immunoglobulin λ light chain variable domain is suppressed or inactivated by one or more of:
a. deleting or mutating all endogenous rodent V_{λ} gene segment coding sequences
b. deleting or mutating all endogenous rodent J_{λ} gene segment coding sequences; and
c. deleting or mutating all endogenous C_{λ} coding sequences or splice sites.

19. The transgenic rodent or rodent cell according to any of the preceding claims, wherein the engineered bovine immunoglobulin light chain locus comprises a rodent intronic κ enhancer (iE _{K}) and 3'E _{K} regulatory sequence.

20. The transgenic rodent or rodent cell according to any of the preceding claims, wherein the transgenic rodent or rodent cell comprises an engineered partly bovine immunoglobulin heavy chain locus comprising bovine immunoglobulin heavy chain variable region gene coding sequences and rodent non-coding regulatory or scaffold sequences of the rodent immunoglobulin heavy chain locus.

21. The transgenic rodent or rodent cell according to claim 20, wherein the engineered bovine immunoglobulin heavy chain locus comprises bovine V_{H}, D and J_{H} gene segments, preferably wherein each bovine V_{H}, D or J_{H} coding gene segment comprises V_{H}, D or J_{H} coding sequence embedded in rodent non-coding regulatory or scaffold sequences of the rodent immunoglobulin heavy chain locus, preferably wherein the heavy chain rodent non-coding regulatory or scaffold sequences are interspersed by functional ADAM6A genes, ADAM6B genes, or a combination thereof.

22. The transgenic rodent or rodent cell according to any of the preceding claims, wherein the rodent regulatory or scaffold sequences comprise enhancer, promoters, splice sites, introns, recombination signal sequences, or combinations thereof.

23. The transgenic rodent or rodent cell according to any of the preceding claims, wherein the rodent is a mouse or a rat.

24. The transgenic rodent or rodent cell according to any of the preceding claims, wherein the rodent cell is an embryonic stem (ES) cell or a cell of an early stage embryo.
